# EUROPEAN PATENT SPECIFICATION

(11) **EP 2 245 014 B1**
(45) Date of publication and mention of the grant of the patent: **02.11.2011**
(21) Application number: 09709660.6
(22) Date of filing: 10.02.2009
(51) Int. Cl.: C07D 221/26, C07D 401/06, C07D 401/14, C07D 413/06, C07D 413/10, C07D 471/04, C07D 471/08, C07D 487/04, C07D 495/04, C07D 498/08, A61K 31/55, A61P 3/00

(54) **UREA DERIVATIVES OF BENZOMORPHANES AND RELATED SCAFFOLDS, MEDICAMENTS CONTAINING SUCH COMPOUNDS AND THEIR USE**
HARNSTOFF-DERIVATE VON BENZOMORPHANEN, ENTSPRECHENDE GERÜSTE, MEDIKAMENTE MIT SOLCHEN VERBINDUNGEN UND IHRE VERWENDUNG
DERIVES D'UREE DE BENZOMORPHANES ET STRUCTURES ASSOCIEES, MEDICAMENTS CONTENANT DE TELS COMPOSES ET LEUR UTILISATION

(30) Priority: 12.02.2008 EP 08151295
(43) Date of publication of application: 03.11.2010
(73) Proprietor: Boehringer Ingelheim International GmbH, 55216 Ingelheim am Rhein (DE)
(72) Inventor: ECKHARDT, Matthias, 55216 Ingelheim am Rhein (DE); HIMMELSBACH, Frank, 55216 Ingelheim am Rhein (DE); HAMILTON, Bradford, 55216 Ingelheim am Rhein (DE); PETERS, Stefan, 55216 Ingelheim am Rhein (DE)
(86) International application number: PCT/EP2009/000905
(87) International publication number: WO 2009/100872

(56) References cited:
- WO-A-2006/024628
- WO-A-2006/040329
- WO-A-2007/051810
- WO-A-2008/000951
- GB-A- 1 077 711
- US-A- 3 341 538

## Description

The present invention relates to compounds derived from the following chemical scaffold which is structurally defined by the formula I 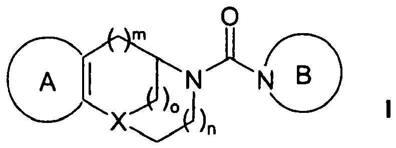 wherein the groups A, B, X, m, n and o are as defined hereinafter, including the tautomers, the stereoisomers, the mixtures thereof and the salts thereof. The invention further relates to pharmaceutical compositions containing a compound of formula I according to the invention as well as the use of a compound according to the invention for preparing a pharmaceutical composition for the treatment of metabolic disorders. In addition, the invention relates to processes for preparing a pharmaceutical composition as well as a compound according to the invention.

In the literature, compounds which have an inhibitory effect on the enzyme 11β-hydroxysteroid dehydrogenase (HSD) 1 are proposed for the treatment of the metabolic syndrome, in particular diabetes type 2, obesity and dyslipidemia.

In the US 3,341,538 derivatives of benzomorphanes of the general formula 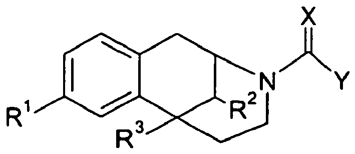 wherein R¹, R², R³, X and Y are as defined therein, are described as non-toxic analgesics having an activity of the same order of magnitude as codeine or morphine and as being free of addictive properties.

In the US 3,539,637 derivatives of 2,3,4,5-tetrahydro-1,4-methano-1H-3-benzazepines of the general formula 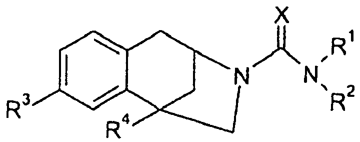 wherein R¹ R², R³, R⁴ and X are as defined therein, are described as non-toxic analgesics in the treatment of pain with little or no addiction liability. R¹ and R² are hydrogen or (lower)alkyl or R¹ and R² taken together with the nitrogen to which they are attached are morpholino, piperidino or pyrrolidino. Inter alia the following compounds are mentioned therein:

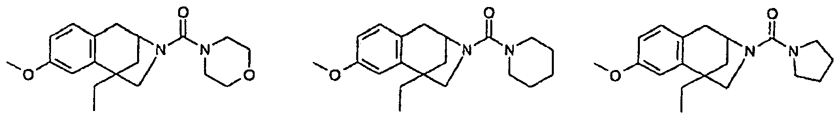

In the GB 1,077,711 derivatives of benzomorphanes of the general formula 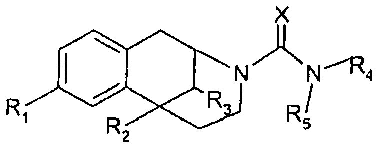 wherein R₁, R₂, R₃, R₄, R₅ and X are as defined therein, are described as analgesics free of addictive properties and having antitussive action. R₄ and R₅ together with the adjacent nitrogen atom represent a saturated heterocycle, in which the nitrogen atom is the only hetero atom, having at most 7 carbon atoms, or a morpholino radical. Inter alia the following compound is mentioned therein:

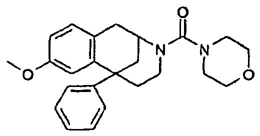

WO2008/0009 relates to urea derivatives of tropane and their use as inhibitor of 11β-Hydroxysteroid Dehydrogenase type 1 (11βHSD1).

The inventors are not aware that urea derivatives of benzomorphanes have been described as inhibitors of 11β-hydroxysteroid dehydrogenase (HSD) 1.

### Aim of the invention

The aim of the present invention is to find new benzomorphanes or related compounds, particularly those which are active with regard to the enzyme 11β-hydroxysteroid dehydrogenase (HSD) 1. A further aim of the present invention is to discover benzomorphanes or related compounds which have an inhibitory effect on the enzyme 11β-hydroxysteroid dehydrogenase (HSD) 1 *in vitro* and/or *in vivo* and possess suitable pharmacological and pharmacokinetic properties to use them as medicaments.

A further aim of the present invention is to provide new pharmaceutical compositions which are suitable for the prevention and/or treatment of metabolic disorders, particularly diabetes and dyslipidemia.

Other aims of the present invention will become apparent to the skilled man directly from the foregoing and following remarks.

### Object of the invention

In a first aspect the present invention relates to compounds derived from the following chemical scaffold which is structurally defined by the formula I 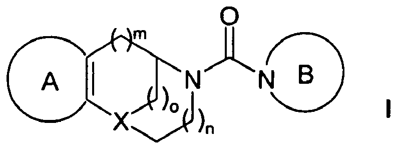 wherein
X denotes CH or N,
m, n, o independently of each other denote 0, 1 or 2,
wherein the C₅₊ₘ₊ₙ-azacycloalkene core structure of general formula I including the bridging group -(CH₂)ₒ- is optionally substituted with 1, 2 or more substituents independently of each other selected from the group consisting of R¹¹ and R¹²,
   - A: denotes a benzo, pyrido, pyrrolo, furo, thieno, pyridazino, pyrimido, or pyrazino ring wherein each of said rings is substituted with one or more substituents independently of each other selected from R¹ and wherein 2 adjacent C-atoms of each of said rings are optionally substituted with R² and R³; or
   a pyrazolo, imidazo, oxazolo, thiazolo, isoxazolo, or isothiazolo ring wherein each of said rings is optionally substituted with R¹; or
   a 1,2,3-triazolo ring optionally substituted with R^{N}; and
   - B: denotes a 3- to 8-membered monocylic, 7- to 12-membered spirocyclic, 6- to 12-membered bicyclic or 9- to 15-membered tricyclic azacycloalk-1-yl group, which is saturated or partially or fully unsaturated, wherein 1 or 2 -CH₂- groups may be replaced by -NR^{N}-,
   wherein 1 to 4 -CH₂- groups may be replaced independently of each other by O, S, carbonyl, or sulfonyl,
   wherein 1 or 2 -CH= groups may be replaced by -N=, and
   wherein said azacycloalkyl group may be substituted with one or more substituents independently of each other selected from L¹, wherein said azacycloalkyl group may substituted with 1 or 2 substituents independently of each other selected from L², wherein 2 adjacent C-atoms of said azacycloalkyl group may be substituted with L³ and L⁴, and wherein 2 adjacent C-atoms of said azacycloalkyl group may be substituted with L⁵ and L⁶;
   - R^{N}: independently of each other denotes hydrogen, C₁₋₆-alkyl, C₃₋₆-alkenyl, C₃₋₆-alkynyl, (het)aryl, C₁₋₄-alkylcarbonyl, C₁₋₄-alkylsulfonyl, (het)arylcarbonyl, (het)arylsulfonyl,
   wherein each alkyl, alkenyl, and alkynyl group may be mono- or polysubstituted with fluorine, and may be monosubstitued with hydroxy, C₁₋₄-alkoxy, C₁₋₄-alkylsulfanyl, C₁₋₄-alkylsulfinyl, C₁₋₄-alkylsulfonyl, amino, C 1 -4-alkyl amino, di-(C₁₋₄-alkyl)amino, C₁₋₄-alkylcarbonylamino, cyano, carboxy, C₁₋₄-alkoxycarbonyl, aminocarbonyl, C₁₋₄-alkylaminocarbonyl, di-(C₁₋₄-alkyl)aminocarbonyl, or (het)aryl,
   - R¹: independently of each other denotes fluorine, chlorine, bromine, iodine, cyano, nitro, C₁₋₄-alkyl, difluoromethyl, trifluoromethyl, difluoromethoxy, trifluoromethoxy, trifluoromethyl-hydroxy-C₁₋₂-alkyl, 2,2,2-trifluoro-1-hydroxy-1-(trifluoromethyl)ethyl, C₃₋₆-cycloal kyl, C₃₋₆-cycloalkyloxy, C₃₋₆-cycloalkyl-C₁₋₃-alkyl, C₃₋₆-cycloalkyl-C₁₋₃-alkyloxy, tetrahydrofuran-3-yloxy, tetrahydropyran-3-yloxy, tetrahydropyran-4-yloxy, tetrahydrofuranyl-C₁₋₃-alkyloxy, tetrahydropyranyl-C₁₋₃-alkyloxy, (het)aryl, (het)aryloxy, (het)aryl-C₁₋₃-alkyl, (het)aryl-C₁₋₃-alkyloxy, (het)aryloxy-C₁₋₃-alkyl, C₁₋₃-alkyl-carbonyl, (het)aryl-carbonyl,
   amino, C₁₋₄-alkylamino, di-(C₁₋₄-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl; 3-oxo-piperazin-1-yl, 4-(C₁₋₄-alkyl)-piperazin-1-yl, 4-(C₁₋₄-alkylcarbonyl)-piperazin-1-yl, 4-(C₃₋₆-cycloalkylcarbonyl)-piperazin-1-yl, 4-(C₁₋₄-alkyloxycarbonyl)-piperazin-1-yl, 4-(C₁₋₄-alkylsulfonyl)-piperazin-1-yl, 2-oxo-4-(C₁₋₄-alkyl)-piperazin-1-yl, 3-oxo-4-(C₁₋₄-alkyl)-piperazin-1-yl,
   C₁₋₄-alkyl-carbonylamino, (het)aryl-carbonylamino, (het)aryl-C₁₋₄alkyl-carbonylamino, C₁₋₄-alkyloxy-carbonylamino, aminocarbonylamino, C₁₋₄-alkyl-aminocarbonylamino, di-(C₁₋₄-alkyl)aminocarbonylamino, pyrrolidin-1-yl-carbonylamino, piperidin-1-yl-carbonylamino, morpholin-4-yl-carbonylamino, piperazin-1-yl-carbonylamino, 4-(C₁₋₄-alkyl)-piperazin-1-yl-carbonylamino, C₁₋₄-alkyl-sulfonylamino, aminosulfonylamino, C₁₋₄-alkylamino-sulfonylamino, di-(C₁₋₄-alkyl)-amino-sulfonylamino, pyrrolidin-1-yl-sulfonylamino, piperidin-1-yl-sulfonylamino, morpholin-4-yl-sulfonylamino, piperazin-1-yl-sulfonylamino, 4-(C₁₋₄-alkyl)-piperazin-1-yl-sulfonylamino, (C₁₋₄-alkyloxy-carbonylamino)carbonylamino, (het)arylsulfonylamino, (het)aryl-C₁₋₄-alkyl-sulfonylamino, N-(C₁₋₄-alkyl)-C₁₋₄-alkyl-carbonylamino, N-(C₁₋₄-alkyl)-(het)arylcarbonylamino, N-(C₁₋₄-alkyl)-(het)aryl-C₁₋₄-alkyl-carbonylamino, N-(C₁₋₄-alkyl)-C₁₋₄-alkyloxy-carbonylamino, N-(aminocarbonyl)-C₁₋₄-alkylamino, N-(C₁₋₄-alkyl-amino-carbonyl)-C₁₋₄-alkylamino,N-[di-(C₁₋₄-alkyl)aminocarbonyl]-C₁₋₄-alkylamino,
   N-(C₁₋₄-alkyl)-C₁₋₄-alkyl-sulfonylamino,N-(C₁₋₄-alkyl)-(het)arylsulfonylamino, N-(C₁₋₄-alkyl)-(het)aryl-C₁₋₄-alkyl-sulfonylamino,
   oxo-imidazolidin-1-yl, 2,4-dioxo-imidazolidin-1-yl, 2,5-dioxo-imidazolidin-1-yl, 2-oxo-hexahydropyrimidin-1-yl, wherein the nitrogen atom in position 3 of the aforementioned groups is optionally substituted with methyl or ethyl,
   (hydroxyimino)aminomethyl, (C₁₋₃-alkyloxyimino)aminomethyl, carboxy, C₁₋₄-alkyloxy-carbonyl, aminocarbonyl, C₁₋₄-alkyl-aminocarbonyl, di-(C₁₋₄-alkyl)-aminocarbonyl, pyrrolidin-1-yl-carbonyl, piperidin-1-yl-carboriyl, morpholin-4-yl-carbonyl, piperazin-1-yl-carbonyl, 4-(C₁₋₄-alkyl)-piperazin-1-yl-carbonyl,
   carboxy-C₁₋₄-alkyl, C₁₋₄-alkyloxy-carbonyl-C₁₋₄-alkyl, cyano-C₁₋₄-alkyl, aminocarbonyl-C₁₋₄-alkyl, C₁₋₄-alkyl-aminocarbonyl-C₁₋₄-alkyl, di-(C₁₋₄-alkyl)-aminocarbonyl-C₁₋₄-alkyl, pyrrolidin-1-yl-carbonyl-C₁₋₄-alkyl, piperidin-1-yl-carbonyl-C₁₋₄-alkyl, morpholin-4-yl-carbonyl-C₁₋₄-alkyl, piperazin-1-yl-carbonyl-C₁₋₄-alkyl, 4-(C₁₋₄-alkyl)-piperazin-1-yl-carbonyl-C₁₋₄-alkyl,
   carboxy-C₁₋₄-alkyloxy, C₁₋₄-alkyloxy-carbonyl-C₁₋₄-alkyloxy, cyano-C₁₋₄-alkyl-oxy, aminocarbonyl-C₁₋₄-alkyloxy, C₁₋₄-alkyl-aminocarbonyl-C₁₋₄-alkyloxy, di-(C₁₋₄-alkyl)-aminocarbonyl-C₁₋₄-alkyloxy, pyrrolidin-1-yl-carbonyl-C₁₋₄-alkyl-oxy, piperidin-1-yl-carbonyl-C₁₋₄-alkyloxy, morpholin-4-yl-carbonyl-C₁₋₄-alkyl-oxy, piperazin-1-yl-carbonyl-C₁₋₄-alkyloxy, 4-(C₁₋₄-alkyl)-piperazin-1-yl-carbonyl-C₁₋₄-alkyloxy,
   hydroxy-C₁₋₄-alkyl, C₁₋₄-alkyloxy-C₁₋₄-alkyl, amino-C₁₋₄-alkyl. C₁₋₄-alkylamino-C₁₋₄-alkyl, di-(C₁₋₄-alkyl)-amino-C₁₋₄-alkyl, pyrrolidin-1-yl-C₁₋₄-alkyl, C₁₋₄-alkyl-carbonyl-amino-C₁₋₄-alkyl, N-(₁₋₄-alkyl)-C₁₋₄-alkylcarbonyl-ami no-C1-₄-alkyl, 2-oxo-pyrrolidin-yl-C₁₋₄-alkyl, piperidin-1-yl-C₁₋₄-alkyl, 2-oxo-piperidin-1-yl- C₁₋₄-alkyl, morpholin-4-yl-C₁₋₄-alkyl, 3-oxo-morpholin-4-yl-C₁₋₄-alkyl, piperazin-1-yl-C₁₋₄-alkyl, 2-oxo-piperazin-1-yl-C₁₋₄-alkyl, 3-oxo-piperazin-1-yl-C₁₋₄-alkyl, 4-(C₁₋₄-alkyl)-piperazin-1-yl-C₁₋₄-alkyl, 2-oxo-4-(C₁₋₄-alkyl)-piperazin-1-yl-C₁₋₄-alkyl, 3-oxo-4-(C₁₋₄-alkyl)-piperazin-1-yl-C₁₋₄-alkyl,
   hydroxy-C₁₋₄-alkyloxy, C₁₋₄-alkyloxy-C₁₋₄-alkyloxy, C₁₋₄-alkylsulfanyl-C₁₋₄-alkyl-oxy, C₁₋₄-alkylsulfinyl-C₁₋₄-alkyloxy, C₁₋₄-alkylsulfonyl-C₁₋₄-alkyloxy, amino-C₁₋₄-alkyloxy, C₁₋₄-alkylamino-C₁₋₄-alkyloxy, di-(C₁₋₄-alkyl)-amino-C₁₋₄-alkyloxy, pyrrolidin-1-yl-C₁₋₄-alkyloxy, 2-oxo-pyrrolidin-1-yl-C₁₋₄-alkyloxy, piperidin-1-yl-C₁₋₄-alkyloxy, 2-oxo-piperidin-1-yl-C₁₋₄-alkyloxy, morpholin-4-yl-C₁₋₄-alkyloxy, 3-oxo-morpholin-4-yl-C₁₋₄-alkyloxy, piperazin-1-yl-C₁₋₄-alkyloxy, 2-oxo-piperazin-1-yl-C₁₋₄-alkyloxy, 3-oxo-piperazin-1-yl-C₁₋₄-alkyloxy, 4-(C₁₋₄-alkyl)-piperazin-1-yl-C₁₋₄-alkyloxy, 2-oxo-4-(C₁₋₄-alkyl)-piperazin-1-yl-C₁₋₄-alkyloxy, 3-oxo-4-(C₁₋₄-alkyl)-piperazin-1-yl-C₁₋₄-alkyloxy,
   C₁₋₄-alkylsulfanyl, C₁₋₄-alkysulfinyl, C₁₋₄-alkylsulfonyl, C₁₋₄-alkylsulfonyloxy, (het)arylsulfonyl, (het)arylsulfonyloxy, trifluoromethylsulfanyl, trifluoromethyl-sulfinyl, trifluoromethylsulfonyl, C₃₋₆-cycloalkylsulfanyl, C₃₋₆-cycloalkylsulfinyl, C₃₋₆-cycloalkylsulfonyl, C₃₋₆-cydoalkyl-C₁₋₃-alkylsulfanyl, C₃₋₆-cycloalkyl-C₁₋₃-alkylsulfinyl, C₃₋₆-cydoalkyl-C₁₋₃-alkylsulfonyl,
   aminosulfonyl, C₁₋₄-alkyl-aminosulfonyl, di-(C₁₋₄-alkyl)-aminosulfonyl, pyrrolidin-1-yl-sulfonyl, piperidin-1-yl-sulfonyl, morpholin-4-yl-sulfonyl, piperazin-1-yl-sulfonyl, or 4-(C₁₋₄-alkyl)-piperazin-1-yl-sulfonyl,
   wherein the above-mentioned saturated heterocycles and cycloalkyl-rings are optionally substituted with one or two groups selected from fluorine, C₁₋₃-alkyl, C₁₋₃-alkoxy, C₁₋₃-alkoxy-C₁₋₃-alkyl, or hydroxy,
   - R², R³: are linked to each other to form a methylenedioxy, ethylenedioxy or C₃₋₅-alkylene bridging group, which may be mono- or disubstituted with methyl, and which may be mono- or polyfluorinated; or
   R², R³ form combined with the carbon atoms to which they are attached a benzo, pyrido, pyrimido, pyrazino, pyridazino, pyrrolo, furano, thieno, pyrazolo, imidazo, triazolo, oxazolo, thiazolo, isoxazolo, or isothiazolo ring, wherein each of said rings may be substituted with one or more, preferably one to three, substituents independently selected from R^{P};
   - R^{P}: denotes halogen, C₁₋₆-alkyl, hydroxy-C₁₋₄-alkyl, C₁₋₃-alkyloxy-C₁₋₃-alkyl, C₃₋₆-cycloalkyl, hydroxy-C₄₋₆-cycloalkyl, C₁₋₃-alkyloxy-C₃₋₆-cycloalkyl, azetidinyl, 1-(C₁₋₃-alkyl)-azetidinyl, 1-(C₁₋₃-alkylcarbonyl)-azetidinyl, pyrrolidinyl, 1-(C₁₋₃-alkyl)-pyrrolidinyl, 1-(C₁₋₃-alkylcarbonyl)-pyrrolidinyl, piperidinyl, 1-(C₁₋₃-alkyl)-piperidinyl, 1-(C₁₋₃-alkylcarbonyl)piperidinyl, tetrahydrofuranyl, tetrahydropyranyl, difluoromethyl, trifluoromethyl, cyano, nitro, amino, C₁₋₃-alkylamino, di-(C₁₋₃-alkyl)amino, C₁₋₃-alkylcarbonylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, aminosulfonyl, methylsulfanyl, methylsulfinyl, methylsulfonyl, hydroxy, C₁₋₃-alkyloxy, difluoromethoxy, trifluoromethoxy, phenyl, or
   pyrrolyl, furanyl, thienyl, pyridyl, where in any of these groups 1 or 2 CH are optionally replaced by N atoms, or
   1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl,
   wherein each of the aromatic or heteroaromatic groups mentioned above is optionally substituted with one or two groups independently selected from fluorine, chlorine, C₁₋₃-alkyl, difluoromethyl, trifluoromethyl, cyano, amino, acetylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, hydroxy, C₁₋₃-alkyloxy, difluoromethoxy, and trifluoromethoxy,
   - R¹⁰: independently of each other enotes halogen, C₁₋₃-alkyl, difluoromethyl, trifluoromethyl, cyano, nitro, amino, C₁₋₃-alkylamino, di-(C₁₋₃-alkyl)amino, acetylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, aminosulfonyl, methylsulfanyl, methylsulfinyl, methylsulfonyl, hydroxy, C₁₋₃-alkyloxy, difluoromethoxy, trifluoromethoxy, or phenyl optionally substituted with 1 or 2 substituents independently of each other selected from fluorine, methyl, methoxy, cyano, or hydroxy,
   - R¹¹: independently of each other denotes fluorine, C₁₋₄-alkyl, (het)aryl, hydroxy, C₁₋₄-alkyloxy, cyano, carboxy, C₁₋₄-alkyloxycarbonyl, aminocarbonyl, C₁₋₄-alkylamino-carbonyl, di-(C₁₋₄-alkyl)-aminocarbonyl, hydroxy-C₁₋₄-alkyl or C₁₋₃-alkyloxy-C₁₋₄-alkyl,
   - R¹²: independently of each other denotes fluorine or C₁₋₄-alkyl, and
   - L¹: independently of each other denotes halogen, C₁₋₄-alkyl, trifluoromethyl, hydroxy, C₁₋₄-alkyloxy, or cyano;
   - L²: independently of each other denotes fluorine, chlorine, bromine, iodine, nitro, cyano, hydroxy, C₃₋₆-cycloalkyl, C₃₋₆-cycloalkyloxy, tetrahydrofuran-3-yloxy, tetrahydropyran-3-yloxy, tetrahydropyran-4-yloxy, tetrahydrofuranyl-C₁₋₃-alkyloxy, tetrahydropyranyl-C₁₋₃-alkyloxy, (het)aryl, (het)aryloxy,
   C₁₋₆-alkyl, C₂₋₆-alkenyl, C₂₋₆-alkynyl, C₁₋₄-alkyloxy, wherein in each group one CH₂ group may be replaced by carbonyl or sulfonyl, and wherein each group may be mono or polyfluorinated, and wherein each group may additionally be substituted with
   hydroxy, chlorine, C₁₋₃-alkyloxy, amino, C₁₋₃-alkylamino, di-(C₁₋₄-alkyl)-amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₃-alkyl)-piperazin-1-yl, 2-oxo-4-(C₁₋₃-alkyl)-piperazin-1-yl, 3-oxo-4-(C₁₋₃-alkyl)-piperazin-1-yl, carboxy, C₁₋₃-alkyloxy-carbonyl, cyano, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, C₁₋₃-alkylcarbonylamino, arylcarbonylamino, C₁₋₃-alkylsulfanyl, C₁₋₃-alkylsulfinyl, C₁₋₃-alkylsulfonyl, C₃₋₆-cycloalkyl, (het)aryl, or (het)aryloxy;
   amino, C₁₋₃-alkylamino, di-(C₁₋₃-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₃-alkyl)-piperazin-1-yl, 4-(C₁₋₄-alkylcarbonyl)-piperazin-1-yl, 4-(C₃₋₆-cycloalkylcarbonyl)-piperazin-1-yl, 4-(C₁₋₃-alkyloxycarbonyl)-piperazin-1-yl, 4-(C₁₋₄-alkylsulfonyl)-piperazin-1-yl, 2-oxo-4-(C₁₋₃-alkyl)-piperazin-1-yl, 3-oxo-4-(C₁₋₃-alkyl)-piperazin-1-yl,
   C₁₋₄-alkyl-carbonylamino, (het)aryl-carbonylamino, (het)aryl-C₁₋₃-alkyl-carbonylamino, C₁₋₃-alkyloxy-carbonylamino, aminocarbonylamino, C₁₋₃-alkyl-aminocarbonylamino, di-(C₁₋₃-alkyl)aminocarbonylamino, pyrrolidin-1-yl-carbonylamino, piperidin-1-yl-carbonylamino, morpholin-4-yl-carbonylamino, piperazin-1-yl-carbonylamino, 4-(C₁₋₃-alkyl)-piperazin-1-yl-carbonylamino, C₁₋₃-alkyl-sulfonylamino, aminosulfonylamino, C₁₋₃-alkylamino-sulfonylamino, di-(C₁₋₃-alkyl)amino-sulfonylamino, pyrrolidin-1-yl-sulfonylamino, piperidin-1-yl-sulfonylamino, morpholin-4-yl-sulfonylamino, piperazin-1-yl-sulfonylamino, 4-(C₁₋₃-alkyl)-piperazin-1-yl-sulfonylamino, (C₁₋₃-alkyloxy-carbonylamino)carbonylamino, (het)arylsulfonylamino, (het)aryl-C₁₋₃-alkyl-sulfonylamino,
   N-(C₁₋₃-alkyl)-C₁₋₃-alkyl-carbonylamino, N-(C₁₋₃-alkyl)-(het)arylcarbonylamino, N-(C₁₋₃-alkyl)-(het)aryl-C₁₋₃-alkyl-carbonylamino, N-(C₁₋₃-alkyl)-C₁₋₃-alkyloxy-carbonylamino, N-(aminocarbonyl)-C₁₋₃-alkylamino, N-(C₁₋₃-alkyl-aminocarbonyl)-C₁₋₃-alkylamino, N-[di-(C₁₋₃-alkyl)aminocarbonyl]-C₁₋₃-alkylamino,
   N-(C₁₋₃-alkyl)-C₁₋₃-alkyl-sulfonylamino, N-(C₁₋₃-alkyl)-(het)arylsulfonylamino, N-(C₁₋₃-alkyl)-(het)aryl-C₁₋₃-alkyl-sulfonylamino,
   carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, (het)arylaminocarbonyl, N-(C₁₋₃-alkyl)-(het)arylaminocarbonyl, (het)aryl-C₁₋₃-alkylaminocarbonyl, N-(C₁₋₃-alkyl)-(het)aryl-C₁₋₃-alkylaminocarbonyl,
   C₁₋₃-alkylsulfanyl, C₁₋₃-alkysulfinyl, (het)arylsulfonyl, trifluoromethylsulfanyl, trifluoromethylsulfinyl,
   aminosulfonyl, C₁₋₃-alkyl-aminosulfonyl, di-(C₁₋₃-alkyl)-aminosulfonyl, pyrrolidin-1-yl-sulfonyl, piperidin-1-yl-sulfonyl, morpholin-4-yl-sulfonyl, piperazin-1-yl-sulfonyl, 4-(C₁₋₃-alkyl)-piperazin-1-yl-sulfonyl,
   wherein the above-mentioned saturated heterocycles and cycloalkyl-rings are optionally substituted with one or two groups selected from fluorine, C₁₋₃-alkyl, C₁₋₃-alkoxy, C₁₋₃-alkoxy-C₁₋₃-alkyl, or hydroxy, and
L³ and L⁴ are linked to each other and
L⁵ and L⁶ are linked to each other, such that independently of each other and in each case together with the 2 adjacent C-atoms to which they are attached an aryl- or heteroaryl-group is formed which is fused to the cyclic group B, and which aryl- or heteroaryl-group is optionally substituted with 1, 2 or 3 identical or different R¹⁰,
while by aryl is meant phenyl or naphthtyl and by heteroaryl is meant pyrrolyl, furanyl, thienyl, pyridyl, indolyl, benzofuranyl, benzothiophenyl, quinolinyl, isoquinolinyl, or pyrrolyl, furanyl, thienyl, pyridyl wherein in said pyrrolyl, furanyl, thienyl, pyridyl 1 or 2 CH groups are each replaced by N, or indolyl, benzofuranyl, benzothiophenyl, quinolinyl, isoquinolinyl wherein in said indolyl, benzofuranyl, benzothiophenyl, quinolinyl, isoquinolinyl 1 to 3 CH groups are each replaced by N, or tetrazolyl,
while the above-mentioned (het)aryl is an aryl group as defined hereinbefore, or a heteroaryl group as defined hereinbefore, or a ring selected from the group consisting of 1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl, 1,2,3,4-tetrahydro-2,3-dioxo-pyrazinyl, 2,3-dihydro-2-oxoindolyl, 2,3-dihydrobenzofuranyl, 2,3-dihydro-2-oxo-1H-benzimidazolyl, 2,3-dihydro-2-oxo-benzoxazolyl, 1,2-dihydro-2-oxo-quinolinyl, 1,4-dihydro-4-oxo-quinolinyl, 1,2-dihydro-1-oxo-isoquinolinyl, 1,4-dihydro-4-oxo-cinnolinyl, 1,2-dihydro-2-oxoquinazolinyl, 1,4-dihydro-4-oxo-quinazolinyl, 1,2,3,4-tetrahydro-2,4-dioxoquinazolinyl, 1,2-dihydro-2-oxoquinoxalinyl, 1,2,3,4-tetrahydro-3-oxo-quinoxalinyl, 1,2,3,4-tetrahydro-2,3-dioxo-quinoxalinyl, 1,2-dihydro-1-oxo-phthalazinyl, 1,2,3,4-tetrahydro-1,4-dioxo-phthalazinyl, chromanyl, coumarinyl, 2,3-dihydrobenzo[1,4]dioxinyl and 3,4-dihydro-3-oxo-2H-benzo[1,4]oxazinyl, wherein each of said rings is optionally substituted with 1, 2 or 3 substituents independently of each other selected from R¹⁰,
whilst each of the above-mentioned alkyl or alkylene moieties may be branched or unbranched,
the tautomers, the stereoisomers thereof, the mixtures thereof, and the salts thereof,
while the following compounds (M1) to (M4) are excluded,

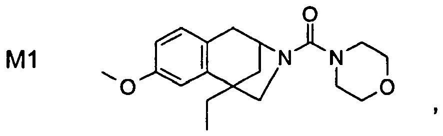

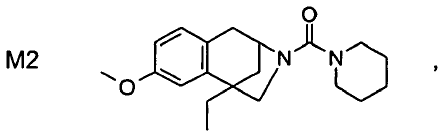

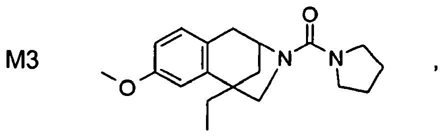

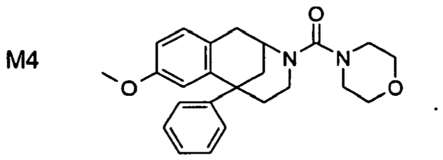

The compounds of general formula I according to the invention and the physiologically acceptable salts thereof have valuable pharmacological properties, particularly an inhibitory effect on the enzyme 11β-hydroxysteroid dehydrogenase (HSD) 1.

A further aspect of the invention also relates to the physiologically acceptable salts of the compounds of general formula I according to this invention with inorganic or organic acids.

In a further aspect this invention relates to pharmaceutical compositions, containing at least one compound of general formula I or a physiologically acceptable salt according to the invention, optionally together with one or more inert carriers and/or diluents.

In a further aspect this invention relates to the compounds according to general formula I, including the compounds (M1), (M2), (M3) and (M4), or the physiologically acceptable salts thereof, for treatment or prevention of diseases or conditions which can be influenced by inhibiting the enzyme 11β-hydroxysteroid dehydrogenase (HSD) 1, such as metabolic disorders.

In a further aspect this invention relates to the use of at least one compound according to general formula I, including the compounds (M1), (M2), (M3) and (M4), or one of the physiologically acceptable salts thereof for preparing a pharmaceutical composition which is suitable for the treatment or prevention of diseases or conditions which can be influenced by inhibiting the enzyme 11β-hydroxysteroid dehydrogenase (HSD) 1, such as metabolic disorders.

In a further aspect the present invention relates to a process for preparing the compounds of general formula I, characterized in that

in order to prepare compounds of general formula I which are defined as hereinbefore and hereinafter,
one of the amines of general formula II 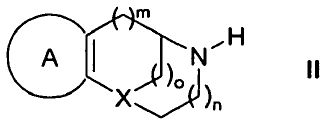 wherein
the groups A, X, m, n and o are defined as hereinbefore and hereinafter,
or of general formula III 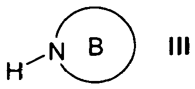 wherein B is definded as hereinbefore and hereinafter,
is reacted with a carbonic acid derivative Y-CO-Y yielding a compound either of general formula IV or V as intermediate 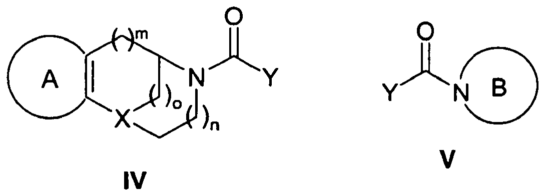 which is optionally isolated and optionally purified,
and the intermediate of the formula IV or V is reacted with the other amine of the formula III or II to yield the compound of the formula I,
wherein Y is a leaving group and in particular denotes fluorine, chlorine, bromine, cyano, C₁₋₄-alkoxy, C₂₋₄-alkenyloxy, C₂₋₄-alkynyloxy, partially or fully fluorinated C₂₋₁₀-alkoxy, oxyarylotriazol, oxyheteroarylotriazol, heteroar-N-yl, 3-methyl-imidazol-1-yl, succinyl-N-oxy, di-(C₁₋₄-alkyl)aminocarbonyloxy, pyrrolylcarbonyloxy, piperidinylcarbonyloxy, morpholinylcarbonyloxy, aryloxy, or heteroaryloxy,
while the alkyl, alkenyl, and alkynyl groups mentioned in the definition of the above groups, either alone or as part of another group, may be substituted with one or more substituents independently of each other selected from fluorine, chlorine, C₁₋₃-alkyl, or C₁₋₃-alkoxy,
while the aryl groups mentioned in the definition of the above groups, either alone or as part of another group, denote phenyl or naphthyl and the heteroaryl groups mentioned in the definition of the above groups, either alone or as part of another group, denote pyridinyl, pyrimidinyl, triazinyl, imidazolyl, pyrazolyl, triazolyl, tetrazolyl, whilst both the aryl and heteroaryl groups optionally are substituted with one or more substituents independently of each other selected from fluorine, chlorine, bromine, C₁₋₃-alkyl, C₁₋₃-alkyloxy, nitro, cyano, or di-(C₁₋₃-alkyl)amino groups,
while the two Y in Y-CO-Y may be identical or different,
while the second Y to be replaced may also be transformed to a more reactive Y after the first Y is replaced with one of the two amines,
optionally in the presence of an organic base such as an amine, e.g. ethyldiisopropylamine, triethylamine, imidazole or pyridine, or an inorganic base, e.g. potassium carbonate or calcium oxide, and/or an additive such as 4-dimethylaminopyridine or 1-hydroxybenzotriazol; wherein the reactions are preferably conducted between 0 and 120 °C; wherein preferred solvents are selected from tetrahydrofuran, 1,2-dimethoxyethane, ether, 1,4-dioxane, dimethylformamide, N,N-dimethylacetamide, N-methylpyrrolidinone, acetonitrile, dichloromethane, 1,2-dichloroethane, toluene, benzene, hexanes, ethyl acetate, but also aqueous and alcoholic solutions may be usable for some of the combinations listed above;
and, if necessary any protective group used in the reactions described above is cleaved concurrently or subsequently;
if desired a compound of general formula I thus obtained is resolved into its stereoisomers;
if desired a compound of general formula I thus obtained is converted into the salts thereof, particularly for pharmaceutical use into the physiologically acceptable salts thereof.

### Detailed Description of the invention

Unless otherwise stated, the groups, residues, and substituents, particularly A, B, R^{N}, R¹ R², R³, R¹⁰, R¹¹, R¹², R^{P}, L¹, L², L³, L⁴, L⁵, L⁶, X, m, n, and o are defined as above and hereinafter. If residues, substituents, or groups occur several times in a compound they may have the same or different meanings. Some preferred meanings of individual scaffolds, groups, and substituents of the compounds according to the invention will be given hereinafter.

The indexes m, n and o each denote independently of each other 0, 1 or 2. Preferably m, n and o are chosen such that the sum of m + n + o is 2, 3 or 4.

Preferred embodiments of this invention are described by each of the formulae I.1 to I.10 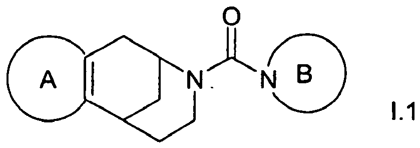 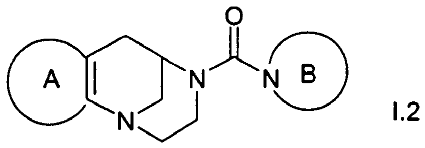 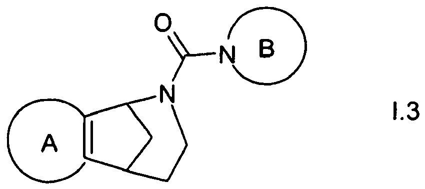 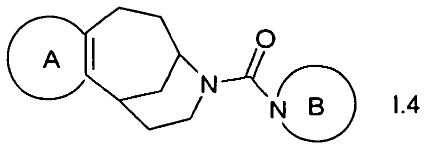 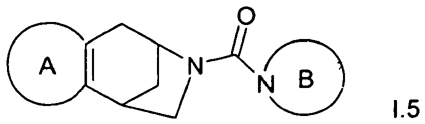 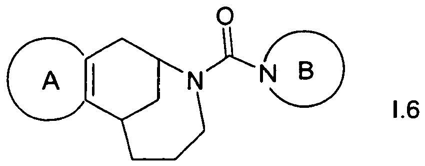 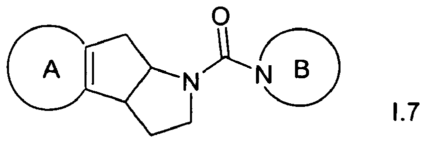 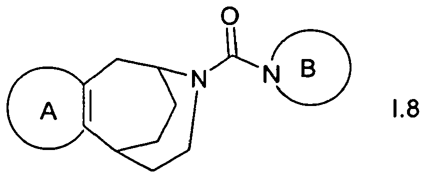 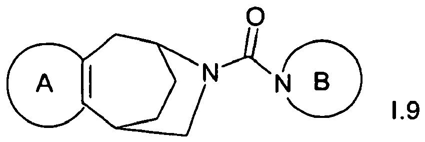 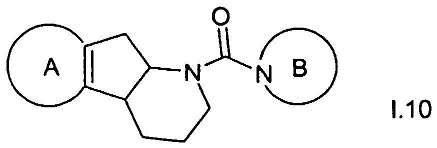 wherein the C₅₊ₘ₊ₙ-azacycloalkene core structure of general formulae I.1 to I.10 including the bridging group -(CH₂)ₒ- is optionally substituted with 1, 2 or more substituents independently of each other selected from the group consisting of R¹¹ and R¹² , and
wherein the rings A and B are defined as hereinbefore and hereinafter, their tautomers, their stereoisomers, mixtures thereof and the salts thereof, while the compounds (M1), (M2), (M3) and (M4) as defined hereinbefore, are excluded.

According to a preferred embodiment of the general formula I.1 compounds of the invention are described by the formula I.1-RR 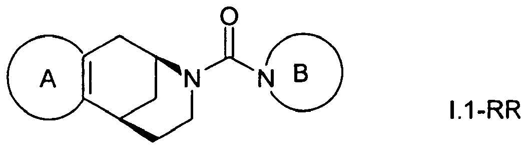 wherein the 2,6-methano-azocin core structure with the stereochemical configuration as depicted is optionally substituted with 1, 2 or more substituents independently of each other selected from the group consisting of R¹¹ and R¹², and wherein the rings A, B and R¹¹, R¹² are defined as hereinbefore and hereinafter, their tautomers, their stereoisomers, mixtures thereof and the salts thereof.

According to another preferred embodiment of the general formula I.1a compounds of the invention are described by the formula I.1-SS 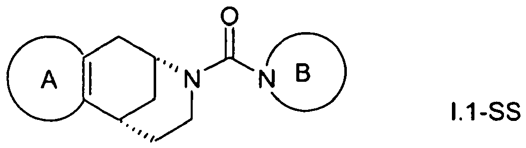 wherein the 2,6-methano-azocin core structure with the stereochemical configuration as depicted is optionally substituted with 1, 2 or more substituents independently of each other selected from the group consisting of R¹¹ and R¹², and wherein the rings A, B and R¹¹, R¹² are defined as hereinbefore and hereinafter, their tautomers, their stereoisomers, mixtures thereof and the salts thereof.

Even more preferred compounds are described by the formulae I.1 to I.10 and I.1-RR and I.1-SS wherein the C₅₊ₘ₊ₙ-azacycloalkene core structure including the bridging group -(CH₂)ₒ- is optionally mono-substituted with R¹¹ and optionally substituted with 1, 2 or 3 substituents independently of each other selected from R¹².

The nitrogen containing ring B preferably denotes azetidin-1-yl, pyrrolidin-1-yl, piperidin-1-yl, azepan-1-yl, azocan-1-yl,
azetidin-1-yl, wherein one -CH₂- group is replaced by O S, NR^{N}, carbonyl, or sulfonyl, or
pyrrolidin-1-yl, piperidin-1-yl, azepan-1-yl, azocan-1-yl, which may be partially or fully unsaturated and wherein one or two -CH₂- groups are independently of each other replaced by O S, carbonyl, or sulfonyl, and wherein one -CH₂- group may be replaced by -NR^{N}-,
aza-bicyclohept-N-yl, aza-bicyclooct-N-yl, aza-bicyclonon-N-yl, aza-bicyclodec-N-yl, bicycloundec-N-yl, bicyclododec-N-yl, each of which may be partially unsaturated, and in each of which one or two -CH₂- groups may be replaced independently of each other by O S, -NR^{N}-, carbonyl, or sulfonyl, and in each of which one -CH= group may be replaced by -N=,
aza-tricyclonon-N-yl, aza-tricyclodec-N-yl, aza-tricycloundec-N-yl, aza-tricyclododec-N-yl, aza-tricyclotridec-N-yl, aza-tricyclotetradec-N-yl, in each of which one or two -CH₂- groups may be replaced independently of each other by O, S, NR^{N}, carbonyl, or sulfonyl, and in each of which one -CH= group may be replaced by -N=,
wherein each of the above rings B may be substituted with one or more substituents independently of each other selected from L¹,
wherein each of the above rings B may be substituted with 1 or 2 substituents independently of each other selected from L²,
wherein 2 adjacent C-atoms of each of the above rings B may be substituted with L³ and L⁴, and
wherein 2 adjacent C-atoms of each of the above rings B may be substituted with L⁵ and L⁶.

More preferably, the ring B denotes pyrrolidin-1-yl, piperidin-1-yl, azepan-1-yl, azocan-1-yl, which may be partially or fully unsaturated, and wherein one or two -CH₂- groups may be independently of each other replaced by O, S, carbonyl, or sulfonyl, and wherein one -CH₂- group may be replaced by -NR^{N}-,
octahydro-cyclopentapyrrol-N-yl, octahydro-indol-N-yl, octahydro-isoindol-N-yl, octahydropyrindin-N-yl, decahydro-quinolin-N-yl, decahydro-isoquinolin-N-yl, decahydro-cyclopentaazepin-N-yl, each of which may be partially unsaturated, and in each of which one or two CH₂ groups may be replaced independently of each other by O, S, -NR^{N}-, carbonyl, or sulfonyl, and in each of which one -CH= group may be replaced by -N=,
aza-bicyclo[3.2.1]octan-N-yl, aza-bicyclo[3.3.1]nonan-N-yl, aza-bicyclo[4.2.1]nonan-N-yl, aza-bicydo[3.2.2]nonan-N-yl, aza-bicyclo[5.2.1]decan-N-yl, aza-bicyclo-[4.2.2]decan-N-yl, aza-bicydo[3.3.2]decan-N-yl, each of which may be partially unsaturated, and in each of which one or two -CH₂- groups may be replaced independently of each other by O, S, -NR^{N}-, carbonyl, or sulfonyl, and in each of which one -CH= group may be replaced by -N=,
2-aza-adamant-2-yl, in which one -CH₂- group may be replaced by O, S, -NR^{N}-, carbonyl, or sulfonyl, and in each of which one -CH= group may be replaced by -N=,
wherein each of the above rings B may be substituted with one or more substituents independently of each other selected from L¹,
wherein each of the above rings B may be substituted with 1 or 2 substituents independently of each other selected from L²,
wherein 2 adjacent C-atoms of each of the above rings B may be substituted with L³ and L⁴, and
wherein 2 adjacent C-atoms of each of the above rings B may be substituted with L⁵ and L⁶.

Most preferably, the ring B denotes pyrrolidin-1-yl, piperidin-1-yl, azepan-1-yl, azocan-1-yl, 2-oxo-pyrrolidin-1-yl, 2-oxo-imidazolidin-1-yl, 2-oxo-piperidin-1-yl, piperazin-1-yl, morpholin-4-yl, 2-oxo-piperazin-1-yl, 3-oxo-piperazin-1-yl, 2-oxomorpholin-4-yl, 3-oxo-morpholin-4-yl, 2,3-dihydro-indol-1-yl, 5,6-dihydro-8H-[1,2,4]triazolo[4,3-a]pyrazin-7-yl, 1,2,3,4-tetrahydro-isoquinolin-2-yl, 2,3-dihydrobenzo[1,4]oxazin-4-yl, 8-aza-bicyclo[3.2.1]octan-8-yl, 3-aza-bicyclo[3.2.1]octan-3-yl, 3-oxo-8-aza-bicyclo[3.2.1]oct-8-yl, 9-aza-bicyclo[3.3.1]nonan-9-yl, 2-azabicyclo[3.3.1]nonan-2-yl, 3-aza-bicyclo[3.3.1]nonan-3-yl, 9-aza-bicyclo[4.2.1]nonan-9-yl, 3-aza-bicyclo[3.2.2]nonan-3-yl, 10-aza-bicyclo[5.2.1]decan-10-yl, 3-azabicyclo[4.2.2]decan-3-yl, 3-aza-bicyclo[3.3.2]decan-3-yl, 2-aza-adamant-2-yl, 1-oxo-2,7-diaza-spiro[4.5]dec-7-yl, 7-oxo-2,8-diaza-spiro[5.5]undec-2-yl, particularly, azetidin-1-yl, pyrrolidin-1-yl, piperidin-1-yl, azepan-1-yl, morpholin-4-yl, piperazin-1-yl, 2,3-dihydro-indol-1-yl, 5,6-dihydro-8H-[1,2,4]triazolo[4,3-a]pyrazin-7-yl, 1,2,3,4-tetrahydro-isoquinolin-2-yl, 2,3-dihydro-benzo[1,4]oxazin-4-yl, 8-azabicyclo[3.2.1]octan-8-yl, 9-aza-bicyclo[3.3.1]nonan-9-yl, 1-oxo-2,7-diazaspiro[4.5]dec-7-yl, 7-oxo-2,8-diaza-spiro[5.5]undec-2-yl, 2-oxo-imidazolidin-1-yl,
wherein each of the above rings B may be substituted with one or more substituents independently of each other selected from L¹,
wherein each of the above rings B may be substituted with 1 or 2 substituents independently of each other selected from L²,
wherein 2 adjacent C-atoms of each of the above rings B may be substituted with L³ and L⁴, and
wherein 2 adjacent C-atoms of each of the above rings B may be substituted with L⁵ and L⁶.

Examples of preferred meanings of the ring B are shown in the following: 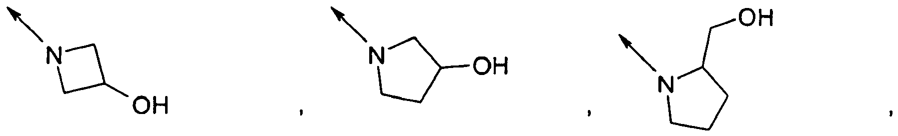 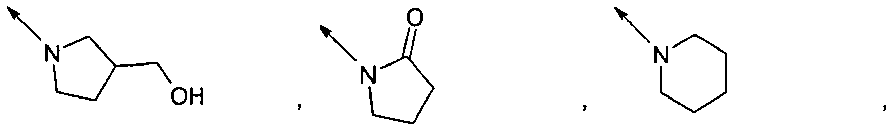 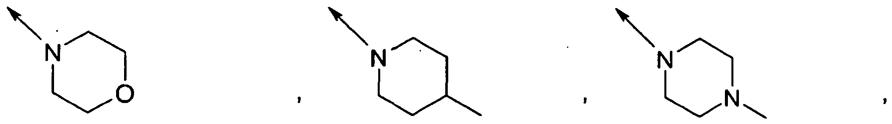 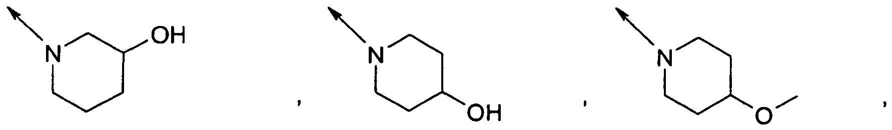 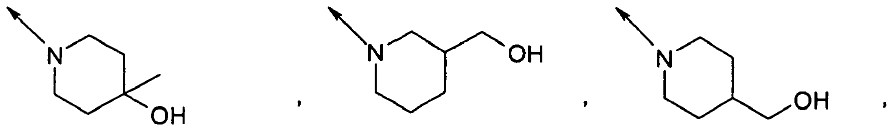 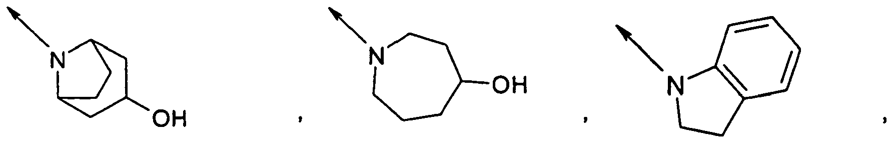 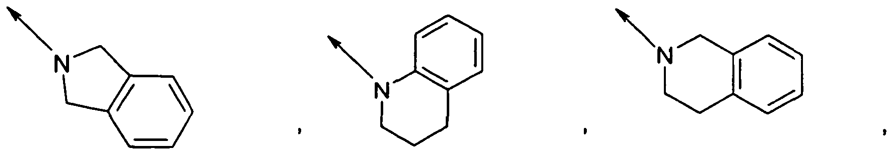 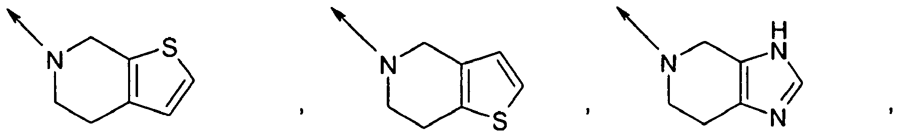 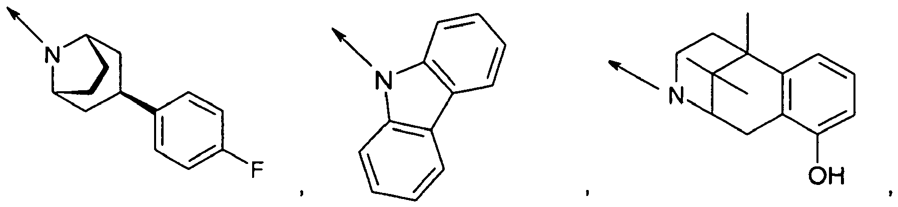 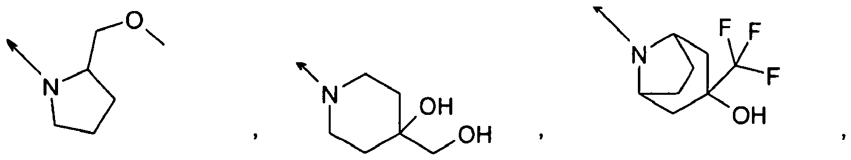 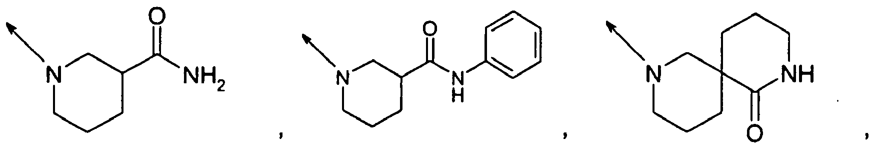 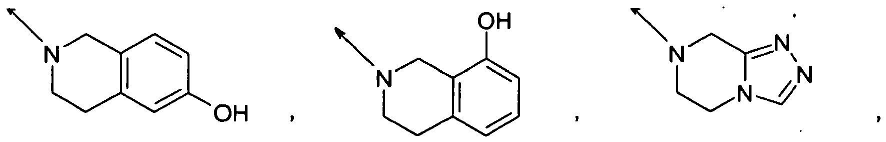 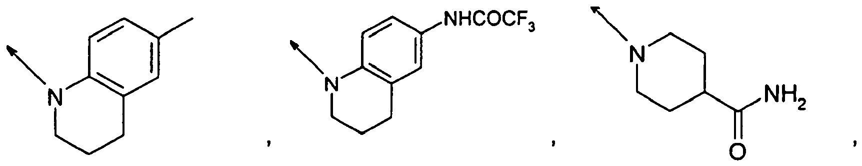 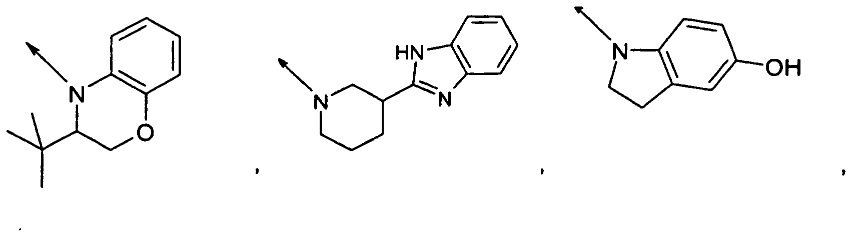 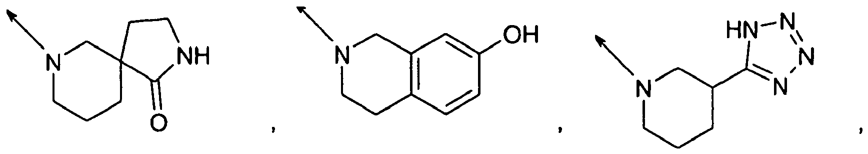 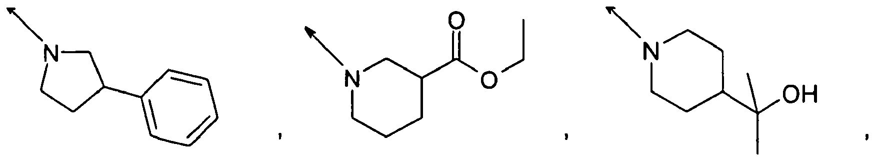 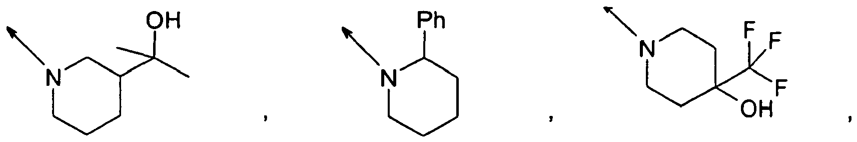 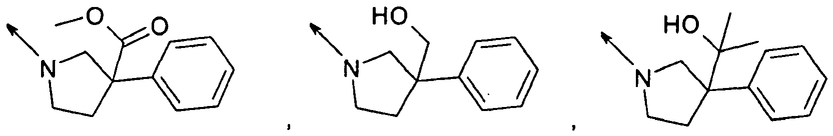 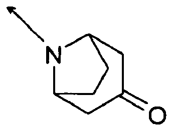 wherein these groups may additionally be monosubstitued with L¹ and additionally monosubstitued with L².

Preferably, the ring A denotes a benzo, pyrido, pyrrolo, furo, thieno, pyridazino, pyrimido, or pyrazino ring wherein each of said rings is optionally substituted with one or more substituents independently of each other selected from R¹, and wherein 2 adjacent C-atoms of each of said rings are optionally substituted with R² and R³; or a pyrazolo, oxazolo, thiazolo, or imidazo ring each of which is optionally substituted with R¹.

More preferably, the ring A denotes a benzo or pyrido ring, most preferably a benzo ring, wherein each of said rings is optionally substituted with one or more substituents independently of each other selected from R¹, and wherein 2 adjacent C-atoms of each of said rings are optionally substituted with R² and R³.

Preferably, R^{N} denotes hydrogen, C₁₋₆-alkyl, C₃₋₆-alkenyl, phenyl, C₁₋₄-alkylcarbonyl, C₁₋₄-alkylsulfonyl, phenylcarbonyl, phenylsulfonyl, wherein each alkyl group may be mono- or polysubstituted with fluorine and may be monosubstituted with hydroxy, C₁₋₄-alkoxy, C₁₋₄-alkylcarbonylamino, cyano, carboxy, C₁₋₄-alkoxycarbonyl, aminocarbonyl, C₁₋₄-alkylaminocarbonyl, di-(C₁₋₄-alkyl)aminocarbonyl or phenyl, and wherein each phenyl group may be monosubstitued with R¹⁰. More preferably, R^{N} denotes hydrogen, C₁₋₆-alkyl, phenyl, C₁₋₄-alkylcarbonyl, C₁₋₄-alkylsulfonyl, wherein each alkyl group may be mono- or polysubstituted with fluorine and may be monosubstitued with hydroxy, C₁₋₄-alkoxy, or phenyl, and wherein each phenyl group may be monosubstitued with R¹⁰. Most preferably, R^{N} denotes hydrogen, methyl, ethyl, isopropyl, phenyl, acetyl, methylsulfonyl.

Preferably, the one or more substituents L¹, which may be identical or different, denote fluorine, chlorine, C₁₋₄-alkyl, trifluoromethyl, hydroxy, C₁₋₄-alkoxy, cyano, more preferably fluorine, methyl, ethyl, trifluoromethyl, hydroxy, methoxy, ethoxy, cyano, particularly fluorine, methyl, trifluoromethyl, hydroxy, and methoxy.

Preferably, the one or more substituents L², which may be identical or different, denote fluorine, chlorine, bromine, cyano, hydroxy, C₁₋₆-alkyl, C₂₋₆-alkenyl, C₂₋₆-alkynyl, C₁₋₄-alkyloxy, difluoromethyl, trifluoromethyl, difluoromethoxy, trifluoromethoxy, C₃₋₆-cycloalkyl, C₃₋₆-cycloalkyloxy, C₃₋₆-cycloalkyl-C₁₋₃-alkyl, C₃₋₆-cycloalkyl-C₁₋₃-alkyloxy, (het)aryl, (het)aryloxy, (het)aryl-C₁₋₃-alkyl, (het)aryl-C₁₋₃-alkyloxy, (het)aryloxy- C₁₋₃-alkyl,
amino, C₁₋₃-alkylamino, di-(C₁₋₃-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₃-alkyl)-piperazin-1-yl, 4-(C₁₋₄-alkylcarbonyl)-piperazin-1-yl, 4-(C₃₋₆-cycloalkylcarbonyl)-piperazin-1-yl, 4-(C₁₋₄-alkyloxycarbonyl)-piperazin-1-yl, 4-(C₁₋₄-alkylsulfonyl)-piperazin-1-yl, 2-oxo-4-(C₁₋₃-alkyl)-piperazin-1-yl, 3-oxo-4-(C₁₋₃-alkyl)-piperazin-1-yl,
C₁₋₃-alkyl-carbonylamino, (het)aryl-carbonylamino, (het)aryl-C₁₋₃-alkyl-carbonylamino, C₁₋₃-alkyloxy-carbonylamino, aminocarbonylamino, C₁₋₃-alkyl-aminocarbonylamino, di-(C₁₋₃-alkyl)aminocarbonylamino, pyrrolidin-1-yl-carbonylamino, piperidin-1-yl-carbonylamino, morpholin-4-yl-carbonylamino, piperazin-1-yl-carbonylamino, 4-(C₁₋₃-alkyl)-piperazin-1-yl-carbonylamino, C₁₋₃-alkyl-sulfonylamino, aminosulfonylamino, C₁₋₃-alkylamino-sulfonylamino, di-(C₁₋₃-alkyl)amino-sulfonylamino, pyrrolidin-1-yl-sulfonylamino, piperidin-1-yl-sulfonylamino, morpholin-4-yl-sulfonylamino, piperazin-1-yl-sulfonylamino, 4-(C₁₋₃-atkyl)-piperazin-1-yl-sulfonylamino, (C₁₋₃-alkyloxycarbonylamino)carbonylamino, (het)arylsulfonylamino, (het)aryl-C₁₋₃-alkylsulfonylamino,
N-(C₁₋₃-alkyl)-C₁₋₃-alkyl-carbonylamino, N-(C₁₋₃-alkyl)-(het)arylcarbonylamino, N-(C₁₋₃-alkyl)-(het)aryl-C₁₋₃-alkyl-carbonylamino, N-(C₁₋₃-alkyl)-C₁₋₃-alkyloxy-carbonylamino, N-(aminocarbonyl)-C₁₋₃-alkylamino, N-(C₁₋₃-alkyl-aminocarbonyl)-C₁₋₃-alkylamino, N-[di-(C₁₋₃-alkyl)aminocarbonyl]-C₁₋₃-alkylamino,
N-(C₁₋₃-alkyl)-C₁₋₃-alkyl-sulfonylamino, N-(C₁₋₃-alkyl)-(het)arylsulfonylamino, N-(C₁₋₃-alkyl)-(het)aryl-C₁₋₃-alkyl-sulfonylamino,
carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃alkyl)-piperazin-1-yl-carbonyl, (het)arylaminocarbonyl, N-(C₁₋₃-alkyl)-(het)arylaminocarbonyl, (het)aryl-C₁₋₃-alkylaminocarbonyl, N-(C₁₋₃-alkyl)-(het)aryl-C₁₋₃-alkylaminocarbonyl,
C₁₋₃-alkyl-carbonyl, (het)aryl-carbonyl,
carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃-alkyl-aminocarbonyl-C₁₋₃-alkyl, di-(C₁₋₃-alkyl)-aminocarbonyl-C₁₋₃-alkyl, pyrrolidin-1-yl-carbonyl-C₁₋₃-alkyl, piperidin-1-yl-carbonyl-C₁₋₃-alkyl, morpholin-4-yl-carbonyl-C₁₋₃-alkyl, piperazin-1-yl-carbonyl-C₁₋₃-alkyl, 4-(C₁₋₃-alkyl)-piperazin-1-yl-carbonyl-C₁₋₃-alkyl,
carboxy-C₁₋₃-alkyloxy, C₁₋₃-alkyloxy-carbonyl-C₁₋₃-alkyloxy, cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy, pyrrolidin-1-yl-carbonyl-C₁₋₃-alkyl-oxy, piperidin-1-yl-carbonyl-C₁₋₃-alkyloxy, morpholin-4-yl-carbonyl-C₁₋₃-alkyl-oxy, piperazin-1-yl-carbonyl-C₁₋₃-alkyloxy, 4-(C₁₋₃-alkyl)-piperazin-1-yl-carbonyl-C₁₋₃-alkyloxy,
hydroxy-C₁₋₃-alkyl, C₁₋₃-alkyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, pyrrolidin-1-yl-C₁₋₃-alkyl, 2-oxo-pyrrolidin-1-yl-C₁₋₃-alkyl, piperidin-1-yl-C₁₋₃-alkyl, 2-oxo-piperidin-1-yl-C₁₋₃-alkyl, morpholin-4-yl-C₁₋₃-alkyl, 3-oxo-morpholin-4-yl-C₁₋₃-alkyl, piperazin-1-yl-C₁₋₃-alkyl, 2-oxo-piperazin-1-yl-C₁₋₃-alkyl, 3-oxo-piperazin-1-yl-C₁₋₃-alkyl, 4-(C₁₋₃-alkyl)-piperazin-1-yl-C₁₋₃-alkyl, 2-oxo-4-(C₁₋₃-alkyl)-piperazin-1-yl-C₁₋₃-alkyl, 3-oxo-4-(C₁₋₃-alkyl)-piperazin-1-yl-C₁₋₃-alkyl,
C₁₋₃-alkylcarbonylamino-C₁₋₃-alkyl, arylcarbonylamino-C₁₋₃-alkyl,
hydroxy-C₁₋₃-alkyloxy, C₁₋₃-alkyloxy-C₁₋₃-alkyloxy, C₁₋₃-alkylsulfanyl-C₁₋₃-alkyloxy, C₁₋₃-alkylsulfinyl-C₁₋₃-alkyloxy, C₁₋₃-alkylsulfonyl-C₁₋₃-alkyloxy, amino-C₁₋₃-alkyloxy, C₁₋₃-alkylamino-C₁₋₃-alkyloxy, di-(C₁₋₃-alkyl)-amino-C₁₋₃-alkyloxy, pyrrolidin-1-yl-C₁₋₃-alkyloxy, 2-oxo-pyrrolidin-1-yl-C₁₋₃-alkyloxy, piperidin-1-yl-C₁₋₃-alkyloxy, 2-oxo-piperidin-1-yl-C₁₋₃-alkyloxy, morpholin-4-yl-C₁₋₃-alkyloxy, 3-oxo-morpholin-4-yl-C₁₋₃-alkyloxy, piperazin-1-yl-C₁₋₃-alkyloxy, 2-oxo-piperazin-1-yl-C₁₋₃-alkyloxy, 3-oxo-piperazin-1-yl-C₁₋₃-alkyloxy, 4-(C₁₋₃-alkyl)-piperazin-1-yl-C₁₋₃-alkyloxy, 2-oxo-4-(C₁₋₃-alkylFpiperazin-1-yl-C₁₋₃-alkyloxy, 3-oxo-4-(C₁₋₃-alkyl)-piperazin-1-yl-C₁₋₃-alkyloxy,
C₁₋₃-alkylsulfanyl, C₁₋₃-alkylsulfinyl, C₁₋₃-alkylsulfonyl, (het)arylsulfonyl, trifluoromethylsulfanyl, trifluoromethylsulfinyl, trifluoromethylsulfonyl,
wherein the above-mentioned saturated heterocycles and cycloalkyl-rings are optionally substituted with one or two groups independently of each other selected from C₁₋₃-alkyl, C₁₋₃-alkoxy, C₁₋₃-alkoxy-C₁₋₃-alkyl, or hydroxy, and
wherein the above-mentioned (het)aryl is phenyl, naphthyl, pyrrolyl, furanyl, thienyl, pyridyl, indolyl, benzofuranyl, benzothiophenyl, quinolinyl, isoquinolinyl, or pyrrolyl, furanyl, thienyl, pyridyl in which 1 or 2 CH are each replaced by N, or indolyl, benzofuranyl, benzothiophenyl, quinolinyl, isoquinolinyl in which 1 to 3 CH are each replaced by N, or tetrazolyl. and
wherein the above-mentioned (het)aryl groups are optionally substituted with one or two R¹⁰ which may be identical or different.

More preferably, the one or more substituents L², which may be identical or different, denote fluorine, chlorine, bromine, cyano, C₁₋₆-alkyl, C₂₋₆-alkenyl, C₂₋₆-alkynyl, hydroxy, C₁₋₄-alkyloxy, trifluoromethyl, difluoromethoxy, trifluoromethoxy, C₃₋₆-cycloalkyl, C₃₋₆-cycloalkyloxy, phenyl, phenoxy, tetrazolyl, benzimidazolyl,
amino, C₁₋₃-alkylamino, di-(C₁₋₃-alkyl)amino, C₁₋₃-alkyl-carbonylamino, phenyl-carbonylamino, C₁₋₃-alkyl-sulfonylamino, N-(C₁₋₃-alkyl)-C₁₋₃-alkyl-carbonylamino, N-(C₁₋₃-alkyl)-phenylcarbonylamino, N-(C₁₋₃-alkyl)-C₁₋₃-alkyl-sulfonylamino,
carboxy, C₁₋₃-alkyloxy-carbonyl, aminocarbonyl, C₁₋₃alkyl-aminocarbonyl, di-(C₁₋₃-alkyl)-aminocarbonyl, phenylaminocarbonyl, N-(C₁₋₃-alkyl)-phenylaminocarbonyl,
carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃-alkyl-aminocarbonyl-C₁₋₃-alkyl, di-(C₁₋₃-alkyl)-aminocarbonyl-C₁₋₃-alkyl,
carboxy-C₁₋₃-alkyloxy, C₁₋₃alkyloxy-carbonyl-C₁₋₃-alkyloxy, cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy,
hydroxy-C₁₋₃-alkyl, C₁₋₃-alkyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl,
C₁₋₃-alkylcarbonylamino-C₁₋₃-alkyl,
hydroxy-C₁₋₃-alkyloxy, C₁₋₃-alkyloxy-C₁₋₃-alkyloxy, amino-C₁₋₃-alkyloxy, C₁₋₃-alkylamino-C₁₋₃-alkyloxy, di-(C*₁₋₃-alkyl)-amino-C₁₋₃-alkyloxy,
C₁₋₃-alkylsulfanyl, C₁₋₃-alkylsulfinyl, C₁₋₃-alkylsulfonyl,
and wherein the above-mentioned phenyl groups are optionally substituted with one or two R¹⁰ which may be identical or different.

Most preferably, the one or more substituents L², which may be identical or different, denote fluorine, C₁₋₄-alkyl, hydroxy, C₁₋₃-alkoxy, hydroxy-C₁₋₃-alkyl , C₁₋₃-alkyloxy-C₁₋₃-alkyl, C₁₋₃-alkyloxy-carbonyl, aminocarbonyl, phenylaminocarbonyl, tetrazolyl, benzimidazolyl, or phenyl which may be monosubstituted with R¹⁰, particularly methyl, tert-butyl, hydroxy, methoxy, hydroxymethyl, 2-hydroxy-prop-2-yl, methoxymethyl, ethoxycarbonyl, aminocarbonyl, phenylaminocarbonyl, phenyl, 4-fluorophenyl, tetrazol-5-yl, or benzimidazol-2-yl.

Preferably, L³ and L⁴, which are linked to each other, form with the atoms to which they are linked an aryl- or heteroaryl-group which is fused to the cyclic group B, and which is optionally substituted with 1, 2 or 3 identical or different R¹⁰,
while said aryl- or heteroaryl group is selected from the group consisting of phenyl, pyrrolyl, furanyl, thienyl, pyridyl, indolyl, benzofuranyl, benzothiophenyl, quinolinyl, isoquinolinyl, and
pyrrolyl, furanyl, thienyl, pyridyl wherein in said pyrrolyl, furanyl, thienyl and pyridyl one -CH= group is replaced by -N=, and
indolyl, quinolinyl, isoquinolinyl wherein in said indolyl, quinolinyl and isoquinolinyl one or two -CH= groups are each replaced by -N=.

More preferably, L³ and L⁴, which are linked to each other, form with the atoms to which they are linked an aryl- or heteroaryl-group which is fused to the cyclic group B, wherein said fused aryl- or heteroaryl-group is selected from the group consisting of benzo, pyrido, pyrimido, pyrazino, pyridazino, pyrrolo, furano, thieno, imidazo, pyrazolo, oxazolo, isoxazolo, thiazolo, isothiazolo, each of which is optionally substituted with one to three identical or different R¹⁰.

Even more preferably L³ and L⁴, which are linked to each other, form with the atoms to which they are linked an aryl- or heteroaryl-group which is fused to the cyclic group B, wherein said fused aryl- or heteroaryl-group is selected from the group consisting of benzo, pyrido, pyrimido, pyrazino, pyridazino, pyrrolo, furano, thieno, imidazo, pyrazolo, oxazolo, isoxazolo, thiazolo and isothiazolo, each of which is optionally substituted with 1, 2 or 3 identical or different R¹⁰.

Most preferably L³ and L⁴, which are linked to each other, form with the atoms to which they are linked an aryl- or heteroaryl-group which is fused to the cyclic group B, wherein said fused aryl- or heteroaryl-group is selected from the group consisting of benzo, pyrido, pyrimido, pyrrolo, furano, thieno, imidazo and oxazolo group, each of which is optionally substituted with 1, 2 or 3 identical or different R¹⁰, particularly benzo, thieno, and imidazo, each of which is optionally substituted with 1, 2 or 3 identical or different R¹⁰.

Preferably, L⁵ and L⁶, which are linked to each other, form with the atoms to which they are linked an aryl- or heteroaryl-group which is fused to the cyclic group B, wherein said fused aryl- or heteroaryl-group is selected from the group consisting of benzo, pyrido, pyrimido, pyrazino, pyridazino, pyrrolo, furano, thieno, imidazo, pyrazolo, oxazolo, isoxazolo, thiazolo, and isothiazolo, each of which is optionally substituted with 1, 2 or 3 identical or different R¹⁰, more preferably selected from the group consisting of benzo, pyrido, pyrimido, pyrazino, and pyridazino, each of which is optionally substituted with one to three identical or different R¹⁰, most preferably benzo, which is optinally substituted with 1, 2 or 3 identical or different R¹⁰.

Regarding the definitions of L³ and L⁴ and of L⁵ and L⁶ in cases where an N-containing heteroaryl-group fused to the cyclic group B is formed, and where said N-containing heteroaryl-group is substituted with hydroxy at the carbon atom adjacent to the nitrogen, then a tautomeric amide substructure may be formed and is part of the invention. Examples of substructures of the ring B wherein two adjacent C-atoms are substituted with L³ and L⁴, wherein a tautomeric amide is formed are depicted in the following table:

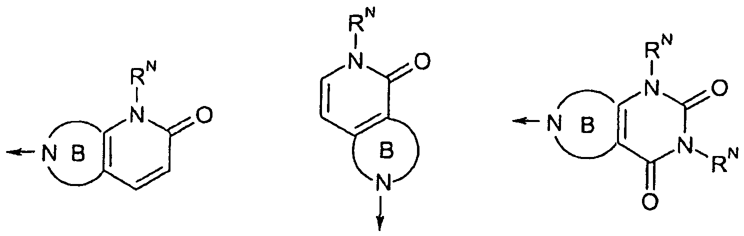

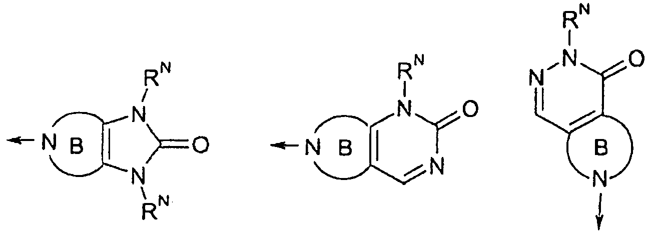

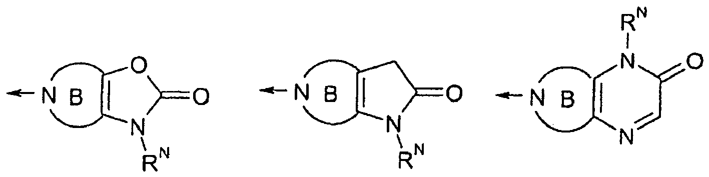

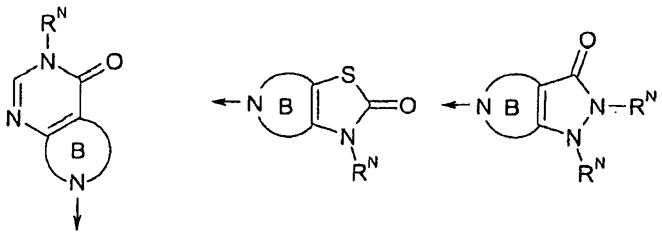

Preferably, the substituent R¹ denotes fluorine, chlorine, cyano, C₁₋₄-alkyl, difluoromethyl, trifluoromethyl, trifluoromethyl-hydroxy-C₁₋₂-alkyl, difluoromethoxy, trifluoromethoxy, C₃₋₆-cycloalkyl, C₃₋₆-cycloalkyloxy, C₃₋₆-cycloalkyl-C₁₋₃-alkyl, C₃₋₆-cycloalkyl-C₁₋₃-alkyloxy, tetrahydrofuran-3-yloxy, tetrahydropyran-3-yloxy, tetrahydropyran-4-yloxy, tetrahydrofuranyl-C₁₋₃-alkyloxy, tetrahydropyranyl-C₁₋₃-alkyloxy, (het)aryl, (het)aryloxy, (het)aryl-C₁₋₃-alkyl, (het)aryl-C₁₋₃-alkyloxy, (het)aryloxy- C₁₋₃-alkyl, C₁₋₃-alkyl-carbonyl, (het)aryl-carbonyl,
amino, C₁₋₃-alkylamino, di-(C₁₋₃-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, 3-oxo-piperazin-1-yl, 4-(C₁₋₄-alkylcarbonyl)-piperazin-1-yl,
C₁₋₃-alkyl-carbonylamino, (het)aryl-carbonylamino, C₁₋₃-alkyloxy-carbonylamino, C₁₋₃-alkyl-aminocarbonylamino, di-(C₁₋₃-alkyl)aminocarbonylamino, pyrrolidin-1-yl-carbonylamino, piperidin-1-yl-carbonylamino, morpholin-4-yl-carbonylamino, C₁₋₃-alkyl-sulfonylamino, C₁₋₃-alkylamino-sulfonylamino, di-(C₁₋₃-alkyl)aminosulfonylamino, pyrrolidin-1-yl-sulfonylamino, piperidin-1-yl-sulfonylamino, morpholin-4-yl-sulfonylamino, (het)arylsulfonylamino,
N-(C₁₋₃-alkyl)-C₁₋₃-alkyl-carbonylamino, N-(C₁₋₃-alkyl)-(het)arylcarbonylamino, N-(C₁₋₃-alkyl)-C₁₋₃-alkyloxy-carbonylamino, N-(C₁₋₃-alkyl-aminocarbonyl)C₁₋₃-alkylamino, N-[di-(C₁₋₃-alkyl)aminocarbonyl]-C₁₋₃-alkylamino,
N-(C₁₋₃-alkyl)-C₁₋₃-alkyl-sulfonylamino, N-(C₁₋₃-alkyl)-(het)arylsulfonylamino,
(hydroxyimino)aminomethyl, (C₁₋₃-alkyloxyimino)aminomethyl, carboxy, C₁₋₃-alkyloxycarbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl,
carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃-alkyl-aminocarbonyl-C₁₋₃-alkyl, di-(C₁₋₃-alkylraminocarbonyl-C₁₋₃-alkyl, pyrrolidin-1-yl-carbonyl-C₁₋₃-alkyl, piperidin-1-yl-carbonyl-C₁₋₃-alkyl, morpholin-4-yl-carbonyl-C₁₋₃-alkyl,
carboxy-C₁₋₃-alkyloxy, C₁₋₃-alkyloxy-carbonyl-C₁₋₃-alkyloxy, cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy, pyrrolidin-1-yl-carbonyl-C₁₋₃-alkyl-oxy, piperidin-1-yl-carbonyl-C₁₋₃-alkyloxy, morpholin-4-yl-carbonyl-C₁₋₃-alkyl-oxy,
hydroxy-C₁₋₃-alkyl, C₁₋₃-alkyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, pyrrolidin-1-yl-C₁₋₃-alkyl, 2-oxo-pyrrolidin-1-yl-C_{1- 3}-alkyl, C₁₋₄-alkylcarbonyl-amino-C₁₋₃-alkyl, N-(C₁₋₃-alkyl)-C₁₋₄-alkylcarbonyl-amino-C₁₋₃-alkyl, 2-oxo-piperidin-1-yl-C₁₋₃-alkyl, 3-oxo-morpholin-4-yl-C₁₋₃-alkyl,
hydroxy-C₁₋₃-alkyloxy, C₁₋₃-alkyloxy-C₁₋₃-alkyloxy, C₁₋₃-alkylsulfinyl-C₁₋₃-alkyloxy, C₁₋₃-alkylsulfonyl-C₁₋₃-alkyloxy, di-(C₁₋₃-alkyl)-amino-C₁₋₃-alkyloxy, 2-oxo-pyrrolidin-1-yl-C₁₋₃-alkyloxy, 2-oxo-piperidin-1-yl-C₁₋₃-alkyloxy, morpholin-4-yl-C₁₋₃-alkyloxy, 3-oxo-morpholin-4-yl-C₁₋₃-alkyloxy,
aminosulfonyl, C₁₋₃-alkyl-aminosulfonyl, di-(C₁₋₃-alkyl)-aminosulfonyl, pyrrolidin-1-yl-sulfonyl, piperidin-1-yl-sulfonyl,-morpholin-4-yl-sulfonyl,
wherein the above-mentioned (het)aryl is defined as described hereinbefore and hereinafter.

More preferably, R¹ denotes fluorine, chlorine, cyano, C₁₋₄-alkyl, trifluoromethyl, trifluoromethyl-hydroxy-C₁₋₂-alkyl, difluoromethoxy, trifluoromethoxy, C₃₋₆-cycloalkyloxy, tetrahydrofuran-3-yloxy, tetrahydropyran-3-yloxy, tetrahydropyran-4-yloxy, tetrahydrofuranyl-C₁₋₃-alkyloxy, tetrahydropyranyl-C₁₋₃-alkyloxy, (het)aryl, (het)aryloxy, (het)aryl-C₁₋₃-alkyloxy, C₁₋₃-alkyl-carbonyl, or
amino, C₁₋₃-alkylamino, 2-oxo-pyrrolidin-1-yl, 2-oxo-piperidin-1-yl, morpholin-4-yl, 3-oxo-morpholin-4-yl, C₁₋₃-alkyl-carbonylamino, (het)aryl-carbonylamino, C₁₋₃-alkylsulfonylamino, N-(C₁₋₃-alkyl)-C₁₋₃-alkyl-carbonylamino, N-(C₁₋₃-alkyl)-(het)arylcarbonylamino,
N-(C₁₋₃-alkyl)-C₁₋₃-alkyl-sulfonylamino, N-(C₁₋₃-alkyl)-(het)arylsulfonylamino,
carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl,
carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃-alkyl-aminocarbonyl-C₁₋₃-alkyl, di-(C₁₋₃-alkyl)-aminocarbonyl-C₁₋₃-alkyl, pyrrolidin-1-yl-carbonyl-C₁₋₃-alkyl, piperidin-1-yl-carbonyl-C₁₋₃-alkyl, morpholin-4-yl-carbonyl-C₁₋₃-alkyl,
cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy, pyrrolidin-1-yl-carbonyl-C₁₋₃-alkyl-oxy, piperidin-1-yl-carbonyl-C₁₋₃-alkyloxy, morpholin-4-yl-carbonyl-C₁₋₃-alkyl-oxy,
hydroxy-C₁₋₃-alkyl, C₁₋₃-alkyloxy-C₁₋₃-alkyl, C₁₋₄-alkylcarbonyl-amino-C₁₋₃-alkyl, N-(C₁₋₃-alkyl)-C₁₋₃-alkylcarbonyl-amino-C₁₋₃-alkyl, 2-oxo-pyrrolidin-1-yl-C₁₋₃-alkyl, 2-oxo-piperidin-1-C₁₋₃-alkyl, 3-oxo-morpholin-4-yl-C₁₋₃-alkyl,
hydroxy-C₁₋₃-alkyloxy, C₁₋₃-alkyloxy-C₁₋₃-alkyloxy,
aminosulfonyl, C₁₋₃-alkyl-aminosulfonyl, di-(C₁₋₃-alkyl)-aminosulfonyl,
wherein the above-mentioned (het)aryl groups are selected from the group consisting of phenyl, furanyl, thienyl, oxazolyl, thiazolyl, isoxazolyl, imidazolyl, pyrazolyl, oxadiazolyl, triazolyl, tetrazolyl, pyridinyl, pyrimidinyl, pyrazinyl, pyridazinyl, 1,2-dihydro-1-methyl-2-oxo-pyridinyl, 2,3-dihydro-2-methyl-3-oxo-pyridazinyl, and triazinyl, wherein each group is optionally mono- or disubstituted with identical or different R¹⁰.

Even more preferably, R¹ denotes fluorine, chlorine, C₁₋₃-alkyl, amino, C₁₋₃-alkyl-carbonylamino, C₁₋₃-alkyl-sulfonylamino, cyano, carboxy, C₁₋₃-alkyloxy-carbonyl, aminocarbonyl, C₁₋₃-alkyl-aminocarbonyl, di-(C₁₋₃-alkyl)-aminocarbonyl, hydroxy-C₁₋₃-alkyl, trifluoromethyl-hydroxy-C₁₋₃-alkyl, C₁₋₃-alkyloxy-C₁₋₃-alkyl, C₁₋₃-alkyl-carbonyl-amino-C₁₋₃-alkyl, cyano-C₁₋₃-alkyloxy, hydroxy-C₁₋₃-alkyloxy, C₁₋₃-alkyloxy-C₁₋₃-alkyloxy, trifluoromethyl, difluoromethoxy, trifluoromethoxy, C₁₋₃-alkylcarbonyl, aminosulfonyl, C₁₋₃-alkyl-aminosulfonyl, di-(C₁₋₃-alkyl)-aminosulfonyl, cyano-C₁₋₃-alkyloxy, phenyl-C₁₋₃-alkyloxy, phenoxy, phenyl, pyridinyl, 1,2-dihydro-1-methyl-2-oxo-pyridinyl, pyrimidinyl, pyridazinyl, 2,3-dihydro-2-methyl-3-oxo-pyridazinyl, 4-methyl-4H-[1,2,4]triazol-3-yl, or oxadiazolyl, wherein each (het)aryl mentioned is optionally mono- or disubstituted with identical or different R¹⁰.

Most preferably R¹ denotes fluorine, chlorine, methyl, hydroxy, methoxy, amino, acetylamino, methylsulfonylamino, cyano, methoxycarbonyl, ethoxycarbonyl, acetyl, 1-hydroxyethyl, 1-hydroxy-1-methyl-ethyl, 2,2,2-trifluoro-1-hydroxy-1-methyl-ethyl, methylsulfonyl, aminosulfonyl, methylaminosulfonyl, dimethylaminosulfonyl, cyanomethoxy, benzyloxy, phenoxy, phenyl, pyridin-3-yl, pyridin-4-yl, 1,2-dihydro-1-methyl-2-oxo-pyridin-5-yl, 1,2-dihydro-1-methyl-2-oxo-pyridin-4-yl, pyrimidin-4-yl, 2-methyl-pyrimidin-4-yl, 2-methyl-pyrimidin-5-yl, 6-methyl-pyridazin-3-yl, 2,3-dihydro-2-methyl-3-oxo-pyridazin-6-yl, 4,5-dimethyl-4H-[1,2,4]triazol-3-yl, oxadiazolyl, or methyloxadiazolyl.

If R² and R³ are linked to each other to form a bridging group they are preferably selected from the group consisting of methylenedioxy, difluoromethylenedioxy, ethylenedioxy and C₃₋₅-alkylene bridging group, even more preferably consisting of methylenedioxy, ethylenedioxy, propylene, and butylene, most preferably methylenedioxy or ethylenedioxy.

Additionally R² and R³ may form combined with the carbon atoms to which they are attached a group preferably selected from the group consisting of a benzo, pyrazino, pyrazolo, imidazo, N-(C₁₋₃-alkyl)-pyrazolo, N-(C₁₋₃-alkyl)-imidazo, oxazolo, thiazolo, isoxazolo, and isothiazolo ring, wherein each of the five-membered rings is optionally monosubstituted with R^{P} and each of the six-membered rings is optionally substituted with one or two substituents independently of each other selected from R^{p}, or N-(C₁₋₃-alkyl)-triazolo or triazolo.

If R² and R³ combined with the carbon atoms to which they are attached form a group as described hereinbefore said group is even more preferably selected from the group consisting of a benzo, pyrazino, pyrazolo, imidazo, N-(C₁₋₃-alkyl)-pyrazolo, N-(C₁₋₃-alkyl)-imidazo, oxazolo, thiazolo, isoxazolo, and isothiazolo ring, wherein each of the five-membered rings is optionally monosubstituted with R^{p} and each of the six-membered rings is optionally substituted with one or two substituents independently of each other selected from R^{p}, or N-(C₁₋₃-alkyl)-triazolo or triazolo.

If R² and R³ combined with the carbon atoms to which they are attached form a group as described hereinbefore said group is most preferably selected from the group consisting of a benzo, pyrazino, imidazo, N-(C₁₋₃-alkyl)imidazo, N-(C₁₋₃-alkyl)-triazolo, oxazolo, or thiazolo ring, wherein the benzo and pyrazino ring are optionally substituted with one or two substituents independently of each other selected from R^{p} and the imidazo, N-(C₁₋₃-alkyl)-imidazo, oxazolo, and thiazolo ring are optionally additionally monosubstituted with R^{p}.

Most preferably, R² and R³ together denote methylenedioxy or together with the carbon atoms to which they are attached form an optionally additionally with methyl, tert-butyl, cyclopropyl, tetrahydrofuran-2-yl, 1-acetyl-piperidin-4-yl, pyridin-3-yl, 1,2-dihydro-1-methyl-2-oxo-pyridin-5-yl, pyridazin-4-yl, pyrazinyl, or 5-methyl-pyrazin-2-yl substituted oxazolo, imidazo, or N-methyl-imidazo group, an optionally with methyl substituted triazolo group, or an optionally methyl or dimethyl substituted benzo or pyrazino ring.

R^{p} preferably is fluorine, C₁₋₄-alkyl. C₃₋₆-cycloalkyl, pyrrolidinyl, 1-methyl-pyrrolidinyl, 1-acetyl-pyrrolidinyl, piperidinyl, 1-methyl-piperidinyl, 1-acetyl-piperidinyl, tetrahydrofuranyl, tetrahydropyranyl, trifluoromethyl, cyano, amino, acetylamino, methylsulfonylamino, carboxy, C₁₋₃-alkyloxycarbonyl, aminocarbonyl, methylaminocarbonyl, dimethylaminocarbonyl, aminosulfonyl, hydroxy, C₁₋₃-alkyloxy, or phenyl, pyridyl, pyridazinyl, pyrazinyl, pyrimidinyl, 1,2-dihydro-2-oxo-pyridinyl, which are optionally substituted with one or two groups independently selected from fluorine, chlorine, C₁₋₃-alkyl, difluoromethyl, trifluoromethyl, cyano, amino, acetylamino, methylsulfonylamino, carboxy, C₁₋₃-alkyloxycarbonyl, aminocarbonyl, methylaminocarbonyl, dimethylamino-carbonyl, hydroxy, methoxy, difluoromethoxy, and trifluoromethoxy.

More preferably, R^{p} is fluorine, methyl, ethyl, tert-butyl, C₃₋₆-cycloalkyl, pyrrolidinyl, 1-methyl-pyrrolidinyl, 1-acetyl-pyrrolidinyl, piperidinyl, 1-methyl-piperidinyl, 1-acetyl-piperidinyl, tetrahydrofuranyl, tetrahydropyranyl, trifluoromethyl, cyano, amino, acetylamino, methylsulfonylamino, carboxy, methoxycarbonyl, ethoxycarbonyl, aminocarbonyl, methylaminocarbonyl, dimethylaminocarbonyl, aminosulfonyl, hydroxy, methoxy, or
phenyl, pyridyl, pyridazinyl, pyrazinyl, pyrimidinyl, 1,2-dihydro-2-oxo-pyridinyl, which are optionally substituted with one or two groups independently selected from
fluorine, methyl, trifluoromethyl, cyano, amino, acetylamino, methylsulfonylamino, carboxy, aminocarbonyl, methylaminocarbonyl, dimethylaminocarbonyl, hydroxy, and methoxy.

Most preferably, R^{P} is fluorine, methyl, cyclopropyl, 1-acetyl-piperidinyl, tetrahydrofuranyl, acetylamino, methylsulfonylamino, carboxy, hydroxy, methoxy, or pyridyl, pyridazinyl, pyrazinyl, 1,2-dihydro-2-oxo-pyridinyl, which are optionally substituted with one or two methyl groups; particularly, L is methyl, tert-butyl, cyclopropyl, tetrahydrofuran-2-yl, 1-acetyl-piperidin-4-yl, pyrid-3-yl, pyridazin-3-yl, pyrazinyl, 5-methylpyrazin-2-yl, 1,2-dihydro-2-oxo-pyridin-5-yl.

Preferably the one or more substituents R¹⁰ independently of each other denote fluorine, chlorine, bromine, C₁₋₃-alkyl, difluoromethyl, trifluoromethyl, cyano, nitro, amino, acetylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, aminosulfonyl, methylsulfanyl, methylsulfinyl, methylsulfonyl, phenyl, hydroxy, C₁₋₃-alkyloxy, difluoromethoxy, or trifluoromethoxy.

More preferably, R¹⁰ denotes fluorine, chlorine, methyl, difluoromethyl, trifluoromethyl, cyano, hydroxy, methoxy, difluoromethoxy, or trifluoromethoxy. Most preferably, R¹⁰ denotes fluorine or methyl.

Preferably the one or more substituents R¹¹ independently of each other denote fluorine, C₁₋₃-alkyl, phenyl, hydroxy, C₁₋₃-alkyloxy, cyano, carboxy, C₁₋₄-alkyloxycarbonyl, aminocarbonyl, C₁₋₄-alkylamino-carbonyl, di-(C₁₋₄-alkyl)-aminocarbonyl, hydroxy-C₁₋₄-alkyl, or C₁₋₃-alkyloxy-C₁₋₄-alkyl. More preferably R¹¹ denotes fluorine, C₁₋₃-alkyl, hydroxy, or C₁₋₃-alkyloxy. Most preferably, R¹¹ denotes methyl, ethyl, propyl, hydroxy, or methoxy, particularly methyl or hydroxy.

Preferably the one or more substituents R¹² independently of each other denote fluorine, or C₁₋₃-alkyl, more preferably methyl or ethyl.

Particularly preferred embodiments of this invention are described by each of the formulae I.1 a to I.10a 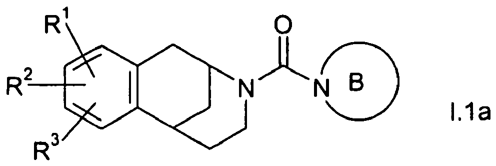 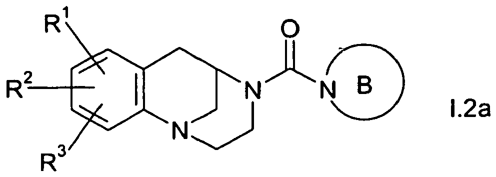 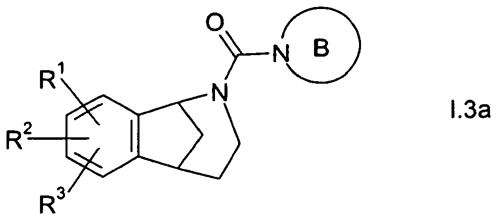 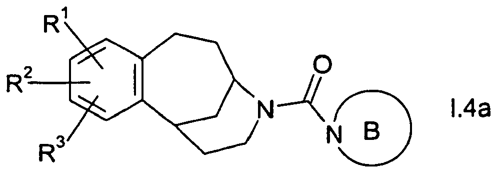 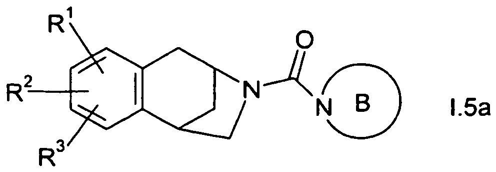 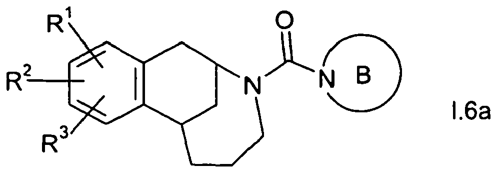 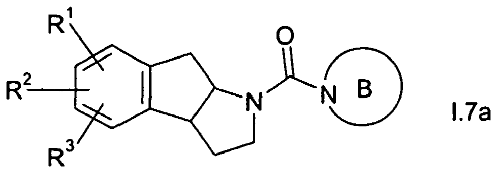 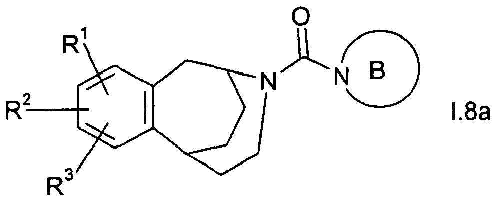 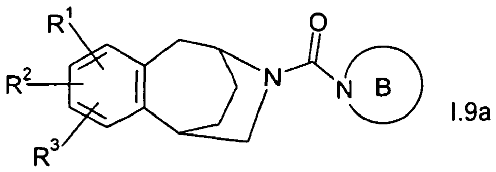 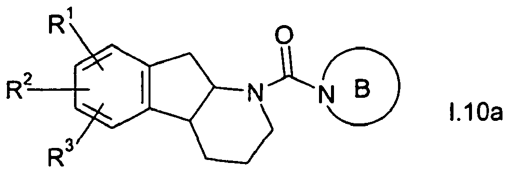 wherein the C₅₊ₘ₊ₙ-azacycloalkene core structure of general formulae I.1a to I.10a including the bridging group -(CH₂)ₒ- is optionally substituted with 1, 2 or more substituents independently of each other selected from the group consisting of R¹¹ and R¹², and
wherein the ring B and R¹, R², R³, R¹¹, R¹² are defined as hereinbefore and hereinafter, their tautomers, their stereoisomers, mixtures thereof and the salts thereof, while the compounds (M1), (M2), (M3) and (M4) as defined hereinbefore, are excluded.

According to a preferred embodiment of the general formula I.1a compounds of the invention are described by the formula I.1a-RR 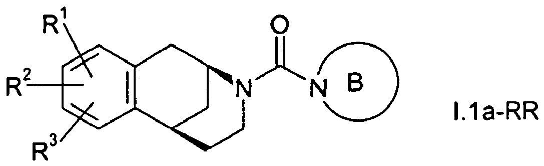 wherein the 2,6-methano-azocin core structure with the stereochemical configuration as depicted is optionally substituted with 1, 2 or more substituents independently of each other selected from the group consisting of R¹¹ and R¹² , and wherein the ring B and R¹, R², R³, R¹¹, R¹² are defined as hereinbefore and hereinafter, their tautomers, their stereoisomers, mixtures thereof and the salts thereof.

According to another preferred embodiment of the general formula I.1a compounds of the invention are described by the formula I.1a-SS 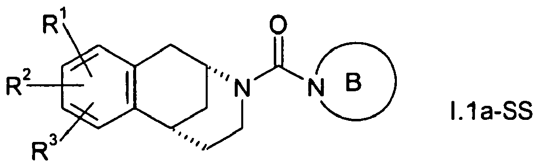 wherein the 2,6-methano-azocin core structure with the stereochemical configuration as depicted is optionally substituted with 1, 2 or more substituents independently of each other selected from the group consisting of R¹¹ and R¹² , and wherein the ring B and R¹, R², R³, R¹¹, R¹² are defined as hereinbefore and hereinafter, their tautomers, their stereoisomers, mixtures thereof and the salts thereof.

Even more preferred compounds are described by the formulae I.1 a to I.10a and I.1a-RR and I.1a-SS wherein the C₅₊ₘ₊ₙ-azacycloalkene core structure including the bridging group -(CH₂)ₒ- is optionally mono-substituted with R¹¹ and optionally substituted with 1, 2 or 3 substituents independently of each other selected from R¹² wherein the ring B and R¹, R², R³, R¹¹, R¹² are defined as hereinbefore and hereinafter, their tautomers, their stereoisomers, mixtures thereof and the salts thereof.

According to another embodiment the invention relates to compounds of the formula I, in particular of the formulae **I.**1 to I.10, most preferably of the formulae I.1a to I.10a, in particular of the formula I.5 and I.5a, defined as hereinbefore wherein compounds of the formula I.5a-1 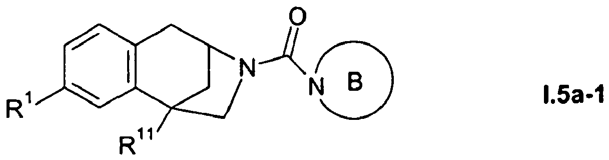 wherein R¹ is hydrogen, hydroxy or C₁₋₄-alkyloxy, and R¹¹ is C₁₋₄-alkyl or phenyl, and the ring B is morpholin-4-yl, piperidin-1-yl or pyrrolidin-1-yl, and all remaining substituents are hydrogen, are excluded.

According to another embodiment the invention relates to compounds of the formula I, in particular of the formulae I.1 to I.10, most preferably of the formulae I.1 a to I.10a, in particular of the formula I.1 and I.1a, defined as hereinbefore wherein compounds of the formula I.1a-1 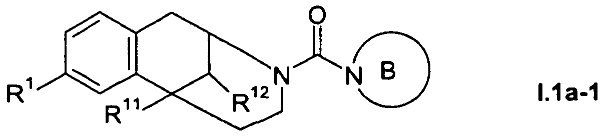 wherein R¹ is hydrogen, hydroxy or C₁₋₄-alkyloxy, and R¹¹ is hydrogen, C₁₋₄-alkyl, 2-methoxy-ethyl, or phenyl, wherein said phenyl is optionally substituted with halogen, hydroxy or C₁₋₃-alkyloxy, and R¹² is hydrogen, or C₁₋₄-alkyl, and the ring B is a 3- to 8-membered monocylic azacycloalk-1-yl group or morpholin-4-yl, and all remaining substituents are hydrogen, and their salts, are excluded.

Some terms used above and hereinafter to describe the compounds according to the invention will now be defined more closely.

The term "substituted" as used herein, means that any one or more hydrogens on the designated atom is replaced with a selection from the indicated group, provided that the designated atom's normal valence is not exceeded, and that the substitution results in a stable compound.

The term "partially unsaturated" as used herein, means that in the designated group or moiety 1, 2 or more, preferably 1 or 2, double bonds are present. Preferably as used herein, the term "partially unsaturated" does not cover fully unsaturated groups or moieties.

The term "fully unsaturated" as used herein, means that in the designated group or moiety conjugated double bonds are present such that an aromatic or heteroaromatic system is formed.

The term halogen denotes an atom selected from the group consisting of F, Cl, Br and I.

The term C₁₋ₙ-alkyl, wherein n may have a value of 1 to 18, denotes a saturated, branched or unbranched hydrocarbon group with 1 to n C atoms. Examples of such groups include methyl, ethyl, n-propyl, iso-propyl, butyl, iso-butyl, sec-butyl, tert-butyl, n-pentyl, iso-pentyl, neo-pentyl, tert-pentyl, n-hexyl, iso-hexyl, etc.

The term C₂₋ₙ-alkenyl, wherein n has a value of 3 to 6, denotes a branched or unbranched hydrocarbon group with 2 to n C atoms and a C=C double bond. Examples of such groups include ethenyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl etc.

The term C₂₋ₙ-alkynyl, wherein n has a value of 3 to 6, denotes a branched or unbranched hydrocarbon group with 2 to n C atoms and a C≡C triple bond. Examples of such groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl etc. Unless otherwise stated alkynyl groups are connected to the remainder of the molecule via the C atom in position 1. Therefore terms such as 1-propynyl, 2-propynyl, 1-butynyl, etc. are equivalent to the terms 1-propyn-1-yl, 2-propyn-1-yl, 1-butyn-1-yl, etc.. This also applies analogously to C₂₋ₙ-alkenyl groups.

The term C₁₋ₙ-alkoxy denotes a C₁₋ₙ-alkyl-O group, wherein C₁₋ₙ-alkyl is as hereinbefore defined. Examples of such groups include methoxy, ethoxy, n-propoxy, iso-propoxy, n-butoxy, iso-butoxy, sec-butoxy, tert-butoxy, n-pentoxy, iso-pentoxy, neo-pentoxy, tert-pentoxy, n-hexoxy, iso-hexoxy, etc.

The term C₁₋ₙ-alkylcarbonyl denotes a C₁₋ₙ-alkyl-C(=O) group, wherein C₁₋ₙ-alkyl is as hereinbefore defined. Examples of such groups include methylcarbonyl, ethylcarbonyl, n-propylcarbonyl, iso-propylcarbonyl, n-butylcarbonyl, iso-butylcarbonyl, sec-butylcarbonyl, tert-butylcarbonyl, n-pentylcarbonyl, iso-pentylcarbonyl, neo-pentylcarbonyl, tert-pentylcarbonyl, n-hexylcarbonyl, iso-hexylcarbonyl, etc.

The term C₃₋ₙ-cycloalkyl denotes a saturated mono-, bi-, tri- or spirocarbocyclic group with 3 to n C atoms. Examples of such groups include cyclopropyl, cyclobutyl, cyclopentyl, cyclohexyl, cycloheptyl, cyclooctyl, cyclononyl, cyclododecyl, bicyclo[3.2.1.]octyl, spiro[4.5]decyl, norpinyl, norbonyl, norcaryl, adamantyl, etc. Preferably the term C₃₋₇-cycloalkyl denotes saturated monocyclic groups.

The term C₅₋ₙ-cycloalkenyl denotes a C₅₋ₙ-cycloalkyl group which is as hereinbefore defined and additionally has at least one C=C double bond.

The term C₃₋ₙ-cycloalkylcarbonyl denotes a C₃₋ₙ-cycloalkyl-C(=O) group wherein C₃₋ₙ-cycloalkyl is as hereinbefore defined.

The term tri-(C₁₋₄-alkyl)silyl comprises silyl groups which have identical or two or three different alkyl groups.

The term di-(C₁₋₃-alkyl)amino comprises amino groups which have identical or two different alkyl groups.

If groups or residues are optionally substituted, this applies to any form of the group or residue. For instance, if an alkyl group is optionally mono- or polyfluorinated this comprises also alkyl residues which are part of larger groups, e.g. alkyloxy, alkylcarbonyl, alkoxyalkyl, etc., or if a (het)aryl group is optionally mono- or polysubstituted with a certain substituent or a set of substituents this also includes (het)aryl groups which are part of larger groups, e.g. (het)aryl-C₁₋ₙ-alkyl, (het)aryloxy, (het)aryloxy-C₁₋ₙ-alkyl, (het)aryl-C₁₋ₙ-alkyloxy, etc.. Accordingly, in cases where R¹ or L² have e.g. the meaning (het)aryloxy, while (het)aryl residues are optionally mono- or polyfluorinated and (het)aryl denotes inter alia phenyl, the meanings mono-, di-, tri-, tetra-, and pentafluorophenoxy are also comprised. The same applies to groups or residues in which a part of the group or residue is replaced as e.g. a CH₂ group is optionally replaced with O S, NR, CO, or SO₂. For instance, a residue having inter alia the meaning hydroxy-C₁₋₃-alkyl, in which a CH₂ group is optionally replaced by CO, this also comprises carboxy, carboxymethyl, hydroxymethylcarbonyl, carboxyethyl, hydroxyl-methylcarbonylmethyl, and hydroxyethylcarbonyl.

The compounds according to the invention may be obtained using methods of synthesis known in principle. Preferably, the compounds are obtained by the following methods according to the invention which are described in more detail hereinafter.

A general strategy to access compounds of the invention is delineated in Scheme 1; A, X, m, n, and o have the meanings as defined hereinbefore and hereinafter. The key reaction to assemble the bicyclic framework is an intramolecular reaction of an amino functionality with a carboxy group that results in the formation of an amide linkage. The fusion of the carboxylic acid function and the amino group may be carried out with or without an additive at elevated temperatures, preferably between 20 and 200 °C. Additives that remove the water forming during the reaction, such as molecular sieves or orthoesters, or other additives such as bases, e.g. hexamethyldisilazides, or boronic acids may facilitate the reaction. Though, more preferably the reaction is done with a more reactive entity of the carboxy function such as an acyl halide, ester, thioester, anhydride, mixed anhydride, or ketene which may be generated in a separate preceding reaction step or in situ. Preferred acyl halides or pseudohalides are acyl chloride, acyl fluoride, and acylcyanide. Preferred esters and thioesters are derived from e.g. methanol/methylthiol, ethanol/ethylthiol, 2,2,2-trifluoroethanol, phenol/thiophenol, substituted phenol/thiophenol such as 4-nitrophenol or pentafluorophenol, hydroxy heteroaryl such as hydroxybenzotriazol, hydroxypyridotriazol, or hydroxytriazines, or N-hydroxysuccinimid. Preferred mixed anhydrides are derived from alkylcarboxylic acids, e.g. pivalic acid, carbonates, e.g. methyl and ethyl carbonate, carbamates, e.g. N,N-dimethyl carbamate, phosphoric acids, e.g. dimethylphosphoric acid or (Me₂N)₂P(O)OH, or ureas, e.g. dicyclohexylurea, dimethylurea, or tetramethylurea. Additionally, N acylated derivatives derived from azaheteroaromatics such as imidazole, triazole, tetrazole, or pyridine such as e.g. 4-dimethylaminopyridine may be used as well. Some of the more popular reagents used for the activation of the carboxylic acid function are N,N'-carbonyldiimidazol, dicyclohexylcarbodiimide, (benzotriazol-1-yloxy)dipiperidinocarbenium hexafluorophosphate or tetrafluoroborate, (benzotriazol-1-yloxy)dipyrrolidinocarbenium hexafluorophosphate or tetrafluoroborate, 1-(3-dimethylaminopropyl)-3-ethylcarbodiimide methiodide, POCl₃, SOCl₂, (COCl)₂, COCl₂, arylboronic acid, TiCl₄, (MeO)₂POCl, cyanuric chloride, 1-hydroxybenzotriazol, 1-hydroxy-7-azabenzotriazol, benzoltriazol-1-yloxytris(dimethylamino)phosphonium hexafluorophosphate or tetrafluoroborate, benzoltriazol-1-yloxytripyrrolidinophosphonium hexafluorophosphate or tetrafluoroborate, (7-aza-benzoltriazol-1-yloxy)tripyrrolidinophosphonium hexafluorophosphate or tetrafluoroborate, O-(benzotriazol-1-yl)-N,N,N',N'-tetramethyluronium hexafluorophosphate or tetrafluoroborate, O-(7-aza-1H-benzotriazol-1-yl)-1,1,3,3-tetramethyluronium hexafluorophosphate or tetrafluoroborate. This compilation of reagents represents only a few possibilities to activate an carboxylic acid function. A host of additional reagents is known and may be used here as well. The reactive carboxylic acid derivatives may also serve as intermediates for other acylating reagents also sufficiently reactive for this transformation. The activation step and the ensuing amide forming step are often best carried out in the presence of additional additives such as bases, e.g. ethyldiisopropylamine, triethylamine, alkali metal carbonate, pyridine, 4-dimethylaminopyridine, imidazole, dimethylaluminum amides, lithium amides, alkali metal cyanide, or alkali metal hexamethyldisilazide. The reactions are preferably conducted in organic solvents but may also be carried out in aqueous solvents. Among the organic solvents ordinarily used are dimethylformamide, N,N-dimethylacetamide, N-methylpyrrolidinone, dimethylsulfoxide, tetrahydrofuran, hexane, ether, 1,4-dioxane, 1,2-dimethoxyethane, dichloromethane, dichloroethane, toluene, benzene, ethyl acetate, quinoline, pyridine, or mixtures thereof. The reactions may be carried out at -80 °C to 220 °C, preferably between -10 °C and 120 °C. Subsequently, the lactam group is reduced to give the secondary amine. This transformation is a well established reaction that may be carried out, for example, using LiAlH₄, hydrogen in the presence of a catalyst, NaBH₄ in the presence of e.g. iodine, LiBH₄, borane, sodium in propanol, Cl₃SiH, silanes, e.g. Et₃SiH, in the presence of a transition metal such as rhenium, 9-BBN, LiBH₃NMe₂, or Et₃SiH combined with LiEt₃BH. Solvents such as e.g. tetrahydrofuran, ether, 1,2-dimethoxyethane, 1,4-dioxane, hexane, benzene, toluene, dichloromethane, alcohols, water, or mixtures thereof may be employed at - 78°C to 200 °C, preferably between -10 °C and 120 °C; though, in combination with some reducing reagents only a few of these solvents are usable. This strategy is well suited for the synthesis of the scaffolds I.1 to I.10.

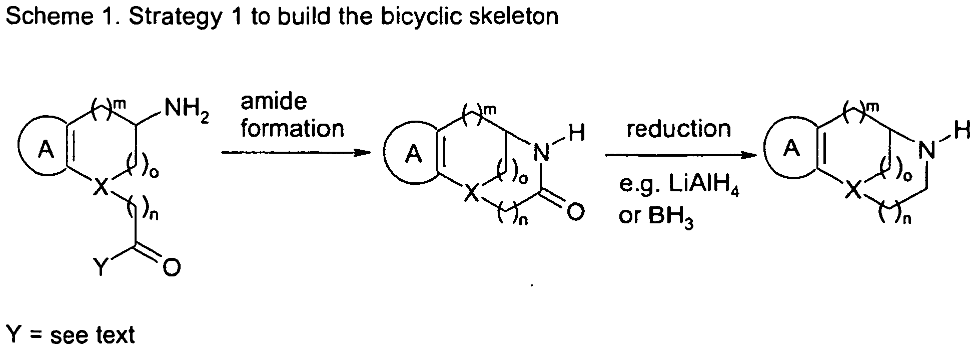

Another common synthetic route to acquire the compounds of the invention is summarized in Scheme 2; A, X, m, n, and o have the meanings as defined hereinbefore and hereinafter. The bicyclic framework is formed via an intramolecular reductive amination reaction of a primary amine with a ketone functionality. Reductive aminations have large precedence in organic chemistry and may be carried out e.g. using hydrogen in the presence of a transition metal catalyst such as one derived from Ni, Rh, Pd, or Pt, borohydride reagents, e.g. sodium borohydride, sodium cyanoborohydride, or sodium triacetoxyborohydride, zinc in combination with hydrochloric acid, PhSiH₃ with Bu₂SnCl₂, BioHi₄, or formic acid or salts thereof. Some of these reagents are preferably used in combination with an additive such as acid, e.g. acetic acid or mineral acid. The reactions are preferably conducted in organic solvents or aqueous mixtures, e.g. dimethylformamide, N,N-dimethylacetamide, N-methylpyrrolidinone, dimethylsulfoxide, tetrahydrofuran, hexane, ether, 1,4-dioxane, 1,2-dimethoxyethane, dichloromethane, dichloroethane, toluene, benzene, alcohols, water, or mixtures thereof. The reactions may be carried out at -80 °C to 200 °C, preferably between -10 °C and 100 °C.

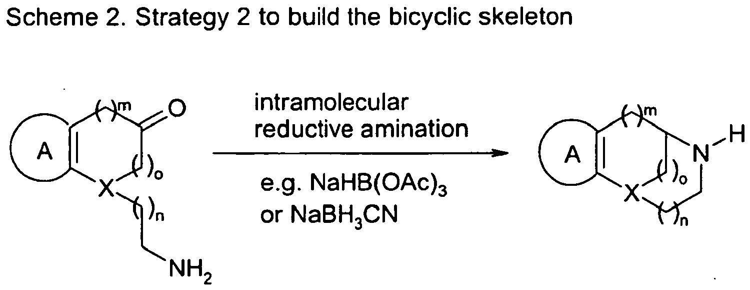

The strategy shown in Scheme 3, wherein A, X, m, n, and o have the meanings as defined hereinbefore and hereinafter, is another valid approach based on the reductive amination reaction already delineated in Scheme 2. Reaction conditions described there may be employed analogously here.

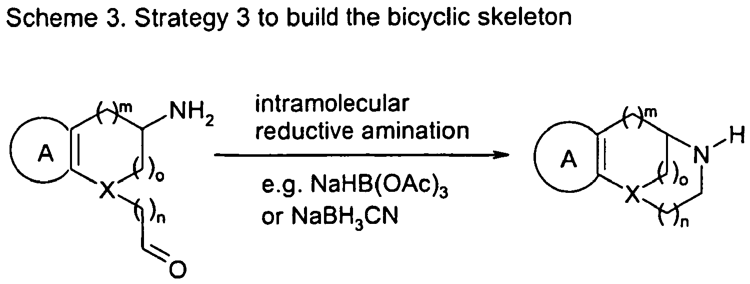

Scheme 4, wherein A, X, m, n, and o have the meanings as defined hereinbefore and hereinafter, shows another approach to assemble the bicyclic framework. This approach is an intramolecular alkylation of the nitrogen group with an appropriate electrophile of the side-chain. The nitrogen group may be an amino group, i.e. R^{a} denotes e.g. hydrogen, methyl, allyl, benzyl, or dimethoxybenzyl, or an amide group, i.e. R^{a} denotes e.g. methoxycarbonyl, benzyloxycarbonyl, allyloxycarbonyl, tertbutoxycarbonyl, trifluormethylcarbonyl, acetyl, 2,2,2-trichloroethoxycarbonyl, tolylsulfonyl, phenylsulfonyl, methoxyphenylsulfonyl, nitrophenylsulfonyl, 2,2,2-trichloroethylsulfonyl, or 2-trimethylsilylethylsulfonyl. The nitrogen function is reacted with an electrophilic Cₛₚ₃-center in the side-chain, i.e. LG in Scheme 4 denotes e.g. chlorine, bromine, iodine, mesyloxy, tosyloxy, or trifluoromethlysulfonyloxy, in the presence of a base such as e.g. triethylamine, ethyldiisopropylamine, diazabicycloundecene, alkali metal carbonate, alkali metal terfbutoxide, alkali metal diisopropylamide, butyllithium, or sodium hydride. The stronger bases among them are preferably used in combination with the amides in e.g. N-methylpyrrolidinone, dimethylsulfoxide, tetrahydrofuran, hexane, ether, 1,4-dioxane, 1,2-dimethoxyethane, toluene, benzene, tertbutanol, isopropanol, or mixtures thereof at temperatures between -70 and 100 °C, preferably between -30 and 60 °C. The milder bases listed are preferably used in combination with the amines in dichloromethane, dimethylformamide, N-methylpyrrolidinone, dimethylsulfoxide, tetrahydrofuran, hexane, ether, 1,4-dioxane, 1,2-dimethoxyethane, toluene, benzene, methanol, ethanol, tertbutanol, isopropanol, water, or mixtures thereof at temperatures between 0 and 140 °C, preferably between 20 and 120 °C. For the amides the conditions originally reported by Mitsunobu may be used as well. Accordingly, the side-chain leaving group LG is generated in situ from the hydroxy group (LG = OH) using a phosphine, e.g. triphenylphosphine or tributylphosphine, in combination with an azodicarboxylate, e.g. diethyl azodicarboxylate, diisopropyl azodicarboxylate, or azodicarboxylic dipiperidide. Suited solvents may be selected from among dimethylformamide, N-methylpyrrolidinone, dichloromethane, tetrahydrofuran, hexane, ether, 1,4-dioxane, 1,2-dimethoxyethane, toluene, benzene, and mixtures thereof. The reaction is preferably conducted at temperatures between 0 and 100 °C. The opposite way around, i.e. LG denotes NHR^{a} and NHR^{a} denotes LG, may be applicable as well. Reaction conditions are equivalent to the original way around.

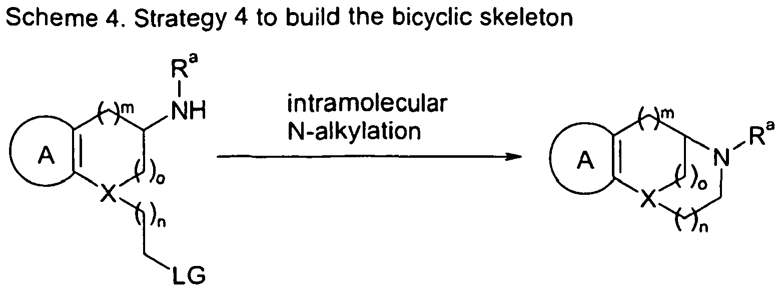

A further generally applicable approach is based on an electrophilic aromatic substitution reaction (Scheme 5); A, X, m, n, and o have the meanings as defined hereinbefore and hereinafter. Thereby the aromatic part of the molecule reacts with an activated carbon atom of the azacycle to form the bicyclic framework. The reactive intermediate bears a (partially) positively charged carbon atom in the azacycle that may be generated by the addition of an acid to an olefinic bond or by the activation of an appropriately positioned leaving group. A huge number of Bronstedt and Lewis acids have been described for this classical reaction that may also be used here. The following enumeration is supposed to give a few more widely used of them: hydrobromic acid, hydroiodic acid, hydrochloric acid, sulfuric acid, phosphoric acid, P₄O₁₀, trifluoroacetic acid, methanesulfonic acid, toluenesulfonic acid, trifluormethanesulfonic acid, Sc(OSO₂CF₃)₃, SnCl₄, FeCl₃, AlBr₃, AlCl₃, SbCl₅, BCl₃, BF₃, ZnCl₂, montmorillonites, POCl₃, and PCl₅. Depending on the inclination of the leaving group to be substituted and the electronic nature of the aromatic a more or less powerful acid catalyst has to be used. Besides the acid catalysts mentioned silver salts, e.g. AgOSO₂CF₃, may be useful in the reactions using halides as leaving group. Preferred solvents are hydrocarbons such as hexanes or cyclohexane, chlorinated hydrocarbons such as dichloromethane or dichloroethane, perfluorinated hydrocarbons, nitrobenzene, chlorinated benzenes, heteroaromatics such as quinoline, 1,2-dimethoxyethane, 1,4-dioxane, ether, ionic liquids, or mixtures thereof. The reactions may be carried out between -10 °C and 220 °C, preferably between 20 °C and 180 °C. The reactions may also be conducted under microwave irradiation. This synthetic strategy is particularly suited for the scaffolds I.1 and 1.3 to I.10 bearing an electron rich aromatic.

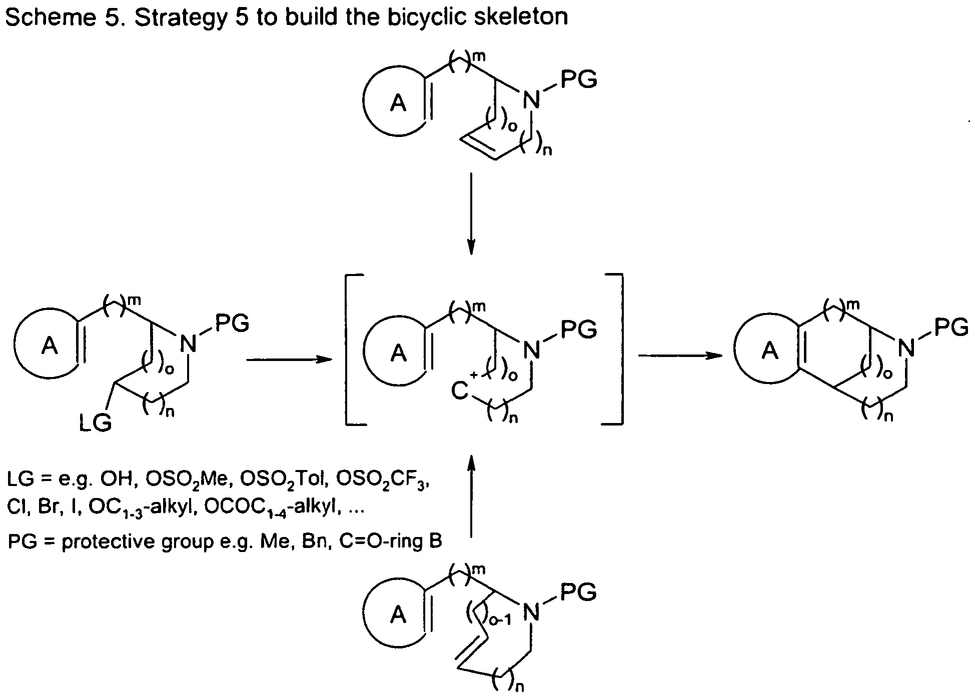

Besides the strategies presented a host of additional approaches to construct the bicyclic systems of the present invention can be envisaged and are also reported in the literature (see e.g. J. Med. Chem. 1970, 13, 630-634; Chem. Rev. 1977, 77, 1-36; J. Med. Chem. 1979, 22, 537-553; J. Org. Chem. 1984, 49, 4033-4044; J. Med. Chem. 1996, 39, 1956-1966; Heterocycles 1996, 43, 15-22; J. Med. Chem. 2002, 45, 3755-3764; J. Org. Chem. 2006, 71, 2046-2055; and references quoted therein). Therefore, the preceding strategies are in no way meant to restrict the possible synthetic pathways to access the compounds of the invention but are only supposed to show a few routes by way of example.

The synthetic routes presented may rely on the use of protecting groups. Suitable protecting groups for the respective functionalities and their removal have been described hereinbefore and may be employed analogously (see also: Protecting Groups, Philip J. Kocienski, 3rd edition, Georg Thieme Verlag, Stuttgart, 2004 and references quoted therein).

If in the process of manufacture according to the invention a compound of general formula I is obtained which contains an amino, alkylamino or imino group, this may be converted by acylation or sulfonylation into a corresponding acyl or sulfonyl compound of general formula I.

If a compound of general formula I is obtained which contains a hydroxy group, this may be converted by acylation or sulfonylation into a corresponding acyl or sulfonyl compound of general formula **I**.

If a compound of general formula I is obtained which contains a hydroxy group, this may be converted by alkylation into a corresponding ether of general formula I.

If a compound of general formula I is obtained which contains an amino, alkylamino or imino group, this may be converted by alkylation or reductive alkylation into a corresponding alkyl compound of general formula I.

If a compound of general formula I is obtained which contains a nitro group, this may be converted by reduction into a corresponding amino compound.

If a compound of general formula I is obtained which contains an imino group, this may be converted by nitrosation and subsequent reduction into a corresponding N-amino-imino compound.

If a compound of general formula I is obtained which contains a C₁₋₃-alkyloxycarbonyl group, this may be converted by cleavage of the ester into the corresponding carboxy compound.

If a compound of general formula I is obtained which contains a carboxy group, this may be converted by esterification into a corresponding ester of general formula I.

If a compound of general formula I is obtained which contains a carboxy or ester group, this may be converted by reaction with an amine into a corresponding amide of general formula I.

If a compound of general formula I is obtained which contains an aromatic substructure, this may be derivatized with a chlorine, bromine, or iodine atom or a nitro, sulfonic acid, chlorosulfonyl, or acyl group to a corresponding compound of general formula I by an electrophilic substitution reaction.

If a compound of general formula I is obtained which contains an aromatic amino group, this may be transformed into a corresponding cyano, fluoro, chloro, bromo, iodo, hydroxy, mercapto, or azido compound of general formula I by diazotization and subsequent replacement of the diazo group with cyanide, fluoride, chloride, bromide, iodide, hydroxide, alkyl or hydrogen sulfide, or azide, respectively.

If a compound of general formula I is obtained which contains an aromatic amino group, this may be converted into a corresponding aryl derivatized aromatic compound of general formula I by diazotization and subsequent replacement of the diazo group with an appropriate aryl nucleophile mediated by a suited transition metal species.

If a compound of general formula I is obtained which contains an aromatic chloro, bromo, iodo, trifluoromethylsulfonyloxy, mesyloxy, or tosyloxy group, this may be converted into a corresponding aryl, alkenyl, alkynyl, or alkyl derivatized compound of general formula I by replacement of the respective group by aryl, alkenyl, alkynyl, or alkyl using a transition metal species mediated process.

If a compound of general formula I is obtained which contains an aromatic chloro, bromo, iodo, trifluoromethylsulfonyloxy, mesyloxy, or tosyloxy group, this may be replaced for hydrogen to give a corresponding aromatic compound of general formula I.

If a compound of general formula I is obtained which contains two adjacent heteroatoms that are amino and hydroxy, amino, or mercapto, these heteroatoms may be linked via a carboxy carbon atom to form a cyclic amidine, imino ester, or imino thioester substructure that may be part of an aromatic ring.

If a compound of general formula I is obtained which contains a cyano group, this may be converted into an amino alkyl derivatized compound of general formula I by reduction.

If a compound of general formula I is obtained which contains a cyano group, this may be converted into a N-hydroxycarbamimidoyl group by the treatment with hydroxylamine.

If a compound of general formula I is obtained which contains an N-hydroxycarbamimidoyl group, this may be converted to an oxadiazole derivatized compound of general formula I by the treatment with a carboxylic or related group.

If a compound of general formula I is obtained which contains an aminocarbonyl group, this may be converted by dehydration into a corresponding cyano compound of general formula I.

If a compound of general formula I is obtained which contains a keto or aldehydic group, this may be converted by reduction into a corresponding hydroxyl compound of general formula I.

The subsequent esterification is optionally carried out in a solvent or mixture of solvents such as methylene chloride, dimethylformamide, benzene, toluene, chlorobenzene, tetrahydrofuran, benzene/tetrahydrofuran or 1,4-dioxane or particularly advantageously in the corresponding alcohol optionally in the presence of an acid such as hydrochloric acid or in the presence of a dehydrating agent. Isobutyl chloroformate, thionyl chloride, trimethylchlorosilane, sulfuric acid, methanesulfonic acid, p-toluenesulfonic acid, phosphorus trichloride, phosphorus pentoxide, N,N'-dicyclohexylcarbodiimide, N,N'-dicyclohexylcarbodiimide/N-hydroxysuccinimide, N,N'-carbonyldiimidazole, triphenylphosphine/carbon tetrachloride, or combinations thereof optionally in the presence of 4-dimethylamino-pyridine and/or 1-hydroxy-benzotriazole are among the routinely used reagents to accomplish this transformation. The reactions are conducted between 0 and 150 °C, preferably between 0 and 80 °C.

The subsequent ester formation may also be carried out by reacting a compound which contains a carboxy group with a corresponding alkyl halide.

The subsequent acylation or sulfonylation is optionally carried out in a solvent or mixture of solvents such as methylene chloride, dimethylformamide, benzene, toluene, chlorobenzene, tetrahydrofuran, benzene/tetrahydrofuran, or 1,4-dioxane with a corresponding acyl or sulfonyl derivative optionally in the presence of a tertiary organic base or in the presence of an inorganic base or in the presence of a dehydrating agent. Routinely used agents are e.g. isobutyl chloroformate, thionyl chloride, trimethylchlorosilane, sulfuric acid, methanesulfonic acid, p-toluenesulfonic acid, phosphorus trichloride, phosphorus pentoxide, N,N'-dicyclohexylcarbodiimide, N,N'-dicyclohexylcarbodiimide/N-hydroxysuccinimide, N,N'-carbonyldiimidazole, triphenylphosphine/carbon tetrachloride, or combinations thereof that may be employed in the presence of 4-dimethylamino-pyridine and/or 1-hydroxy-benzotriazole at temperatures between 0 and 150 °C, preferably between 0 and 80 °C.

The subsequent alkylation is optionally carried out in a solvent or mixture of solvents such as methylene chloride, dimethylformamide, benzene, toluene, chlorobenzene, tetrahydrofuran, benzene/tetrahydrofuran, or 1,4-dioxane with an alkylating agent such as a corresponding halide or sulfonic acid ester, e.g. methyl iodide, ethyl bromide, dimethylsulfate, or benzyl chloride, optionally in the presence of a tertiary organic base or in the presence of an inorganic base at temperatures between 0 and 150 °C, preferably between 0 and 100 °C.

The subsequent reductive alkylation is carried out with a corresponding carbonyl compound such as e.g. formaldehyde, acetaldehyde, propionaldehyde, acetone, or butyraldehyde in the presence of a complex metal hydride such as sodium borohydride, lithium borohydride, sodium triacetoxyborohydride, or sodium cyanoborohydride conveniently at a pH of 6-7 and at ambient temperature or using hydrogen in the presence of a transition metal catalyst, e.g. palladium/charcoal at a hydrogen pressure of 1 to 5 bar. Methylation may also be carried out in the presence of formic acid as reducing agent at elevated temperature, e.g. between 60 and 120 °C.

The subsequent reduction of a nitro group is carried out, for example, with hydrogen and a catalyst such as palladium on carbon, platinum dioxide, or Raney nickel, or using other reducing agents such as iron or zinc in the presence of an acid such as acetic acid.

The subsequent nitrosation of an imino group followed by reduction to obtain the N-amino-imino compound is carried out, for example, with an alkyl nitrite such as isoamyl nitrite to form the N-nitroso-imino compound that is then reduced to the N-amino-imino compound using, for example, zinc in the presence of an acid such as acetic acid.

The subsequent cleaving of a C₁₋₃-alkyloxycarbonyl group to obtain the carboxy group is carried out, for example, by hydrolysis with an acid such as hydrochloric acid or sulfuric acid or an alkali metal hydroxide such as lithium hydroxide, sodium hydroxide, or potassium hydroxide.

The subsequent amide formation is carried out by reacting a corresponding reactive carboxylic acid derivative with a corresponding amine optionally in a solvent or mixture of solvents such as methylene chloride, dimethylformamide, benzene, toluene, chlorobenzene, tetrahydrofuran, benzene/tetrahydrofuran or 1,4-dioxane, while the amine used may also serve as solvent, optionally in the presence of a tertiary organic base or in the presence of an inorganic base or with a corresponding carboxylic acid in the presence of a dehydrating agent. Isobutyl chloroformate, thionyl chloride, trimethylchlorosilane, phosphorus trichloride, phosphorus pentoxide, N,N'-dicyclohexylcarbodiimide, N,N'-dicyclohexylcarbodiimide/N-hydroxysuccinimide, 1-hydroxy-benzotriazole, N,N'-carbonyldiimidazole or triphenylphosphine/carbon tetrachloride, or combinations thereof optionally in the presence of 4-dimethylaminopyridine at temperatures between 0 and 150 °C, preferably between 0 and 80 °C, may be applied to achieve the coupling.

The subsequent introduction of a chlorine, bromine, or iodine atom onto an aromatic substructure may be carried out by reacting the aromatic compound with an appropriate electrophile of the halogen atom. Suited chlorine and bromine electrophiles may be e.g. N-halosuccinimide, HOCI, HOBr, *tert*BuOCl, tertBuOBr, chlorine, bromine, dibromoisocyanuric acid, pyridinium dichlorobromate, pyridinium tribromide, or sulfuryl chloride that may be used alone or in combination with an acid, e.g. hydrochloric acid, hydrobromic acid, tetrafluoroboric acid, triflic acid, sulfuric acid, or acetic acid, or a Lewis acid, e.g. iron(III) halide, borontrifluoride hydrate, borontrifluoride etherate, or aluminum halide. Further useful combinations may be LiBr and ceric ammonium nitrate, KCI or KBr with Oxone^{®}, or KBr and sodium perborate. Suited iodine electrophiles may be generated from iodine combined with an oxidizing agent such as nitric acid, sulfur trioxide, manganese dioxide, H10₃, hydrogen peroxide, sodium periodate, peroxydisulfates, and Oxone^{®}. Further suited iodine electrophiles may be e.g. iodine chloride, dichloroiodates, and N-iodosuccinimide. These iodine electrophiles may be used without an additive or in the presence of an acid such as e.g. acetic acid, trifluoroacetic acid, or sulfuric acid, or a Lewis acid such as borontrifluoride hydrate, or copper salts. If a nitro group is to be introduced appropriate nitro electrophiles may be generated from, for example, nitric acid, acetyl nitrate, ceric ammonium nitrate, sodium nitrate, N₂O₅, alkyl nitrate, and nitronium tetrafluoroborate. Some of these reagents may be used without an additive, though, several of them are better used in combination with an acid, e.g. sulfuric acid or triflic acid, acetic anhydride, trifluoroacetic anhydride, Lewis acid, e.g. ytterbium triflate or iron acetate, P₂O₅, or a base. The SO₃H group may be introduced by reacting the aromatic compound with, for example, concentrated sulfuric acid, SO₃, ClSO₃H, or ClSO₂NMe₂ combined with indium triflate. Reacting the aromatic compound with ClSO₃H gives the corresponding chlorosulfonylated derivative that may be hydrolyzed to the sulfonic acid. Acylating the aromatic part is conducted using an acyl electrophile that may be generated from the respective acyl halide, e.g. chloride, or acyl anhydride and a Lewis acid such as e.g. aluminum halide, diethylaluminum halide, indium halide, iron(III) halide, tin(IV) halide, borontrifluoride, titanium(IV) halide, or a Bronsted acid, e.g. sulfuric acid or triflic acid. The formyl group is best introduced using the so-called Vilsmeier or Vilsmeier-Haack conditions: dialkylformamide combined with phosgene, thionyl chloride, POCl₃, or oxalyl chloride. Preferred solvents for the electrophilic substitutions described may differ depending on the electrophile employed; in the following some more generally applicable are mentioned: methylene chloride, dichloroethane, chlorobenzene, dichlorobenzene, ether, fluorinated hydrocarbons, hexanes, quinoline, or acetonitrile. The temperatures preferably applied range from 0 to 180 °C.

The subsequent replacement of an aromatic amino group is initiated by diazotization of the amino group using a nitrous acid or nitrosonium source or equivalent such as a nitrite salt combined with an acid, e.g. sodium nitrite and hydrochloric acid, nitrosonium tetrafluoroborate, or an alkylnitrite, e.g. *tert*butylnitrite or *i*soamylnitrite. The diazotization is optionally carried out in methylene chloride, dichloroethane, dimethylformamide, N-methylpyrrolidinone, benzene, toluene, chlorobenzene, tetrahydrofuran, water, ethyl acetate, alcohol, ether, 1,2-dimethoxyethane, 1,4-dioxane, or mixtures thereof at temperatures between -10 °C and 100 °C (diazotization of amino groups is detailed in, for example, Angew. Chem. Int. Ed. 1976, 15, 251). The subsequent displacement of the diazo group for a cyano group, chlorine, or bromine using cuprous cyanide, chloride, or bromide, respectively, is known as the Sandmeyer reaction (see e.g. March's Advanced Organic Chemistry, Michael B. Smith and Jerry March, John Wiley & Sons Inc., 6. Ed., New Jersey, 2007 and references quoted therein); the reaction is optionally conducted between -10 °C and 120 °C in one of the solvents or mixtures mentioned above. The replacement of the diazo group for a fluorine atom may be achieved with a tetrafluoroborate salt or tetrafluoroboric acid and heating to 20 to 160 °C; the reaction is known as the Schiemann reaction. Iodine may be introduced by treatment of the diazo compound with an iodide salt, e.g. sodium iodide, preferably using water or an aqueous solvent mixture at temperatures between 0 and 120 °C. The diazo group is replaced for hydroxy using water or an aqueous solvent mixture at temperatures between 0 and 180 °C. The reaction usually works without further additives but the addition of cuprous oxide or strong acid may be advantageous. Mercapto or alkylmercapto may be introduced via their corresponding disulfide salts or dialkyldisulfides at temperatures between 0 and 120 °C; depending on the sulfur species used an inert solvent or aqueous solvent system may be preferred (see e.g. Synth. Commun. 2001, 31, 1857 and references quoted therein).

The subsequent replacement of an aromatic amino group by an aryl group may be carried out via the corresponding diazo compound obtainable as described above. The reaction with an aryl nucleophile, preferably an aryl boronic acid, boronic ester, trifluoroborate, zinc halide, or stannane, is conducted in the presence of a transition metal species derived from palladium, nickel, rhodium, copper, or iron, preferably palladium. The active catalyst may be a complex of the transition metal with ligands such as e.g. phosphines, phosphites, imdiazole carbenes, imidazolidine carbenes, dibenzylideneacetone, allyl, or nitriles, an elemental form of the transition metal such as palladium on carbon or nanoparticles, or salts such as chloride, bromide, acetate, or trifluoroacetate. In these reactions the diazo compound is preferably employed as its tetrafluoroborate salt optionally in methylene chloride, dimethylformamide, N-methylpyrrolidinone, benzene, toluene, tetrahydrofuran, water, ethyl acetate, alcohol, ether, 1,2-dimethoxyethane, 1,4-dioxane, or mixtures thereof at temperatures between 10 °C and 180 °C, preferably between 20 °C and 140 °C.

The subsequent replacement of an aromatic chloro, bromo, iodo atom or an aromatic trifluoromethylsulfonyloxy, mesyloxy, or tosyloxy group for an aryl, alkenyl, alkynyl, or alkyl residue is preferably mediated by a transition metal species derived from palladium, nickel, rhodium, copper, or iron. The active catalyst may be a complex of the transition metal with ligands such as e.g. phosphines (e.g. tri*tert*butylphosphine, tricyclohexylphosphine, substituted biphenyldicyclohexylphosphines, substituted biphenyldi*tert*butylphosphines, triphenylphosphine, tritolylphosphine, trifurylphosphine, 1,1'-bis(diphenylphosphino)ferrocene), phosphites, imdiazole carbenes, imidazolidine carbenes, dibenzylideneacetone, allyl, or nitriles, an elemental form of the transition metal such as palladium on carbon or nanoparticles of iron or palladium, or a salt such as fluoride, chloride, bromide, acetate, triflate, or trifluoroacetate. The replacement is preferably conducted with a trifluoroborate, boronic acid, or boronic ester (Suzuki or Suzuki-type reaction), zinc halide (Negishi or Negishi-type reaction), stannane (Stille reaction), silane (Hiyama or Hiyama-type reaction), magnesium halide (Kumada or Kumada-type reaction) of the aryl, alkenyl, or alkyl residue to be introduced. The terminal alkyne is preferably used as it is or as the zinc acetylide derivative. Depending on the electrophilic and nucleophilic reaction partners additives such as halide salts, e.g. lithium chloride, potassium fluoride, tetrabutylammonium fluoride, hydroxide sources such as potassium hydroxide or potassium carbonate, silver salts such as silver oxide or triflate, copper salts such as copper chloride or copper thiophenecarboxylate may be advantageous or even essential. Copper iodide is a preferred additive in the coupling with a terminal alkyne group

(Sonogashira reaction). The coupling reactions are optionally conducted in methylene chloride, dimethylformamide, N-methylpyrrolidinone, benzene, toluene, tetrahydrofuran, water, ethyl acetate, alcohol, ether, dimethylsulfoxide, 1,2-dimethoxyethane, 1,4-dioxane, or mixtures thereof, though, depending on the nucleophile some of them are less or not suited at all. Preferred temperatures are in the range from -10 °C to 180 °C.

The subsequent replacement of an aromatic chlorine, bromine, or iodine atom or an aromatic trifluoromethylsulfonyloxy, mesyloxy, or tosyloxy group for a hydrogen atom is preferably mediated by a transition metal species derived from palladium, nickel, platinum, or rhodium. The active catalyst may be a complex of the transition metal with ligands, an elemental form, or a salt of the transition metal as mentioned above. Raney nickel or palladium on carbon are among the preferred catalyst species. Suited hydrogen sources may be hydrogen, preferably at pressures of 1 to 5 bar, silanes, e.g. trialkoxysilane, boranes, hydrides, e.g. alkali metal borohydride, formic acid, or formates, e.g. ammonium formate. The reactions are preferably carried out in methylene chloride, dimethylformamide, N,N-dimethylacetamide, N-methylpyrrolidinone, benzene, toluene, tetrahydrofuran, water, ethyl acetate, alcohol, ether, 1,2-dimethoxyethane, 1,4-dioxane, or mixtures thereof at -10 °C to 180 °C, more preferably at 20 °C to 140 °C.

The subsequent cyclization of two adjacent heteroatoms is optionally conducted with a carboxy equivalent such as nitrile, carboxylic chloride or fluoride, carboxylic acid, ketene, carboxylic ester, or carboxylic thioester. The overall transformation consists of two reaction steps: attachment of the carboxy equivalent to one of the two heteroatoms followed by cyclization with the other heteroatom. The first step is an amide formation with the amino functionality that may be carried out as described hereinbefore. The ensuing reaction step, cyclization with the second heteroatom, may be accomplished by heating in the presence of an acid, e.g. acetic acid, trifluoroacetic acid, sulfuric acid, or hydrochloric acid, or a base, e.g. sodium hydroxide, sodium ethoxide, or sodium *tert*butoxide. The use of dehydrating reagents such as anhydrides, e.g. acetic anhydride, orthoesters, e.g. trimethylorthoformate, thionylchloride, phosgene, diphosgene, triphosgene, phosphorous oxychloride, phosphorous pentachloride, dialkylcarbodiimides, combinations of phosphines, e.g. triphenylphosphine or trialkylphosphine with dialkyl azodicarboxylates, bromine, iodine, or 1,2-dihaloethanes, e.g. 1,2-dibromotetrafluoroethane, may be advantageous. The reactions are preferably carried out in inert solvents or mixtures such as methylene chloride, dichloroethane, benzene, toluene, tetrahydrofuran, ether, or combinations thereof, though, cyclization in the presence of an acid or a base may also be conducted in water or an alcohol, e.g. methanol, ethanol, *iso*propanol, or *tert*butanol, or combinations with these solvents. The reactions are carried out at temperatures between 0 °C and 200 °C, preferably between 20 °C and 140 °C.

The subsequent reduction of a cyano group to obtain an aminomethyl group is optionally conducted with hydrogen in the presence of a transition metal species or with a hydride. Suited transition metals may be derived from palladium, nickel, platinum, rhodium, or ruthenium such as, for example, palladium on charcoal, palladium hydroxide, platinum oxide, or Raney nickel that may be used in solvents such as ethyl acetate, alcohols, e.g. methanol or ethanol, dichloromethane, tetrahydrofuran, ether, benzene, toluene, dimethylformamide, or N-methylpyrrolidinone at hydrogen pressures between 1 and 10 bar, preferably between 1 and 5 bar, and at temperatures between 0 and 180 °C, preferably between 20 and 120 °C. Additives such as acids, e.g. hydrochloric acid, methanesulfonic acid, sulfuric acid, or acetic acid, may be beneficial for the hydrogenation. Appropriate hydride sources may be selected from e.g. borohydrides, e.g. sodium borohydride, potassium trisecbutylborohydride, borane, or lithium triethylborohydride, or alanates, e.g. lithium aluminum hydride or diisobutylaluminum hydride. Some of these reagents are best used in combination with nickel chloride or cobalt chloride as sodium borohydride. These reagents may be used in e.g. tetrahydrofuran, ether, 1,4-dioxane, 1,2-dimethoxyethane, dichloromethane, 1,2-dichloroethane, benzene, or toluene; some are also compatible with alcoholic solutions. Preferred reaction temperatures range from -80 °C to 160 °C, more preferred from -40 °C to 80 °C.

The subsequent formation of a N-hydroxycarbamimidoyl group from a cyano group may be carried out by the treatment of the cyano compound with hydroxylamine. The reaction is preferably conducted in aqueous or alcoholic solvents at temperatures between 0 °C and 140 °C.

The subsequent formation of an oxadiazole from an N-hydroxycarbamimidoyl is optionally conducted with a carboxy equivalent such as nitrile, carboxylic chloride or fluoride, carboxylic acid, ketene, carboxylic ester, or carboxylic thioester. The transformation is related to the formation of a ring starting from two adjacent heteroatoms described above and may be carried out analogously.

The subsequent formation of a cyano group from an amino carbonyl group is optionally conducted by using a dehydrating reagent such as e.g. anhydride, e.g. acetic anhydride, trifluoroacetic anhydride, or triflic anhydride, phosgene, thionyl chloride, oxalyl chloride, POCl₃, PCl₅, P₄O₁₀, triphenylphosphite, or triphenyl- or trialkylphosphine combined with tetrachloromethane, 1,2-dibromotetrafluoroethane, or bromine. The reactions are preferably carried out in dichloromethane, 1,2-dichloroethane, hexanes, ether, 1,4-dioxane, benzene, toluene, acetonitrile, mixtures thereof, or without a solvent at temperatures between 0 °C and 140 °C. Additives such as amines, e.g. pyridine or triethylamine, or dimethylformamide may be beneficial.

The subsequent reduction of a keto or an aldehydic group to obtain a secondary or primary alcohol may be carried out with a complex metal hydride such as sodium borohydride, lithium borohydride, lithium triethylborohydride, diisobutylaluminum hydride, or lithium aluminum hydride. The reductions may be conducted in e.g. dichloromethane, 1,2-dichloroethane, hexanes, ether, 1,4-dioxane, tetrahydrofuran, dimethylformamide, N-methylpyrrolidinone, benzene, toluene, alcohols, e.g. methanol, water, or mixtures thereof, though, not all reducing agents are compatible with all of these solvents. Preferred temperatures are between -80 °C and 140 °C depending on the reducing power of the reagent. Alternatively, hydrogen in the presence of a transition metal catalyst may be used for the reduction.

In the reactions described hereinbefore, any reactive group present such as hydroxy, carboxy, amino, alkylamino, or imino group may be protected during the reaction by conventional protecting groups which are cleaved again after the reaction.

For example, a protecting group for a hydroxy group may be a trimethylsilyl, *tert*butyldimethylsilyl, triisopropylsilyl, acetyl, pivaloyl, benzoyl, methyl, *tert*-butyl, allyl, trityl, benzyl, 4-methoxybenzyl, tetrahydropyranyl, methoxymethyl, ethoxymethyl, or 2-trimethylsilylethoxymethyl group,
protecting groups for a carboxy group may be trimethylsilyl, methyl, ethyl, *tert*butyl, allyl, benzyl, or tetrahydropyranyl,
protecting groups for a ketone or aldehyde may be a ketal or acetal, respectively, e.g. derived from methanol, glycol, or propane-1,3-diol,
protecting groups for an amino, alkylamino, or imino group may be methyl, formyl, acetyl, trifluoroacetyl, ethoxycarbonyl, *tert*-butoxycarbonyl, benzyloxycarbonyl, benzyl, methoxybenzyl, or 2,4-dimethoxybenzyl and for the amino group additionally phthalyl, and
protecting groups for a terminal alkyne may be trimethylsilyl, trisopropylsilyl, *tert*butyldimethylsilyl, or 2-hydroxy-isopropyl.

Any acyl protecting group may be cleaved, for example, hydrolytically in an aqueous solvent, e.g. in water, isopropanol/water, acetic acid/water, tetrahydrofuran/water, or 1,4-dioxane/water, in the presence of an acid such as trifluoroacetic acid, hydrochloric acid, or sulfuric acid or in the presence of an alkali metal base such as lithium hydroxide, sodium hydroxide, or potassium hydroxide or aprotically, e.g. in the presence of iodotrimethylsilane, at temperatures between 0 and 120 °C, preferably between 10 and 100 °C. A trifluoroacetyl group is preferably cleaved by treating with an acid such as hydrochloric acid, optionally in a solvent such as acetic acid, at temperatures between 50 and 120 °C or by treating with sodium hydroxide solution, optionally in an additional solvent such as tetrahydrofuran or methanol, at temperatures between 0 and 80 °C.

Any acetal or ketal protecting group used may be cleaved, for example, hydrolytically in an aqueous solvent, e.g. in water, isopropanol/water, acetic acid/water, tetrahydrofuran/water, or 1,4-dioxane/water, in the presence of an acid such as trifluoroacetic acid, hydrochloric acid, or sulfuric acid or aprotically, e.g. in the presence of iodotrimethylsilane, at temperatures between 0 and 120 °C, preferably between 10 and 100 °C.

A trimethylsilyl group is cleaved, for example, in water, an aqueous solvent mixture or an alcohol, such as methanol or ethanol, in the presence of a base such as lithium hydroxide, sodium hydroxide, potassium carbonate, or sodium methoxide.
Acids such as e.g. hydrochloric acid, trifluoroacetic acid, or acetic acid may also be suitable. The cleavage usually takes place at comparatively low temperatures, e.g. between -60 and 60 °C. Silyl groups other than trimethylsilyl are preferentially cleaved in the presence of an acid, e.g. trifluoroacetic acid, hydrochloric acid, or sulfuric acid, at temperatures between 0 °C and 100 °C. A particularly suited cleaving method for silyl groups is based on the use of fluoride salts, e.g. tetrabutylammonium fluoride, hydrogen fluoride, or potassium fluoride, in organic solvents, such as for example diethyl ether, tetrahydrofuran, 1,4-dioxane, 1,2-dimethoxyethane, toluene, benzene, dichloroethane, or dichloromethane at temperatures between -20 and 100 °C.

A benzyl, methoxybenzyl, or benzyloxycarbonyl group is advantageously cleaved hydrogenolytically, e.g. with hydrogen in the presence of a catalyst such as palladium on carbon, palladium hydroxide, or platinum oxide in a solvent such as methanol, ethanol, ethyl acetate, or glacial acetic acid, optionally in the presence of an acid, such as hydrochloric acid, at temperatures between 0 and 100 °C, preferably between 20 and 60 °C, and at hydrogen pressures of 1 to 7 bar, preferably 3 to 5 bar. Trimethylsilyl iodide, boron trichloride, or boron trifluoride in the presence of a scavenger such as anisol, thioanisol, or pentamethylbenzene may also be used with benzylether derivatives. An electron-rich benzyl residue, such as methoxybenzyl, may also be cleaved oxidatively with e.g. 2,3-dichloro-5,6-dicyano-1,4-benzoquinone (DDQ) or ceric ammonium nitrate (CAN) preferably in an alcoholic or aqueous solvent at temperatures between 10 and 120 °C. A 2,4-dimethoxybenzyl group is preferably cleaved in trifluoroacetic acid in the presence of a scavenger such as anisole.

A *tert*butyl or *tert*butyloxycarbonyl group is preferably cleaved by treating with an acid such as trifluoroacetic acid, sulfuric acid, or hydrochloric acid or by treating with iodotrimethylsilane optionally using a solvent such as methylene chloride, 1,4-dioxane, methanol, isopropanol, water, or diethylether.

A methyl group at an tertiary amine may be cleaved by the treatment with 1-chloroethyl chloroformate. Hydrobromic acid and borontribromide are particularly suited for the cleavage of methylethers.

The compounds of general formula I may be resolved into their enantiomers and/or diastereomers, as mentioned before. Thus, for example, *cis*/*trans* mixtures may be resolved into their *cis* and *trans* isomers, and racemic compounds may be separated into their enantiomers.

The *cis*/*trans* mixtures may be resolved, for example, by chromatography into the cis and *trans* isomers thereof. The compounds of general formula I which occur as racemates may be separated by methods known *per se* (cf. Allinger N. L. and Eliel E. L. in "Topics in Stereochemistry", Vol. 6, Wiley Interscience, 1971) into their optical antipodes and diastereomeric mixtures of compounds of general formula I may be resolved into their diastereomers by taking advantage of their different physico-chemical properties using methods known *per se,* e.g. chromatography and/or fractional crystallization; if the compounds obtained thereafter are racemates, they may be resolved into the enantiomers as mentioned above.

The racemates are preferably resolved by column chromatography on chiral phases or by crystallisation from an optically active solvent or by reacting with an optically active substance which forms salts or derivatives, such as e.g. esters or amides, with the racemic compound. Salts may be formed with enantiopure acids for basic compounds and with enantiopure bases for acidic compounds. Diastereomeric derivatives are formed with enantiopure auxiliary compounds such as e.g. acids, their activated derivatives, or alcohols. Separation of the diastereomeric mixture of salts or derivatives thus obtained may be achieved by taking advantage of their different physico-chemical properties, e.g. differences in solubility; the free antipodes may be released from the pure diastereomeric salts or derivatives by the action of suitable agents. Optically active acids in common use for such a purpose are e.g. the D- and L-forms of tartaric acid, dibenzoyltartaric acid, di-o-tolyltartaric acid, malic acid, mandelic acid, camphorsulfonic acid, glutamic acid, aspartic acid, or quinic acid. Optically active alcohols applicable as auxiliary may be, for example, (+) or (-)-menthol and optically active acyl groups in amides may be, for example, (+)- or (-)-menthyloxycarbonyl.

As mentioned above, the compounds of formula I may be converted into salts, particularly for pharmaceutical use into the physiologically acceptable salts with inorganic or organic acids provided that compound I bears a basic residue. Acids which may be used for this purpose include for example hydrochloric acid, hydrobromic acid, sulfuric acid, methanesulfonic acid, phosphoric acid, fumaric acid, succinic acid, lactic acid, citric acid, tartaric acid, or maleic acid.

If the compounds of formula I contain an acidic residue like, for example, a carboxy group, they may be converted into the salts thereof with inorganic or organic bases, particularly for pharmaceutical use into the physiologically acceptable salts thereof. Suitable bases for this purpose include, for example, sodium hydroxide, potassium hydroxide, calcium hydroxide, calcium isopropoxide, magnesium hydroxide, magnesium ethoxide, ammonium hydroxide, cyclohexylamine, ethanolamine, diethanolamine, triethanolamine, N-methyl-D-glucamine, L-lysine, L-arginine, and piperazine.

The compounds according to the invention are advantageously also obtainable using the methods described in the examples that follow, which may also be combined for this purpose with methods known to the skilled man from the literature.

As already mentioned, the compounds of general formula I according to the invention and the physiologically acceptable salts thereof have valuable pharmacological properties, particularly an inhibitory effect on the enzyme 11β-hydroxysteroid dehydrogenase (HSD) 1.

The biological properties of the new compounds may be investigated as follows:
In vitro inhibition of 11β-HSD1 by test compounds was determined with HTRF (Homogeneous Time-Resolved Fluorescence) technology (cisbio international, France) detecting cortisol generated from cortisterone by human liver microsomes.

Briefly, compounds were incubated for 1 hour at 37°C in Tris buffer (20 mM tris, 5 mM EDTA, pH 6.0) containing NADPH (200µM) and cortisone (80nM). Cortisol generated in the reaction is then detected with a competitive immunoassay, involving two HTRF conjugates: cortisol linked to XL665 and anti-cortisol antibody labeled with Europium cryptate. The incubation period for detection reaction was typically 2 hours. The amount of cortisol is determined by reading the time-resolved fluorescence of the wells (Ex 320/75 nm; Em 615/8.5 nm and 665/7.5 nm). The ratio of the two emission signals is then calculated (Em665*10000/Em615). Each assay contained incubations with vehicle controls instead of compound as controls for non-inhibited cortisol generation (100% CTL; 'high values') and incubations with carbenoxolone as controls for fully inhibited enzyme and cortisol background (0% CTL; 'low values'). Each assay also contained a calibration curve with cortisol to transform the fluorescent data into cortisol concentrations. Percent inhibition of each compound was determined relative to the carbenoxolone signal and IC₅₀ curves were generated.

The compounds of general formula I according to the invention for example have IC₅₀ values below 10000 nM, particularly below 1000 nM, most preferably below 200 nM. The %CTL values of some example compounds at a concentration of 1 µM are provided in the following Table 2 wherein 100% indicates no inhibition and a value of zero or below zero indicates complete inhibition. The measurement of %CTL is described hereinbefore.

**Table 2. Inhibition of 11β-HSD 1 mediated by the compounds described in the experimental section**

| Example | %CTL at 1 µM | Example | %CTL at 1 µM | Example | %CTL at 1 µM |
|---|---|---|---|---|---|
| 1 | -46 | 26 | -41 | 51 | 94 |
| 2 | 18 | 27 | -41 | 52 | 27 |
| 3 | 84 | 28 | 36 | 53 | 7 |
| 7 | -32 | 29 | 30 | 54 | -25 |
| 8 | -27 | 30 | -54 | 55 | -31 |
| 9 | -14 | 31 | -6 | 58 | -37 |
| 10 | 53 | 32 | 82 | 59 | 70 |
| 11 | 18 | 33 | 23 | 60 | -19 |
| 12 | -39 | 35 | -30 | 61 | 7 |
| 13 | -53 | 36 | -31 | 62 | 13 |
| 14 | -46 | 37 | -7 | 63 | 47 |
| 15 | 14 | 39 | 23 | 64 | -33 |
| 16 | -41 | 40 | -17 | 65 | 2 |
| 17 | -43 | 41 | -56 | 66 | -16 |
| 18 | -8 | 42 | 32 | 67 | 71 |
| 19 | -47 | 43 | -45 | 68 | 87 |
| 20 | -39 | 44 | -4 | 69 | 6 |
| 21 | -28 | 46 | -10 | 70 | -15 |
| 22 | -4 | 47 | 94 | 71 | -18 |
| 23 | -9 | 48 | -18 | 72 | -11 |
| 24 | 70 | 49 | 23 | 73 | -6 |
| 25 | -43 | 50 | -20 | 74 | -17 |
| 75 | 26 | 76 | -10 | 77 | 30 |
| 78 | 61 | 79 | 13 | 80 | 70 |

In view of their ability to inhibit the enzyme 11β-hydroxysteroid dehydrogenase (HSD) 1, the compounds of general formula I according to the invention and the corresponding pharmaceutically acceptable salts thereof are theoretically suitable for the treatment and/or preventative treatment of all those conditions or diseases which may be affected by the inhibition of the 11β-hydroxysteroid dehydrogenase (HSD) 1 activity. Therefore, compounds according to the invention are particularly suitable for the prevention or treatment of diseases, particularly metabolic disorders, or conditions such as type 1 diabetes mellitus, type 2 diabetes mellitus, complications of diabetes (such as e.g. retinopathy, nephropathy or neuropathies, diabetic foot, ulcers, macroangiopathies, slow or poor wound healing), metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, edema and hyperuricaemia. These substances are also suitable for preventing beta-cell degeneration such as e.g. apoptosis or necrosis of pancreatic beta cells. The substances are also suitable for improving or restoring the functionality of pancreatic cells, and also of increasing the number and size of pancreatic beta cells. The compounds according to the invention may also be used as diuretics or antihypertensives and are suitable for the prevention and treatment of acute renal failure.

Additionally, inhibition of 11β-hydroxysteroid dehydrogenase (HSD) 1 has been shown to lower intraocular pressure in subjects with ocular hypertension, therefore the compounds could be used to treat glaucoma.

In view of the role of 11β-hydroxysteroid dehydrogenase (HSD) 1 in modulating cortisol levels for interaction with the glucocorticoid receptor, and the known role of excess glucocorticoids in bone loss, the compounds may have beneficial effects in treatment or prevention of osteoporosis.

Stress and/or glucocorticoids have been shown to influence cognitive function, and excess cortisol has been associated with brain neuronal loss or dysfunction. Treatment with an 11β-hydroxysteroid dehydrogenase (HSD) 1 inhibitor may result in amelioration or prevention of cognitive impairment. Such compounds may also be useful in treating anxiety or depression.

The dynamic interaction between the immune system and the HPA (hypothalamopituitary-adrenal) axis is known, and glucocorticoids help balance between cell-mediated responses and humoral responses. The immune reaction is typically biased towards a humoral response in certain disease states, such as tuberculosis, leprosy, and psoriasis. More appropriate would be a cell-based response. An 11β-hydroxysteroid dehydrogenase (HSD) 1 inhibitor would bolster a temporal immune response in association with immunization to ensure that a cell based response would be obtained, and as such could be useful in immunomodulation.

In particular, the compounds according to the invention, including the physiologically acceptable salts thereof, are suitable for the prevention or treatment of diabetes, particularly type 1 diabetes mellitus, type 2 diabetes mellitus, and diabetic complications.

The dosage required to achieve the corresponding activity for treatment or prevention usually depends on the compound which is to be administered, the patient, the nature and gravity of the illness or condition and the method and frequency of administration and is for the patient's doctor to decide. Expediently, the dosage may be from 1 to 100 mg, preferably 1 to 30 mg, by intravenous route, and 1 to 1000 mg, preferably 1 to 100 mg, by oral route, in each case administered 1 to 4 times a day. For this purpose, the compounds of formula I prepared according to the invention may be formulated, optionally together with other active substances, together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof, to produce conventional galenic preparations such as plain or coated tablets, capsules, powders, suspensions or suppositories.

The compounds according to the invention may also be used in conjunction with other active substances, particularly for the treatment and/or prevention of the diseases and conditions mentioned above. Other active substances which are suitable for such combinations include for example those which potentiate the therapeutic effect of an 11β-hydroxysteroid dehydrogenase (HSD) 1 antagonist according to the invention with respect to one of the indications mentioned and/or which allow the dosage of an 11β-hydroxysteroid dehydrogenase (HSD) 1 antagonist according to the invention to be reduced. Therapeutic agents which are suitable for such a combination include, for example, antidiabetic agents such as metformin, sulfonylureas (e.g. glibenclamide, tolbutamide, glimepiride), nateglinide, repaglinide, thiazolidinediones (e.g. rosiglitazone, pioglitazone), SGLT 2 inhibitors (e.g. dapagliflozin, sergliflozin), PPAR-gamma-agonists (e.g. GI 262570) and antagonists, PPAR-gamma/alpha modulators (e.g. KRP 297), alpha-glucosidase inhibitors (e.g. acarbose, voglibose), DPPIV inhibitors (e.g. Sitagliptin, Vildagliptin, Saxagliptin, Alogliptin, Linagliptin), alpha2-antagonists, insulin and insulin analogues, GLP-1 and GLP-1 analogues (e.g. exendin-4) or amylin. The list also includes inhibitors of protein tyrosinephosphatase 1, substances that affect deregulated glucose production in the liver, such as e.g. inhibitors of glucose-6-phosphatase, or fructose-1,6-bisphosphatase, glycogen phosphorylase, glucagon receptor antagonists and inhibitors of phosphoenol pyruvate carboxykinase, glycogen synthase kinase or pyruvate dehydrokinase and glucokinase activators, lipid lowering agents such as for example HMG-CoA-reductase inhibitors (e.g. simvastatin, atorvastatin), fibrates (e.g. bezafibrate, fenofibrate), nicotinic acid and the derivatives thereof, PPAR-alpha agonists, PPAR-delta agonists, ACAT inhibitors (e.g. avasimibe) or cholesterol absorption inhibitors such as, for example, ezetimibe, bile acid-binding substances such as, for example, cholestyramine, inhibitors of ileac bile acid transport, HDL-raising compounds such as CETP inhibitors or ABC1 regulators or active substances for treating obesity, such as sibutramine or tetrahydrolipostatin, SDRIs, axokine, leptin, leptin mimetics, antagonists of the cannabinoidl receptor, MCH-1 receptor antagonists, MC4 receptor agonists, NPY5 or NPY2 antagonists or β3-agonists such as SB-418790 or AD-9677 and agonists of the 5HT2c receptor.

Moreover, combinations with drugs for influencing high blood pressure, chronic heart failure or atherosclerosis such as e.g. A-II antagonists or ACE inhibitors, ECE inhibitors, diuretics, β-blockers, Ca-antagonists, centrally acting antihypertensives, antagonists of the alpha-2-adrenergic receptor, inhibitors of neutral endopeptidase, thrombocyte aggregation inhibitors and others or combinations thereof are suitable. Examples of angiotensin II receptor antagonists are candesartan cilexetil, potassium losartan, eprosartan mesylate, valsartan, telmisartan, irbesartan, EXP-3174, L-158809, EXP-3312, olmesartan, medoxomil, tasosartan, KT-3-671, GA-0113, RU-64276, EMD-90423, BR-9701, etc.. Angiotensin II receptor antagonists are preferably used for the treatment or prevention of high blood pressure and complications of diabetes, often combined with a diuretic such as hydrochlorothiazide.

A combination with uric acid synthesis inhibitors or uricosurics is suitable for the treatment or prevention of gout.

A combination with GABA-receptor antagonists, Na-channel blockers, topiramat, protein-kinase C inhibitors, advanced glycation end product inhibitors or aldose reductase inhibitors may be used for the treatment or prevention of complications of diabetes.

The dosage for the combination partners mentioned above is usefully 1/5 of the lowest dose normally recommended up to 1/1 of the normally recommended dose.

Therefore, in another aspect, this invention relates to the use of a compound according to the invention or a physiologically acceptable salt of such a compound combined with at least one of the active substances described above as a combination partner, for preparing a pharmaceutical composition which is suitable for the treatment or prevention of diseases or conditions which can be affected by inhibiting the enzyme 11β-hydroxysteroid dehydrogenase (HSD) 1. These are preferably metabolic diseases, particularly one of the diseases or conditions listed above, most particularly diabetes or diabetic complications.

The use of the compound according to the invention, or a physiologically acceptable salt thereof, in combination with another active substance may take place simultaneously or at staggered times, but particularly within a short space of time. If they are administered simultaneously, the two active substances are given to the patient together; while if they are used at staggered times the two active substances are given to the patient within a period of less than or equal to 12 hours, but particularly less than or equal to 6 hours.

Consequently, in another aspect, this invention relates to a pharmaceutical composition which comprises a compound according to the invention or a physiologically acceptable salt of such a compound and at least one of the active substances described above as combination partners, optionally together with one or more inert carriers and/or diluents.

Thus, for example, a pharmaceutical composition according to the invention comprises a combination of a compound of formula I according to the invention or a physiologically acceptable salt of such a compound and at least one angiotensin II receptor antagonist optionally together with one or more inert carriers and/or diluents.

The compound according to the invention, or a physiologically acceptable salt thereof, and the additional active substance to be combined therewith may both be present together in one formulation, for example a tablet or capsule, or separately in two identical or different formulations, for example as a so-called kit-of-parts.

The Examples that follow are intended to illustrate the present invention without restricting it. The terms "ambient temperature" and "room temperature" are used interchangeably and designate a temperature of about 20°C.

### Preparation of the starting compounds:

### Example I

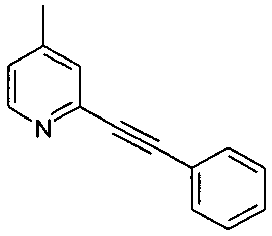

### 4-Methyl-2-phenylethynyl-pyridine

Phenylacetylene (15.4 mL) is added to a mixture of 2-bromo-4-methyl-pyridine (20.0 g), Cul (2.2 g), and Pd(PPh₃)₂Cl₂ (4.1 g) in triethylamine (600 mL) kept under argon atmosphere. The mixture is stirred at ambient temperature overnight. Then, water is added and the resulting mixture is extracted with diethylether. The combined organic extracts are washed with brine and dried (MgSO₄). The solvent is removed under reduced pressure and the residue is purified by chromatography on silica gel (cyclohexane/ethyl acetate 9:1->4:1) to give the product as an oil.
Yield: 18.6 g (83% of theory)
Mass spectrum (ESI⁺): m/z = 194 [M+H]⁺

### Example II

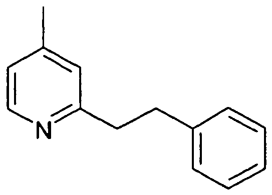

### 4-Methyl-2-phenethyl-pyridine

A mixture of 4-methyl-2-phenylethynyl-pyridine (18.2 g) and 10% palladium on carbon (2.0 g) in methanol (300 mL) is stirred under hydrogen atmosphere (50 psi) at ambient temperature until the triple bond is completely reduced (20 h). The mixture is filtrered and the solvent is removed under reduced pressure.
Yield: 17.6 g (95% of theory)
Mass spectrum (ESI⁺): m/z = 198 [M+H]⁺

### Example III

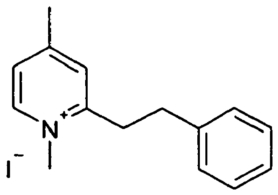

### 1,4-Dimethyl-2-phenethyl-pyridinium iodide

lodomethane (8.3 mL) is added to a solution of 4-methyl-2-phenethyl-pyridine (17.5 g) in acetonitrile (70 mL). The resulting solution is stirred at room temperature overnight before another portion of iodomethane (2.8 mL) is added and the solution is further stirred at ca. 35 °C for another 14 h. After cooling to room temperature, the precipitate is separated by filtration, washed with acetonitrile, and dried at 50 °C.
Yield: 20.9 g (69% of theory)
Mass spectrum (ESI⁺): m/z = 212 [1,4-dimethyl-2-phenethyl-pyridinium]⁺

### Example IV

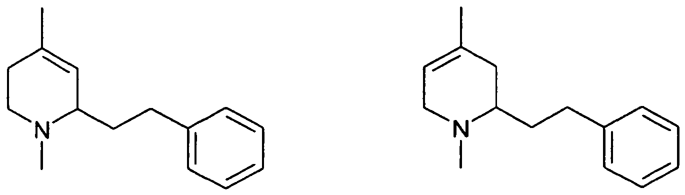

### 1,4-Dimethyl-6-phenethyl-1,2,3,6-tetrahydro-pyridine and 1,4-dimethyl-2-phenethyl-1,2,3,6-tetrahydro-pyridine

Sodium borohydride (2.9 g) is added in one portion to a mixture of 1,4-dimethyl-2-phenethyl-pyridinium iodide (20.9 g) and sodium hydroxide (23.9 g) in water (60 mL) and methanol (75 mL). The mixture is stirred at 60 °C for 1 h and then cooled to room temperature. The reaction mixture is extracted with diethylether and the organic extracts are dried (MgSO₄). After removing the solvent, the residue is purified by chromatography on silica gel (dichloromethane/methanol 30:1->9:1) to give a mixture of the two title compounds (ca. 3:1).
Yield: 16.4 g (61% of theory)
Mass spectrum (ESI⁺): m/z = 216 [M+H]⁺

### Example V

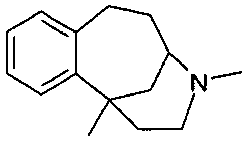

### 1,11-Dimethyl-11-aza-tricyclo[8,3,1,0*2,7*]tetradeca-2,4,6-triene

A mixture of 1,4-dimethyl-6-phenethyl-1,2,3,6-tetrahydro-pyridine and 1,4-dimethyl-2-phenethyl-1,2,3,6-tetrahydro-pyridine (ca. 3:1, 1.0 g) dissolved in polyphosphoric acid (5 mL) is stirred at 150 °C for 2 d. After cooling to ca. 80 °C, water (30 mL) is added and the mixture is stirred vigorously for another 5 min. Then, the mixture is cooled in an ice bath, more water is added, and the mixture is basified using 40% NaOH in water. The resulting mixture is extracted with ethyl acetate, the combined organic extracts are washed with brine and dried (MgSO₄). The solvent is removed under reduced pressure to yield the title compound.
Yield: 0.76 g (76% of theory)
Mass spectrum (ESI⁺): m/z = 216 [M+H]⁺

The following compound may be obtained analogously to Example V:

### (1) 1-Methyl-10-aza-tricyclo[7.2.1.0*2,7*]dodeca-2,4,6-triene

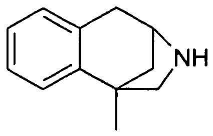

Mass spectrum (ESI⁺): m/z = 174 [M+H]⁺
2-Benzyl-4-methyl-2,5-dihydro-pyrrole-1-carboxylic acid tert-butyl ester is used as the starting material.

### Example VI

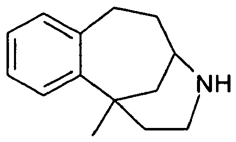

### 1-Methyl-11-aza-tricyclo[8.3.1.0*2,7*]tetradeca-2,4,6-triene

1-Chloroethyl chloroformate (3.8 mL) is added dropwise to a mixture of 1,11-dimethyl-11-aza-tricyclo[8.3.1.0*2,7*]tetradeca-2,4,6-triene (0.75 g) and NaHCO₃ (2.9 g) in 1,2-dichloroethane (3.5 mL) chilled in an ice bath. The reaction mixture is warmed to room temperature in the cooling bath and stirred overnight. Then, dichloromethane (20 mL) is added and the precipitate is removed by filtration. The filtrate is concentrated under reduced pressure and the residue is dissolved in methanol (20 mL). The resulting solution is stirred at reflux temperature for 2 h. After cooling to ambient temperature, the solution is concentrated and the residue is purified by HPLC (water/MeCN/NH₃) to give the title compound.
Yield: 0.11 g (16% of theory)

The following compounds may be obtained analogously to Example VI:

### (1) 11,11-Dimethyl-2,3,4,5-tetrahydro-1H-2,6-methano-benzo[d]azocin-6-ol

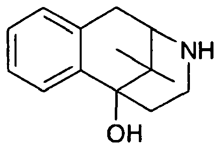

The starting material, 3,11,11-trimethyl-2,3,4,5-tetrahydro-1H-2,6-methanobenzo[d]azocin-6-ol, may be obtained in analogy to EP 28717 (1981) from 2-benzyl-1,3,3-trimethyl-piperidinone.

### (2) 8-Hydroxy-2,3,4,5-tetrahydro-1H-2,6-methano-benzo[d]azocine-6-carboxylic acid methyl ester

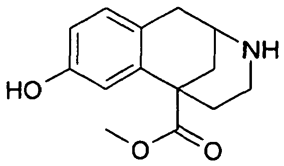

The starting material, 8-hydroxy-3-methyl-2,3,4,5-tetrahydro-1H-2,6-methano-benzo[d]azocine-6-carboxylic acid methyl ester, may be obtained in analogy to J. Med. Chem. 1962, 5, 357-361 and US 3687957 (1972) from 8-methoxy-3-methyl-1-oxo-2,3,4,5-tetrahydro-1H-2,6-methano-benzo[d]azocine-6-carbonitrile. The methoxy group on the aromatic ring may be cleaved by using boron tribromide in dichloromethane or hydrobromic acid in acetic acid (see e.g. J. Med. Chem. 1992, 35, 4135-4142; J. Med. Chem. 2004, 47, 165-174).

The starting material may also be obtained by reacting compound Example XXII(1) with boron tribromide in dichloromethane.

### (3) 11,11-Dimethyl-6-phenyl-1,2,3,4,5,6-hexahydro-2,6-methano-benzo[d]azocin-8-ol

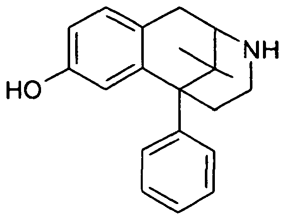

The starting material, 3,11,11-trimethyl-6-phenyl-1,2,3,4,5,6-hexahydro-2,6-methano-benzo[d]azocin-8-ol, may be obtained as described in DE 2027077 (1970).

### (4) 6-Propyl-1,2,3,4,5,6-hexahydro-2,6-methano-benzo[d]azocin-8-ol

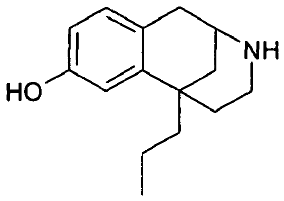

The starting material, 3-methyl-6-propyl-1,2,3,4,5,6-hexahydro-2,6-methano-benzo[d]azocin-8-ol, may be obtained as described in J. Med. Chem. 1963, 6, 322-5.

### (5) 6-Methyl-1,2,3,4,5,6-hexahydro-2,6-methano-benzo[d]azocin-8-ol

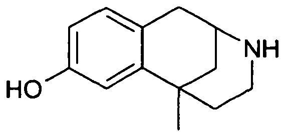

The starting material, 3,6-Dimethyl-1,2,3,4,5,6-hexahydro-2,6-methano-benzo[d]azocin-8-ol, may be obtained as described in J. Org. Chem. 1960, 25, 1386-8.

### Example VII

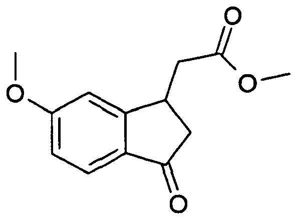

### (6-Methoxy-3-oxo-indan-1-yl)-acetic acid methyl ester

Concentrated sulfuric acid (3.0 mL) is added to 5-methoxy-1-indanone-3-acetic acid (13.0 g) dissolved in methanol (100 mL). The solution is stirred at reflux temperature for 4 h and then cooled to room temperature. About two third of the methanol is removed under reduced pressure and water (100 mL) and ethyl acetate (200 mL) are added to the remainder. The organic phase is separated and washed with water, 1 M NaOH solution, and brine. The organic phase is dried (MgSO₄) and the solvent is evaporated to give the product as a yellow oil.
Yield: 13.2 g (95% of theory)
Mass spectrum (ESI⁺): m/z = 235 (M+H]⁺

### Example VIII

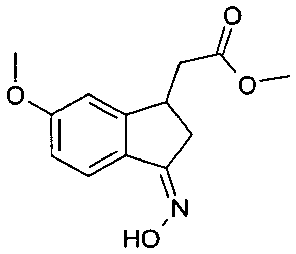

### (3-Hydroxyimino-6-methoxy-indan-1-yl)-acetic acid methyl ester

(6-Methoxy-3-oxo-indan-1-yl)-acetic acid methyl ester (12.0 g), hydroxylamine hydrochloride (4.6 g), and sodium acetate (5.5 g) dissolved in water (40 mL) and methanol (50 mL) are stirred at reflux temperature for 3 h. After cooling to room temperature, water (100 mL) is added and the solution is extracted with ethyl acetate. The combined organic extracts are washed with water and brine and dried (MgSO₄). The solvent is evaporated to give the product as a brown oil.
Yield: 12.5 g (98% of theory)
Mass spectrum (ESI⁺): m/z = 250 [M+H]⁺

### Example IX

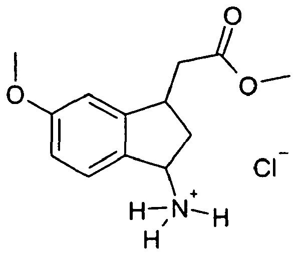

### 5-Methoxy-3-methoxycarbonylmethyl-indan-1-yl-ammonium chloride

A mixture of 10% palladium on carbon (3.0 g), (3-hydroxyimino-6-methoxy-indan-1-yl)-acetic acid methyl ester (12.5 g), and concentrated hydrochloric acid (4.7 mL) in methanol (150 mL) is stirred under hydrogen atmosphere at room temperature overnight. The mixture is filtered and the filtrate is concentrated under reduced pressure. The residue is azeotropically dried using toluene and triturated with diisopropylether to give the product as a white solid after drying at 50 °C.
Yield: 13.0 g (100% of theory)
Mass spectrum (ESI⁺): m/z = 236 [M+H]⁺ ([M+H]⁺ of (3-amino-6-methoxy-indan-1-yl)-acetic acid methyl ester)

### Example X

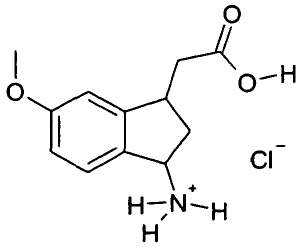

### 3-Carboxymethyl-5-methoxy-indan-1-yl-ammonium chloride

5-Methoxy-3-methoxycarbonylmethyl-indan-1-yl-ammonium chloride (12.5 g) dissolved in 2 M hydrochloric acid (120 mL) is stirred at reflux temperature for 3 h. Then, the solvent is removed and the residue is azeotropically dried using toluene and further purified by washing with diisopropylether. The product is dried at 50°C.
Yield: 11.8 g (100% of theory)
Mass spectrum (ESI⁺): m/z = 222 [M+H]⁺ ([M+H]⁺ of (3-amino-6-methoxy-indan-1-yl)-acetic acid)

### Example XI

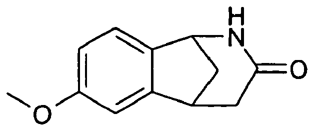

### 4-Methoxy-9-aza-tricyclo[6.3.1.0*2,7*]dodeca-2,4,6-trien-10-one

3-Carboxymethyl-5-methoxy-indan-1-yl-ammonium chloride (13.2 g) and 1-cyclohexyl-3-(2-morpholinoethyl)carbodiimide methyl-p-toluenesulfonate (21.7 g) dissolved in pyridine (500 mL) are stirred at room temperature for 7 d. Then, the pyridine is removed under reduced pressure and the residue is taken up in water (200 mL) and dichloromethane (200 mL). The organic phase is separated and the aqueous phase is extracted twice with dichloromethane. The combined organic phases are washed with 1 M hydrochloric acid, 1 M NaOH solution, and water. After drying (MgSO₄), the solvent is evaporated under reduced pressure to yield the product as a beige solid.
Yield: 3.0 g (29% of theory)
Mass spectrum (ESI⁺): m/z = 204 [M+H]⁺

### Example XII

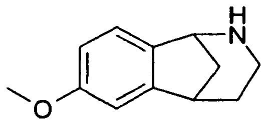

### 4-Methoxy-9-aza-tricyclo[6.3.1.0*2,7*]dodeca-2,4,6-triene

1 M Borane tetrahydrofuran complex (70 mL) is added dropwise to a solution of 4-methoxy-9-aza-tricyclo[6.3.1.0*2,7*]dodeca-2,4,6-trien-10-one (3.0 g) in THF (20 mL) chilled in an ice bath. The resulting solution is stirred at reflux temperature for 5 h and then at room temperature overnight. The solution is cooled to ca. -10 °C and half-concentrated hydrochloric acid (50 mL) is added carefully. The mixture is stirred at room temperature for 1 h and an additional hour at reflux temperature. The solvent is removed and 2 M NaOH solution (50 mL) is added to the residue. The resulting mixture is extracted with dichloromethane and the combined organic extracts are dried (MgSO₄). After removal of the solvent, the residue is taken up in ethanol (20 mL) and the resulting solution is treated with oxalic acid (3 mL) to obtain the oxalate salt of the title compound.
Yield: 0.8 g (19% of theory)
Mass spectrum (ESI⁺): m/z = 190 [M+H]⁺

### Example XIII

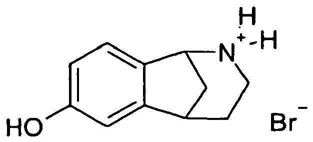

### 4-Hydroxy-9-azonia-tricyclo[6.3.1.0*2,7*]dodeca-2,4,6-triene bromide

A solution of 4-methoxy-9-aza-tricyclo[6.3.1.0*2,7*]dodeca-2,4,6-triene (0.50 g of oxalate salt) in hydrobromic acid (48% in water, 10 mL) is stirred at reflux temperature for 3 h. Then, the solution is concentrated under reduced pressure and the residue is azetropically dried using toluene and ethanol. The residue is washed with acetone and dried to give the product as a solid.
Yield: 0.23 g (49% of theory)
Mass spectrum (ESI⁺): m/z = 176 [M+H]⁺ (of free amine)

The following compound may be obtained analogously to Example XIII:

### (1) (2R,6S)-6,11,11-Trimethyl-1,2,3,4,5,6-hexahydro-2,6-methano-benzo[d]azocin-9-ol

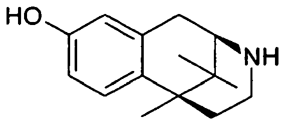

Mass spectrum (ESI⁺): m/z = 232 [M+H]⁺
The compound is prepared from (2*R*,6*S*)-9-methoxy-6,11,11-trimethyl-1,2,3,4,5,6-hexahydro-2,6-methano-benzo[d]azocine [tartaric acid salt, for preparation see WO 9959976] and isolated as the hydrogen bromide salt.

### Example XIV

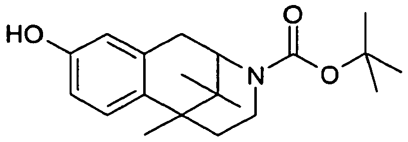

### 9-Hydroxy-6,11,11-trimethyl-1,2,5,6-tetrahydro-4H-2,6-methano-benzo[d]azocine-3-carboxylic acid tert-butyl ester

Di-*tert*butyl dicarbonate (8.7 g) is added to a solution of 6,11,11-trimethyl-1,2,3,4,5,6-hexahydro-2,6-methano-benzo[d]azocin-9-ol (12.0 g) and triethylamine (8 ml) in 1,4-dioxane (100 mL) and water (100 mL). The solution is stirred at room temperature overnight. Then, ethyl acetate is added and the organic phase is separated. The aqueous phase is extracted with ethyl acetate and the organic extract and phase are combined. The organic phase is washed with 1 M hydrochloric acid, water, and brine, and then dried (MgSO₄). After removal of the solvent under reduced pressure, the residue is crystallized from diisopropylether to give the title compound.
Yield: 6.5 g (51% of theory)
Mass spectrum (ESI⁺): m/z = 332 [M+H]⁺

The following compounds may be obtained analogously to Example XIV:

### (1) (2R,6S)-10-Hydroxy-6,11,11-trimethyl-1,2,5,6-tetrahydro-4H-2,6-methano-benzo[d]azocine-3-carboxylic acid tert-butyl ester

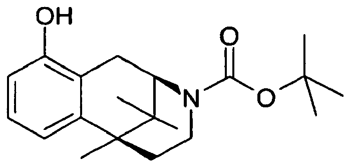

Mass spectrum (ESI⁺): m/z = 332 [M+H]⁺

### (2) (2R,6R,11 S)-8-Hydroxy-6,11-dimethyl-1,2,5,6-tetrahydro-4H-2,6-methano-benzo[d]azocine-3-carboxylic acid tert-butyl ester

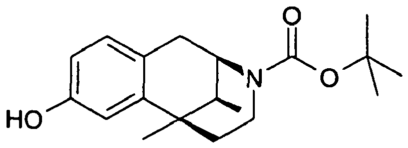

### (3) (2S,6R)-8-Hydroxy-6,9,11,11-tetramethyl-1,2,5,6-tetrahydro-4H-2,6-methano-benzo[d]azocine-3-carboxylic acid tert-butyl ester

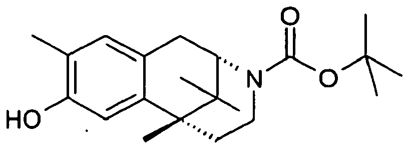

The compound may be obtained by resolution of the racemic mixture by HPLC on chiral phase

### (4) (2R,6S)-8-Hydroxy-6,9,11,11-tetramethyl-1,2,5,6-tetrahydro-4H-2,6-methano-benzo[d]azocine-3-carboxylic acid tert-butyl ester

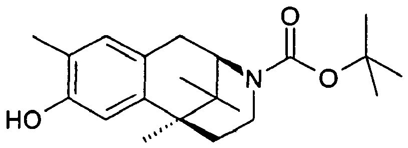

The compound may be obtained by resolution of the racemic mixture by HPLC on chiral phase

### (5) (2S,6R)-9-Hydroxy-6,8,11,11-tetramethyl-1,2,5,6-tetrahydro-4H-2,6-methano-benzo[d]azocine-3-carboxylic acid tert-butyl ester

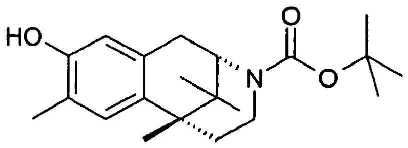

The compound may be obtained by resolution of the racemic mixture by HPLC on chiral phase

### (6) (2R,6S)-9-Hydroxy-6,8,11,11-tetramethyl-1,2,5,6-tetrahydro-4H-2,6-methano-benzo[d]azocine-3-carboxylic acid tert-butyl ester

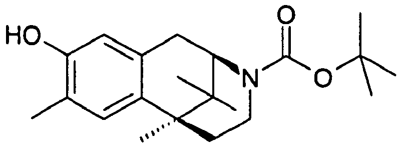

The compound may be obtained by resolution of the racemic mixture by HPLC on chiral phase

### (7) 8-Hydroxy-6,11,11-trimethyl-1,2,5,6-tetrahydro-4H-2,6-methano-benzo[d]azocine-3-carboxylic acid tert-butyl ester

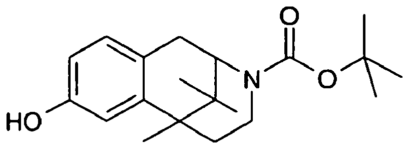

### 8) (2R,6S)-9-Hydroxy-6,11,11-trimethyl-1,2,5,6-tetrahydro-4H-2,6-methano-benzo[d]azocine-3-carboxylic acid tert-butyl ester

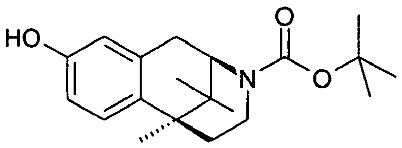

Mass spectrum (ESI⁺): m/z = 332 [M+H]⁺

The compound may be obtained by resolution of the racemic mixture by HPLC on chiral phase or by using the enantiomerically pure starting material that in turn may be obtained as described in Example XIII(1) or by resolution of the racemic mixture by HPLC on chiral phase. The synthesis of the racemic starting material is described in EP 521422 (1993).

### (9) 7-Hydroxy-6,11,11-trimethyl-1,2,5,6-tetrahydro-4H-2,6-methano-benzo[d]azocine-3-carboxylic acid tert-butyl ester

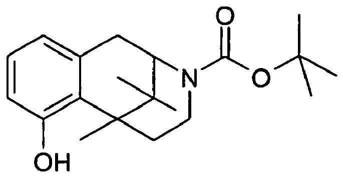

Mass spectrum (ESI⁺): m/z = 332 [M+H]⁺

### (10) (2R,6S)-8-Acetyl-6,11,11-trimethyl-1,2,5,6-tetrahydro-4H-2,6-methano-benzo[d]azocine-3-carboxylic acid tert-butyl ester

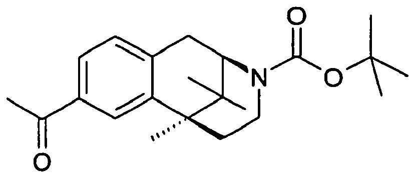

Mass spectrum (ESI⁺): m/z = 358 [M+H]⁺

### Example XV

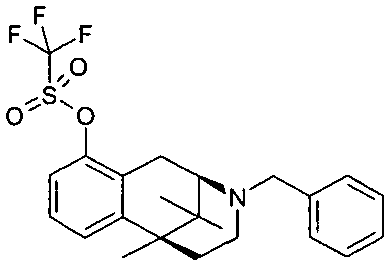

### (2R,6S)-Trifluoro-methanesulfonic acid 3-benzyl-6,11,11-trimethyl-1,2,3,4,5,6-hexahydro-2,6-methano-benzo[d]azocin-10-yl ester

Trifluoromethanesulfonic anhydride (9.7 mL) is added to a solution of (2*R*,6*S*)-3-benzyl-6,11,11-trimethyl-1,2,3,4,5,6-hexahydro-2,6-methano-benzo[d]azocin-10-ol (13.7 g), triethylamine (43 mL), and 4-dimethylaminopyridine (50 mg) in dichloromethane (135 mL) chilled to -10°C under argon atmosphere. The solution is stirred at ca. -5°C for 30 min and then at room temperature overnight. The solution is added to ice-cold water and then aqueous ammonia solution is added. The resulting mixture is extracted with dichloromethane and then the combined organic extracts are washed with water and dried (MgSO₄). The solvent is removed under reduced pressure to give the crude product that is used without further purification.
Yield: 18.0 g (93% of theory)
Mass spectrum (ESI⁺): m/z = 454 [M+H]⁺

The following compounds may be obtained analogously to Example XV:

### (1) 6,11,11-Trimethyl-9-trifluoromethanesulfonyloxy-1,2,5,6-tetrahydro-4H-2,6-methano-benzo[d]azocine-3-carboxylic acid tert-butyl ester

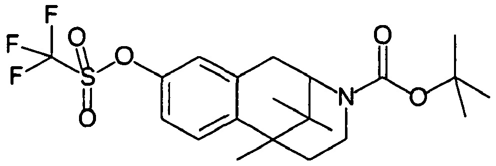

Mass spectrum (ESI⁺): m/z = 464 [M+H]⁺

### (2) (2R,6S)-6,11,11-Trimethyl-10-trifluoromethanesulfonyloxy-1,2,5,6-tetrahydro-4H-2,6-methano-benzo[d]azocine-3-carboxylic acid tert-butyl ester

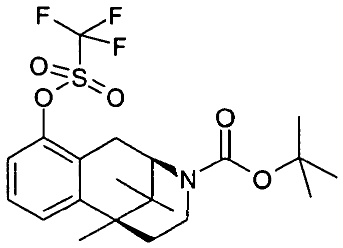

Mass spectrum (ESI⁺): m/z = 481 [M+NH₄]⁺

### (3) (2R,6R,11 S)-6,11-Dimethy)-8-trifluoromethanesulfonyloxy-1,2,5,6-tetrahydro-4H-2,6-methano-benzo[d]azocine-3-carboxylic acid tert-butyl ester

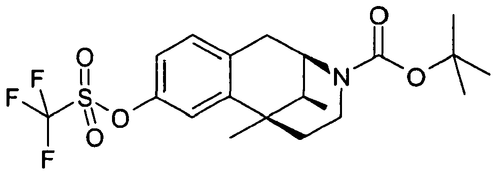

Mass spectrum (ESI⁺): m/z = 450 [M+H]⁺

### (4) 6,11,11-Trimethyl-8-trifluoromethanesulfonyloxy-1,2,5,6-tetrahydro-4H-2,6-methano-benzo[d]azocine-3-carboxylic acid tert-butyl ester

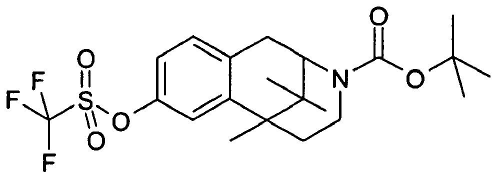

### (5) (2R,6S)-6,11,11-Trimethyl-9-trifluoromethanesulfonyloxy-1,2,5,6-tetrahydro-4H-2,6-methano-benzo[d]azocine-3-carboxylic acid tert-butyl ester

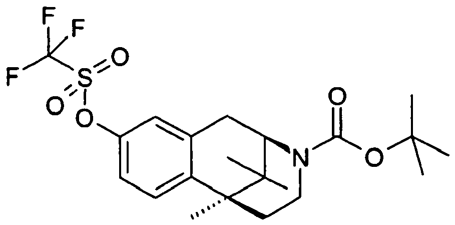

Mass spectrum (ESI⁺): m/z = 464 [M+H]⁺

### (6) (2R,6S)-Trifluoro-methanesulfonic acid 9-cyano-6,11,11-trimethyl-3-(piperidine-1-carbonyl)-1,2,3,4,5,6-hexahydro-2,6-methano-benzo[d]azocin-10-yl ester

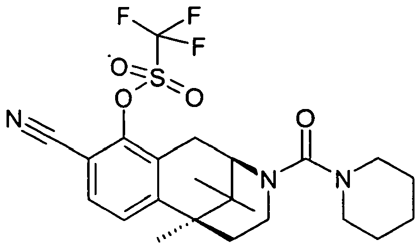

Mass spectrum (ESI⁺): m/z = 500 [M+H]⁺

The compound is prepared from end compound Example 35

### (7) (2R,6R,11R)-Trifluoro-methanesulfonic acid 9-cyano-6,11-dimethyl-3-(2,2,2-trifluoro-acetyl)-1,2,3,4,5,6-hexahydro-2,6-methano-benzo[d]azocin-8-yl ester

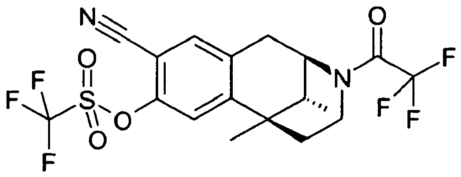

Mass spectrum (ESI⁺): m/z = 488 [M+NH₄]⁺

### (8) 6,11,11-Trimethyl-7-trifluoromethanesulfonyloxy-1,2,5,6-tetrahydro-4H-2,6-methano-benzo[d]azocine-3-carboxylic acid tert-butyl ester

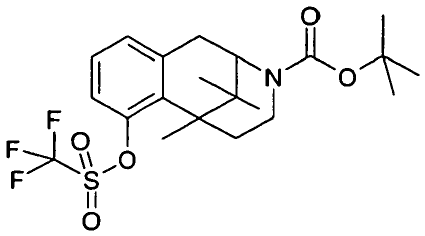

Mass spectrum (ESI⁺): m/z = 464 [M+H]⁺

### (9) Trifluoro-methanesulfonic acid (2R,6R,11 S)-6,11-dimethyl-3-(2,2,2-trifluoroacetyl)-1,2,3,4,5,6-hexahydro-2,6-methano-benzo[d]azocin-8-yl ester

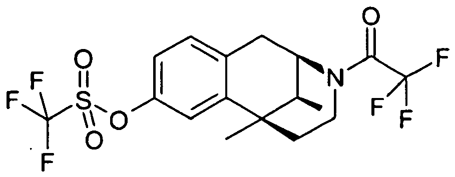

Mass spectrum (ESI⁺): m/z = 446 [M+H]⁺

### Example XVI

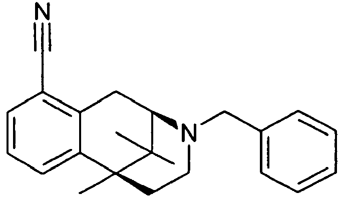

### (2R,6S)-3-Benzyl-6,11,11-trimethyl-1,2,3,4,5,6-hexahydro-2,6-methano-benzo[d]a zocine-10-carbonitrile

Tetrakis(triphenylphosphine)palladium(0) (2.79 g) is added to a mixture of (2*R*,6*S*)-trifluoro-methanesulfonic acid 3-benzyl-6,11,11-trimethyl-1,2,3,4,5,6-hexahydro-2,6-methano-benzo[d]azocin-10-yl ester (7.30 g) and zinc cyanide (2.85 g) in dimethylformamide (35 mL) kept in argon atmosphere. The resulting mixture is stirred at 100 °C for 6 h. After cooling to room temperature, water (300 mL), concentrated ammonia solution (10 mL), and ethyl acetate (150 mL) are added and the forming precipitate is separated by filtration. The organic layer of the filtrate is separated and the aqueous layer is extracted twice with ethyl acetate. The combined organic phases are washed with brine and dried (MgSO₄). The solvent is removed under reduced pressure and the residue is purified by chromatography on silica gel (cyclohexane/ethyl acetate 19:1) to give the product.
Yield: 4.43 g (62% of theory)
Mass spectrum **(ESI⁺):** m/z = 331 [M+H]⁺

The following compounds may be obtained analogously to Example XVI:

### (1) (2R,6R,11 S)-8-Cyano-6,11-dimethyl-1,2,5,6-tetrahydro-4H-2,6-methano-benzo[d]azocine-3-carboxylic acid tert-butyl ester

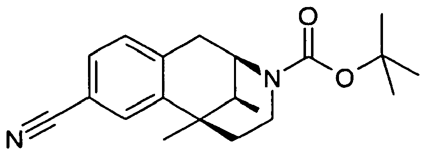

Mass spectrum (ESI⁺): m/z = 327 [M+NH₄]⁺

### (2) 9-Cyano-6,11,11-trimethyl-1,2,5,6-tetrahydro-4H-2,6-methano-benzo[d]azocine-3-carboxylic acid tert-butyl ester

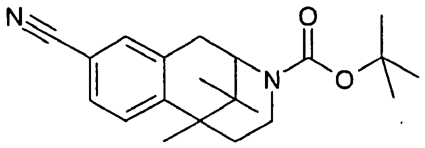

Mass spectrum (ESI⁺): m/z = 341 [M+H]⁺

### (3) (2R,6S)-9-Cyano-6,11,11-trimethyl-1,2,5,6-tetrahydro-4H-2,6-methano-benzo[d]azocine-3-carboxylic acid tert-butyl ester

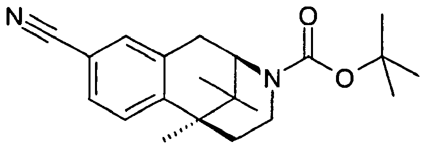

Mass spectrum (ESI⁺): m/z = 341 [M+H]⁺

### (4) 7-Cyano-6,11,11-trimethyl-1,2,5,6-tetrahydro-4H-2,6-methano-benzo[d]azocine-3-carboxylic acid tert-butyl ester

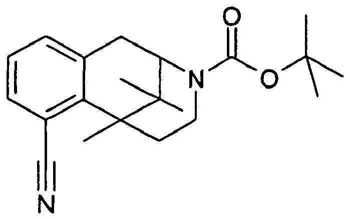

Mass spectrum (ESI⁺): m/z = 341 [M+H]⁺

### Example XVII

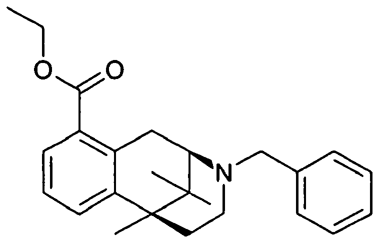

### (2R,6S)-3-Benzyl-6,11,11-trimethyl-1,2,3,4,5,6-hexahydro-2,6-methano-benzo[d]azocine-10-carboxylic acid ethyl ester

A solution of (2*R*,6*S*)-3-benzyl-6,11,11-trimethyl-1,2,3,4,5,6-hexahydro-2,6-methano-benzo[d]azocine-10-carbonitrile (1.14 g) in 80% sulfuric acid (4 mL) is stirred at 150 °C for 1 h. After cooling to room temperature, ethanol (30 mL) is added and the solution is stirred at 100 °C for 2 d. Then, the cooled solution is added to water (100 mL) and the mixture is basified using 40% aqueous NaOH solution. The resulting mixture is extracted twice with ethyl acetate and the combined extracts are dried (MgSO₄). The solvent is removed under reduced pressure to give the crude product.
Yield: 1.14 g (87% of theory)
Mass spectrum (ESI⁺): m/z = 378 [M+H]⁺

The following compounds may be obtained analogously to Example XVII:

### (1) 6,11,11-Trimethyl-1,2,3,4,5,6-hexahydro-2,6-methano-benzo[d]azocine-9-carboxylic acid ethyl ester

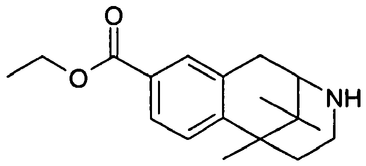

Mass spectrum (ESI⁺): m/z = 288 [M+H]⁺

The compound is prepared from 6,11,11-trimethyl-1,2,3,4,5,6-hexahydro-2,6-methano-benzo[d]azocine-9-carbonitrile applying the procedure described above.

### (2) (2R,6R,11 S)-6,11-Dimethyl-1,2,3,4,5,6-hexahydro-2,6-methano-benzo[d]azocine-8-carboxylic acid ethyl ester

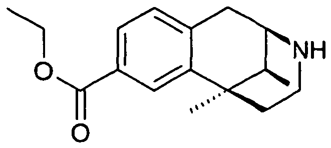

Mass spectrum (ESI⁺): m/z = 274 [M+H]⁺

The compound is prepared from (2*R*,6*R*,11*S*)-6,11-dimethyl-1,2,3,4,5,6-hexahydro-2,6-methano-benzo[d]azocine-8-carbonitrile applying the procedure described above.

### (3) 1-Hydroxy-8-methoxy-3-methyl-2,3,4,5-tetrahydro-1H-2,6-methano-benzo[d]azocine-6-carboxylic acid methyl ester

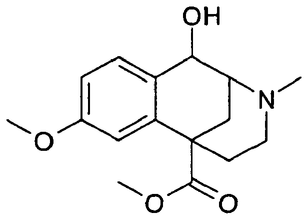

The compound may be prepared from 1-hydroxy-8-methoxy-3-methyl-2,3,4,5-tetrahydro-1H-2,6-methano-benzo[d]azocine-6-carbonitrile [for synthesis see US 3687957 (1972)] as described above using methanol instead of ethanol.

### Example XVIII

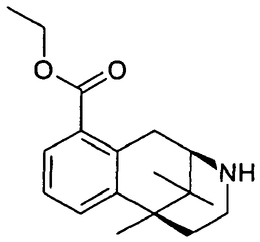

### (2R,6S)-6,11,11-Trimethyl-1,2,3,4,5,6-hexahydro-2,6-methano-benzo[d]azocine-10-carboxylic acid ethyl ester

Pd(OH)₂ (0.20 g) is added to a solution of (2*R*,6*S*)-3-benzyl-6,11,11-trimethyl-1,2,3,4,5,6-hexahydro-2,6-methano-benzo[d]azocine-10-carboxylic acid ethyl ester (1.13 g) in ethanol (20 mL). The resulting mixture is stirred under hydrogen atmosphere (50 psi) at room temperature overnight. Then, the catalyst is separated by filtration and the filtrate is concentrated under reduced pressure to give the product.
Yield: 0.61 g (71 % of theory)
Mass spectrum (ESI⁺): m/z = 288 [M+H]⁺

The following compounds may be obtained analogously to Example XVIII:

### (1) (2R,6S)-6,11,11-Trimethyl-1,2,3,4,5,6-hexahydro-2,6-methano-benzo[d]azocine-10-carbonitrile

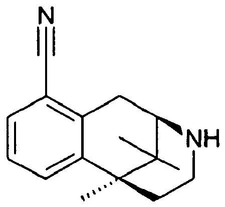

### (2) 2,3,4,5,6,7-Hexahydro-2,6-methano-1H-azocino[5,4-b]indole (racemic mixture of the diastereomer shown)

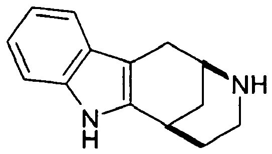

### (3) 5,6,7,8,9,10-Hexahydro-6,10-methano-pyrido[3,2-d]azocine (racemic mixture of the diastereomer shown)

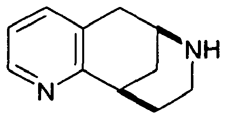

Mass spectrum (ESI⁺): m/z = 175 [M+H]⁺

The debenzylation is carried out in the presence of 1 equivalent of 1 M hydrochloric acid as described above.

### (4) 4-Methyl-3,5,9-triaza-tricyclo[6.3.1.0*2,6*]dodeca-2(6),4-diene (racemic mixture of the diastereomer shown)

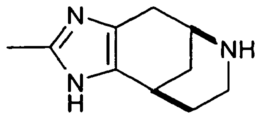

Mass spectrum (ESI⁺): m/z = 178 [M+H]⁺

### Example XIX

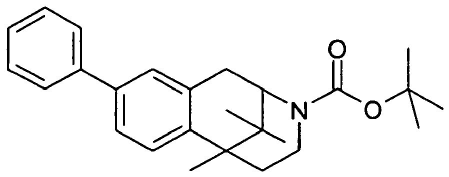

### 6,11,11-Trimethyl-9-phenyl-1,2,5,6-tetrahydro-4H-2,6-methano-benzo[d]azocine-3-carboxylic acid tert-butyl ester

Aqueous 2 M Na₂CO₃ solution (5 mL) is added to a mixture of 6,11,11-trimethyl-9-trifluoromethanesulfonyloxy-1,2,5,6-tetrahydro-4H-2,6-methano-benzo[d]azocine-3-carboxylic acid tert-butyl ester (1.00 g) and phenylboronic acid (0.34 g) in dimethylformamide (5 mL) in argon atmosphere. The resulting mixture is flushed with argon and then 1,1'-bis(diphenylphosphino)ferrocene-palladium(II) dichloride dichloromethane complex (0.18 g) is added. The mixture is heated to 100°C and stirred at this temperature for 4 h. After cooling to room temperature, water is added and the resulting mixture is extracted with ethyl acetate. The combined organic extracts are dried (MgSO₄) and the solvent is removed under reduced pressure. The residue is purified by chromatography on silica gel (cyclohexane/ethyl acetate 9:1->1:1) to give the product as a colorless oil.
Yield: 0.35 g (41 % of theory)
Mass spectrum (ESI⁺): m/z = 392 [M+H]⁺

The following compounds may be obtained in analogy to Example XIX:

### (1) (2R,6R,11S)-6,11-Dimethyl-8-phenyl-1,2,5,6-tetrahydro-4H-2,6-methano-benzo[d]azocine-3-carboxylic acid tert-butyl ester

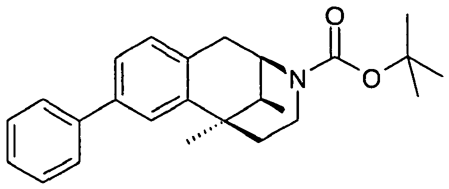

Mass spectrum (ESI⁺): m/z = 378 [M+H]⁺

### (2) (2R,6R,11S)-6,11-Dimethyl-8-pyridin-3-yl-1,2,5,6-tetrahydro-4H-2,6-methano-benzo[d]azocine-3-carboxylic acid tert-butyl ester

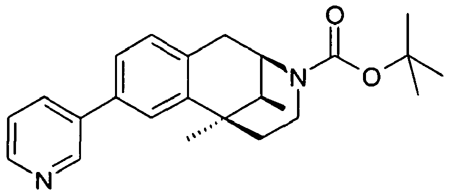

Mass spectrum (ESI⁺): m/z = 379 [M+H]⁺

### (3) (2R,6R,11S)-6,11-Dimethyl-8-pyridin-4-yl-1,2,5,6-tetrahydro-4H-2,6-methano-benzo[d]azocine-3-carboxylic acid tert-butyl ester

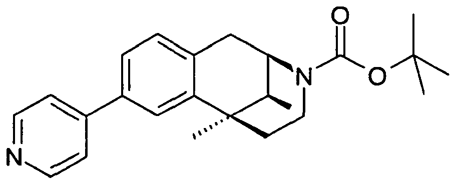

Mass spectrum (ESI⁺): m/z = 379 [M+H]⁺

### (4) (2R,6R,11S)-6,11-Dimethyl-8-pyrimidin-5-yl-1,2,5,6-tetrahydro-4H-2,6-methano-benzo[d]azocine-3-carboxylic acid tert-butyl ester

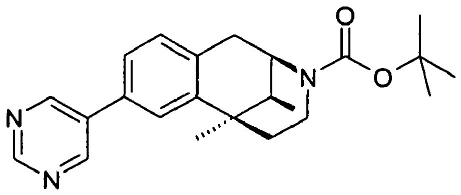

Mass spectrum (ESI⁺): m/z = 380 [M+H]⁺

### (5) 6,11,11 -Trimethyl-7-pyridin-3-yl-1,2,5,6-tetrahydro-4H-2,6-methano-benzo[d]azocine-3-carboxylic acid tert-butyl ester

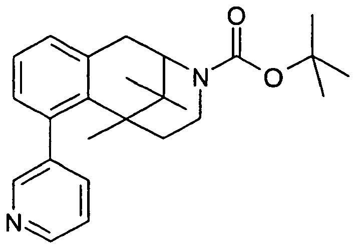

Mass spectrum (ESI⁺): m/z = 393 [M+H]⁺

### Example XX

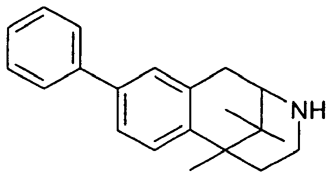

6,11,11-Trimethyl-9-phenyl-1,2,3,4,5,6-hexahydro-2,6-methano-benzo[d]azocine Trifluoroacetic acid (0.5 mL) is added to a solution of 6,11,11-trimethyl-9-phenyl-1,2,5,6-tetrahydro-4H-2,6-methano-benzo[d]azocine-3-carboxylic acid tert-butyl ester (0.30 g) in dichloromethane (2.5 mL). The solution is stirred at ambient temperature for 1 h and is then concentrated under reduced pressure. The crude trifluoroacetic acid salt of the title compound is used without further purification.
Yield: 0.31 g (100% of theory)

The transformation may also be carried out analogously using HCl in 1,4-dioxane or isopropanol instead of trifluoroacetic acid in dichloromethane.

The following compounds may be obtained analogously to Example XX:
(Alternatively, in cases in which the purity of the product after applying the procedure described above is insufficient, the compounds are purified by HPLC on reversed phase (MeCN/water) to obtain the pure compounds.)

### (1) (2R,6R,11S)-6,11-Dimethyl-1,2,3,4,5,6-hexahydro-2,6-methano-benzo[d]azocine-8-carbonitrile

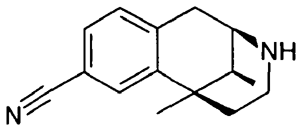

Mass spectrum (ESI⁺): m/z = 227 [M+H]⁺

The compound is obtained as its trifluoroacetic acid salt.

### (2) 6,11,11-Trimethyl-1,2,3,4,5,6-hexahydro-2,6-methano-benzo[d]azocine-9-carbonitrile

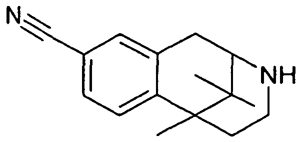

Mass spectrum (ESI⁺): m/z = 241 [M+H]⁺

The compound is obtained as its trifluoroacetic acid salt.

### (3) (2R,6S)-6,11,11-Trimethyl-1,2,3,4,5,6-hexahydro-2,6-methano-benzo[d]azocin-10-ylamine

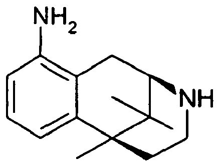

Mass spectrum (ESI⁺): m/z = 231 [M+NH₄]⁺

The compound is obtained as its double trifluoroacetic acid salt.

### (4) 6,11,11-Trimethyl-1,2,3,4,5,6-hexahydro-2,6-methano-benzo[d]azocin-9-ylamine

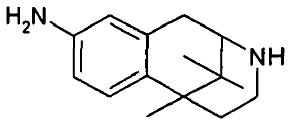

Mass spectrum (ESI⁺): m/z = 231 [M+H]⁺

The compound is obtained as its double trifluoroacetic acid salt.

### (5) (2S,6R)-8-Methoxy-6,9,11,11-tetramethyl-1,2,3,4,5,6-hexahydro-2,6-methano-benzo[d]azocine

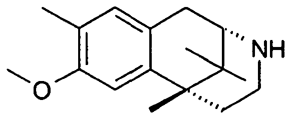

The compound may be obtained by resolution of the racemic mixture by HPLC on chiral phase or by using the enantiomerically pure (2*S*,6*R*)-8-methoxy-6,9,11,11-tetramethyl-1,2,5,6-tetrahydro-4H-2,6-methano-benzo[d]azocine-3-carboxylicacid tert-butyl ester.

### (6) (2R,6S)-8-Methoxy-6,9,11,11-tetramethyl-1,2,3,4,5,6-hexahydro-2,6-methano-benzo[d]azocine

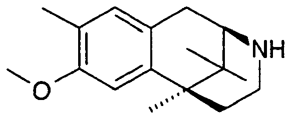

The compound may be obtained by resolution of the racemic mixture by HPLC on chiral phase or by using the enantiomerically pure (2*R*,6*S*)-8-methoxy-6,9,11,11-tetramethyl-1,2,5,6-tetrahydro-4H-2,6-methano-benzo[d]azocine-3-carboxylicacid tert-butyl ester.

### (7) (2S,6R)-9-Methoxy-6,8,11,11-tetramethyl-1,2,3,4,5,6-hexahydro-2,6-methano-benzo[d]azocine

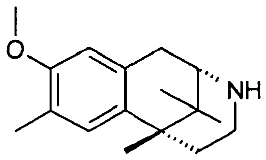

The compound may be obtained by resolution of the racemic mixture by HPLC on chiral phase or by using the enantiomerically pure (2*S*,6*R*)-9-methoxy-6,8,11,11-tetramethyl-1,2,5,6-tetrahydro-4H-2,6-methano-benzo[d]azocine-3-carboxylicacid tert-butyl ester.

### (8) (2R,6S)-9-Methoxy-6,8,11,11-tetramethyl-1,2,3,4,5,6-hexahydro-2,6-methano-benzo[d]azocine

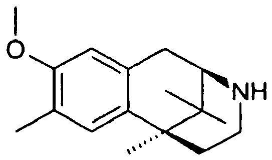

The compound may be obtained by resolution of the racemic mixture by HPLC on chiral phase or by using the enantiomerically pure (2*R*,6*S*)-9-methoxy-6,8,11,11-tetramethyl-1,2,5,6-tetrahydro-4H-2,6-methano-benzo[d]azocine-3-carboxylic acid tert-butyl ester.

### (9) 8,9-Dimethoxy-6,11,11-trimethyl-1,2,3,4,5,6-hexahydro-2,6-methano-benzo[d]azocine

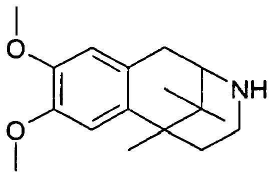

### (10) 8-Methoxy-6,11,11-trimethyl-1,2,3,4,5,6-hexahydro-2,6-methano-benzo[d]azocin-9-ol

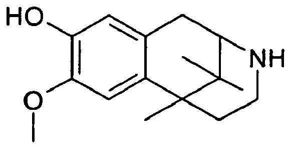

### (11) 9-Methoxy-6,1 1,1 1-trimethyl-1,2,3,4,5,6-hexahydro-2,6-methano-benzo[d]azocin-8-ol

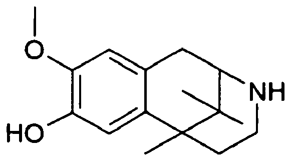

### (12) (2R,6S)-6,11,11-Trimethyl-1,2,3,4,5,6-hexahydro-2,6-methano-benzo[d]azocine-9-carbonitrile

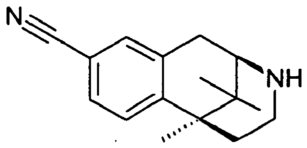

Mass spectrum (ESI⁺): m/z = 241 [M+H]⁺

The compound is obtained as its trifluoroacetic acid salt.

### (13) (2S,6R)-9-Methoxy-6,11,11-trimethyl-1,2,3,4,5,6-hexahydro-2,6-methano-benzo[d]azocine

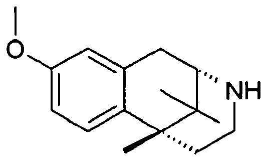

The compound may be obtained from the racemic mixture by HPLC on reversed phase.

### (14) (2R,6R,11S)-6,11-Dimethyl-8-phenyl-1,2,3,4,5,6-hexahydro-2,6-methano-benzo[d]azocine

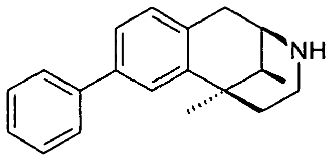

Mass spectrum (ESI⁺): m/z = 278 [M+H]⁺

### (15) (2R,6R,11S)-6,11-Dimethyl-8-pyridin-3-yl-1,2,3,4,5,6-hexahydro-2,6-methano-benzo[d]azocine

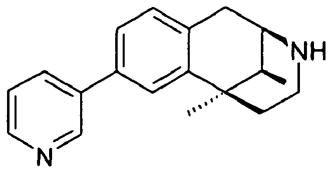

Mass spectrum (ESI⁺): m/z = 279 [M+H]⁺

### (16) (2R,6R,11S)-6,11-Dimethyl-8-pyridin-4-yl-1,2,3,4,5,6-hexahydro-2,6-methano-benzo[d]azocine

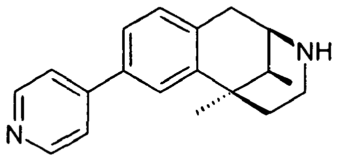

Mass spectrum (ESI⁺): m/z = 279 [M+H]⁺

### (17) (2R,6R, 11S)-6,11-Dimethyl-8-pyrimidin-5-yl-1,2,3,4,5,6-hexahydro-2,6-methano-benzo[d]azocine

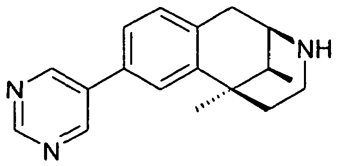

Mass spectrum (ESI⁺): m/z = 280 [M+H]⁺

### (18) 6,11,11-Trimethyl-7-pyridin-3-yl-1,2,3,4,5,6-hexahydro-2,6-methano-benzo[d]azocine

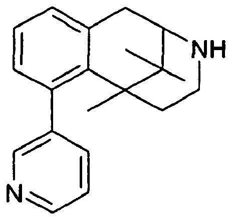

Mass spectrum (ESI⁺): m/z = 293 [M+H]⁺

### (19) 6,11,11-Trimethyl-1,2,3,4,5,6-hexahydro-2,6-methano-benzo[d]azocine-7-carbonitrile

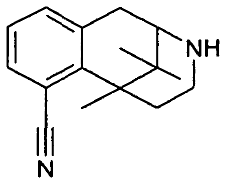

Mass spectrum (ESI⁺): m/z = 241 [M+H]⁺

### (20) 2,8-Diaza-spiro[5.5]undecan-1-one

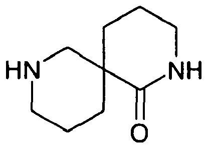

The compound is obtained as the HCl salt from 7-oxo-2,8-diaza-spiro[5.5]undecane-2-carboxylic acid tert-butyl ester using HCl in 1,4-dioxane.

### (21) (2R,6R,11S)-6,11-Dimethyl-8-(5-methyl-[1,3,4]oxadiazol-2-yl)-1,2,3,4,5,6-hexahydro-2,6-methano-benzo[d]azocine

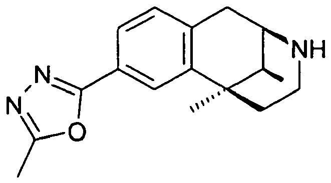

Mass spectrum (ESI⁺): m/z = 284 [M+H]⁺

The compound is isolated as its trifluoroacetic acid salt.

### (22) (2R,6R,11S)-6,11-Dimethyl-8-[1,3,4]oxadiazol-2-yl-1,2,3,4,5,6-hexahydro-2,6-methano-benzo[d]azocine

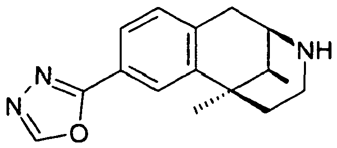

Mass spectrum (ESI⁺): m/z = 270 [M+H]⁺

### (23) 1,1,1-Trifluoro-2-[(2R,6S)-6,11,11-trimethyl-1,2,3,4,5,6-hexahydro-2,6-methano-benzo[d]azocin-8-yl]-propan-2-ol

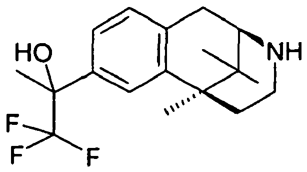

Mass spectrum (ESI⁺): m/z = 328 [M+H]⁺

### (24) (2R,6S)-9-Methanesulfonyl-6,11,11-trimethyl-1,2,3,4,5,6-hexahydro-2,6-ethano-benzo[d]azocine

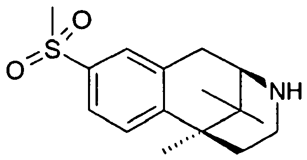

Mass spectrum (ESI⁺): m/z = 294 [M+H]⁺

### (25) (3-Phenyl-pyrrolidin-3-yl)-acetic acid methyl ester

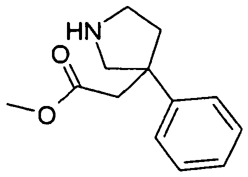

Mass spectrum (ESI⁺): m/z = 220 [M+H]⁺

The compound is obtained from 3-methoxycarbonylmethyl-3-phenyl-pyrrolidine-1-carboxylic acid tert-butyl ester employing the procedure described above.

### Example XXI

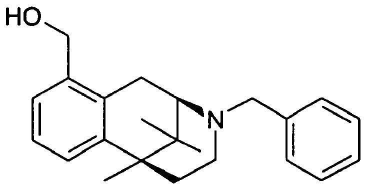

### [(2R,6S)-3-Benzyl-6,11,11-trimethyl-1,2,3,4,5,6-hexahydro-2,6-methano-benzo[d]azocin-10-yl]-methanol

A solution of (2*R*,6*S*)-3-benzyl-6,11,11-trimethyl-1,2,3,4,5,6-hexahydro-2,6-methano-benzo[d]azocine-10-carboxylic acid ethyl ester (0.96 g) in tetrahydrofuran (2 mL) is added dropwise to LiAlH₄ (1.6 mL, 2.4 mol/L in THF) in tetrahydrofuran (1.5 mL). The reaction mixture is stirred at ambient temperature for 90 min. Then, water (4 mL) is added carefully and the resulting mixture is extracted with ethyl acetate. The combined organic extracts are washed with water and brine and dried (MgSO₄). The solvent is removed under reduced pressure to give the product.
Yield: 0.62 g (72% of theory)
Mass spectrum (ESI⁺): m/z = 336 [M+H]⁺

### Example XXII

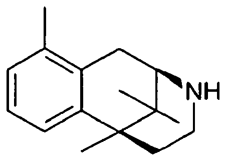

### (2R,6S)-6,10,11,11-Tetramethyl-1,2,3,4,5,6-hexahydro-2,6-methano-benzo[d]azocine

10% Palladium on carbon (0.10 g) is added to a solution of [(2R,6S)-3-benzyl-6,11,11-trimethyl-1,2,3,4,5,6-hexahydro-2,6-methano-benzo[d]azocin-10-yl]-methanol (0.60 g) in methanol (10 mL). The mixture is stirred under hydrogen atmosphere (50 psi) at room temperature overnight. Then, another portion of 10% palladium on carbon (0.2 g) and 4 M hydrochloric acid (1 mL) are added and the mixture is further stirred in hydrogen atmosphere for 4 h. After the catalyst is separated by filtration, the filtrate is concentrated under reduced pressure to give the hydrochloric acid salt of the title compound.
Yield: 0.50 g (100% of theory)

The following compound may be obtained analogously to Example XXII:

### (1) 8-Methoxy-3-methyl-2,3,4,5-tetrahydro-1H-2,6-methano-benzo[d]azocine-6-carboxylic acid methyl ester

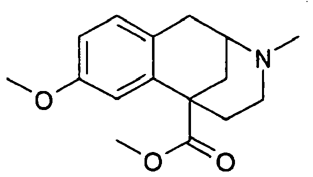

The compound may be obtained from 1-hydroxy-8-methoxy-3-methyl-2,3,4,5-tetrahydro-1H-2,6-methano-benzo[d]azocine-6-carboxylic acid methyl ester employing the procedure described above. Alternatively, the reduction may be conducted in analogy to J. Org. Chem. 1987, 52, 5233-5239.

### Example XXIII

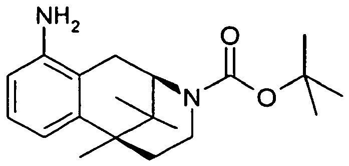

### (2R,6S)-10-Amino-6,11,11-trimethyl-1,2,5,6-tetrahydro-4H-2,6-methano-benzo[d]azocine-3-carboxylic acid tert-butyl ester

A flask charged with a stir bar, (2*R*,6*S*)-6,11,11-trimethyl-10-trifluoromethanesulfonyloxy-1,2,5,6-tetrahydro-4H-2,6-methano-benzo[d]azocine-3-carboxylic acid tert-butyl ester (4.0 g), benzhydrylideneamine (3.2 mL), Cs₂CO₃ (5.6 g), and toluene (80 mL) is flushed with argon for 10 min. Then, 2,2'-bis-diphenylphosphanyl-[1,1']binaphthalenyl (0.35 g) and tris(dibenzylideneacetone)dipalladium (0.18 g) are added and the resulting mixture is stirred at reflux temperature overnight. After cooling to room temperature, the reaction mixture is washed with water and concentrated. The residue is taken up in tetrahydrofuran and 2 M hydrochloric acid is added. The mixture is stirred at ambient temperature for 4 h. The precipitate is separated by filtration and the filtrate is concentrated under reduced pressure. The residue is purified by chromatography on silica gel (cyclohexane/ethyl acetate 1:7) to give the product as a brown oil.
Yield: 0.83 g (29% of theory)
Mass spectrum (ESI⁺): m/z = 331 [M+H]⁺

The following compounds may be obtained analogously to Example XXIII:

### (1) 9-Amino-6,11,11-trimethyl-1,2,5,6-tetrahydro-4H-2,6-methano-benzo[d]azocine-3-carboxylic acid tert-butyl ester

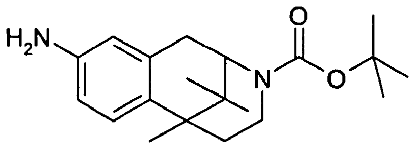

Mass spectrum (ESI⁺): m/z = 331 [M+H]⁺

### (2) 8-Amino-6,11,11-trimethyl-1,2,5,6-tetrahydro-4H-2,6-methano-benzo[d]azocine-3-carboxylic acid tert-butyl ester

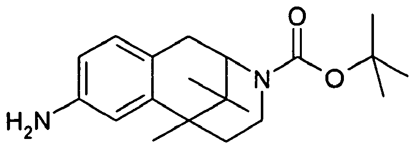

Mass spectrum (ESI⁺): m/z = 331 [M+H]⁺

### Example XXIV

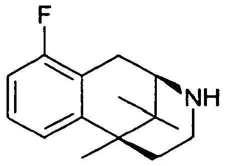

### (2R,6S)-10-Fluoro-6,11,11-trimethyl-1,2,3,4,5,6-hexahydro-2,6-methano-benzo[d]azocine

A solution of nitrosonium tetrafluoroborate (0.25 g) in 1,4-dioxane (2 mL) is added to a solution of (2*R*,6*S*)-10-amino-6,11,11-trimethyl-1,2,5,6-tetrahydro-4H-2,6-methano-benzo[d]azocine-3-carboxylic acid tert-butyl ester (0.10 g) in 1,4-dioxane (2 mL). The solution is heated to 50 °C and stirred at this temperature overnight. After cooling to ambient temperature, the reaction solution is diluted with methanol and then concentrated under reduced pressure. The residue is purified by HPLC on reversed phase (MeCN/H₂O/F₃CCO₂H) to yield the title product.
Yield: 25 mg (36% of theory)
Mass spectrum (ESI⁺): m/z = 234 [M+H]⁺

The following compounds may be obtained analogously to Example XXIV:

### (1) 8-Fluoro-6,11,11-trimethyl-1,2,3,4,5,6-hexahydro-2,6-methano-benzo[d]azocine

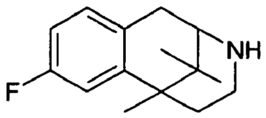

### (2) 9-Fluoro-6,11,11-trimethyl-1,2,3,4,5,6-hexahydro-2,6-methano-benzo[d]azocine

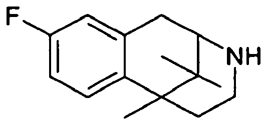

In cases in which the *tert*butyloxycarbonyl group is not completely cleaved off after the reaction the crude product is treated with trifluoroacetic acid in dichloromethane.

### Example XXV

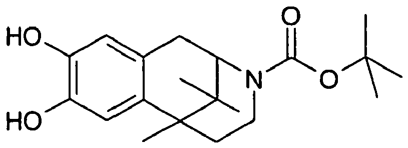

### 8,9-Dihydroxy-6,11,11-trimethyl-1,2,5,6-tetrahydro-4H-2,6-methano-benzo[d]azocine-3-carboxylic acid tert-butyl ester

Di-*tert*-butyl dicarbonate (0.34 g) is added to a solution of 6,11,11-trimethyl-1,2,3,4,5,6-hexahydro-2,6-methano-benzo[d]azocine-8,9-diol (0.44 g) and triethylamine (0.43 mL) in dichloromethane (5 mL). The solution is stirred at room temperature for 2 h. Then, the solution is washed twice with water and once with brine. After drying (MgSO₄), the solvent is removed under reduced pressure to yield the product.
Yield: 0.43 g (80% of theory)
Mass spectrum (ESI⁻): m/z = 346 [M-H]⁻

### Example XXVI

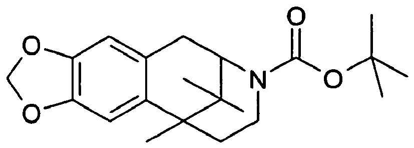

### 8,9-Methylenedioxy-6,11,11-trimethyl-1,2,5,6-tetrahydro-4H-2,6-methano-benzo[d]azocine-3-carboxylic acid tert-butyl ester

A mixture of 8,9-dihydroxy-6,11,11-trimethyl-1,2,5,6-tetrahydro-4H-2,6-methano-benzo[d]azocine-3-carboxylic acid tert-butyl ester (0.21 g), K₂CO₃ (0.19 g) and diiodomethane (54 µL) in dimethylformamide (5 mL) is heated to 100°C and stirred at this temperature for 2 h. Then, another portion of diiodomethane (54 µL) and K₂CO₃ (0.18 g) is added and the mixture is further stirred at 100°C for 5 h. After cooling to room temperature, water is added and the resulting mixture is extracted with ethyl acetate. The combined organic extracts are washed with brine and dried (MgSO₄). After removal of the solvent, the residue is purified by chromatography on silica gel (cyclohexane/ethyl acetate 1:1).
Yield: 0.20 g (93% of theory)
Mass spectrum (ESI⁺): m/z = 360 [M+H]⁺

### Example XXVII

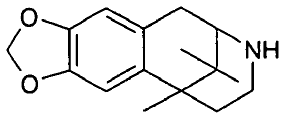

### 8,9-Methylenedioxy-6,11,11-trimethyl-1,2,3,4,5,6-hexahydro-2,6-methano-benzo[d]azocine

Isopropanolic hydrochloric acid (5 mol/L, 0.55 mL) is added to 8,9-methylenedioxy-6,11,11-trimethyl-1,2,5,6-tetrahydro-4H-2,6-methano-benzo[d]azocine-3-carboxylic acid tert-butyl ester (0.19 g) dissolved in dichloromethane (2 mL). The resulting solution is stirred for 2 h at room temperature. Then, the solution is concentrated under reduced pressure to give the title product as its hydrochloric acid salt.
Yield: 0.15 g (97% of theory)
Mass spectrum (ESI⁺): m/z = 260 [M+H]⁺

### Example XXVIII

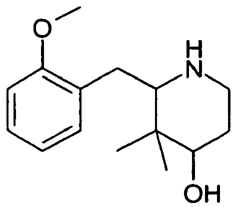

### 2-(2-Methoxy-benzyl)-3,3-dimethyl-piperidin-4-ol

Sodium borohydride (0.31 g) is added to 2-(2-methoxy-benzyl)-3,3-dimethyl-piperidin-4-one (2.00 g, prepared according to J. Med. Chem. 2002, 45, 3755-3765 from racemic starting material) dissolved in methanol (20 mL). The solution is stirred for 3 h at room temperature and then 1 M sodium hydroxide solution (40 mL) is added. After stirring for 10 min, the mixture is extracted with dichloromethane. The combined organic extracts are washed with water and dried (MgSO₄). The solvent is evaporated to give the title product.
Yield: 2.00 g (99% of theory)
Mass spectrum (ESI⁺): m/z = 250 [M+H]⁺

### Example XXIX

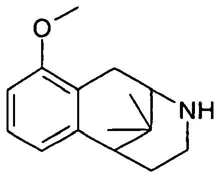

### 10-Methoxy-11,11-dimethyl-1,2,3,4,5,6-hexahydro-2,6-methano-benzo[d]azocine

A solution of 2-(2-methoxy-benzyl)-3,3-dimethyl-piperidin-4-ol (0.80 g) in polyphosphoric acid (10 mL) is stirred at 120°C overnight. After cooling the solution to ca. 80°C, water (300 mL) is added and the mixture is stirred vigorously for another 10 min. Then, the mixture is cooled in an ice bath, more water is added, and the mixture is basified using 10 M aqueous NaOH. The resulting mixture is extracted with ethyl acetate and the combined organic extracts are washed with brine and dried (MgSO₄). The solvent is removed under reduced pressure to yield the title product that is used without further purification.
Yield: 0.36 g (49% of theory)

The following compound may be obtained analogously to Example XXIX:

### (1) (2S,6R)-9-Methoxy-6,11,11-trimethyl-1,2,3,4,5,6-hexahydro-2,6-methano-benzo[d]azocine

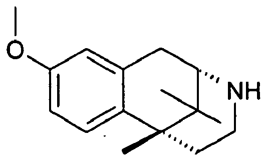

The racemic product mixture is resolved into its enantiomers by using HPLC on chiral phase.

The compound may also be obtained in analogy to the procedure described in J. Med. Chem. 1997, 40, 2922-2930.

### Example XXX

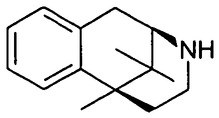

### (2R,6S)-6,11,11-Trimethyl-1,2,3,4,5,6-hexahydro-2,6-methano-benzo[d]azocine

10% Pd/C (0.20 g) is added to a solution of (2*R*,6*S*)-trifluoro-methanesulfonic acid 3-benzyl-6,11,11-trimethyl-1,2,3,4,5,6-hexahydro-2,6-methano-benzo[d]azocin-10-yl ester (0.50 g) in ethanol (10 mL). The resulting mixture is shaken under hydrogen atmosphere (50 psi) at room temperature overnight. Then, the catalyst is separated by filtration and Pd(OH)₂ (0.2 g) is added to the filtrate (the benzyl group was not completely removed after the treatment in the presence of Pd/C). The mixture is shaken for another 16 h in hydrogen atmosphere (50 psi) at room temperature. The catalyst is separated and the filtrate is concentrated under reduced pressure to give the crude product that is used without further purification.
Yield: 0.23 g (98% of theory)

The following compound may be obtained analogously to Example XXX:

### (1) 3,5,9-Triaza-tricyclo[6.3.1.0*2,6*]dodeca-2(6),4-diene (racemic mixture of the diastereomer shown)

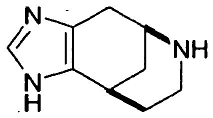

### Example XXXI

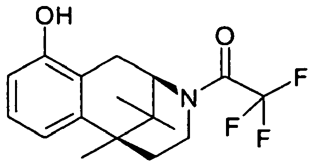

### 2,2,2-Trifluoro-1-[(2R,6S)-10-hydroxy-6,11,11-trimethyl-1,2,5,6-tetrahydro-4H-2,6-methano-benzo[d]azocin-3-yl]-ethanone

Trifluoroacetic anhydride (5.0 mL) is added to a solution of the hydrobromic acid salt of (2*R*,2*S*)-6,11,11-trimethyl-1,2,3,4,5,6-hexahydro-2,6-methano-benzo[d]azocin-10-ol (5.0 g) and triethylamine (5.5 mL) in dichloromethane (50 mL) chilled in an ice bath. The resulting solution is stirred at ambient temperature overnight. Then, water is added, the resulting mixture is stirred for an additional 15 min, and the organic phase is separated. The organic phase is washed with water and brine, dried (Na₂SO₄), and the solvent is evaporated. The residue is purified by chromatography on silica gel (ethyl acetate/cyclohexane 1:4) to give the product as a foam-like solid.
Yield: 3.34 g (64% of theory)
Mass spectrum (ESI⁺): m/z = 328 [M+H]⁺

The following compounds may be obtained analogously to Example XXXI:

### (1) 2,2,2-Trifluoro-1-[(2R,6S)-6,11,11-trimethyl-1,2,5,6-tetrahydro-4H-2,6-methano-benzo[d]azocin-3-yl]-ethanone

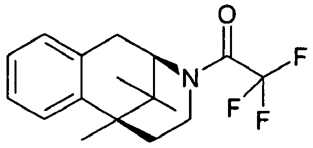

Mass spectrum (ESI⁺): m/z = 312 [M+H]⁺

### (2) 2,2,2-Trifluoro-1-[(2R,6R,11S)-8-hydroxy-6,11-dimethyl-1,2,5,6-tetrahydro-4H-2,6-methano-benzo[d]azocin-3-yl]-ethanone

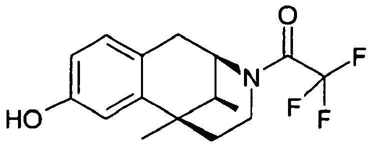

Mass spectrum (ESI⁺): m/z = 314 [M+H]⁺

### (3) 2,2,2-Trifluoro-1-[(2R,6R,11R)-8-hydroxy-6,11-dimethyl-1,2,5,6-tetrahydro-4H-2,6-methano-benzo[d]azocin-3-yl]-ethanone

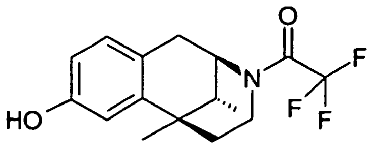

Mass spectrum (ESI⁺): m/z = 314 [M+H]⁺

### (4) 2,2,2-Trifluoro-1-[(2R,6S)-9-hydroxy-6,11,11-trimethyl-1,2,5,6-tetrahydro-4H-2,6-methano-benzo[d]azocin-3-yl]-ethanone

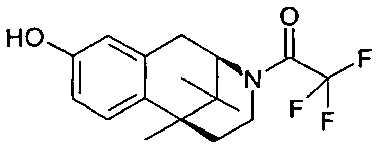

### Example XXXII

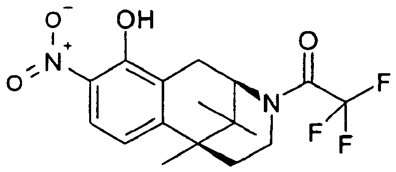

### 2,2,2-Trifluoro-1-[(2R,6S)-10-hydroxy-6,11,11-trimethyl-9-nitro-1,2,5,6-tetrahydro-4H-2,6-methano-benzo[d]azocin-3-yl]-ethanone

Nitric acid (0.4 mL) is slowly added to a solution of 2,2,2-trifluoro-1-[(2*R,*6*S*)-10-hydroxy-6,11,11-trimethyl-1,2,5,6-tetrahydro-4H-2,6-methano-benzo[d]azocin-3-yl]-ethanone (2.9 g) in acetic acid (5 mL) chilled in an ice bath. The ice bath is removed and the solution is stirred at ambient temperature overnight. The solution is poured into ice-cold water and the resulting mixture is extracted with ethyl acetate. The combined extracts are washed with brine and dried (Na₂SO₄). After removal of the solvent under reduced pressure, the residue is purified by chromatography on silica gel (ethyl acetate/cyclohexane 1:9->1:3).
Yield: 1.3 g (39% of theory)
Mass spectrum (ESI⁻): m/z = 371 [M-H]⁻

The following compounds may be obtained analogously to Example XXXII:

### (1) 2,2,2-Trifluoro-1-[(2R,6R,11S)-8-hydroxy-6,11-dimethyl-9-nitro-1,2,5,6-tetrahydro-4H-2,6-methano-benzo[d]azocin-3-yl]-ethanone

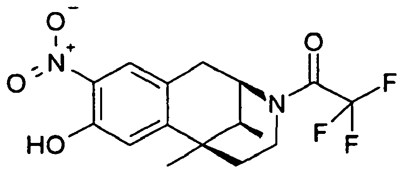

Mass spectrum (ESI⁺): m/z = 359 [M+H]⁺

### (2) 2,2,2-Trifluoro-1-[(2R,6R,11S)-8-hydroxy-6,11-dimethyl-7-nitro-1,2,5,6-tetrahydro-4H-2,6-methano-benzo[d]azocin-3-yl]-ethanone

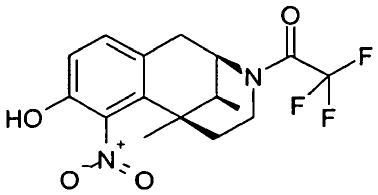

Mass spectrum (ESI⁺): m/z = 359 [M+H]⁺

The compound is obtained in a mixture with compound Example XXXII(1) that is separated by chromatography as described above.

### (3) 2,2,2-Trifluoro-1-[(2R,6S)-9-hydroxy-6,11,11-trimethyl-8-nitro-1,2,5,6-tetrahydro-4H-2,6-methano-benzo[d]azocin-3-yl]-ethanone

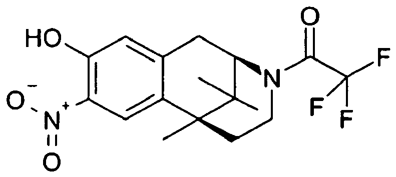

Mass spectrum (ESI⁺): m/z = 373 [M+H]⁺

The compound is obtained in a mixture with compound Example XXXII(4) that is separated by chromatography as described above.

### (4) 2,2,2-Trifluoro-1-[(2R,6S)-9-hydroxy-6,11,11-trimethyl-10-nitro-1,2,5,6-tetrahydro-4H-2,6-methano-benzo[d]azocin-3-yl]-ethanone

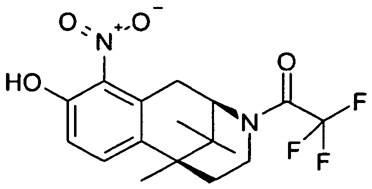

Mass spectrum (ESI⁺): m/z = 373 [M+H]⁺

The compound is obtained in a mixture with compound Example XXXII(3) that is separated by chromatography as described above.

### Example XXXIII

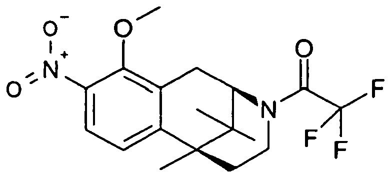

### 2,2,2-Trifluoro-1-[(2R,6S)-10-methoxy-6,11,11-trimethyl-9-nitro-1,2,5,6-tetrahydro-4H-2,6-methano-benzo[d]azocin-3-yl]-ethanone

Methyl iodide (80 µL) is added to a mixture of 2,2,2-trifluoro-1-[(2*R*,6*S*)-10-hydroxy-6,11,11-trimethyl-9-nitro-1,2,5,6-tetrahydro-4H-2,6-methano-benzo[d]azocin-3-yl]-ethanone (0.40 g) and potassium carbonate (0.17 g) in dimethylformamide (5 mL). The mixture is stirred at room temperature overnight, before another portion of methyl iodide (80 µL) and potassium carbonate (0.16 g) are added. The mixture is stirred for another 6 h at room temperature. Then, water and ethyl acetate are added, the organic phase is separated, and the aqueous phase is extracted with ethyl acetate. The combined organic phases are washed with brine and dried (Na₂SO₄). The solvent is evaporated to give the crude product that is used without further purification.
Yield: 0.41 g (100% of theory)
Mass spectrum (ESI⁺): m/z = 387 [M+H]⁺

The following compounds may be obtained analogously to Example XXXIII:

### (1) (2S,6R)-8-Methoxy-6,9,11,11-tetramethyl-1,2,5,6-tetrahydro-4H-2,6-methano-benzo[d]azocine-3-carboxylic acid tert-butyl ester

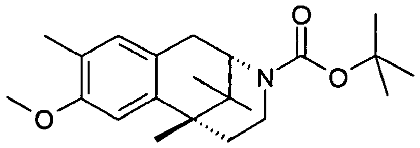

The compound may be obtained by resolution of the racemic mixture by HPLC on chiral phase or by using the enantiomerically pure (2*S*,6*R*)-8-hydroxy-6,9,11,11-tetramethyl-1,2,5,6-tetrahydro-4H-2,6-methano-benzo[d]azocine-3-carboxylic acid tert-butyl ester.

### (2) (2R,6S)-8-Methoxy-6,9,11,11-tetramethyl-1,2,5,6-tetrahydro-4H-2,6-methano-benzo[d]azocine-3-carboxylic acid tert-butyl ester

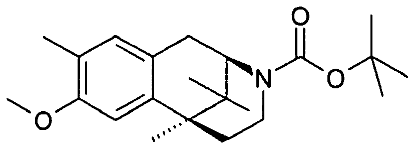

The compound may be obtained by resolution of the racemic mixture by HPLC on chiral phase or by using the enantiomerically pure (2*R*,6*S*)-8-hydroxy-6,9,11,11-tetramethyl-1,2,5,6-tetrahydro-4H-2,6-methano-benzo[d]azocine-3-carboxylic acid tert-butyl ester.

### (3) (2S,6R)-9-Methoxy-6,8,11,11-tetramethyl-1,2,5,6-tetrahydro-4H-2,6-methano-benzo[d]azocine-3-carboxylic acid tert-butyl ester

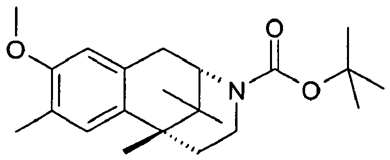

The compound may be obtained by resolution of the racemic mixture by HPLC on chiral phase or by using the enantiomerically pure (2*S*,6*R*)-9-hydroxy-6,8,11,11-tetramethyl-1,2,5,6-tetrahydro-4H-2,6-methano-benzo[d]azocine-3-carboxylicacid tert-butyl ester.

### (4) (2R,6S)-9-Methoxy-6,8,11,11-tetramethyl-1,2,5,6-tetrahydro-4H-2,6-methano-benzo[d]azocine-3-carboxylic acid tert-butyl ester

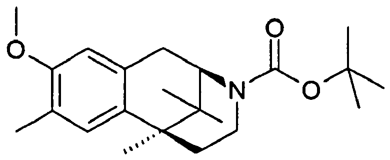

The compound may be obtained by resolution of the racemic mixture by HPLC on chiral phase or by using the enantiomerically pure (2*R*,6*S*)-9-hydroxy-6,8,11,11-tetramethyl-1,2,5,6-tetrahydro-4H-2,6-methano-benzo[d]azocine-3-carboxylic acid tert-butyl ester.

### (5) 8,9-Dimethoxy-6,11,11-trimethyl-1,2,5,6-tetrahydro-4H-2,6-methano-benzo[d]azocine-3-carboxylic acid tert-butyl ester

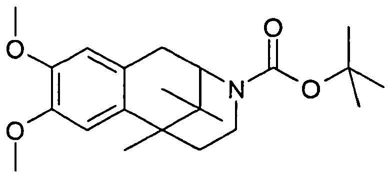

Twice the amount of methyl iodide and potassium carbonate as described in the procedure above are employed to prepare the compound from 8,9-dihydroxy-6,11,11-trimethyl-1,2,5,6-tetrahydro-4H-2,6-methano-benzo[d]azocine-3-carboxylic acid tert-butyl ester.

### (6) 9-Hydroxy-8-methoxy-6,11,11-trimethyl-1,2,5,6-tetrahydro-4H-2,6-methano-benzo[d]azocine-3-carboxylic acid tert-butyl ester

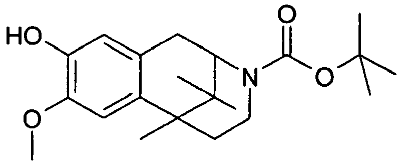

The compound is obtained in a mixture with 8-hydroxy-9-methoxy-6,11,11-trimethyl-1,2,5,6-tetrahydro-4H-2,6-methano-benzo[d]azocine-3-carboxylicacid tert-butyl ester and 8,9-dimethoxy-6,11,11-trimethyl-1,2,5,6-tetrahydro-4H-2,6-methano-benzo[d]azocine-3-carboxylic acid tert-butyl ester from 8,9-dihydroxy-6,11,11-trimethyl-1,2,5,6-tetrahydro-4H-2,6-methano-benzo[d]azocine-3-carboxylic acid tert-butyl ester that may be separated by HPLC on reversed phase (MeCN/H₂O).

### (7) 8-Hydroxy-9-methoxy-6,11,11-trimethyl-1,2,5,6-tetrahydro-4H-2,6-methano-benzo[d]azocine-3-carboxylic acid tert-butyl ester

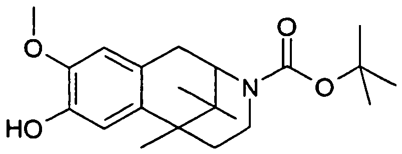

The compound is obtained in a mixture with 9-hydroxy-8-methoxy-6,11,11-trimethyl-1,2,5,6-tetrahydro-4H-2,6-methano-benzo[d]azocine-3-carboxylicacid tert-butyl ester and 8,9-dimethoxy-6,11,11-trimethyl-1,2,5,6-tetrahydro-4H-2,6-methano-benzo[d]azocine-3-carboxylic acid tert-butyl ester from 8,9-dihydroxy-6,11,11-trimethyl-1,2,5,6-tetrahydro-4H-2,6-methano-benzo[d]azocine-3-carboxylic acid tert-butyl ester that may be separated by HPLC on reversed phase (MeCN/H₂O).

### (8) 9-Methoxy-6,11,11-trimethyl-1,2,3,4,5,6-hexahydro-2,6-methano-benzo[d]azocine

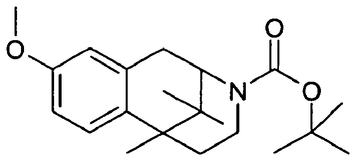

### (9) (2S,6R)-9-Methoxy-6,11,11-trimethyl-1,2,3,4,5,6-hexahydro-2,6-methano-benzo[d]azocine

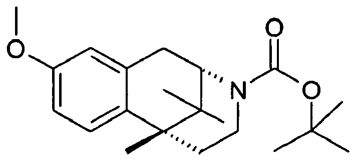

The compound may be obtained from the racemic mixture by HPLC on chiral phase.

### (10) 2,2,2-Trifluoro-1-[(2R,6R,11S)-8-methoxy-6,11-dimethyl-9-nitro-1,2,5,6-tetrahydro-4H-2,6-methano-benzo[d]azocin-3-yl]-ethanone

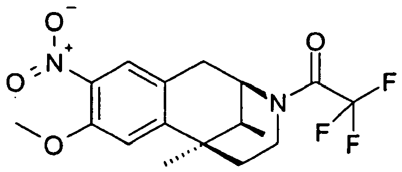

Mass spectrum (ESI⁺): m/z = 373 [M+H]⁺

### (11) 2,2,2-Trifluoro-1-[(2R,6R,11S)-8-methoxy-6,11-dimethyl-7-nitro-1,2,5,6-tetrahydro-4H-2,6-methano-benzo[d]azocin-3-yl]-ethanone

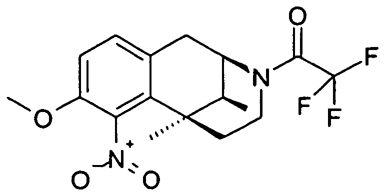

Mass spectrum (ESI⁺): m/z = 373 [M+H]⁺

### (12) 3-Methoxycarbonylmethyl-3-phenyl-pyrrolidine-1-carboxylic acid tert-butyl ester

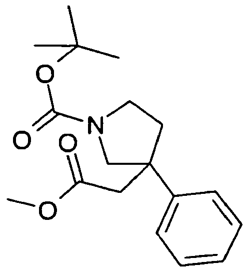

Mass spectrum (ESI⁺): m/z = 320 [M+H]⁺

The compound is obtained from 3-carboxymethyl-3-phenyl-pyrrolidine-1-carboxylic acid tert-butyl ester employing the procedure described above.

### Example XXXIV

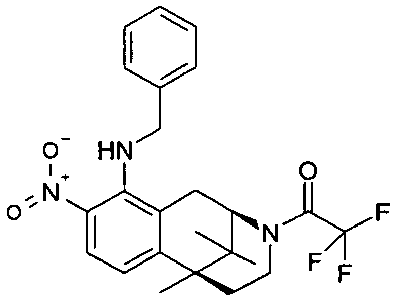

### 1-[(2R,6S)-10-Benzylamino-6,11,11-trimethyl-9-nitro-1,2,5,6-tetrahydro-4H-2,6-methano-benzo[d]azocin-3-yl]-2,2,2-trifluoro-ethanone

2,2,2-Trifluoro-1-[(2*R*,6*S*)-10-methoxy-6,11,11-trimethyl-9-nitro-1,2,5,6-tetrahydro-4H-2,6-methano-benzo[d]azocin-3-yl]-ethanone (0.41 g) is combined with benzylamine (0.7 mL) and the resulting mixture is stirred at 70°C overnight. After cooling to room temperature, the mixture is purified by HPLC on reversed phase (MeCN/H₂O/F₃CCO₂H) to give the product as an oil.
Yield: 0.19 g (38% of theory)
Mass spectrum (ESI⁺): m/z = 462 [M+H]⁺

The following compound may be obtained analogously to Example XXXIV:

### (1) 1-[(2R,6R,11S)-8-Benzylamino-6,11-dimethyl-9-nitro-1,2,5,6-tetrahydro-4H-2,6-methano-benzo[d]azocin-3-yl]-2,2,2-trifluoro-ethanone

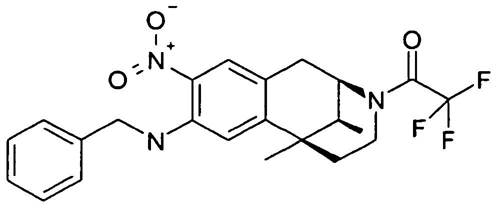

The reaction mixture is stirred at 170°C for 5 h.

### Example XXXV

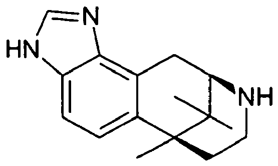

### (5R,9S)-4,5,6,7,8,9-hexahydro-9,12,12-trimethyl-5,9-methano-1H-imidazo[5,4-i][3]benzazocine

A mixture of Raney-Ni (0.1 g), [(2*R*,6*S*)-1-10-benzylamino-6,11,11-trimethyl-9-nitro-1,2,5,6-tetrahydro-4H-2,6-methano-benzo[d]azocin-3-yl]-2,2,2-trifluoro-ethanone (0.19 g), and formic acid (10 mL) is stirred in hydrogen atmosphere at 50 °C overnight. Then, the catalyst is separated by filtration and the filtrate is concentrated. The remainder is taken up in methanol (10 mL) and treated with 4 M NaOH solution (2 mL) at 50 °C overnight. After cooling to room temperature, the solution is neutralized with 2 M hydrochloric acid and the solvent is removed. The residue is purified by HPLC on reversed phase (MeCN/H₂O).
Yield: 35 mg (33% of theory)

The following compound may be obtained analogously to Example XXXV:

### (1) (6R,10R,12S)-5,6,7,8,9,10-Hexahydro-10,12-dimethyl-6,10-methano-1H-imidazo[5,4-i][3]benzazocine

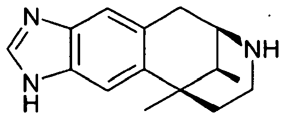

Mass spectrum (ESI⁺): m/z = 242 [M+H]⁺

### Example XXXVI

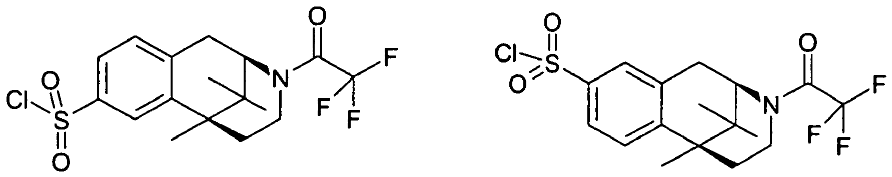

### (2R,6S)-6,11,11-Trimethyl-3-(2,2,2-trifluoro-acetyl)-1,2,3,4,5,6-hexahydro-2,6-methano-benzo[d]azocine-8-sulfonyl chloride and (2R,6S)-6,11,11-trimethyl-3-(2,2,2-trifluoro-acetyl)-1,2,3,4,5,6-hexahydro-2,6-methano-benzo[d]azocine-9-sulfonyl chloride

Chlorosulfonic acid (1.15 mL) is slowly added to a solution of 2,2,2-trifluoro-1-[(2*R*,6*S*)-6,11,11-trimethyl-1,2,5,6-tetrahydro-4H-2,6-methano-benzo[d]azocin-3-yl]-ethanone (0.90 g) in dichloromethane (10 mL) at room temperature. Then, the solution is stirred at ambient temperature overnight. The solution is poured into ice-cold water and the resulting mixture is extracted with ethyl acetate. The combined organic extracts are washed with brine and dried (MgSO₄). The solvent is removed under reduced pressure to give the crude title compounds in a mixture that is used without further purification.
Yield: 1.18 g

### Example XXXVII

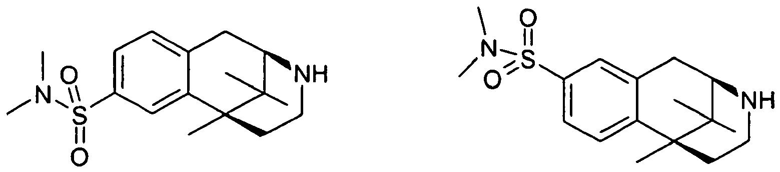

### (2R,6S)-6,11,11-Trimethyl-1,2,3,4,5,6-hexahydro-2,6-methano-benzo[d]azocine-8-sulfonic acid dimethylamide and (2R,6S)-6,11,11-trimethyl-1,2,3,4,5,6-hexahydro-2,6-methano-benzo[d]azocine-9-sulfonic acid dimethylamide

Dimethylamine (3.3 mL, 2 M in THF) is added to a mixture of (2*R*,6*S*)-6,11,11-trimethyl-3-(2,2,2-trifluoro-acetyl)-1,2,3,4,5,6-hexahydro-2,6-methano-benzo[d]azocine-8-sulfonyl chloride and (2*R*,6*S*)-6,11,11-trimethyl-3-(2,2,2-trifluoroacetyl)-1,2,3,4,5,6-hexahydro-2,6-methano-benzo[d]azocine-9-sulfonyl chloride (0.90 g, crude product from Example XXXVII) dissolved in ethanol (5 mL) and chilled in an ice bath. The cooling bath is removed and the solution is stirred at room temperature for 2 h. Then, 4 M NaOH solution (2.2 mL) is added to cleave off the trifluoroacetyl group. After stirring at room temperature for 1 h, the solution is diluted with water and the resulting mixture is extracted with ethyl acetate. The combined extracts are washed with brine and dried (MgSO₄). The solvent is removed and the residue is purified by HPLC on reversed phase (MeCN/H₂O/NH₃) to give the two title compounds separated.
(2*R*,6*S*)-6,11,11-Trimethyl-1,2,3,4,5,6-hexahydro-2,6-methano-benzo[d]azocine-8-sulfonic acid dimethylamide: Yield: 500 mg (71% of theory)
Mass spectrum (ESI⁺): m/z = 323 [M+H]⁺
(2*R*,6*S*)-6,11,11-Trimethyl-1,2,3,4,5,6-hexahydro-2,6-methano-benzo[d]azocine-9-sulfonic acid dimethylamide: Yield: 50 mg (7% of theory)
Mass spectrum (ESI⁺): m/z = 323 [M+H]⁺

The following compounds may be obtained analogously to Example XXXVII:

### (1) (2R,6S)-6,11,11-Trimethyl-1,2,3,4,5,6-hexahydro-2,6-methano-benzo[d]azocine-8-sulfonic acid methylamide

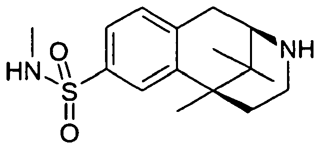

Mass spectrum (ESI⁺): m/z = 309 [M+H]⁺
Methylamine is used as coupling partner.

### (2) (2R,6S)-6,11,11-Trimethyl-1,2,3,4,5,6-hexahydro-2,6-methano-benzo[d]azocine-8-sulfonic acid amide

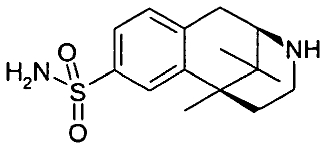

Mass spectrum (ESI⁺): m/z = 295 [M+H]⁺
Ammonia is used as coupling partner.

### Example XXXVIII

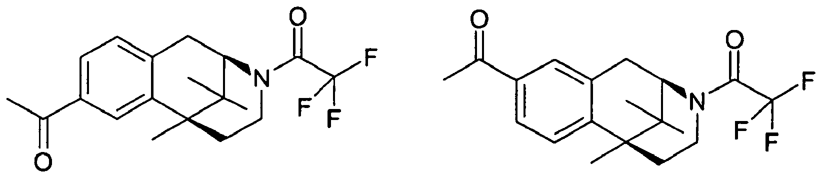

### 1-[(2R,6S)-8-Acetyl-6,11,11-trimethyl-1,2,5,6-tetrahydro-4H-2,6-methano-benzo[d]azocin-3-yl]-2,2,2-trifluoro-ethanone and 1-[(2R,6S)-9-acetyl-6,11,11-trimethyl-1,2,5,6-tetrahydro-4H-2,6-methano-benzo[d]azocin-3-yl]-2,2,2-trifluoro-ethanone

Acetyl chloride (0.25 mL) is added to a suspension of AlCl₃ (1.3 g) in CH₂Cl₂ (5 mL) chilled in an ice bath. After stirring the mixture for 5 min, 2,2,2-trifluoro-1-[(2*R*,6*S*)-6,11,11-trimethyl-1,2,5,6-tetrahydro-4H-2,6-methano-benzo[d]azocin-3-yl]-ethanone (1.0 g) dissolved in dichloromethane (5 mL) is added dropwise. The mixture is stirred at ambient temperature overnight and then poured into ice-cold half-concentrated hydrochloric acid (20 mL). The resulting mixture is extracted with CH₂Cl₂ and the combined organic extracts are washed with water, aqueous NaHCO₃ solution, and brine and dried (MgSO₄). The solvent is removed and the residue is purified by chromatography on silica gel (cyclohexane/ethyl acetate 3:1->1:1) to give the two regioisomeric title compounds in a ca. 3:1 mixture.
Yield: 0.83 g (73% of theory)
Mass spectrum (ESI⁺): m/z = 354 [M+H]⁺

### Example XXXIX

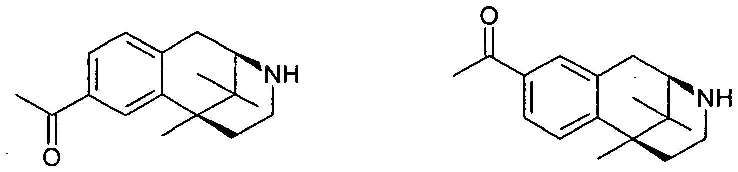

### 1-[(2R,6S)-6,11,11-Trimethyl-1,2,3,4,5,6-hexahydro-2,6-methano-benzo[d]azocin-8-yl]-ethanone and 1-[(2R,6S)-6,11,11-trimethyl-1,2,3,4,5,6-hexahydro-2,6-methano-benzo[d]azocin-9-yl]-ethanone

4 M NaOH solution (2.5 mL) is added to a ca. 3:1 mixture of 1-[(2*R*,6*S*)-8-acetyl-6,11,11-trimethyl-1,2,5,6-tetrahydro-4H-2,6-methano-benzo[d]azocin-3-y)]-2,2,2-trifluoro-ethanone and 1-[(2*R*,6*S*)-9-acetyl-6,11,11-trimethyl-1,2,5,6-tetrahydro-4H-2,6-methano-benzo[d]azocin-3-yl]-2,2,2-trifluoro-ethanone(0.83 g, from Example XXXVIII) in methanol (10 mL). The resulting solution is stirred at room temperature overnight. Then, the solution is neutralized with 1 M hydrochloric acid and concentrated. The residue is purified by HPLC on reversed phase 1(MeCN/water/NH₃) to give the two title compounds separated.
Yield: 0.35 g of 1-[(2*R*,6*S*)-6,11,11-trimethyl-1,2,3,4,5,6-hexahydro-2,6-methano-benzo[d]azocin-8-yl]-ethanone and 0.07 g of 1-[(2*R*,6*S*)-6,11,11-trimethyl-1,2,3,4,5,6-hexahydro-2,6-methano-benzo[d]azocin-9-yl]-ethanone(combined 71 % of theory)
Mass spectrum (ESI⁺): m/z = 258 [M+H]⁺

The following compounds may be obtained analogously to Example XXXIX:

### (1) (2R,6R,11S)-8-Hydroxy-6,11-dimethyl-1,2,3,4,5,6-hexahydro-2,6-methano-benzo[d]azocine-9-carbonitrile

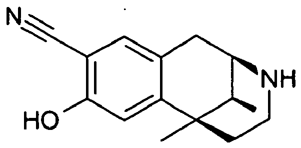

Mass spectrum (ESI⁺): m/z = 243 [M+H]⁺

### (2) (2R,6S)-8-Methanesulfonyl-6,11,11-trimethyl-1,2,3,4,5,6-hexahydro-2,6-methano-benzo[d]azocine

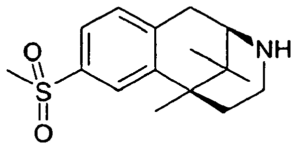

Mass spectrum (ESI⁺): m/z = 294 [M+H]⁺

### (3) (2R,6S)-10-Methanesulfonyl-6,11,11-trimethyl-1,2,3,4,5,6-hexahydro-2,6-methano-benzo[d]azocine

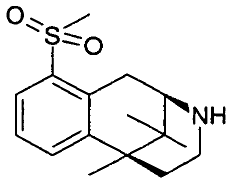

Mass spectrum (ESI⁺): m/z = 294 [M+H]⁺

### (4) (6R,10S)-5,6,7,8,9,10-Hexahydro-2,10,12,12-tetramethyl-6,10-methano-1H-imidazo[5,4-i][3]benzazocine

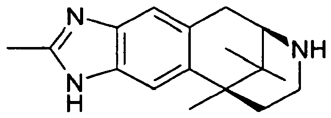

### (5) (6R,10S)-5,6,7,8,9,10-Hexahydro-10,12,12-trimethyl-6,10-methano-1H-imidazo[5,4-i][3]benzazocine

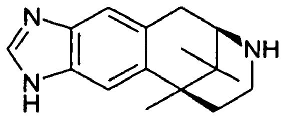

### (6) (2R,6R,11S)-6,11-Dimethyl-7-nitro-1,2,3,4,5,6-hexahydro-2,6-methano-benzo[d]azocin-8-ol

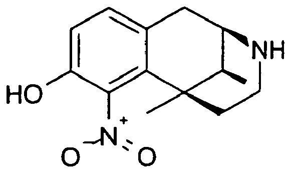

Mass spectrum (ESI⁺): m/z = 227 [M+H]⁺

### (7) (6R,10S)-5,6,7,8,9,10-Hexahydro-10,12,12-trimethyl-6,10-methano-1H-triazolo[5,4-i][3]benzazocine

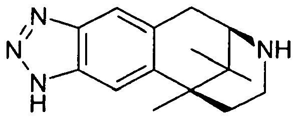

Mass spectrum (ESI⁺): m/z = 257 [M+H]⁺

### (8) (6R,10S)-5,6,7,8,9,10-Hexahydro-10,12,12-trimethyl-2-pyrazin-2-yl-6,10-methano-1H-imidazo[5,4-i][3]benzazocine

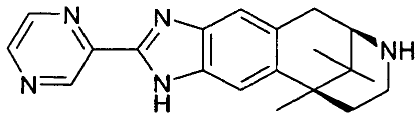

Mass spectrum (ESI⁺): m/z = 334 [M+H]⁺

### (9) (6R,10S)-2-(1-Acetyl-piperidin-4-yl)-5,6,7,8,9,10-hexahydro-10,12,12-trimethyl-6,10-methano-1H-imidazo[5,4-i][3]benzazocine

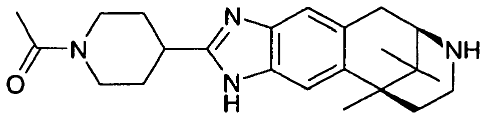

### (10) (6R, 10S)-2-Cyclopropyl-5,6,7,8,9,10-hexahydro-10,12,12-trimethyl-6,10-methano-1H-imidazo[5,4-i][3]benzazocine

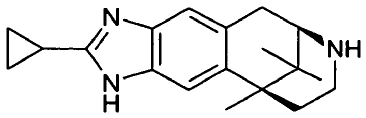

### (11) (6R,10S)-5,6,7,8,9,10-Hexahydro-10,12,12-trimethyl-2-(1-methyl-6-oxo-1,6-dihydro-pyridin-3-yl)-6,10-methano-1H-imidazo[5,4-i][3]benzazocine

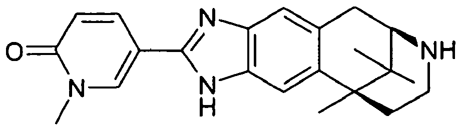

### (12) (6R,10S)-2-tert-Butyl-5,6,7,8,9,10-hexahydro-10,12,12-trimethyl-6,10-methano-1H-imidazo[5,4-i][3]benzazocine

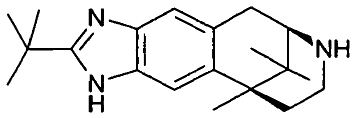

### (13) (6R,10S)-5,6,7,8,9,10-Hexahydro-10,12,12-trimethyl-2-pyridin-3-yl-6,10-methano-1H-imidazo[5,4-i][3]benzazocine

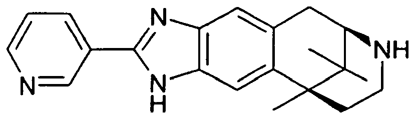

### (14) (6R,10S)-5,6,7,8,9,10-Hexahydro-10,12,12-trimethyl-2-[(S)-tetrahydrofuran-2-yl]-6,10-methano-1H-imidazo[5,4-i][3]benzazocine

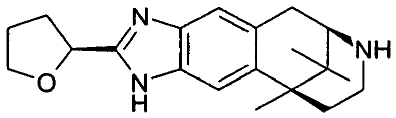

Mass spectrum (ESI⁺): m/z = 326 [M+H]⁺

### (15) (6R,10S)-5,6,7,8,9,10-Hexahydro-10,12,12-trimethyl-2-pyridazin-4-yl-6,10-methano-1H-imidazo[5,4-i][3]benzazocine

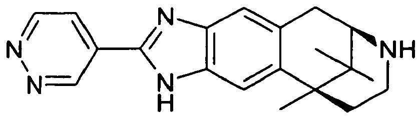

### (16) (6R, 10S)-5,6,7,8,9,10-Hexahydro-10,12,12-trimethyl-2-(5-methyl-pyrazin-2-yl)-6,10-methano-1H-imidazo[5,4-i][3]benzazocine

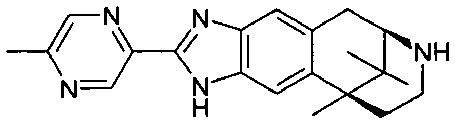

Mass spectrum (ESI⁺): m/z = 348 [M+H]⁺

### (17) (6R,10S)-5,6,7,8,9,10-Hexahydro-10,12,12-trimethy)-2-[(R)-tetrahydrofuran-2-yl]-6,10-methano-1H-imidazo[5,4-i][3]benzazocine

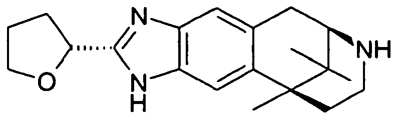

Mass spectrum (ESI⁺): m/z = 326 [M+H]⁺

### (18) (7R,11R,12S)-6,7,8,9,10,11-Hexahydro-2,11,12-trimethyl-7,11-methano-oxazolo[4,5-h][3]benzazocine

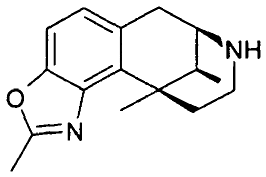

Mass spectrum (ESI⁺): m/z = 257 [M+H]⁺

### (19) (6R,10S)-5,6,7,8,9,10-Hexahydro-2,10,12,12-tetramethyl-6,10-methano-oxazolo[4,5-i][3]benzazocine

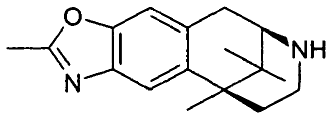

### (20) (6R,10S)-2-Cyclopropyl-5,6,7,8,9,10-hexahydro-10,12,12-trimethyl-6,10-methano-oxazolo[4,5-i][3]benzazocine

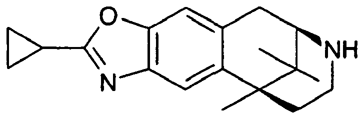

### (21) (6R,10R,12S)-5,6,7,8,9,10-Hexahydro-2,10,12-trimethyl-6,10-methano-oxazolo[5,4-i][3]benzazocine

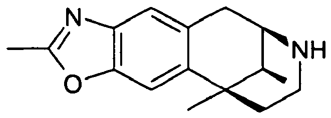

Mass spectrum (ESI⁺): m/z = 257 [M+H]⁺

### (22) (6R,10R,12S)-2-Cyclopropyl-5,6,7,8,9,10-hexahydro-10,12-dimethyl-6,10-methano-oxazolo[5,4-i][3]benzazocine

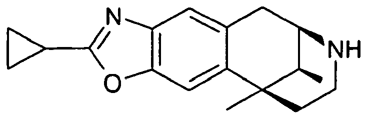

### (23) (6R,10S)-2-tert-Butyl-5,6,7,8,9,10-hexahydro-10,12,12-trimethyl-6,10-methano-oxazolo[4,5-i][3]benzazocine

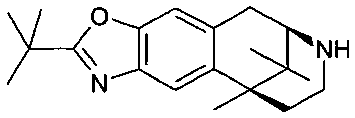

### (24) (6R,10S)-5,6,7,8,9,10-hexahydro-10,12,12-trimethyl-2-(5-methyl-pyrazin-2-yl)-6,10-methano-oxazolo[4,5-i][3]benzazocine

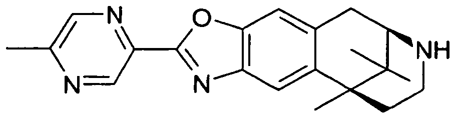

### (25) (6R,10R,12S)-5,6,7,8,9,10-hexahydro-10,12-dimethyl-2-(5-methyl-pyrazin-2-yl)-6,10-methano-oxazolo[5,4-i][3]benzazocine

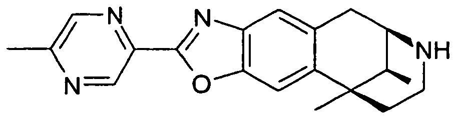

Mass spectrum (ESI⁺): m/z = 335 [M+H]⁺

### (26) (6R,10S)-5,6,7,8,9,10-hexahydro-10,12,12-trimethyl-2-[(R)-tetrahydrofuran-2-yl]-6,10-methano-oxazolo[4,5-i][3]benzazocine

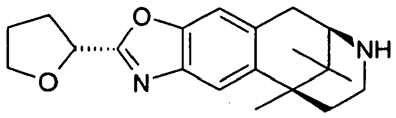

### (27) (6R,10S)-5,6,7,8,9,10-hexahydro-10,12,12-trimethyl-2-[(S)-tetrahydrofuran-2-yl]-6,10-methano-oxazolo[4,5-i][3]benzazocine

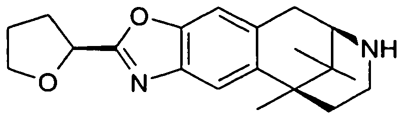

### Example XL

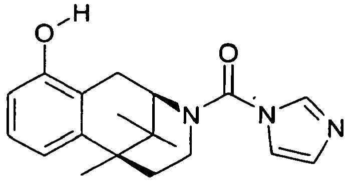

### [(2R,6S)-10-Hydroxy-6,11,11-trimethyl-1,2,5,6-tetrahvdro-4H-2,6-methano-benzo[d]azocin-3-yl]-imidazol-1-yl-methanone

N,N'-Carbonyldiimidazole (6.8 g) is added to (2*R*,6*S*)-6,11,11-trimethyl-1,2,3,4,5,6-hexahydro-2,6-methano-benzo[d]azocin-10-ol (9.66 g) dissolved in tetrahydrofuran (100 mL). The resulting solution is stirred at reflux temperature for 3 h. Then, potassium carbonate (4.0 g) is added and the mixture is stirred at reflux temperature for another 4 h. After cooling to ambient temperature, aqueous NaHCO₃ solution is added and the resulting mixture is extracted with ethyl acetate. The combined extracts are dried (MgSO₄) and the solvent is evaporated. The residue is purified by chromatography on silica gel (dichloromethane/MeOH 1:0->18:1).
Yield: 3.40 g (25% of theory)
Mass spectrum (ESI⁺): m/z = 323 [M+H]⁺

### Example XLI

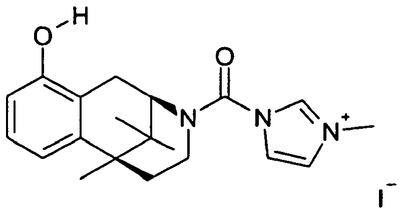

### 3-[(2R,6S)-10-Hydroxy-6.11,11-trimethyl-1,2,5,6-tetrahydro-4H-2,6-methano-benzo[d]azocine-3-carbonyl]-1-methyl-3H-imidazol-1-ium iodide

lodomethane (0.8 mL) is added to a solution of [(2*R*,6*S*)-10-hydroxy-6,11,11-trimethyl-1,2,5,6-tetrahydro-4H-2,6-methano-benzo[d]azocin-3-yl]-imidazol-1-yl-methanone (3.4 g) in acetonitrile (30 mL). After stirring overnight, another portion of iodomethane (1 mL) is added. The solution is stirred for additional 16 h at room temperature and then concentrated under reduced pressure to give the crude title compound that is used without further purification.
Yield: 4.9 g (100% of theory)
Mass spectrum (ESI⁺): m/z = 340 [M]⁺[= 3-(10-hydroxy-6,11,11-trimethyl-1,2,5,6-tetrahydro-4H-2,6-methano-benzo[d]azocine-3-carbonyl)-1-methyl-3H-imidazol-1-ium]

### Example XLII

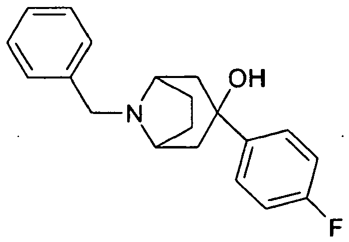

### 8-Benzyl-3-(4-fluoro-phenyl)-8-aza-bicyclo[3.2.1]octan-3-ol

1-Bromo-4-fluoro-benzene (22.7 g) dissolved in diethylether (100 mL) is added to a solution of n-butyllithium (1.7 mol/L in pentane, 86.8 mL) in diethylether (200 mL) cooled to -35 °C. The combined solutions are stirred at -35 - -40 °C for 1 h, before 8-benzyl-8-aza-bicyclo[3.2.1]octan-3-one (22.5 g) dissolved in diethylether (150 mL) is added quickly. The solution is warmed to -10 °C within 1 h and then quenched by the addition of aqueous NH₄Cl solution. The resulting mixture is extracted with ethyl acetate, the combined extracts are washed with brine and 4 M hydrochloric acid is added. The organic phase is separated from the aqueous phase and an oily precipitation formed after the addition. The oily and aqueous phase are basified with 4 M NaOH solution and the resulting mixture is extracted with ethyl acetate. The organic phase and extracts are dried (Na₂SO₄) and the solvent is evaporated to give the title compound.
Yield: 21.0 g (75% of theory)
Mass spectrum (ESI⁺): m/z = 312 [M+H]⁺

### Example XLIII

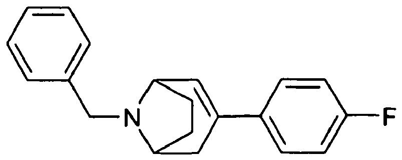

### 8-Benzyl-3-(4-fluoro-phenyl)-8-aza-bicyclo[3.2.1]oct-2-ene

A solution of 8-benzyl-3-(4-fluoro-phenyl)-8-aza-bicyclo[3.2.1]octan-3-ol (21.0 g) in concentrated aqueous hydrochloric acid (80 mL) is stirred at reflux temperature for 1 h. After cooling to ambient temperature, the solution is basified by the addition of 4 M aqueous NaOH solution. The resulting mixture is extracted with ethyl acetate and the combined extracts are dried (Na₂SO₄). The solvent is evaporated and the residue is dissolved in ether. Methanesulfonic acid (4.3 mL) is added and the solvent is removed under reduced pressure to give the methanesulfonic acid salt of the title compound.
Yield: 19.1 g (73% of theory)

### Example XLIV

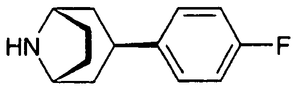

### endo-3-(4-Fluoro-phenyl)-8-aza-bicyclo[3.2.1]octane

A mixture of the methanesulfonic acid salt of 8-benzyl-3-(4-fluoro-phenyl)-8-azabicyclo[3.2.1]oct-2-ene (19.1 g) and 5% palladium on carbon (2 g) in methanol (170 mL) is shaken under hydrogen atmosphere (5 bar) at 55 °C overnight. Then, the catalyst is separated by filtration and the filtrate is concentrated. The residue is taken up in ethyl acetate and washed with saturated aqueous K₂CO₃ solution. The organic phase is concentrated again and the residue is purified by chromatography on silica gel (dichloromethane/methanol 99:1->9:1).
Yield: 3.5 g (35% of theory)
Mass spectrum (ESI⁺): m/z = 206 [M+H]⁺

### Example XLV

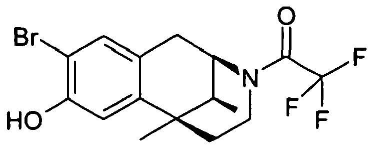

### 1-[(2R,6R,11S)-9-Bromo-8-hydroxy-6,11-dimethyl-1,2,5,6-tetrahydro-4H-2,6-methano-benzo[d]azocin-3-yl]-2,2,2-trifluoro-ethanone

A solution of 2,2,2-trifluoro-1-[(2*R*,6*R*,11*S*)-8-hydroxy-6,11-dimethyl-1,2,5,6-tetrahydro-4H-2,6-methano-benzo[d]azocin-3-yl]-ethanone (3.0 g) and pyridinium tribromide (3.3 g) in acetic acid (2 mL) is stirred at 80 °C for 2 h. After cooling to room temperature, water is added and the resulting mixture is extracted with ethyl acetate. The combined organic extracts are washed with water, aqueous NaHCO₃ solution, and brine. After drying (Na₂SO₄), the solvent is removed and the residue is purified by chromatography on silica gel (cyclohexane/ethyl acetate 4:1->1:1).
Yield: 2.5 g (67% of theory)
Mass spectrum (ESI⁺): m/z = 392/394 (Br) [M+H]⁺

The following compound may be obtained analogously to Example XLV:

### (1) 1-[(2R,6R,11R)-9-Bromo-8-hydroxy-6,11-dimethyl-1,2,5,6-tetrahydro-4H-2,6-methano-benzo[d]azocin-3-yl]-2,2,2-trifluoro-ethanone

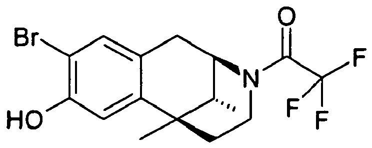

Mass spectrum (ESI⁺): m/z = 392/394 (Br) [M+H]⁺

### Example XLVI

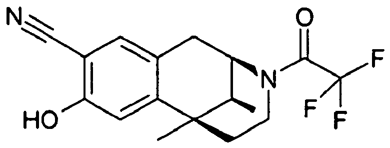

### (2R,6R,11S)-8-Hydroxy-6,11-dimethyl-3-(2,2,2-trifluoro-acetyl)-1,2,3,4,5,6-hexahydro-2,6-methano-benzo[d]azocine-9-carbonitrile

A mixture of 1-[(2*R*,6*R*,11*S*)-9-bromo-8-hydroxy-6,11-dimethyl-1,2,5,6-tetrahydro-4H-2,6-methano-benzo[d]azocin-3-yl]-2,2,2-trifluoro-ethanone (0.50 g) and copper cyanide (0.23 g) in N-methyl-pyrrolidone (2 mL) is stirred in a microwave oven at 180 °C for 1 h. After cooling to room temperature, water is added and the resulting mixture is extracted with ethyl acetate. The combined organic extracts are washed with brine and dried (Na₂SO₄). After removing the solvent, the residue is purified by chromatography on silica gel (cyclohexane/ethyl acetate 2:1->1:2).
Yield: 0.20 g (46% of theory)
Mass spectrum (ESI⁺): m/z = 339 [M+H]⁺

The following compound may be obtained analogously to Example XLVI:

### (1) (2R,6R,11R)-8-Hydroxy-6,11-dimethyl-3-(2,2,2-trifluoro-acetyl)-1,2,3,4,5,6-hexahydro-2,6-methano-benzo[d]azocine-9-carbonitrile

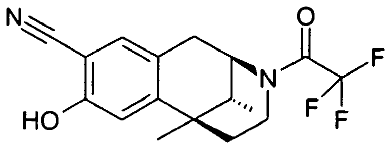

Mass spectrum (ESI⁺): m/z = 339 [M+H]⁺

### Example XLVII

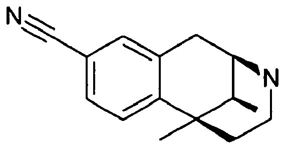

### (2R,6R,11S)-6,11-Dimethyl-1,2,3,4,5,6-hexahydro-2,6-methano-benzo[d]azocine-9-carbonitrile

A solution of KF (76 mg) in water (1 mL) followed by polymethylhydrosiloxane (1.0 g) is added to a mixture of (2R,6R,11 S)-trifluoro-methanesulfonic acid 9-cyano-6,11-dimethyl-3-(2,2,2-trifluoro-acetyl)-1,2,3,4,5,6-hexahydro-2,6-methano-benzo[d]azocin-8-yl ester (0.30 g) and Pd(OAc)₂ (7 mg) in tetrahydrofuran (3 mL). The resulting mixture is stirred at room temperature overnight before 1 M NaOH (20 mL) is added. After stirring vigorously for 1 h, the organic phase is separated and the aqueous phase is extracted with ethyl acetate. The combined organic phases are washed with water and brine and dried (MgSO₄). The solvent is removed and the residue is taken up in 4 M NaOH (1 mL) and methanol (3 mL) and stirred at room temperature overnight. Then, the solution is neutralized with 1 M hydrochloric acid, filtered, concentrated and the residue is purified by HPLC on reversed phase (MeCN/water).
Yield: 0.07 g (48% of theory)
Mass spectrum (ESI⁺): m/z = 227 [M+H]⁺

The following compound may be obtained analogously to Example XLVII:

### (1) (2R,6R,11R)-6,11-Dimethyl-1,2,3,4,5,6-hexahydro-2,6-methano-benzo[d]azocine-9-carbonitrile

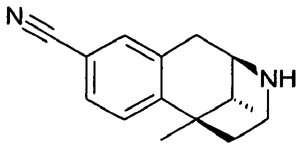

### Example XLVIII

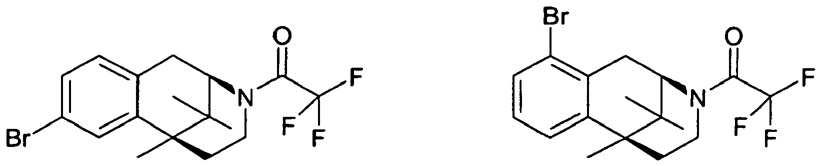

### 1-[(2R,6S)-8-Bromo-6,11,11-trimethyl-1,2,5,6-tetrahydro-4H-2,6-methano-benzo[d]azocin-3-yl]-2,2,2-trifluoro-ethanone and 1-[(2R,6S)-10-bromo-6,11,11-trimethyl-1,2,5,6-tetrahydro-4H-2,6-methano-benzo[d]azocin-3-yl]-2,2,2-trifluoro-ethanone

AlCl₃ (147 mg) is added to a solution of 2,2,2-trifluoro-1-[(2*R*,6*S*)-6,11,11-trimethyl-1,2,5,6-tetrahydro-4H-2,6-methano-benzo[d]azocin-3-yl]-ethanone (275 mg) in 1,2-dichloroethane (10 mL). The resulting mixture is stirred at ambient temperature for 10 min before bromine (52 µL) is added. The mixture is heated to 50 °C. After stirring at 50 °C for 1 h, the mixture is cooled to ambient temperature and diluted with dichloromethane (30 mL) and water (10 mL). The resulting mixture is stirred vigorously for 5 min and then 4 M hydrochloric acid (10 mL) is added. The organic phase is separated and washed with 4 M hydrochloric acid and water and dried (MgSO₄). The solvent is removed under reduced pressure to give the two title compounds in a mixture with a further regioisomerically brominated educt.
Yield: 328 mg (95% of theory)
Mass spectrum (ESI⁺): m/z = 390/392 (Br) [M+H]⁺

### Example IL

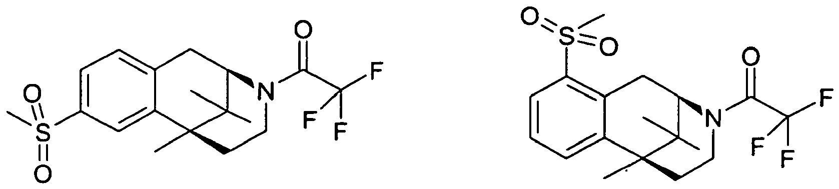

### 2,2,2-Trifluoro-1-[(2R,6S)-8-methanesulfonyl-6,11,11-trimethyl-1,2,5,6-tetrahydro-4H-2,6-methano-benzo[d]azocin-3-yl]-ethanone and 2,2,2-trifluoro-1-[(2R,6S):10-methanesulfonyl-6,11,11-trimethyl-1,2,5,6-tetrahydro-4H-2,6-methano-benzo[d]azocin-3-yl]-ethanone

MeSO₂Na (0.79 g) is added to a mixture of Cul (1.5 g) and 1-[(2*R*,6*S*)-8-bromo-6,11,11-trimethyl-1,2,5,6-tetrahydro-4H-2,6-methano-benzo[d]azocin-3-yl]-2,2,2-trifluoro-ethanone/1-[(2*R*,6*S*)-10-bromo-6,11,11-trimethyl-1,2,5,6-tetrahydro-4H-2,6-methano-benzo[d]azocin-3-yl]-2,2,2-trifluoro-ethanone (300 mg, crude product from Example XLVIII) in dimethylsulfoxide (6 mL). The resulting mixture is heated to 120 °C and stirred at this temperature overnight. After cooling to ambient temperature, the mixture is poured into a solution of concentrated aqueous ammonia (20 mL) and water (80 mL). The resulting mixture is extracted with ethyl acetate and the combined organic extracts are washed with 2 M ammonia solution and brine. After drying (MgSO₄), the solvent is removed under reduced pressure and the residue is purified by HPLC on reversed phase (MeCN/water) to give the two title compounds separated.
2,2,2-Trifluoro-1-[(2*R*,6*S*)-8-methanesulfonyl-6,11,11-trimethyl-1,2,5,6-tetrahydro-4H-2,6-methano-benzo[d]azocin-3-yl]-ethanone: Yield: 150 mg (50% of theory)
Mass spectrum (ESI⁺): m/z = 390 [M+H]⁺
2,2,2-Trifluoro-1-[(2*R*,6*S*)-10-methanesulfonyl-6,11,11-trimethyl-1,2,5,6-tetrahydro-4H-2,6-methano-benzo[d]azocin-3-yl]-ethanone: Yield: 100 mg (33% of theory) Mass spectrum (ESI⁺): m/z = 390 [M+H]⁺

### Example L

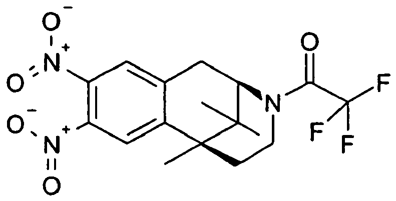

### 2,2,2-Trifluoro-1-[(2R,6S)-6,11,11-trimethyl-8,9-dinitro-1,2,5,6-tetrahydro-4H-2,6-methano-benzo[d]azocin-3-yl]-ethanone

Nitric acid (0.16 mL) is added to a solution of trifluoroacetic acid (0.65 mL) in dichloromethane (4 mL) chilled in an ice bath (ca. 0 °C). After stirring for 10 min, 2,2,2-trifluoro-1-[(2*R*,6*S*)-6,11,11-trimethyl-1,2,5,6-tetrahydro-4H-2,6-methano-benzo[d]azocin-3-yl]-ethanone (0.50 g) in dichloromethane (5 mL) is added. The resulting solution is stirred in the cooling bath for 2 h and then at ambient temperature overnight. The solution is poured into ice-cold water and the resulting mixture is extracted with dichloromethane. The combined organic extracts are washed with aqueous NaHCO₃ solution and dried (MgSO₄). The solvent is removed under reduced pressure and the residue is purified by chromatography on silica gel (cyclohexane/ethyl acetate 1:0->9:1).
Yield: 330 mg (51% of theory)
Mass spectrum (ESI⁺): m/z = 402 [M+H]⁺

### Example LI

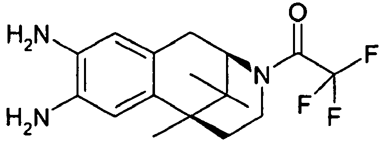

### 1-[(2R,6S)-8,9-Diamino-6,11,11-trimethyl-1,2,5,6-tetrahydro-4H-2,6-methano-benzo[d]azocin-3-yl]-2,2,2-trifluoro-ethanone

A mixture of 10% palladium on carbon (300 mg) and 2,2,2-trifluoro-1-[(2*R*,6*S*)-6,11,11-trimethyl-8,9-dinitro-1,2,5,6-tetrahydro-4H-2,6-methano-benzo[d]azocin-3-yl]-ethanone (330 mg) in methanol (5 mL) is shaken under hydrogen atmosphere at room temperature for 2 h. Then, the catalyst is separated by filtration and the solvent is removed under reduced pressure to give the crude title compound that is used without further purification.
Yield: 260 mg (93% of theory)
Mass spectrum (ESI⁺): m/z = 342 [M+H]⁺

The following compounds may be obtained analogously to Example LI:

### (1) 1-[(2R,6R,11S)-7-Amino-8-hydroxy-6,11-dimethyl-1,2,5,6-tetrahydro-4H-2,6-methano-benzo[d]azocin-3-yl]-2,2,2-trifluoro-ethanone

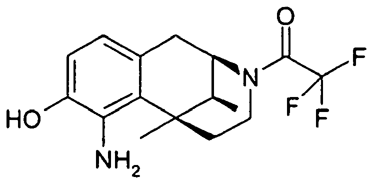

Mass spectrum (ESI⁺): m/z = 329 [M+H]⁺

### (2) 1-[(2R,6S)-8-Amino-9-hydroxy-6,11,11-trimethyl-1,2,5,6-tetrahydro-4H-2,6-methano-benzo[d]azocin-3-yl]-2,2,2-trifluoro-ethanone

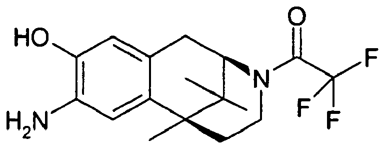

Mass spectrum (ESI⁺): m/z = 343 [M+H]⁺

### (3) 1-[(2R,6R,11S)-9-Amino-8-hydroxy-6,11-dimethyl-1,2,5,6-tetrahydro-4H-2,6-methano-benzo[d]azocin-3-yl]-2,2,2-trifluoro-ethanone

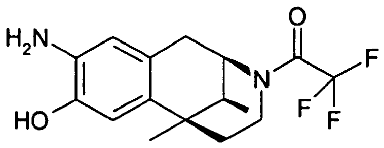

Mass spectrum (ESI⁺): m/z = 329 [M+H]⁺

### Example LII

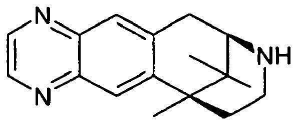

### (7R,11S)-6,7,8,9,10,11-Hexahydro-11,13,13-trimethyl-7,11-methano-pyrazino[2.3-i][3]benzazocine

Glyoxal (40% in water, 95 µL) is added to 1-[(2*R*,6*S*)-8,9-diamino-6,11,11-trimethyl-1,2,5,6-tetrahydro-4H-2,6-methano-benzo[d]azocin-3-yl]-2,2,2-trifluoro-ethanone (260 mg) dissolved in ethanol (3 mL) and chilled in an ice bath. The cooling bath is removed and the solution is stirred at ambient temperature overnight. Then, the solution is concentrated and the residue is taken up in methanol (1 mL) and treated with 4 M aqueous NaOH solution (0.38 mL). After stirring at ambient temperature overnight, brine is added and the resulting mixture is extracted with ethyl acetate. The combined organic extracts are washed with brine, dried (MgSO₄), and the solvent is removed under reduced pressure to give the crude title compound that is used without further purification.
Yield: 204 mg
Mass spectrum (ESI⁺): m/z = 268 [M+H]⁺

The following compounds may be obtained analogously to Example LII:

### (1) (7R,11S)-6,7,8,9,10,11-Hexahydro-2,3,11,13,13-pentamethyl-7,11-methano-pyrazino[2,3-i][3]benzazocine

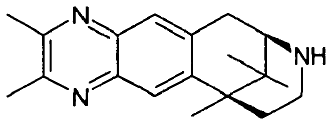

Mass spectrum (ESI⁺): m/z = 296 [M+H]⁺

The compound is obtained by using diacetyl according to the procedure described above.

### (2) (7R,11S)-6,7,8,9,10,11-Hexahydro-3,11,13,13-tetramethyl-7,11-methano-pyrazino[2,3-i][3]benzazocine and (7R,11S)-6,7,8,9,10,11-hexahydro-2,11,13,13-tetramethyl-7,11-methano-pyrazino[2,3-i][3]benzazocine

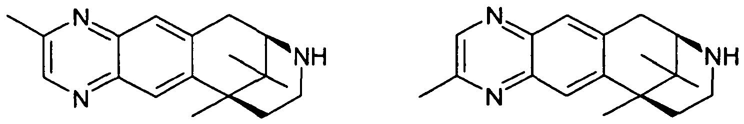

Mass spectrum (ESI⁺): m/z = 296 [M+H]⁺

The compounds are obtained as a mixture of each other by using methylglyoxal.

### Example LIII

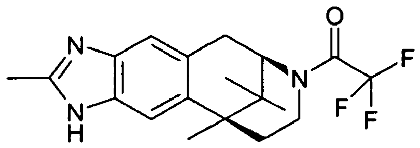

### 2,2,2-Trifluoro-1-[(6R,10S)-5,6,7,8,9,10-hexahydro-2,10,12,12-tetramethyl-6,10-methano-1H-imidazo[5,4-i][3]benzazocin-7-yl]-ethanone

1-[(2*R*,6*S*)-8,9-Diamino-6,11,11-trimethyl-1,2,5,6-tetrahydro-4H-2,6-methano-benzo[d]-azocin-3-yl]-2,2,2-trifluoro-ethanone (600 mg) dissolved in glacial acetic acid is stirred at 130 °C for 3 h. After cooling to ambient temperature, the solution is concentrated under reduced pressure and the residue is taken up in ethyl acetate. The organic solution is washed with aqueous K₂CO₃ solution and brine and dried (MgSO₄). The solvent is removed under reduced pressure to give the crude title compound as a foam-like solid.
Yield: 642 mg
Mass spectrum (ESI⁺): m/z = 366 [M+H]⁺

The following compound may be obtained analogously to Example LIII:

### (1) 2,2,2-Trifluoro-1-[(6R,10S)-5,6,7,8,9,10-hexahydro-10,12,12-trimethyl-6,10-methano-1H-imidazo[5,4-i][3]benzazocin-7-yl]-ethanone

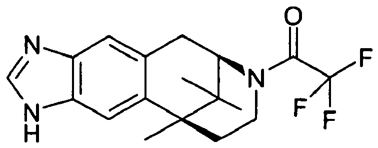

Mass spectrum (ESI⁺): m/z = 352 [M+H]⁺

The reaction is carried out with formic acid instead of acetic acid.

### Example LIV

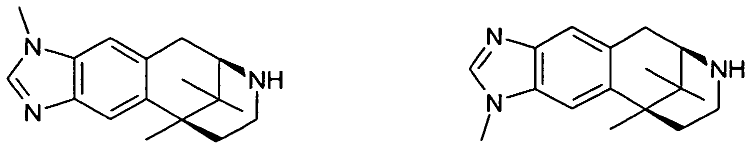

### (6R,10S)-5,6,7,8,9,10-Hexahydro-3,10,12,12-tetramethyl-6,10-methano-imidazo[4,5-i][3]benzazocine and (6R,10S)-5,6,7,8,9,10-hexahydro-1,10,12,12-tetramethyl-6,10-methano-imidazo[5,4-i][3]benzazocine

Methyl iodide (69 µL) is added to a mixture of 2,2,2-trifluoro-1-[(6*R*,10*S*)-5,6,7,8,9,10-hexahydro-10,12,12-trimethyl-6,10-methano-1H-imidazo[5,4-i][3]benzazocin-7-yl]-ethanone (300 mg) and K₂CO₃ (118 mg) in dimethylformamide (2 mL). The resulting mixture is stirred at room temperature overnight. Then, water is added and the mixture is extracted with ethyl acetate. The combined extracts are washed with brine and dried (MgSO₄). The solvent is removed and the residue is taken up in methanol (3 mL) and treated with 4 M aqueous NaOH solution (0.5 mL). The solution is stirred at room temperature overnight and then diluted with ethyl acetate. The resulting solution is washed with water and brine and dried (MgSO₄). The solvent is removed under reduced pressure to give the crude title compounds as a mixture.
Yield: 90 mg (39% of theory)

The following compounds may be obtained analogously to Example LIV:

### (1) (6R,10S)-5,6,7,8,9,10-hexahydro-1,2,10,12,12-pentamethyl-6,10-methano-imidazo[5,4-i][3]benzazocine

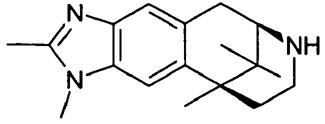

Mass spectrum (ESI⁺): m/z = 284 [M+H]⁺

### (2) (6R,10S)-5,6,7,8,9,10-hexahydro-2,3,10,12,12-pentamethyl-6,10-methano-imidazo[4,5-i][3]benzazocine

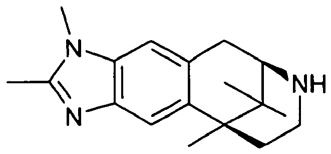

Mass spectrum (ESI^{*}): m/z = 284 [M+H]⁺

The two isomeric compounds (1) and (2) were obtained from the same starting compound and separated by HPLC on reversed phase.

### (3) Mixture of (6R,10S)-5,6,7,8,9,10-hexahydro-1,10,12,12-tetramethyl-6,10-methano-triazolo[5,4-i][3]benzazocine and (6R,10S)-5,6,7,8,9,10-hexahydro-3,10,12,12-tetramethyl-6,10-methano-triazolo[4,5-i][3]benzazocine

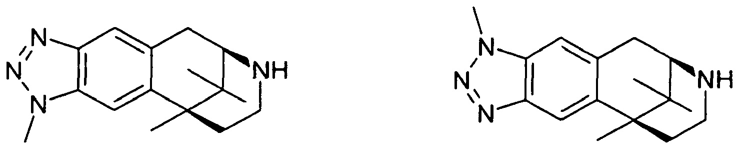

The compounds are obtained from compound Example LXIII after carrying out the reactions described above.

### Example LV

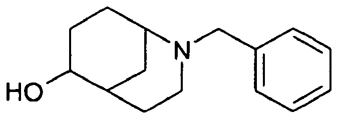

### 2-Benzyl-2-aza-bicyclo[3.3.1]nonan-6-ol

Diisobutylaluminumhydride (1.5 mol/L in toluene, 21 mL) is added to a solution of acetic acid 2-benzyl-3-oxo-2-aza-bicyclo[3.3.1]non-6-yl ester (1.50 g, for synthesis see J. Chem. Soc., Perkin Trans. 1 1999, 1157-1162) in toluene (30 mL) cooled to - 70 °C. The cooling bath is removed and the solution is stirred at ambient temperature overnight. Then, another portion of diisobutylaluminumhydride (1.5 mol/L in toluene, 20 mL) is added and the solution is stirred for additional 4 h at room temperature. Then, the solution is poured into ice-cold water and the resulting mixture is extracted with ethyl acetate. The aqueous phase is acidified using 4 M hydrochloric acid and extracted one more time with ethyl acetate. The combined organic extracts are dried (Na₂SO₄) and the solvent is removed. The residue is purified by chromatography on silica gel (dichloromethane/methanol 1:0->2:1).
Yield: 440 mg (36% of theory)
Mass spectrum (ESI⁺): m/z = 232 [M+H]⁺

### Example LVI

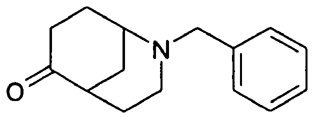

### 2-Benzyl-2-aza-bicyclo[3.3.1]nonan-6-one

Dess-Martin periodinane (1.30 g) is added to a solution of 2-benzyl-2-aza-bicyclo[3.3.1]-nonan-6-ol (0.60 g) in dichloromethane (15 mL) chilled in an ice bath. The cooling bath is removed and the solution is stirred at ambient temperature for 1 h. Then, the solution is diluted with dichloromethane and washed with a mixture of aqueous Na₂S₂O₃ solution and aqueous NaHCO₃ solution. The solution is dried (Na₂SO₄) and the solvent is removed. The residue is purified by chromatography on silica gel (dichloromethane/methanol 1:0->2:1).
Yield: 250 mg (42% of theory)
Mass spectrum (ESI⁺): m/z = 230 [M+H]⁺

### Example LVII

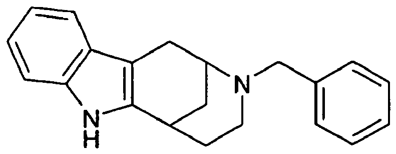

### 3-Benzyl-2,3,4,5,6,7-hexahydro-2,6-methano-1H-azocino[5,4-b]indole

A solution of 2-benzyl-2-aza-bicyclo[3.3.1]nonan-6-one in acetic acid (0.24 g) is added to a solution of PhNHNH₂*HCl (173 mg) in acetic acid (4 mL) heated at reflux temperature. After stirring at this temperature for 2 h, the solution is cooled to room temperature and aqueous K₂CO₃ solution is added. The resulting mixture is extracted with ethyl acetate, the combined organic extracts are dried (Na₂SO₄), and the solvent is removed. The residue is purified by HPLC on reversed phase (MeCN/water). Yield: 160 mg (49% of theory)

### Example LVIII

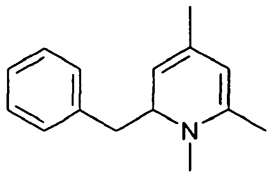

### 2-Benzyl-1,4,6-trimethyl-1,2-dihydro-pyridine

PhCH₂MgCl (1 M in Et₂O, 180 mL) is added dropwise to a solution of 1,2,4-trimethyl-pyridinium iodide (24.3 g) in Et₂O (90 mL) chilled in an ice bath. After stirring in the ice bath for 2 h, the solution is poured into a mixture of 72% aqueous HClO₄ (40 mL) and crushed ice (ca. 900 mL). The resulting mixture is stirred for 1 h and the precipitate formed is separated by filtration. The precipitate is washed with methanol and dried to afford the HClO₄ salt of the title compound.
Yield: 22.6 g (74% of theory)
Mass spectrum (ESI⁺): m/z = 214 [M+H]⁺

### Example LIX

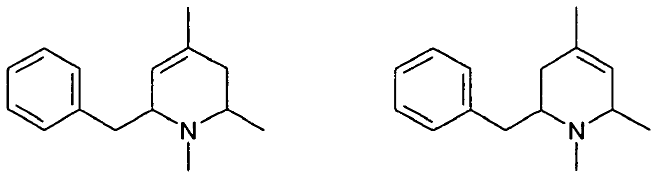

### 6-Benzyl-1,2,4-trimethyl-1,2,3,6-tetrahydro-pyridine and 2-benzyl-1,4,6-trimethyl-1,2,3,6-tetrahydro-pyridine

NaBH₄ (3.8 g) is added portionwise to a solution of 2-benzyl-1,4,6-trimethyl-1,2-dihydro-pyridine (22.6 g) in MeOH (65 mL) and NaOH (1 M in water, 200 mL). The resulting mixture is stirred at room temperature for 20 min and then at 60 °C for 30 min. After cooling to ambient temperature, the mixture is diluted with water (150 mL) and extracted with Et₂O (3x 150 mL). The combined organic extracts are dried (Na₂SO₄) and the solvent is removed to give a mixture of the two title compounds that is used without further purification for the next reaction step.
Yield: 11.7 g (76% of theory)
Mass spectrum (ESI⁺): m/z = 216 [M+H]⁺

### Example LX

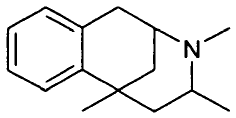

### 3,4,6-Trimethyl-1,2,3,4,5,6-hexahydro-2,6-methano-benzo[d]azocine

A mixture of 6-benzyl-1,2,4-trimethyl-1,2,3,6-tetrahydro-pyridine and 2-benzyl-1,4,6-trimethyl-1,2,3,6-tetrahydropyridine (from Example LIX, 11.7 g) is combined with 48% HBr in water (30 mL) and 33% HBr in acetic acid (20 mL). The mixture is heated to reflux temperature and stirred at this temperature for 4 d. After cooling to ambient temperature, aqueous ammonia (32%, 45 mL) is carefully added and the resulting mixture is extracted with Et₂O (3x 50 mL). The combined organic extracts are extracted with 2 M hydrochloric acid (3x 50 mL), the combined aqueous extracts are basified using 32% aqueous ammonia (20 mL), and the basic aqueous phase is extracted with Et₂O (3x 50 mL). The combined organic extracts are dried (MgSO₄), the solvent is removed, and the residue is purified by chromatography on silica gel (EtOAc/MeOH/NH₃ 95:5:0.5->75:25:2.5). The title compound obtained thereafter is dissolved in *i*PrOH and treated with HCI in iPrOH to precipitate the HCI salt of the title compound from the *i*PrOH solution.
Yield: 1.7 g (15% of theory)
Mass spectrum (ESI⁺): m/z = 216 [M+H]⁺

### Example LXI

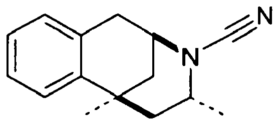

### 4,6-Dimethyl-1,2,5,6-tetrahydro-4H-2,6-methano-benzo[d]azocine-3-carbonitrile (one diastereomer, relative configurations of the substituents given in the structure drawn above are confirmed by NMR experiments)

3,4,6-Trimethyl-1,2,3,4,5,6-hexahydro-2,6-methano-benzo[d]azocine (from Example LX, 1.7 g) dissolved in CH₂Cl₂ (40 mL) is added to a solution of BrCN (1.17 g) in CH₂Cl₂ (10 mL) chilled in an ice bath. The cooling bath is removed and the mixture is stirred at ambient temperature for 1 h and at 45 °C for 2 h. After cooling to ambient temperature, the solution is washed with water, 2 M hydrochloric acid, and 10% aqueous K₂CO₃ solution. The solution is dried (MgSO₄), the solvent is removed, and the residue is triturated with little acetone to give the title compound.
Yield: 0.98 g (54% of theory)
Mass spectrum (ESI⁺): m/z = 227 [M+H]⁺

The following compound may be obtained in analogy to Example LXI:

### (1) 5,6-Dimethyl-1,2,5,6-tetrahydro-4H-2,6-methano-benzo[d]azocine-3-carbonitrile (racemic mixture of diastereomer shown)

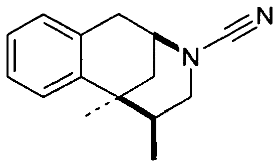

Mass spectrum (ESI⁺): m/z = 227 [M+H]⁺

The starting compound, 3,5,6-trimethyl-1,2,3,4,5,6-hexahydro-2,6-methano-benzo[d]azocine, may be obtained as described in J. Med. Chem. 1971, 14, 565-68.

### Example LXII

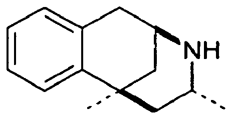

### 3,4,6-Trimethyl-1,2,3,4,5,6-hexahydro-2,6-methano-benzo[d]azocine (racemic mixture of diastereomer shown)

A mixture of 4,6-dimethyl-1,2,5,6-tetrahydro-4H-2,6-methano-benzo[d]azocine-3-carbonitrile (one diastereomer, 925 mg), water (30 mL), and 4 M hydrochloric acid (30 mL) is stirred at reflux temperature for 9 h. After cooling to ambient temperature, the solution is basified using concentrated aqueous ammonia solution and the resulting mixture is extracted with EtOAc (2x 50 mL). The combined organic extracts are washed with brine and dried (MgSO₄). Removal of the solvent under reduced pressure affords the title compound.
Yield: 439 mg (53% of theory)
Mass spectrum (ESI⁺): m/z = 202 [M+H]⁺

The following compound may be obtained in analogy to Example LXII:

### (1) 5,6-Dimethyl-1,2,3,4,5,6-hexahydro-2,6-methano-benzo[d]azocine (racemic mixture of diastereomer shown)

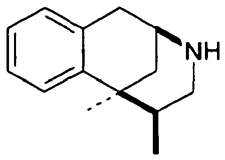

Mass spectrum (ESI⁺): m/z = 202 [M+H]⁺

### Example LXIII

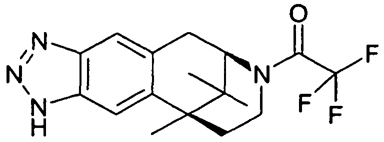

### 2,2,2-Trifluoro-1-[(6R,10S)-5,6,7,8,9,10-hexahydro-10,12,12-trimethyl-6,10-methano-1H-triazolo[5,4-i][3]benzazocin-7-yl]-ethanone

A solution of NaNO₂ (330 mg) in water (2 mL) is slowly added to a flask charged with a stir bar, 1-[(2*R*,6*S*)-8,9-diamino-6,11,11-trimethyl-1,2,5,6-tetrahydro-4H-2,6-methano-benzo[d]azocin-3-yl]-2,2,2-trifluoro-ethanone (650 mg), and acetic acid (15 mL) and chilled in an ice bath. The resulting mixture is stirred in the cooling bath for 2 h and at ambient temperature for 1 h. Then, the solution is poured into ice-cold water and the precipitate formed is separated by filtration and dried to afford the title compound that is used without further purification.
Yield: 610 mg (91 % of theory)
Mass spectrum (ESI⁺): m/z = 353 [M+H]⁺

### Example LXIV

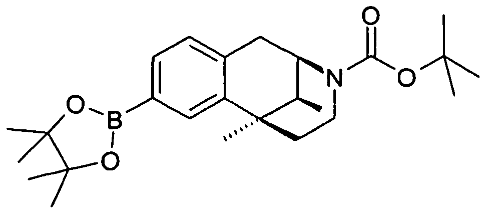

### (2R,6R,11S)-6,11-Dimethyl-8-(4,4,5,5-tetramethyl-[1,3,2]dioxaborolan-2-yl)-1,2,5,6-tetrahydro-4H-2,6-methano-benzo[d]azocine-3-carboxylic acid tert-butyl ester

A flask charged with a stir bar, (2*R*,6*R*,11*S*)-6,11-dimethyl-8-trifluoromethanesulfonyl-oxy-1,2,5,6-tetrahydro-4H-2,6-methano-benzo[d]azocine-3-carboxylic acid tert-butyl ester (9.90 g), bis(pinacolato)diboron (6.15 g), 1,1'-bis(diphenylphosphino)ferrocene (0.73 g), and 1,4-dioxane (50 mL) is flushed with argon for 15 min. Then, 1,1'-bis(diphenylphosphino)-ferrocene-palladium dichloride dichloromethane complex (1.08 g) is added and the mixture is heated to 80 °C. After stirring at 80 °C for 2 d and cooling to ambient temperature, the mixture is diluted with tBuOMe (150 mL) and washed with water (3x 100 mL) and brine (1x 100 mL). The organic phase is dried (MgSO₄) and the solvent is removed under reduced pressure. The residue is purified by chromatography on silica gel (cyclohexane/ethyl acetate 9:1->2:3) to give the title compound as a colorless oil.
Yield: 6.90 g (73% of theory)
Mass spectrum (ESI⁺): m/z = 428 [M+H]⁺

### Example LXV

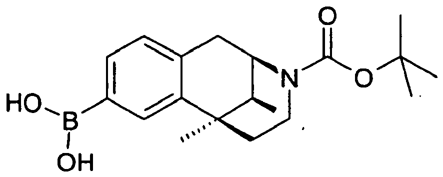

### (2R,6R,11S)-6,11-Dimethyl-8-borono-1,2,5,6-tetrahydro-4H-2,6-methano-benzo[d]azocine-3-carboxylic acid tert-butyl ester

A solution of (2*R*,6*R*,11*S*)-6,11-dimethyl-8-(4,4,5,5-tetramethyl-[1,3,2]dioxaborolan-2-yl)-1,2,5,6-tetrahydro-4H-2,6-methano-benzo[d]azocine-3-carboxylic acid tert-butyl ester (2.50 g) and NalO₄ (5.00 g) in 1 M aqueous NH₄OAc solution (34 mL) and acetone (60 mL) is stirred at room temperature overnight. Then, the solution is concentrated, water is added to the residue, and the resulting mixture is extracted with ethyl acetate. The combined organic extracts are washed with water and brine and dried (Na₂SO₄). The solvent is removed under reduced pressure to give the title compound as a colorless, foam-like solid.
Yield: 1.83 g (91 % of theory)
Mass spectrum (ESI⁻): m/z = 390 [M+HCOO]⁻

### Example LXVI

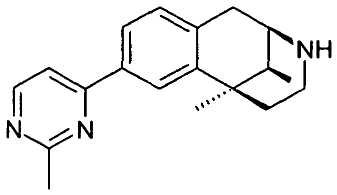

### (2R,6R,11S)-6,11-Dimethyl-8-(2-methyl-pyrimidin-4-yl)-1,2,3,4,5,6-hexahydro-2,6-methano-benzo[d]azocine

Pd(OAc)₂ (3.3 mg) is added to a mixture of (2*R*,6*R*,11*S*)-6,11-dimethyl-8-borono-1,2,5,6-tetrahydro-4H-2,6-methano-benzo[d]azocine-3-carboxylic acid tert-butyl ester (0.30 g), 4-chloro-2-methyl-pyrimidine (93 mg), K₃PO₄ (0.31 g), and 2-dicyclohexyl-phosphino-2',6'-dimethoxy-1,1'-biphenyl (11.5 mg) in n-butanol (2 mL) under argon atmosphere. The resulting mixture is heated to 100 °C and stirred at this temperature overnight. After cooling to room temperature, ethyl acetate is added, the resulting mixture is filtered, and the filtrate is concentrated under reduced pressure. The residue is taken up in CH₂Cl₂ (3 mL) and treated with F₃CCO₂H (0.5 mL) for 1 h. Then, the solution is concentrated and the residue is purified by HPLC on reversed phase (MeCN/H₂O/NH₃) to afford the title compound.
Yield: 0.10 g (48% of theory)
Mass spectrum (ESI⁺): m/z = 294 [M+H]⁺

The following compound may be obtained in analogy to Example LXVI:

### (1) (2R,6R,11S)-6,11-Dimethyl-8-pyrimidin-4-yl-1,2,3,4,5,6-hexahydro-2,6-methano-benzo[d]azocine

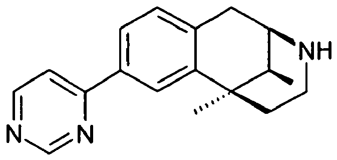

Mass spectrum (ESI⁺): m/z = 280 [M+H]⁺

### ExampleLXVII

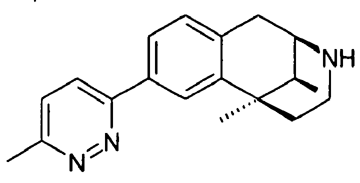

### (2R,6R,11S)-6,11-Dimethvl-8-(6-methyl-pyridazin-3-yl)-1,2,3,4,5,6-hexahydro-2,6-methano-benzo[d]azocine

2 M Aqueous Na₂CO₃ solution (1.13 mL) is added to a mixture of (2*R*,6*R*,11*S*)-6,11-dimethyl-8-(4,4,5,5-tetramethyl-[1,3,2]dioxaborolan-2-yl)-1,2,5,6-tetrahydro-4H-2,6-methano-benzo[d]azocine-3-carboxylic acid tert-butyl ester (483 mg) and 3-chloro-6-methyl-pyridazine (218 mg) in dimethylformamide (2 mL). The resulting mixture is flushed with argon and then 1,1'-bis(diphenylphosphino)ferrocene-palladium dichloride dichloromethane complex (73 mg) is added. The mixture is heated to 100 °C and stirred at this temperature overnight. After cooling to room temperature, water is added and the resulting mixture is extracted with ethyl acetate. The combined organic extracts are washed with water and brine and dried (MgSO₄). The solvent is removed under reduced pressure and the residue is taken up in CH₂Cl₂ (3 mL) and treated with F₃CCO₂H (0.5 mL) for 1 h. Then, the solution is concentrated and the residue is purified by HPLC on reversed phase (MeCN/H₂O/NH₃) to afford the title compound.
Yield: 225 mg (68% of theory)
Mass spectrum (ESI⁺): m/z = 294 [M+H]⁺

The following compounds may be obtained in analogy to Example LXVII:

### (1) (2R,6R,11S)-6,11-Dimethyl-8-(1-methyl-2-oxo-1,2-dihydro-pyridin-4-yl)-1,2,3,4,5,6-hexahydro-2,6-methano-benzo[d]azocine

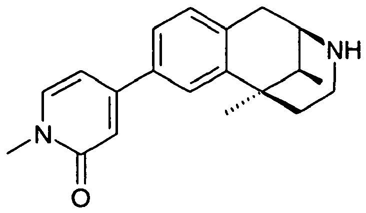

Mass spectrum (ESI⁺): m/z = 309 [M+H]⁺

Trifluoro-methanesulfonic acid 1-methyl-2-oxo-1,2-dihydro-pyridin-4-yl ester or 4-bromo-1-methyl-1 H-pyridin-2-one are used as the coupling partner

### (2) 5-[(2R,6R,11S)-6,11-Dimethyl-1,2,3,4,5,6-hexahydro-2,6-methano-benzo[d]azocin-8-yl]-1-methyl-1H-pyridin-2-one

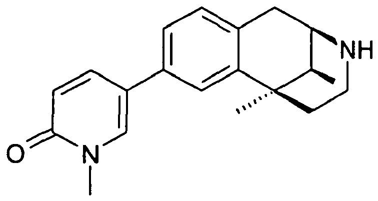

### (3) 6-[(2R,6R,11S)-6,11-Dimethyl-1,2,3,4,5,6-hexahydro-2,6-methano-benzo[d]azocin-8-yl]-2-methyl-2H-pyridazin-3-one

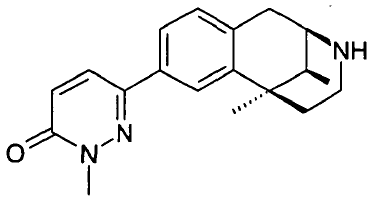

6-Chloro-2-methyl-2H-pyridazin-3-one is used as the coupling partner.

### (4) (2R,6R,11S)-6,11-Dimethyl-8-(2-methyl-pyrimidin-5-yl)-1,2,3,4,5,6-hexahydro-2,6-methano-benzo[d]azocine

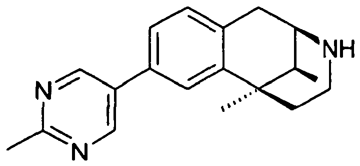

Mass spectrum (ESI⁺): m/z = 294 [M+H]⁺

5-Bromo-2-methyl-pyrimidine is used as the coupling partner.

### Example LXVIII

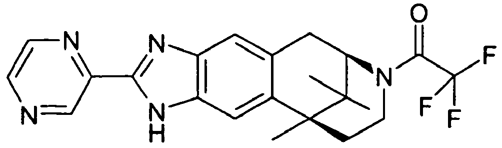

### 2,2,2-Trifluoro-1-[(6R,10S)-5,6,7,8,9,10-hexahydro-10,12,12-trimethyl-2-pyrazin-2-yl-6,10-methano-1H-imidazo[5,4-i][3]benzazocin-7-yl]-ethanone

A solution of pyrazine-2-carboxylic acid (152 mg), 2-(1H-benzotriazol-1-yl)-1,1,3,3-tetramethyluronium tetrafluoroborate (397 mg), and triethylamine (0.5 mL) in dimethylformamide (5 mL) is stirred at room temperature for 30 min, before 1-[(2*R*,6*S*)-8,9-diamino-6,11,11-trimethyl-1,2,5,6-tetrahydro-4H-2,6-methano-benzo[d]azocin-3-yl]-2,2,2-trifluoro-ethanone (300 mg) is added. The solution is stirred at room temperature overnight. Then, the solution is diluted with EtOAc and washed with water and 2 M aqueous K₂CO₃ solution and dried (MgSO₄). The solvent is removed under reduced pressure and the residue is taken up in acetic acid (5 mL). The resulting solution is heated at 80 °C overnight. Then, the solvent is removed under reduced pressure and the residue is evaporated twice with toluene to give the crude title compound that is used without further purification.
Yield: 380 mg (quantitative)
Mass spectrum (ESI⁺): m/z = 430 [M+H]⁺

The following compounds may be obtained in analogy to Example LXVIII:

### (1) 1-[(6R,10S)-2-(1-Acetyl-piperidin-4-yl)-5,6,7,8,9,10-hexahydro-10,12,12-trimethyl-6,10-methano-1H-imidazo[5,4-i][3]benzazocin-7-yl]-2,2,2-trifluoro-ethanone

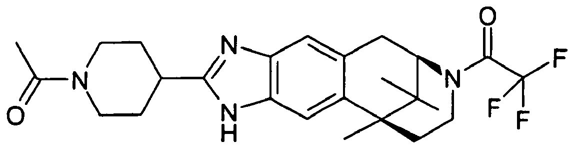

Mass spectrum (ESI⁺): m/z = 477 [M+H]⁺

### (2) 1-[(6R,10S)-2-Cyclopropyl-5,6,7,8,9,10-hexahydro-10,12,12-trimethyl-6,10-methano-1H-imidazo[5,4-i][3]benzazocin-7-yl]-2,2,2-trifluoro-ethanone

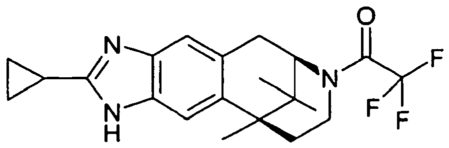

### (3) 2,2,2-Trifluoro-1-[(6R,10S)-5,6,7,8,9,10-hexahydro-10,12,12-trimethyl-2-(1-methyl-6-oxo-1,6-dihydro-pyridin-3-yl)- 6,10-methano-1H-imidazo[5,4-i][3]benzazocin-7-yl]-ethanone

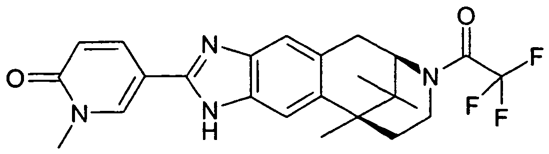

### (4) 1-[(6R,10S)-2-tert Butyl-5,6,7,8,9,10-hexahydro-10,12,12-trimethyl-6,10-methano-1H-imidazo[5,4-i][3]benzazocin-7-yl]-2,2,2-trifluoro-ethanone

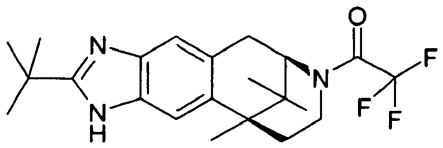

### (5) 2,2,2-Trifluoro-1-[(6R,10S)-5,6,7,8,9,10-hexahydro-10,12,12-trimethyl-2-pyridin-3-yl-6,10-methano-1H-imidazo[5,4-i][3Jbenzazocin-7-yl]-ethanone

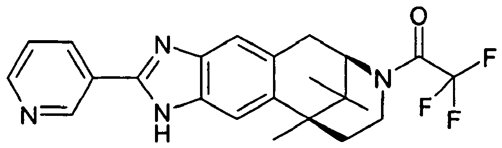

### (6) 2,2,2-Trifluoro-1-{(6R,10S)-5,6,7,8,9,10-hexahydro-10,12,12-trimethyl-2-[(S)-tetrahydrofuran-2-yl]-6,10-methano-1H-imidazo[5,4-i][3]benzazocin-7-yl}-ethanone

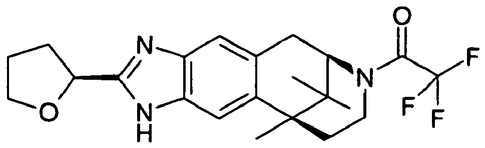

### (7) 2,2,2-Trifluoro-1-[(6R,10S)-5,6,7,8,9,10-hexahydro-10,12,12-trimethyl-2-pyridazin-4-yl-6,10-methano-1H-imidazo[5,4-i][3]benzazocin-7-yl]-ethanone

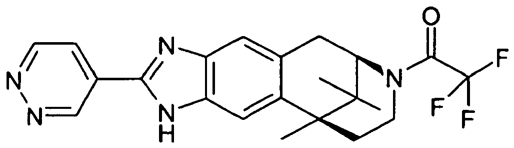

### (8) 2,2,2-Trifluoro-1-[(6R,10S)-5,6,7,8,9,10-hexahydro-10,12,12-trimethyl-2-(5-methyl-pyrazin-2-yl)-6,10-methano-1H-imidazo[5,4-i][3]benzazocin-7-yl]-ethanone

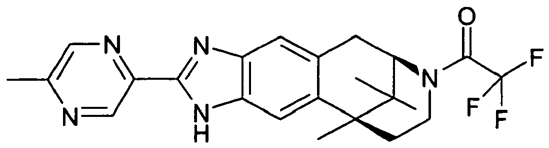

### (9) 2,2,2-Trifluoro-1-{(6R,10S)-5,6,7,8,9,10-hexahydro-10,12,12-trimethyl-2-[(R)-tetrahydrofuran-2-yl]-6,10-methano-1H-imidazo[5,4-i][3]benzazocin-7-yl}-ethanone

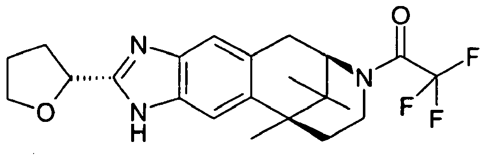

Mass spectrum (ESI⁺): m/z = 422 [M+H]⁺

### Example LXIX

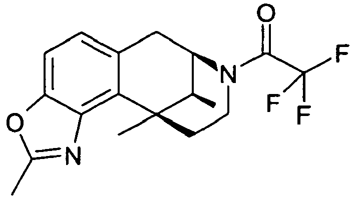

### 2,2,2-Trifluoro-1-[(7R,11R,12S)-6,7,8,9,10,11-hexahydro-2,11,12-trimethyl-7,11-methano-1H-oxazolo[4,5-h][3]benzazocin-8-yl]-ethanone

1-[(2*R*,6*R*,1*S*)-7-Amino-8-hydroxy-6,11-dimethyl-1,2,5,6-tetrahydro-4H-2,6-methano-benzo[d]azocin-3-yl]-2,2,2-trifluoro-ethanone (200 mg) taken up in trimethyl orthoacetate (1 mL) is heated at 100 °C for 3 h. After cooling to ambient temperature, the mixture is concentrated and the residue is triturated with little methanol and dried to give the title compound.
Yield: 100 mg (47% of theory)
Mass spectrum (ESI⁺): m/z = 353 [M+H]⁺

The following compounds may be obtained in analogy to Example LXIX:

### (1) 2,2,2-Trifluoro-1-[(6R,10S)-5,6,7,8,9,10-hexahydro-2,10,12,12-tetramethyl-6,10-methano-oxazolo[4,5-i][3]benzazocin-7-yl]-ethanone

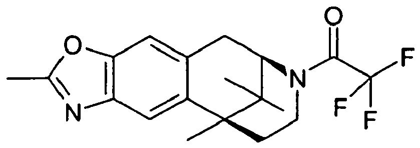

Mass spectrum (ESI⁺): m/z = 367 [M+H]⁺

### (2) 2,2,2-Trifluoro-1-[(6R,10R,12S)-5,6,7,8,9,10-hexahydro-2,10,12-trimethyl-6,10-methano-oxazolo[5,4-i][3]benzazocin-7-yl]-ethanone

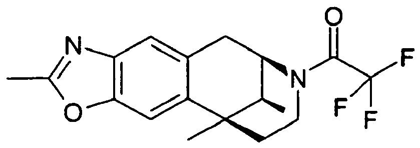

Mass spectrum (ESI⁺): m/z = 353 [M+H]⁺

### Example LXX

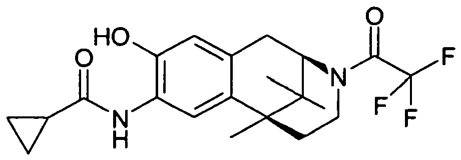

### Cyclopropanecarboxytic acid [(2R,6S)-9-hydroxy-6,11,11-trimethyl-3-(2,2,2-trifluoroacetyl)-1,2,3,4,5,6-hexahydro-2,6-methano-benzo[d]azocin-8-yl]-amide

Cyclopropylcarbonyl chloride (0.13 mL) is added to a solution of 1-[(2*R*,6*S*)-8-amino-9-hydroxy-6,11,11-trimethyl-1,2,5,6-tetrahydro-4H-2,6-methano-benzo[d]azocin-3-yl]-2,2,2-trifluoro-ethanone (0.50 g) and triethylamine (0.25 mL) in dichloromethane (3 mL). After stirring the solution at room temperature overnight, concentrated aqueous ammonia solution (1 mL) and methanol (2 mL) are added and the resulting mixture is stirred for additional 2 h. Then, the solution is concentrated and water is added to the residue. The resulting mixture is extracted with ethyl acetate and the combined organic extracts are washed with brine and dried (MgSO₄). The solvent is removed under reduced pressure to give the crude title compound that is used without further purification.
Yield: 0.62 g (quantitative)

The following compounds may be obtained in analogy to Example LXX:

### (1) Cyclopropanecarboxylic acid [(2R,6R,11S)-9-hydroxy-6,11-dimethyl-3-(2,2,2-trifluoro-acetyl)-1,2,3,4,5,6-hexahydro-2,6-methano-benzo[d]azocin-8-yl]-amide

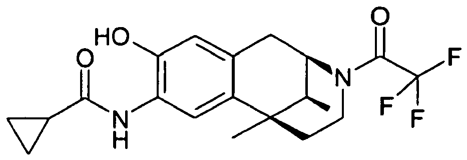

### (2) Cyclopropanecarboxylic acid [(2R,6R,11S)-8-hydroxy-6,11-dimethyl-3-(2,2,2-trifluoro-acetyl)-1,2,3,4,5,6-hexahydro-2,6-methano-benzo[d]azocin-9-yl]-amide

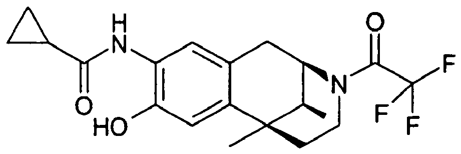

Mass spectrum (ESI⁺): m/z = 397 [M+H]⁺

### (3) N-[(2R,6S)-9-Hydroxy-6,11,11-trimethyl-3-(2,2,2-trifluoro-acetyl)-1,2,3,4,5,6-hexahydro-2,6-methano-benzo[d]azocin-8-yl]-2,2-dimethyl-propionamide

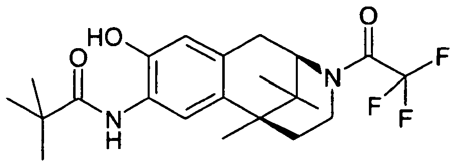

### (4) 5-Methyl-pyrazine-2-carboxylic acid [(2R,6S)-9-hydroxy-6,11,11-trimethyl-3-(2,2,2-trifluoro-acetyl)-1,2,3,4,5,6-hexahydro-2,6-methano-benzo[d]azocin-8-yl]-amide

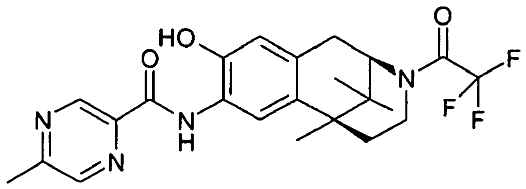

Mass spectrum (ESI⁺): m/z = 463 [M+H]⁺

Alternatively, the compound may be obtained from 5-methyl-pyrazine-2-carboxylic acid using 2-(1H-benzotriazol-1-yl)-1,1,3,3-tetramethyluronium tetrafluoroborate and ethyldiisopropylamine in dimethylformamide as described in Example LXXXII.

### (5) 5-Methyl-pyrazine-2-carboxylic acid [(2R,6R,11S)-8-hydroxy-6,11-dimethyl-3-(2,2,2-trifluoro-acetyl)-1,2,3,4,5,6-hexahydro-2,6-methano-benzo[d]azocin-9-yl]-amide

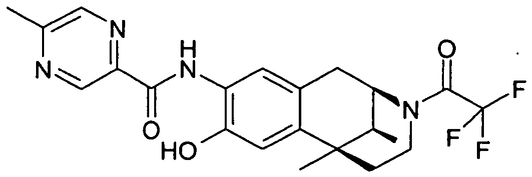

Mass spectrum (ESI⁺): m/z = 449 [M+H]⁺

Alternatively, the compound is obtained from 5-methyl-pyrazine-2-carboxylic acid using 2-(1H-benzotriazol-1-yl)-1,1,3,3-tetramethyluronium tetrafluoroborate and ethyldiisopropylamine in dimethylformamide as described in Example LXXXII.

### (6) (R)-Tetrahydro-furan-2-carboxylic acid [(2R,6S)-9-hydroxy-6,11,11-trimethyl-3-(2,2,2-trifluoro-acetyl)-1,2,3,4,5,6-hexahydro-2,6-methano-benzo[d]azocin-8-yl]-amide

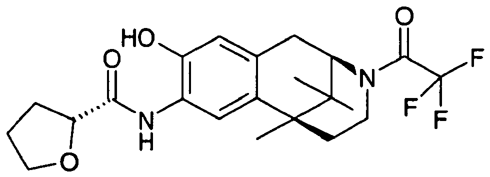

Preferably, the compound is obtained from (*R*)-tetrahydro-furan-2-carboxylic acid using 2-(1H-benzotriazol-1-yl)-1,1,3,3-tetramethyluronium tetrafluoroborate and ethyldiisopropylamine in dimethylformamide as described in Example LXXXII.

### (7) (S)-Tetrahydro-furan-2-carboxylic acid [(2R,6S)-9-hydroxy-6,11,11-trimethyl-3-(2,2,2-trifluoro-acetyl)-1,2,3,4,5,6-hexahydro-2,6-methano-benzo[d]azocin-8-yl]-amide

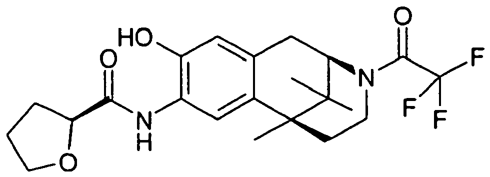

Preferably, the compound is obtained from (*S*)-tetrahydro-furan-2-carboxylic acid using 2-(1H-benzotriazol-1-yl)-1,1,3,3-tetramethyluronium tetrafluoroborate and ethyldiisopropylamine in dimethylformamide as described in Example LXXXII.

### Example LXXI

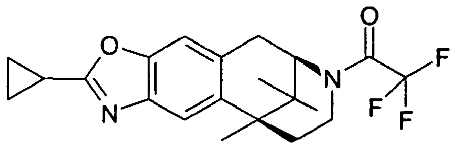

### 1-[(6R,10S)-2-Cyclopropyl-5,6,7,8,9,10-hexahydro-10,12,12-trimethyl-6,10-methano-oxazolo[4,5-i][3]benzazocin-7-yl]-2,2,2-trifluoro-ethanone

A solution of cyclopropanecarboxylic acid [(2*R*,6*S*)-9-hydroxy-6,11,11-trimethyl-3-(2,2,2-trifluoro-acetyl)-1,2,3,4,5,6-hexahydro-2,6-methano-benzo[d]azocin-8-yl]-amide (0.62 g) and pyridinium p-toluenesulfonate (76 mg) in xylene (6 mL) is stirred at reflux temperature for 5 h. After cooling to room temperature, the solution is concentrated, ethyl acetate is added to the residue, and the resulting mixture is washed with water and brine. The organic solution is dried (MgSO₄) and the solvent is evaporated to afford the title compound.
Yield: 0.52 g (89% of theory)

The following compounds may be obtained in analogy to Example LXXI:

### (1) 1-[(6R,10R,12S)-2-Cyclopropyl-5,6,7,8,9,10-hexahydro-10,12-dimethyl-6,10-methano-oxazolo[4,5-i][3]benzazocin-7-yl]-2,2,2-trifluoro-ethanone

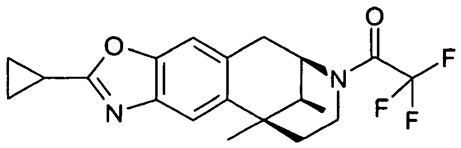

### (2) 1-[(6R,10R,12S)-2-Cyclopropyl-5,6,7,8,9,10-hexahydro-10,12-dimethyl-6,10-methano-oxazolo[5,4-i][3]benzazocin-7-yl]-2,2,2-trifluoro-ethanone

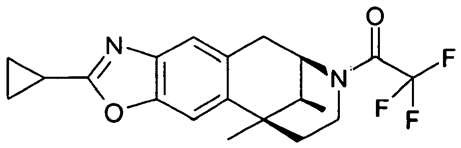

Mass spectrum (ESI⁺): m/z = 379 [M+H]⁺

### (3) 1-[(6R,10S)-2-tert-Butyl-5,6,7,8,9,10-hexahydro-10,12,12-trimethyl-6,10-methano-oxazolo[4,5-i][3]benzazocin-7-yl]-2,2,2-trifluoro-ethanone

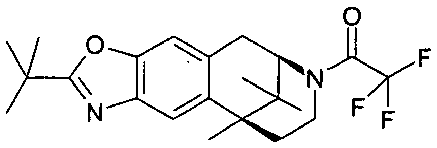

### (4) 2,2,2-Trifluoro-1-[(6R,10S)-5,6,7,8,9,10-hexahydro-10,12,12-trimethyl-2-(5-methyl-pyrazin-2-yl)-6,10-methano-oxazolo[4,5-i][3]benzazocin-7-yl]-ethanone

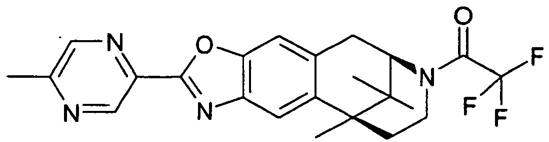

### (5) 2,2,2-Trifluoro-1-[(6R,10R,12S)-5,6,7,8,9,10-hexahydro-10,12-dimethyl-2-(5-methyl-pyrazin-2-yl)-6,10-methano-oxazolo[5,4-i][3]benzazocin-7-yl]-ethanone

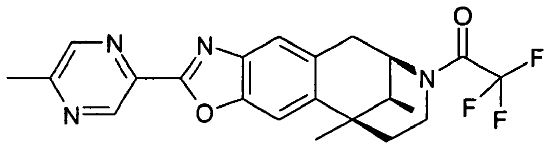

### (6) 2,2,2-Trifluoro-1-[(6R,10S)-5,6,7,8,9,10-hexahydro-10,12,12-trimethyl-2-[(R)-tetrahydrofuran-2-yl]-6,10-methano-oxazolo[4,5-i][3]benzazocin-7-yl]-ethanone

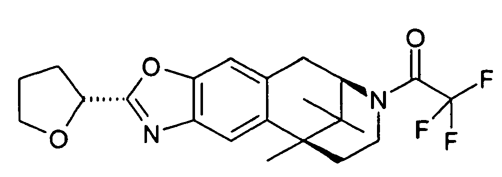

### (7) 2,2,2-Trifluoro-1-[(6R,10S)-5,6,7,8,9,10-hexahydro-10,12,12-trimethyl-2-[(S)-tetrahydrofuran-2-yl]-6,10-methano-oxazolo[4,5-i][3]benzazocin-7-yl]-ethanone

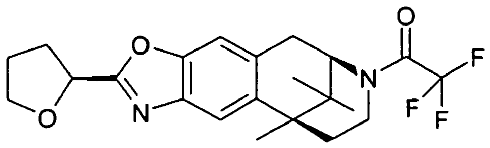

### Example LXXII

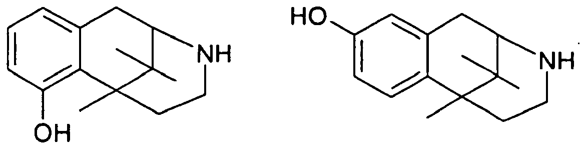

### 6,11,11-Trimethyl-1,2,3,4,5,6-hexahydro-2,6-methano-benzo[d]azocin-7-ol and 6,11,11-trimethyl-1,2,3,4,5,6-hexahydro-2,6-methano-benzo[d]azocin-9-ol

2-(3-Methoxy-benzyl)-3,3-dimethyl-4-methylene-piperidine-1-carbaldehyde (for preparation see J. Med. Chem. 1997, 40, 2928-2939; 47.5 g) is combined with 48% HBr in water (300 mL). The mixture is heated to reflux temperature and stirred at this temperrature for 24 h. After cooling to ambient temperature, the precipitate is separated by filtration, washed with water, and triturated with acetone. Then, the precipitate is taken up in a mixture of 1 N aqueous NaOH solution and CH₂Cl₂. The CH₂Cl₂ phase is separated, dried (Na₂SO₄), and concentrated. The residue is recrystallized from EtOAc to afford 6,11,11-trimethyl-1,2,3,4,5,6-hexahydro-2,6-methano-benzo[d]azocin-7-ol. The filtrate of the reaction mixture is combined with the water and acetone phases (from washing and triturating the precipitate) and basified using concentrated aqueous ammonia solution. The resulting mixture is extracted with CH₂Cl₂, the combined organic extracts are dried (MgSO₄), and the solvent is evaporated. The residue is purified by chromatography on silica gel (EtOAc/MeOH/ NH₄OH 90:10:1->70:30:3) to afford 6,11,11-trimethyl-1,2,3,4,5,6-hexahydro-2,6-methano-benzo[d]azocin-9-ol.
6,11,11-Trimethyl-1,2,3,4,5,6-hexahydro-2,6-methano-benzo[d]azocin-7-ol:
Yield: 5.2 g (13% of theory)
Mass spectrum (ESI⁺): m/z = 232 [M+H]⁺
6,11,11-Trimethyl-1,2,3,4,5,6-hexahydro-2,6-methano-benzo[d]azocin-9-ol: Yield: 9.3 g (23% of theory)
Mass spectrum (ESI⁺): m/z = 232 [M+H]⁺

### Example LXXIII

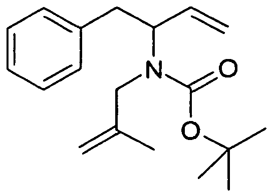

### (1-Benzyl-allyl)-(2-methyl-allyl)-carbamic acid tert-butyl ester

NaH (60% in mineral oil, 0.15 g) is added to a solution of (1-benzyl-allyl)-carbamic acid tert-butyl ester (for preparation see e.g. Eur. J. Org. Chem. 2002, 1, 139-144; 0.86 g) in N-methylpyrrolidinone (5 mL). The resulting mixture is stirred at room temperature for 30 min, before 3-bromo-2-methyl-propene (0.38 mL) is added. After stirring for 5 h, brine is added and the resulting mixture is extracted with EtOAc. The combined organic extracts are dried (Na₂SO₄), the solvent is evaporated, and the residue is purified by chromatography on silica gel (cyclohexane/EtOAc 1:0→1:1).
Yield: 0.79 g (75% of theory)
Mass spectrum (ESI⁺): m/z = 302 [M+H]⁺

The following compound may be obtained in analogy to Example LXXIII:

### (1) (1-Benzyl-allyl)-(4-methyl-pent-4-enyl)-carbamic acid tert-butyl ester

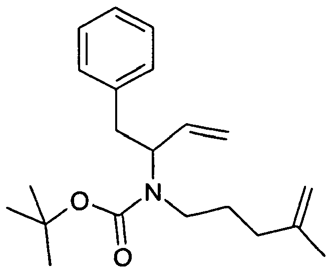

Mass spectrum (ESI⁺): m/z = 330 [M+H]⁺

Methanesulfonic acid 4-methyl-pent-4-enyl ester, prepared from 4-methyl-pent-4-en-1-ol and mesyl chloride in the presence of NEt₃ in dichloromethane, is used as the electrophile.

### Example LXXIV

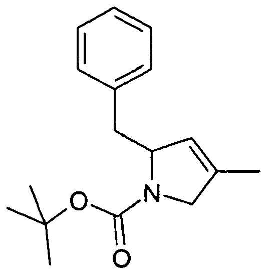

### 2-Benzyl-4-methyl-2,5-dihydro-pyrrole-1-carboxylic acid tert-butyl ester

[(1,3-Bis(2,4,6-trimethylphenyl)-2-imidazolidinylidene]-dichloro-(phenylmethylene)-(tricyclohexylphosphine)-ruthenium (28 mg) is added to a solution of (1-benzyl-allyl)-(2-methyl-allyl)-carbamic acid tert-butyl ester (0.79 g) in toluene (50 mL) under argon atmosphere at room temperature. The resulting mixture is heated to 60 °C and stirred at this temperature for 3 h. After cooling to room temperature, the solvent is removed and the residue is purified by chromatography on silica gel (cyclohexane/ethyl acetate 1:0->1:1).
Yield: 0.40 g (56% of theory)
Mass spectrum (ESI⁺): m/z = 274 [M+H]⁺

The following compound may be obtained in analogy to Example LXXIV:

### (1) 7-Benzyl-5-methyl-2,3,4,7-tetrahydro-azepine-1-carboxylic acid tert-butyl ester

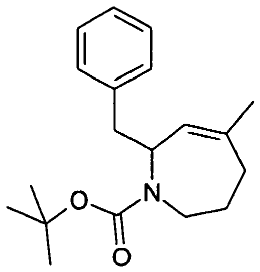

Mass spectrum (ESI⁺): m/z = 302 [M+H]⁺

### Example LXXV

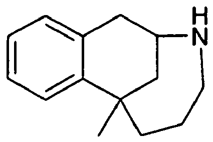

### 1-Methyl-10-aza-tricyclo[7,4,1,0*2,7*]tetradeca-2,4,6-triene

Trifluoromethanesulfonic acid (2.5 mL) is added to a solution of 7-benzyl-5-methyl-2,3,4,7-tetrahydro-azepine-1-carboxylic acid tert-butyl ester (0.30 g) in CH₂Cl₂ (5 mL) chilled in an ice bath. The ice bath is removed and the solution is stirred at room temperature for 5 h. Then, ice-cold water and aqueous K₂CO₃ solution are added and the resulting mixture is extracted with ethyl acetate. The combined extracts are dried (Na₂SO₄), the solvent is evaporated, and the residue is purified by chromatography on silica gel (dichloromethane/methanol 99:1->9:1).
Yield: 0.10g (50% of theory)

### Example LXXVI

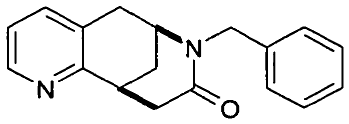

### 7-Benzyl-6,7,9,10-tetrahydro-5H-6,10-methano-pyrido[3,2-d]azocin-8-one (racemic mixture of diastereomer shown)

A flask charged with a stir bar, 2-benzyl-2-aza-bicyclo[3.3.1]nonane-3,6-dione (for preparation see J. Chem. Soc., Perkin Trans. 1, 1999, 1157-1162*;* 0.80 g), NaAuCl₄* 2 H₂O (30 mg), propargylamine (0.45 mL), and ethanol (5 mL) is heated at 100 °C with microwave irradiation for 10 min. After cooling to room temperature, the mixture is filtered and the filtrate is concentrated. The residue is purified by chromatography on silica gel (cyclohexane/ethyl acetate/methanol 6:4:1).
Yield: 0.52 g (56% of theory)

### Example LXXVII

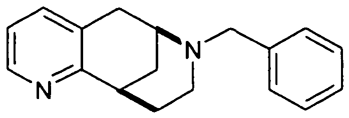

### 7-Benzyl-5,6,7,8,9,10-hexahydro-6,10-methano-pyrido[3,2-d]azocine (racemic mixture of diastereomer shown)

LiAlH₄ (1 M in THF, 4.5 mL) is added dropwise to a solution of 7-benzyl-6,7,9,10-tetrahydro-5H-6,10-methano-pyrido[3,2-d]azocin-8-one (0.55 g) in tetrahydrofuran (3 mL) chilled in an ice bath. The cooling bath is removed and the mixture is stirred at room temperature for 2 h. Ice-cold water and 4 M hydrochloric acid (4 mL) are added and the mixture is stirred for another 15 min. Then, the mixture is basified using 4 M aqueous NaOH solution and the mixture is extracted with ethyl acetate. The combined organic extracts are dried (Na₂SO₄), the solvent is evaporated, and the residue is purified by chromatography on silica gel (cyclohexane/EtOAc/methanol 4:1:0->1:1:1).
Yield: 0.16 g (32% of theory)

The following compounds may be obtained in analogy to Example LXXVII:

### (1) 9-Benzyl-3,5,9-triaza-tricyclo[6.3.1.0*2,6*]dodeca-2(6),4-diene (racemic mixture of diastereomer shown)

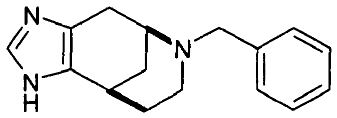

Mass spectrum (ESI⁺): m/z = 254 [M+H]⁺

### (2) 9-Benzyl-4-methyl-3,5,9-triaza-tricyclo[6.3.1.0*2,6*]dodeca-2(6),4-diene (racemic mixture of diastereomer shown)

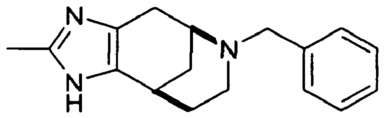

Mass spectrum (ESI⁺): m/z = 268 [M+H]⁺

### (3) 2,3,4,4a,9,9a-Hexahydro-1H-indeno[2,1-b]pyridine (racemic mixture of diastereomer shown)

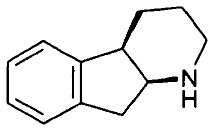

Mass spectrum (ESI⁺): m/z = 174 [M+H]⁺

The reaction mixture is stirred at reflux temperature for 1 h after stirring with LiAlH₄ at ambient temperature for 2 h.

### Example LXXVIII

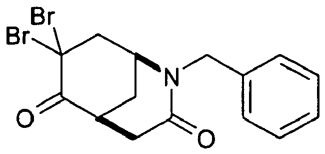

### 2-Benzyl-7,7-dibromo-2-aza-bicyclo[3.3.1]nonane-3,6-dione (racemic mixture of diastereomer shown)

A solution of bromine (1.2 mL) in acetic acid (5 mL) is added to a solution of 2-benzyl-2-aza-bicyclo[3.3.1]nonane-3,6-dione (for preparation see J. Chem. Soc., Perkin Trans. 1, 1999, 1157-1162*;* 3.05 g) in acetic acid (40 mL). The resulting solution is stirred at room temperature for 2 h. Then, the solution is poured into ice-cold water and the resulting mixture is extracted with ethyl acetate. The combined organic extracts are dried (Na₂SO₄) and the solvent is evaporated to afford the title compound as a solid.
Yield: 4.69 g (88% of theory)
Mass spectrum (ESI⁺): m/z = 400/402/404 (2 Br) [M+H]⁺

### Example LXXIX

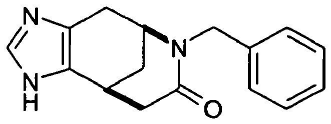

### 9-Benzyl-3,5,9-triaza-tricyclo[6.3.1.0*2.6*]dodeca-2(6),4-dien-10-one (racemic mixture of diastereomer shown)

A mixture of 2-benzyl-7,7-dibromo-2-aza-bicyclo[3.3.1]nonane-3,6-dione (one diastereomer, 2.50 g), paraforamaldehyde (0.19 g), and ca. 7 M ammonia in methanol (25 mL) is stirred at room temperature overnight. Then, the solution is concentrated and the residue is purified by chromatography on silica gel (CH₂Cl₂/MeOH 99:1->9:1).
Yield: 0.85 g (ca. 85% pure, 44% of theory)
Mass spectrum (ESI⁺): m/z = 268 [M+H]⁺

The following compound may be obtained in analogy to Example LXXIX:

### (1) 9-Benzyl-4-methyl-3,5,9-triaza-tricyclo[6.3.1.0*2,6*]dodeca-2(6),4-dien-10-one (racemic mixture of diastereomer shown)

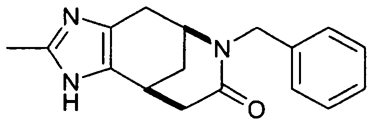

Acetaldehyde is used instead of paraformaldehyde.

### Example LXXX

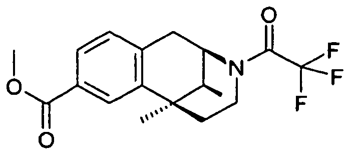

### (2R,6R,11S)-6,11-Dimethyl-3-(2,2,2-trifluoro-acetyl)-1,2,3,4,5,6-hexahydro-2,6-methano-benzo[d]azocine-8-carboxylic acid methyl ester

2,2,6,6-Tetramethylpiperidine (5.4 mL), 1,3-bis(diphenylphosphino)propane (1.30 g), and Pd(OAc)₂ (0.78 g) are added in turn to a flask charged with trifluoromethanesulfonic acid (2*R*,6*R*,11*S*)-6,11-dimethyl-3-(2,2,2-trifluoro-acetyl)-,2,3,4,5,6-hexahydro-2,6-methano-benzo[d]azocin-8-yl ester (7.0 g), dimethylformamide (30 mL), and methanol (30 mL) in argon atmosphere. The reaction flask is put under CO pressure (7 bar) and shaken at 70 °C for 17 h. After cooling to ambient temperature, water is added and the resulting mixture is extracted with Et₂O. The combined organic extracts are washed with water and brine and dried (MgSO₄). The solvent is evaporated to give the title compound as an oil that crystallizes while standing.
Yield: 5.2 g (93% of theory)
Mass spectrum (ESI⁺): m/z = 356 [M+H]⁺

### Example LXXXI

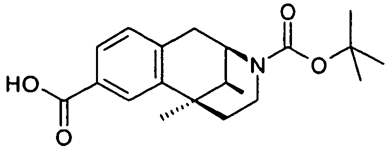

### (2R,6R,11S)-6,11-Dimethyl-1,2,5,6-tetrahydro-4H-2,6-methano-benzo[d]azocine-3,8-dicarboxylic acid 3-tert-butyl ester

4 M aqueous NaOH solution (18.5 mL) is added to a solution of (2*R*,6*R*,11*S*)-6,11-dimethyl-3-(2,2,2-trifluoro-acetyl)-1,2,3,4,5,6-hexahydro-2,6-methano-benzo[d]azocine-8-carboxylic acid methyl ester (5.2 g) in methanol (40 mL). The solution is stirred at room temperature overnight. After neutralization of the solution with MeCOOH, NEt₃ (10 mL) and THF (20 mL) are added and the solution is cooled in an ice bath. Then, di-*tert*butyl dicarbonate (4.0 g) is added, the cooling bath is removed, and the solution is stirred at ambient temperature overnight. 1 M aqueous HCl solution (30 mL) is added and the resulting mixture is extracted with ethyl acetate. The combined organic extracts are washed with brine and dried (MgSO₄). The solvent is evaporated to give the title compound.
Yield: 5.3 g (quantitative)
Mass spectrum (ESI⁺): m/z = 346 [M+H]⁺

### Example LXXXII

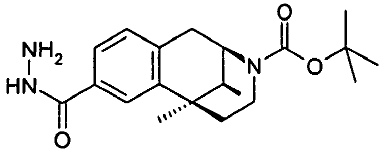

### (2R,6R,11S)-8-Hydrazinocarbonyl-6,11-dimethyl-1,2,5,6-tetrahydro-4H-2,6-methano-benzo[d]azocine-3-carboxylic acid tert-butyl ester

NEt₃ (1.7 mL) and 2-(1H-benzotriazol-1-yl)-1,1,3,3-tetramethyluronium tetrafluoroborate (3.9 g) are added in turn to a solution of (2*R*,6*R*,11*S*)-6,11-dimethyl-1,2,5,6-tetrahydro-4H-2,6-methano-benzo[d]azocine-3,8-dicarboxylic acid 3-*tert*butyl ester (4.2 g) in dimethylformamide (10 mL). The resulting solution is stirred at ambient temperature for 30 min, before hydrazine hydrate (3 mL) is added. The solution is stirred further at room temperature for 1 h and then water (30 mL) is added. The resulting mixture is extracted with ethyl acetate and the combined organic extracts are washed with 1 M aqueous NaOH solution, water, and brine. After drying (MgSO₄), the solvent is evaporated and the residue is purified by chromatography on silica gel (cyclohexane/EtOAc 1:4->0:1) to give the title compound.
Yield: 2.8 g (64% of theory)
Mass spectrum (ESI⁻): m/z = 358 [M-H]⁻

### Example LXXXIII

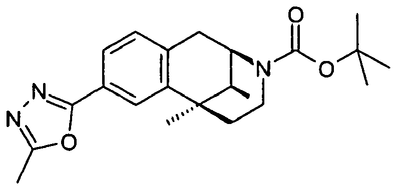

### (2R,6R,11S)-6,11-Dimethyl-8-(5-methyl-[1,3,4]oxadiazol-2-yl)-1,2,5,6-tetrahydro-4H-2,6-methano-benzo[d]azocine-3-carboxylic acid tert-butyl ester

(2*R*,6*R*,11*S*)-8-Hydrazinocarbonyl-6,11-dimethyl-1,2,5,6-tetrahydro-4H-2,6-methano-benzo[d]azocine-3-carboxylic acid tert-butyl ester (0.50 g) in (EtO)₃CMe (2 mL) is heated in a microwave oven at 120 °C for 30 min. After cooling to room temperature, the mixture is concentrated under reduced pressure and the residue is purified by HPLC on reversed phase (MeCN/H₂O/NH₄OH to give the title compound.
Yield: 93 mg (17% of theory)
Mass spectrum (ESI⁺): m/z = 384 [M+H]⁺

The following compound is obtained in analogy to Example LXXXIII:

### (1) (2R,6R,11S)-6,11-Dimethyl-8-[1,3,4]oxadiazol-2-yl-1,2,5,6-tetrahydro-4H-2,6-methano-benzo[d]azocine-3-carboxylic acid tert-butyl ester

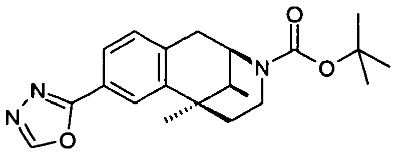

Mass spectrum (ESI⁺): m/z = 370 [M+H]⁺

The reaction is conducted at 145 °C with (EtO)₃CH.

### Example LXXXIV

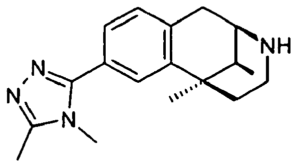

### (2R,6R11S)-8-(4,5-Dimethyl-4H-[1,2,4]triazol-3-yl)-6,11-dimethyl-1,2,3,4,5,6-hexahydro-2,6-methano-benzo[d]azocine

Oxalylic chloride (0.12 mL) is added to a solution N-methylacetamide (102 mg) and 2,6-lutidine (0.33 mL) in dichloromethane (5 mL) chilled in an ice bath. After stirring the solution for 15 min, (2*R*,6*R*,11*S*)-8-hydrazinocarbonyl-6,11-dimethyl-1,2,5,6-tetrahydro-4H-2,6-methano-benzo[d]azocine-3-carboxylic acid tert-butyl ester (0.50 g) is added and the cooling bath is removed. The resulting solution is stirred at ambient temperature for 1 h and then neutralized with aqueous NaHCO₃ solution. The resulting mixture is extracted with dichloromethane, the combined organic extracts are dried (MgSO₄), and the solvent is evaporated. The residue is taken up in acetic acid (3 mL) and stirred at 120 °C for 2.5 h. After cooling to room temperature, the mixture is concentrated under reduced pressure and the residue is taken up in trifluoroacetic acid (1 mL) and dichloromethane (5 mL) to cleave off the tert-butoxycarbonyl group. The solution is stirred at room temperature overnight and then concentrated. The residue is dissolved in little methanol/acetonitrile, neutralized with aqueous ammonia, and purified by HPLC on reversed phase (MeCN/H₂O/NH₄OH) to give the title compound.
Yield: 50 mg (12% of theory)
Mass spectrum (ESI⁺): m/z = 297 [M+H]⁺

### Example LXXXV

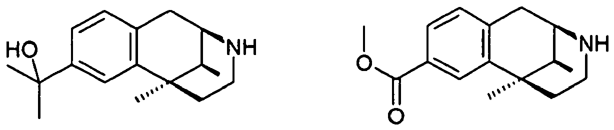

### 2-[(2R,6R,11S-6,11-Dimethyl-1,2,3,4,5,6-hexahdro-2,6-methano-benzo[d]azocin-8-yl]-propan-2-ol and (2R,6R,11S)-6,11-dimethyl-2,3,4,5,6-hexahydro-2,6-methano-benzo[d]azocine-8-carboxylic acid methyl ester

MeMgBr (1.4 mol/L in tetrahydrofuran/toluene, 2.0 mL) is added to a solution of (2*R*,6*R*,11*S*)-6,11-dimethyl-3-(2,2,2-trifluoro-acetyl)-1,2,3,4,5,6-hexahydro-2,6-methano-benzo[d]azocine-8-carboxylic acid methyl ester (0.20 g) in tetrahydrofuran (5 mL) chilled in an ice bath. The solution is stirred with cooling for 2 h, before aqueous NH₄Cl solution is added carefully. The resulting mixture is extracted with ethyl acetate and the combined organic extracts are washed with brine and dried (MgSO₄). The solvent is evaporated to yield a mixture of the title compounds (ca. 4:1 in favor of 2-[(2*R*,6*R*,11*S*)-6,11-dimethyl-1,2,3,4,5,6-hexahydro-2,6-methano-benzo[d]azocin-8-yl]-propan-2-ol).
Yield: 0.15 g
Mass spectrum (ESI⁺): m/z = 260 [M+H]⁺ for both compounds determined with analytical HPLC-MS

### Example LXXXVI

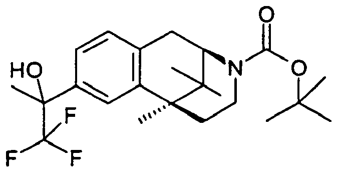

### (2R,6S)-6,11,11-Trimethyl-8-(2,2,2-trifluoro-1-hydroxy-1-methyl-ethyl)-1,2,5,6-tetrahydro-4H-2,6-methano-benzo[d]azocine-3-carboxylic acid tert-butyl ester

Me₃SiCF₃ (2 M in tetrahydrofuran, 0.42 mL) is added dropwise to a mixture of (2*R*,6*S*)-8-acetyl-6,11,11-trimethyl-1,2,5,6-tetrahydro-4H-2,6-methano-benzo[d]azocine-3-carboxylic acid tert-butyl ester (0.30 g) and CsF (13 mg) in tetrahydrofuran (3 mL) cooled to ca. -5 °C. The mixture is stirred at -5 °C for 1.5 h. Then, 1 M aqueous HCI solution (70 mL) is added and the mixture is stirred for 1 h. The mixture is basified using aqueous K₂CO₃ solution and then extracted with ethyl acetate. The combined organic extracts are washed with brine and dried (MgSO₄). The solvent is evaporated to give the crude title compound that is submitted for cleaving the protective group without further purification.
Yield: 0.36 g (crude)

### Example LXXXVII

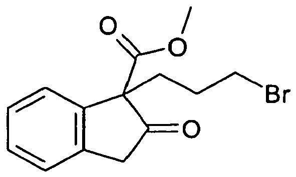

### 1-(3-Bromo-propyl)-2-oxo-indan-1-carboxylic acid methyl ester

A solution of 2-oxo-indan-1-carboxylic acid methyl ester (3.8 g) and NaOH (1 M in water, 20 mL) in ethanol (30 mL) is added dropwise to a solution of 1,3-dibromopropane (10 mL) in ethanol (20 mL) at room temperature. The solution is warmed to 40 °C and stirred at this temperature for 2 d. Then, the solution is concentrated under reduced pressure and ethyl acetate is added to the residue. The resulting mixture is washed with water and brine and dried (MgSO₄). After removing the solvent, the residue is purified by chromatography on silica gel (cyclohexane/EtOAc 20:1->9:1) to give the title compound as an oil.
Yield: 2.1 g (33% of theory)
Mass spectrum (ESI⁺): m/z = 311/313 (Br) **[M+H]⁺**

### Example LXXXVIII

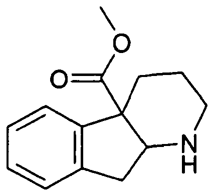

### 1,2,3,4,9,9a-Hexahydro-indeno[2,1-b]pyridine-4a-carboxylic acid methyl ester

NaN₃ (0.44 g) is added to a solution of 1-(3-bromo-propyl)-2-oxo-indan-1-carboxylic acid methyl ester (2.06 g) in dimethylformamide (10 mL) at room temperature. The solution is stirred at room temperature for 4 h and then *^{t}*BuOMe and ethyl acetate are added. The resulting mixture is washed with water and brine and dried (MgSO₄). Most of the organic solvent is evaporated and tetrahydrofuran (10 mL), acetic acid (0.5 mL), and finally 10% Pd/C (150 mg) are added to the residue. The resulting mixture is shaken in hydrogen atmosphere (1 bar) at room temperature for 14 h. Then, the mixture is filtered, the filtrate is concentrated, and the residue is taken up in *^{t}*BuOMe. The organic phase is washed with aqueous Na₂CO₃ solution and brine and dried (MgSO₄). Then, the solvent is evaporated and the residue is dissolved in methanol (10 mL). To the solution is added acetic acid (0.5 mL) and 10% Pd/C (50 mg) and the resulting mixture is shaken under hydrogen atmosphere (1 bar) at room temperature for 6 h. Then, the mixture is filtered and the filtrate is concentrated under reduced pressure to give the crude title compound that is used without further purification.
Yield: 0.44 g (crude)

### Example LXXXIX

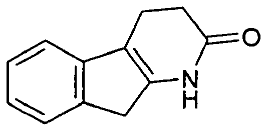

### 1,3,4,9-Tetrahydro-indeno[2,1-b]pyridin-2-one

A mixture of acrylamide (6.15 g) and 1-(1H-Inden-2-yl)-pyrrolidine (for preparation see e.g. J. Org. Chem. 1961, 26, 3761-9; 5.34 g) is stirred under argon atmosphere at 100 °C for 30 min. Then, the mixture is heated to 130 °C and stirred at this temperature for another 15 min. After cooling to ambient temperature, water (50 mL) and acetic acid (5 drops) are added and the resulting mixture is stirred at room temperature for 30 min. Then, ethyl acetate (300 mL) is added and the solution is separated from the precipitate formed thereafter. The organic part of the solution is separated and washed with brine and dried (MgSO₄). The solvent is evaporated and the residue is purified by chromatography on silica gel (cyclohexane/EtOAc 1:1->0:1) to give the title compound as a solid that is triturated with ethyl acetate and dried.
Yield: 1.12 g (21% of theory)
Mass spectrum (ESI⁺): m/z = 186 [M+H]⁺

### Example XC

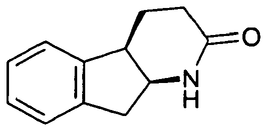

### 1,3,44a,9,9a-Hexahydro-indeno[2,1-b]pyridin-2-one (racemic mixture of diastereomer shown)

10% Palladium on carbon (0.15 g) is added to a solution of 1,3,4,9-tetrahydro-indeno[2,1-b]pyridin-2-one (1.10 g) and acetic acid (0.75 mL) in methanol (20 ml). The resulting mixture is shaken in hydrogen atmosphere (1 bar) at room temperature for 6 h. Then, the mixture is filtered and the filtrate is concentrated. The residue is triturated with *^{t}*BuOMe and dried to yield the title compound as a colorless solid.
Yield: 0.97 g (87% of theory)
Mass spectrum (ESI⁺): m/z = 188 [M+H]⁺

### Example XCI

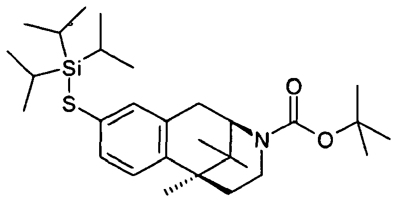

### (2R,6S)-6,11,11-Trimethyl-9-triisopropylsilanylsulfanyl-1,2,5,6-tetrahydro-4H-2,6-methano-benzo[d]azocine-3-carboxylic acid tert-butyl ester

A flask charged with a stir bar, (2*R*,6*S*)-6,11,11-trimethyl-9-trifluoromethanesulfonyl-oxy-1,2,5,6-tetrahydro-4H-2,6-methano-benzo[d]azocine-3-carboxylic acid tert-butyl ester (1.00 g), Cs₂CO₃ (0.92 g), and toluene (20 mL) is sparged with argon for 10 min. Then, triisopropylsilylthiol (0.61 mL) and 1,1'-bis(diphenylphosphino)ferrocene-palladium dichloride dichloromethane complex (135 mg) are added and the mixture is heated to reflux temperature. The mixture is stirred at this temperature for 4 h and then cooled to ambient temperature. Water is added and the resulting mixture is extracted with ethyl acetate. The combined organic extracts are dried (MgSO₄) and the solvent is evaporated. The residue is purified by chromatography on silica gel (cyclohexane/ethyl acetate 20:1->4:1) to give the title compound.
Yield: 0.77 g (70% of theory)
Mass spectrum (ESI⁺): m/z = 504 [M+H]⁺

### Example XCII

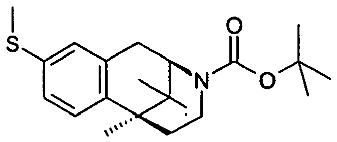

### (2R,6S)-6,11,11-Trimethyl-9-methylsulfanyl-1,2,5,6-tetrahydro-4H-2,6-methano-benzo[d]azocine-3-carboxylicacid tert-butyl ester

Mel (0.38 mL) and CsF (0.11 g) are added to a solution of (2*R*,6*S*)-6,11,11-trimethyl-9-triisopropylsilanyl-sulfanyl-1,2,5,6-tetrahydro-4H-2,6-methano-benzo[d]azocine-3-carboxylic acid tert-butyl ester (0.30 g) in dimethylformamide (2 ml). The resulting mixture is strirred at room temperature overnight. Then, the mixture is concentrated, water is added, and the resulting mixture is extracted with ethyl acetate. The combined extracts are washed with water and brine and dried (MgSO₄) The solvent is evaporated to afford the title compound as a yellow oil.
Yield: 0.21 g (97% of theory)
Mass spectrum (ESI⁺): m/z = 362 [M+H]⁺

### Example XCIII

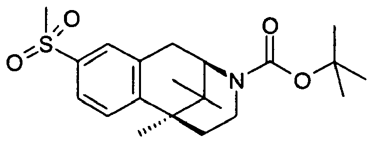

### (2R,6S)-9-Methanesulfonyl-6,11,11-trimethyl-1,2,5,6-tetrahydro-4H-2,6-methano-benzo[d]azocine-3-carboxylic acid tert-butyl ester

3-Chloroperoxybenzoic acid (70%, 0.32 g) is added to a solution of (2*R*,6*S*)-6,11,11-trimethyl-9-methylsulfanyl-1,2,5,6-tetrahydro-4H-2,6-methano-benzo[d]azocine-3-carboxylic acid tert-butyl ester (0.19 g) in dichloromethane (4 ml) chilled in an ice bath. After stirring at room temperature overnight, 10% aqueous K₂CO₃ solution (20 mL) is added to the reaction solution and the resulting mixture is extracted with ethyl acetate. The combined extracts are washed with brine and dried (MgSO₄). The solvent is evaporated to afford the crude title compound as a resin-like solid that is used without further purification.
Yield: 0.21 g (quantitative)
Mass spectrum (ESI⁺): m/z = 394 [M+H]⁺

### Preparation of the end compounds:

### Procedure A (described for Example 1, Table 3)

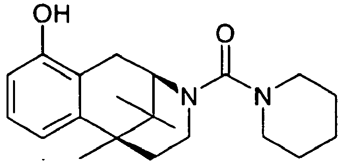

### [(2R,6S)-10-Hydroxy-6,11,11-trimethyl-1,2,5,6-tetrahydro-4H-2,6-methano-benzo[d]azocin-3-yl]-piperidin-1-yl-methanone

Piperidine-1-carbonyl chloride (92 µL) is added to a solution of (2*R*,6*S*)-6,11,11-trimethyl-1,2,3,4,5,6-hexahydro-2,6-methano-benzo[d]azocin-10-ol (0.15 g) and triethylamine (0.15 mL) in dimethylformamide (2 mL). The resulting solution is stirred at room temperature for 3 h. Then, water is added and the mixture is extracted with ethyl acetate. The combined organic extracts are dried (Na₂SO₄) and the solvent is evaporated. The residue is purified by HPLC on reversed phase (H₂O/MeCN).
Yield: 85 mg (38% of theory)
Mass spectrum (ESI⁺): m/z = 343 [M+H]⁺

### Procedure B (described for Example 7, Table 3)

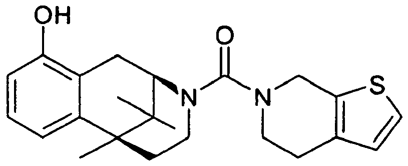

### (4,7-Dihydro-5H-thieno[2,3-c]pyridin-6-yl)-[(2R,6S)-10-hydroxy-6,11,11-trimethyl-1,2,5,6-tetrahydro-4H-2,6-methano-benzo[d]azocin-3-yl]-methanone

4,5,6,7-Tetrahydro-thieno[2,3-c]pyridine (91 mg) is added to a solution of triphosgene (68 mg) and triethylamine (0.23 mL) in dichloromethane (4 mL) chilled in an ice bath. The resulting solution is stirred for 0.5 h before (2*R*,6*S*)-6,11,11-trimethyl-1,2,3,4,5,6-hexahydro-2,6-methano-benzo[d]azocin-10-ol (0.15 g) in dichloromethane (2 mL) is added. The cooling bath is removed and the solution is stirred at room temperature overnight. Then, water is added and the mixture is extracted with ethyl acetate. The combined organic extracts are dried (Na₂SO₄) and the solvent is evaporated. The residue is purified by HPLC on reversed phase (H₂O/MeCN).
Yield: 25 mg (10% of theory)
Mass spectrum (ESI⁺): m/z = 397 [M+H]⁺
Remark: The order of addition of the two amino compounds to phosgene may be reversed.

### Procedure C (described for Example 9, Table 3)

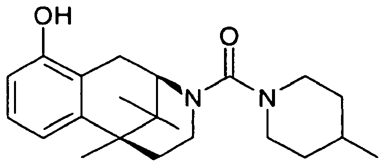

### [(2R,6S)-10-Hydroxy-6,11,11-trimethyl-1,2,5,6-tetrahydro-4H-2,6-methano-benzo[d]azocin-3-yl]-(4-methyl-piperidin-1-yl)-methanone

A mixture of 3-[(2*R*,6*S*)-10-hydroxy-6,11,11-trimethyl-1,2,5,6-tetrahydro-4H-2,6-methano-benzo[d]azocine-3-carbonyl]-1-methyl-3H-imidazol-1-iumiodide (116 mg) and 4-methylpiperidine (0.06 mL) is stirred at 90°C for 3 h. After cooling to ambient temperature, the mixture is purified by HPLC on reversed phase (water/acetonitrile).
Yield: 62 mg (70% of theory)
Mass spectrum (ESI⁺): m/z = 357 [M+H]⁺
Remark: If the amine is a solid the reaction is conducted in tetrahydrofuran (1 mL) as described above.

### Procedure D (described for Example 31, Table 3)

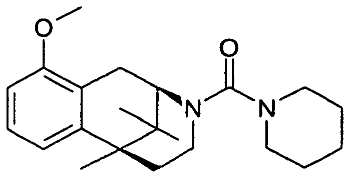

### [(2R,6S)-10-Methoxy-6,11,11-trimethyl-1,2,5,6-tetrahydro-4H-2,6-methano-benzo[d]azocin-3-yl]-piperidin-1-yl-methanone

Methyl iodide (41 µL) is added to a mixture of [(2*R*,6*S*)-10-hydroxy-6,11,11-trimethyl-1,2,5,6-tetrahydro-4H-2,6-methano-benzo[d]azocin-3-yl]-piperidin-1-yl-methanone (200 mg) and potassium carbonate (0.11 g) in dimethylformamide (3 mL). The resulting mixture is stirred at ambient temperature overnight. Then, water is added and the resulting mixture is extracted with ethyl acetate. The combined extracts are dried (Na₂SO₄) and the solvent is evaporated. The residue is purified by HPLC on reversed phase (water/acetonitrile).
Yield: 100 mg (48% of theory)
Mass spectrum (ESI⁺): m/z = 357 [M+H]⁺

### Procedure E (described for Example 34, Table 3)

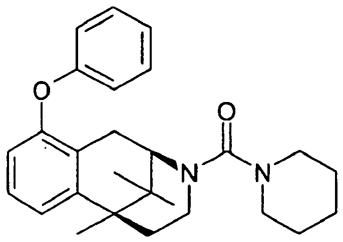

### Piperidin-1-yl-[(2R,6S)-6,11,11-trimethyl-10-phenoxy-1,2,5,6-tetrahydro-4H-2,6-methano-benzo[d]azocin-3-yl]-methanone

Cu(OAc)₂ (0.11 g) is added to a mixture of [(2*R*,6*S*)-10-hydroxy-6,11,11-trimethyl-1,2,5,6-tetrahydro-4H-2,6-methano-benzo[d]azocin-3-yl]-piperidin-1-yl-methanone (0.20 g), phenylboronic acid (0.14 g), molecular sieves 4A (0.4 g), and pyridine (0.25 mL) in dichloromethane (2 mL). The resulting mixture is stirred in air at ambient temperature overnight. Then, the mixture is filtered through Celite and the filtrate is concentrated under reduced pressure. The residue is triturated with methanol to give after drying the pure title compound.
Yield: 45 mg (18% of theory)
Mass spectrum (ESI⁺): m/z = 419 [M+H]⁺

### Procedure F (described for Example 35, Table 3)

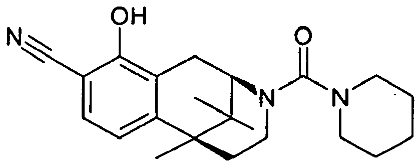

### (2R,6S)-10-Hydroxy-6,11,11-trimethyl-3-(piperidine-1-carbonyl)-1,2,3,4,5,6-hexahydro-2,6-methano-benzo[d]azocine-9-carbonitrile

Boron trichloride (1 M in heptane, 3.5 mL), thiocyanatomethane (0.24 mL), and aluminum chloride (0.39 g) are added in succession to a solution of [(2*R*,6*S*)-10-hydroxy-6,11,11-trimethyl-1,2,5,6-tetrahydro-4H-2,6-methano-benzo[d]azocin-3-yl]-piperidin-1-yl-methanone (1.0 g) in dichloroethane (12 mL) chilled in an ice bath. The resulting mixture is stirred at ambient temperature until a homogenous solution is formed and then heated to 80°C. After stirring for 3 h, another portion of boron trichloride (1 M in heptane, 3.5 mL), thiocyanatomethane (0.24 mL), and aluminum chloride (0.39 g) are added and the mixture is further stirred at 80°C for another 3 h. After cooling to room temperature, 4 M aqueous NaOH solution (9.5 mL) is added and the solution is stirred at 80°C for 30 min. Then, the solution is cooled to room temperature and extracted with dichloromethane. The aqueous phase is acidified using 4 M hydrochloric acid mixture and extracted with ether. The combined organic extracts are dried (Na₂SO₄) and concentrated. The residue is purified by chromatography on silica gel (cyclohexane/ethyl acetate 1:0->1:1).
Yield: 0.25 g (23% of theory)
Mass spectrum (ESI⁺): m/z = 368 [M+H]⁺

### Procedure G (described for Example 36, Table 3)

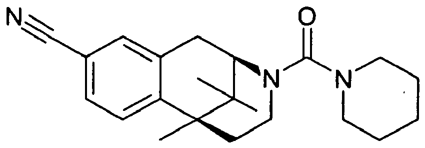

### 2R,6S)-6,11,11-Trimethy-3-(piperidine-1-carbonyl)-1,2,3,4,5,6-hexahydro-2,6-methano-benzo[d]azocine-9-carbonitrile

Polymethylhydrosiloxane (0.94 g) is added to a mixture of (2*R*,6*S*)-trifluoro-methanesulfonic acid 9-cyano-6,11,11-trimethyl-3-(piperidine-1-carbonyl)-1,2,3,4,5,6-hexahydro-2,6-methano-benzo[d]azocin-10-yl ester (0.30 g), KF solution (71 mg in 1 mL water), and palladium(II)acetate (7 mg) in tetrahydrofuran (3 mL) under argon atmosphere. The resulting mixture is stirred at room temperature overnight. Then, 4 M aqueous NaOH solution (5 mL) is added and the mixture is stirred for 4 h. The mixture is extracted with ethyl acetate, the combined extracts are dried (Na₂SO₄) and the solvent is evaporated. The residue is purified by HPLC on reversed phase (water/acetonitrile).
Yield: 30 mg (14% of theory)
Mass spectrum (ESI⁺): m/z = 352 [M+H]⁺

### Procedure H (described for Example 46, Table 3)

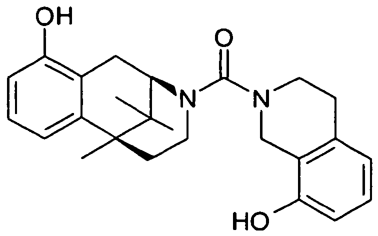

### (8-Hydroxy-3,4-dihydro-1H-isoquinolin-2-yl)-[(2R,6S)-10-hydroxy-6,11,11-trimethyl-1,2,5,6-tetrahydro-4H-2,6-methano-benzo[d]azocin-3-yl]-methanone

Boron tribromide (1 M in CH₂Cl₂, 0.22 mL) is added to a solution of [(2*R*,6*S*)-10-hydroxy-6,11,11-trimethyl-1,2,5,6-tetrahydro-4H-2,6-methano-benzo[d]azocin-3-yl)-(8-methoxy-3,4-dihydro-1H-isoquinolin-2-yl]-methanone (42 mg) in CH₂Cl₂ (4 mL) chilled in an ice bath. The cooling bath is removed and the resulting solution is stirred at ambient temperature overnight. Then, the solution is diluted with ice-cold water and the resulting mixture is extracted with CH₂Cl₂. The combined organic extracts are dried (Na₂SO₄) and the solvent is evaporated. The residue is purified by HPLC on reversed phase (water/MeCN) to give the title compound as a solid.
Yield: 40 mg (99% of theory)
Mass spectrum (ESI⁺): m/z = 407 [M+H]⁺

### Procedure I (described for Example 66, Table 3)

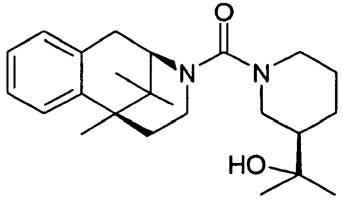

### [(R)-3-(1-Hydroxy-1-methyl-ethyl)-piperidin-1-yl]-[(2R,6S)-6,11,11-trimethyl-1,2,5,6-tetrahydro-4H-2,6-methano-benzo[d]azocin-3-yl]-methanone

1.4 M MeMgBr solution in tetrahydrofuran and toluene (0.65 mL) is added dropwise to a solution of (*R*)-1-[(2*R*,6*S*)-6,11,11-trimethyl-1,2,5,6-tetrahydro-4H-2,6-methano-benzo[d]azocine-3-carbonyl]-piperidine-3-carboxylic acid ethyl ester (120 mg) in tetrahydrofuran (2 mL) chilled in an ice bath. The resulting solution is stirred in the cooling bath for 2 h before the reaction is quenched by the addition of aqueous NH₄Cl solution. The resulting mixture is extracted with *tert*-BuOMe and the combined organic extracts are dried (Na₂SO₄). The solvent is evaporated to give the title compound.
Yield: 115 mg (quantitative)
Mass spectrum (ESI⁺): m/z = 385 [M+H]⁺

### Procedure J (described for Example 71, Table 3)

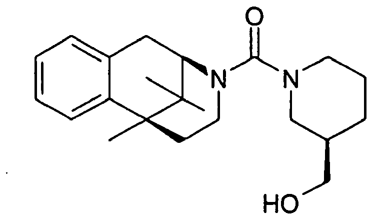

### [(R)-3-Hydroxymethyl-piperidin-1-yl]-[(2R,6S)-6,11,11-trimethyl-1,2,5,6-tetrahydro-4H-2,6-methano-benzo[d]azocin-3-yl)-methanone

LiAlH₄ (1 M in Et₂O 0.25 mL) is added to a solution of (*R*)-1-[(2*R*,6*S*)-6,11,11-trimethyl-1,2,5,6-tetrahydro-4H-2,6-methano-benzo[d]azocine-3-carbonyl]-piperidine-3-carboxylic acid ethyl ester (90 mg) in tetrahydrofuran (3 mL) chilled in an ice bath. The cooling bath is removed and the resulting mixture is stirred at ambient temperature overnight. Then, water is added and the mixture is extracted with *tert-*BuOMe. The combined organic extracts are washed with brine and dried (Na₂SO₄). The solvent is evaporated to give the title compound as an oil.
Yield: 80 mg (ca. 90% pure)
Mass spectrum (ESI⁺): m/z = 357 [M+H]⁺

### Procedure K (described for Example 79, Table 3)

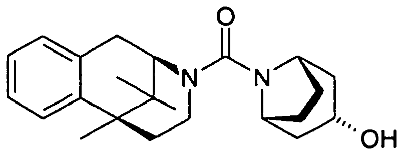

### (exo-3-Hydroxy-8-aza-bicyclo[3.2.1]oct-8-yl)-[(2R,6S)-6,11,11-trimethyl-1,2,5,6-tetrahydro-4H-2,6-methano-benzo[d]azocin-3-yl]-methanone

NaBH₄ (35 mg) dissolved in methanol (1 mL) is added to a solution of 8-[(2*R*,6*S*)-6,11,11-trimethyl-1,2,5,6-tetrahydro-4H-2,6-methano-benzo[d]azocine-3-carbonyl)-8-aza-bicyclo[3.2.1]octan-3-one (90 mg) in tetrahydrofuran (3 mL) and methanol (1 mL). The resulting mixture is stirred at ambient temperature for 2 h and then concentrated under reduced pressure. The residue is purified by HPLC on reversed phase (water/MeCN) to give the title compound and the corresponding diastereomer, (*endo*-3-hydroxy-8-aza-bicyclo[3.2.1]oct-8-yl)-[(2*R*,6*S*)-6,11,11-trimethyl-1,2,5,6-tetrahydro-4H-2,6-methano-benzo[d]azocin-3-yl]-methanone (86 mg, 29% of theory), in separate fractions.
Yield: 128 mg (42% of theory)
Mass spectrum (ESI⁺): m/z = 369 [M+H]⁺

**Table 3. End compounds**

| Example No. | Chemical Name/Structure/Remarks | Prepared in analogy to Procedure | Characterization |
|---|---|---|---|
| 1 | 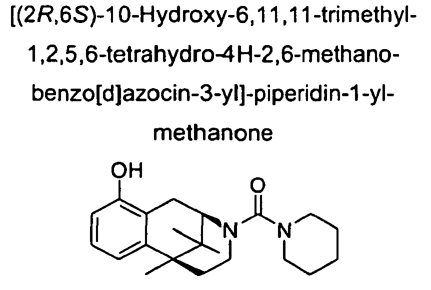 | A | Mass spectrum (ESI⁺): m/z = 343 [M+H]⁺ |
| 2 | 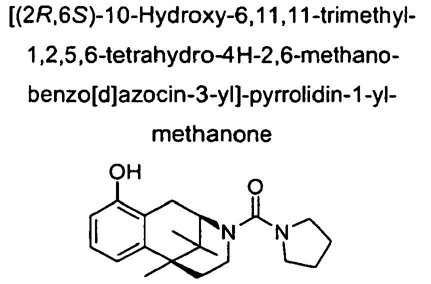 | A | Mass spectrum (ESI⁺): m/z = 329 [M+H]⁺ |
| 3 | 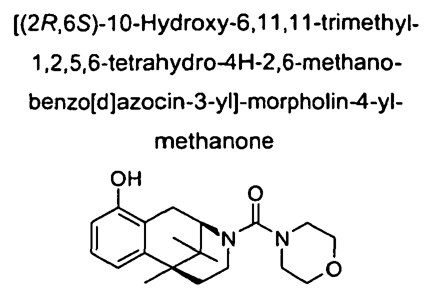 | A | Mass spectrum (ESI⁺): m/z = 345 [M+H]⁺ |
| 4 | 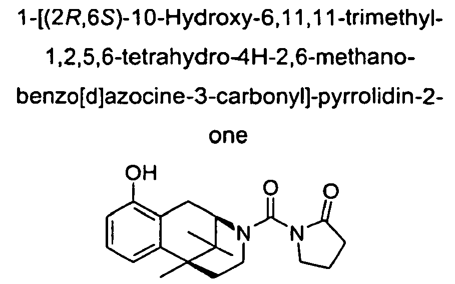 | A | Mass spectrum (ESI⁺): m/z = 344 [M+H]⁺ |
| 5 | 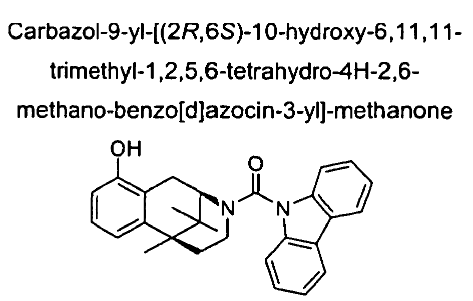 | A | Mass spectrum (ESI⁺): m/z = 425 [M+H]⁺ |
| 6 | 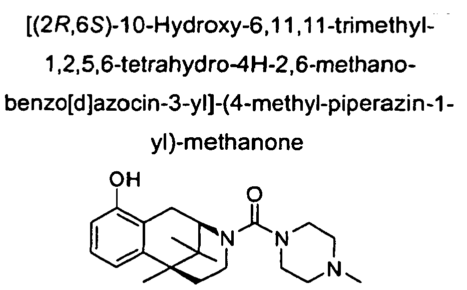 | A | Mass spectrum (ESI⁺): m/z = 358 [M+H]⁺ |
| 7 | 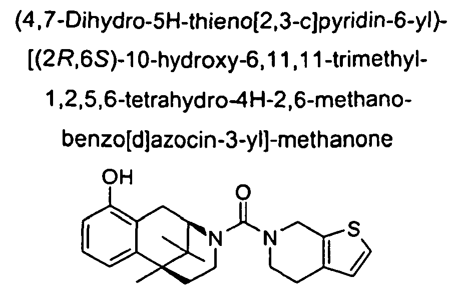 | B | Mass spectrum (ESI⁺): m/z = 397 [M+H]⁺ |
| 8 | 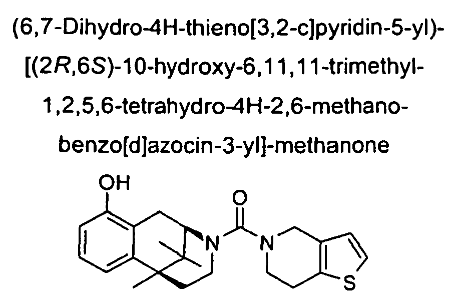 | B | Mass spectrum (ESI⁺): m/z = 397 [M+H]⁺ |
| 9 | 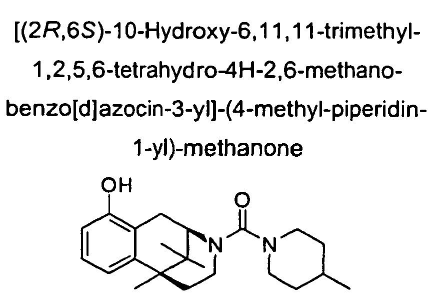 | C | Mass spectrum (ESI⁺): m/z = 357 [M+H]⁺ |
| 10 | 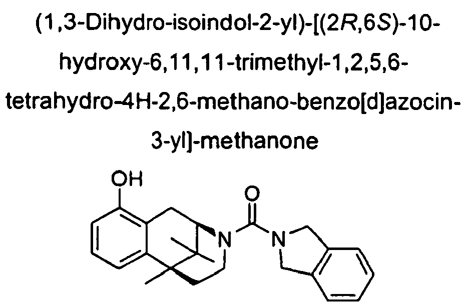 | C | Mass spectrum (ESI⁺): m/z = 377 [M+H]⁺ |
| 11 | 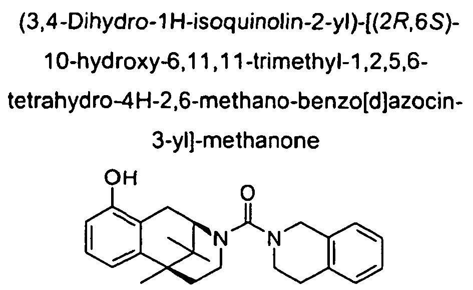 | C | Mass spectrum (ESI⁺): m/z = 391 [M+H]⁺ |
| 12 | 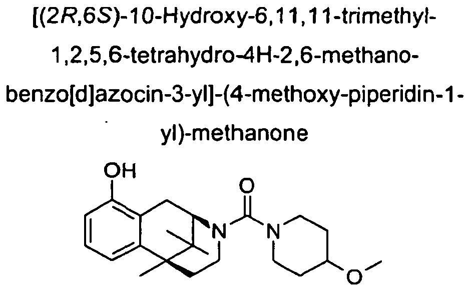 | C | Mass spectrum (ESI⁺): m/z = 373 [M+H]⁺ |
| 13 | 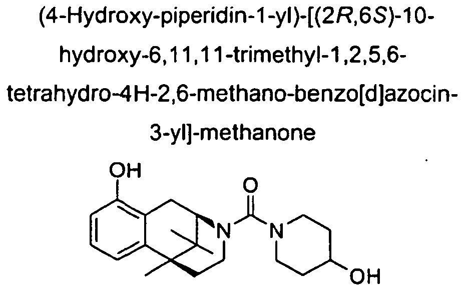 | C | Mass spectrum (ESI⁺): m/z = 359 [M+H]⁺ |
| 14 | 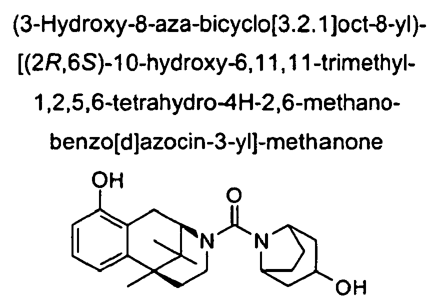 | C | Mass spectrum (ESI⁺): m/z = 385 [M+H]⁺ |
| 15 | 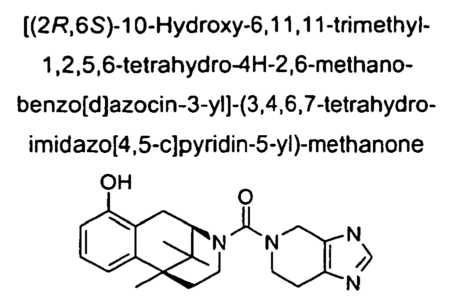 | C | Mass spectrum (ESI⁺): m/z = 381 [M+H]⁺ |
| 16 | 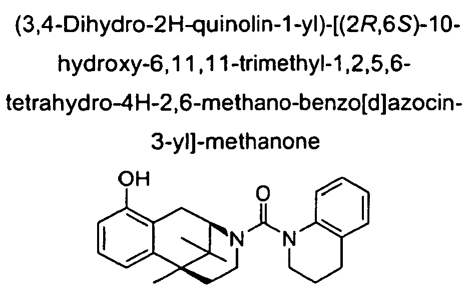 | B | Mass spectrum (ESI⁺): m/z = 391 [M+H]⁺ |
| 17 | 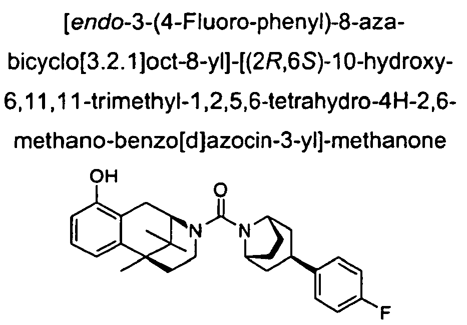 | C | Mass spectrum (ESI⁺): m/z = 463 [M+H]⁺ |
| 18 | 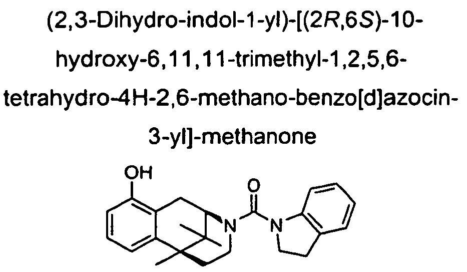 | C | Mass spectrum (ESI⁺): m/z = 377 [M+H]⁺ |
| 19 | 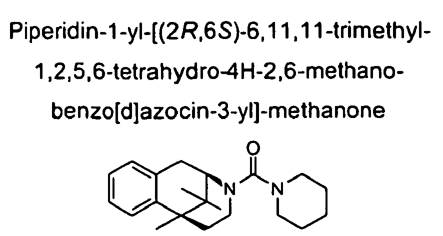 | A | Mass spectrum (ESI⁺): m/z = 327 [M+H]⁺ |
| 20 | 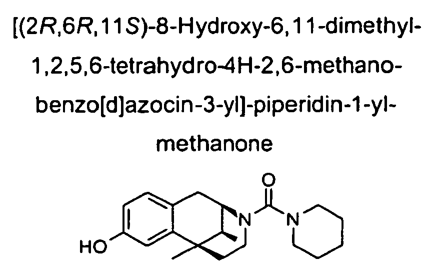 | A | Mass spectrum (ESI⁺): m/z = 329 [M+H]⁺ |
| 21 | 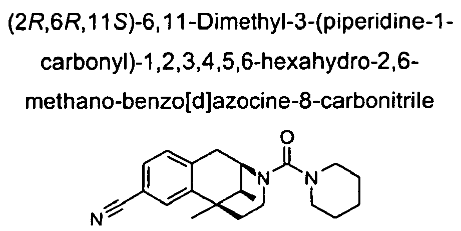 | A | Mass spectrum (ESI⁺): m/z = 338 [M+H]⁺ |
| 22 | 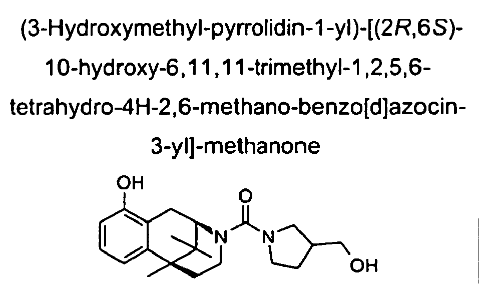 | C | Mass spectrum (ESI⁺): m/z = 359 [M+H]⁺ |
| 23 | 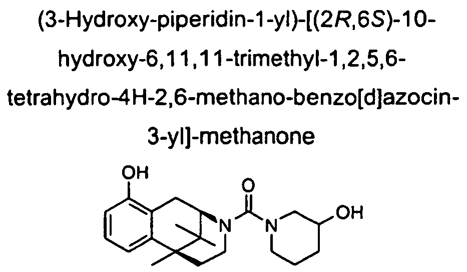 | C | Mass spectrum (ESI⁺): m/z = 359 [M+H]⁺ |
| 24 | 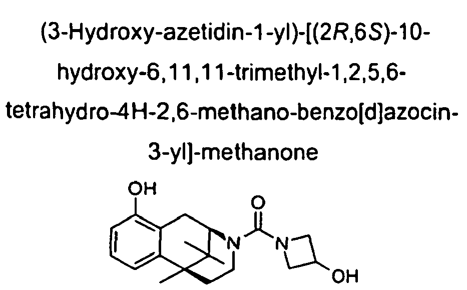 | C | Mass spectrum (ESI⁺): m/z = 331 [M+H]⁺ |
| 25 | 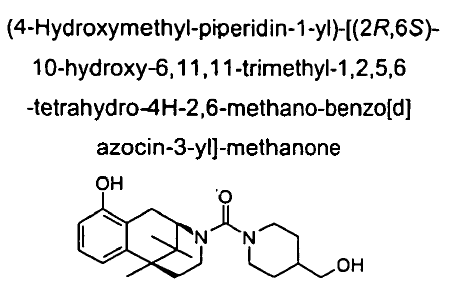 | C | Mass spectrum (ESI⁺): m/z = 373 [M+H]⁺ |
| 26 | 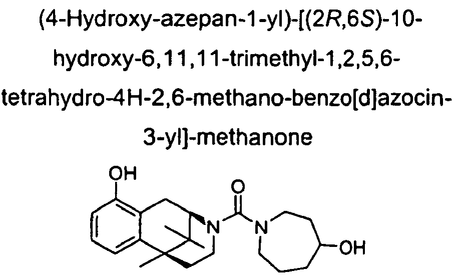 | C | Mass spectrum (ESI⁺): m/z = 373 [M+H]⁺ |
| 27 | 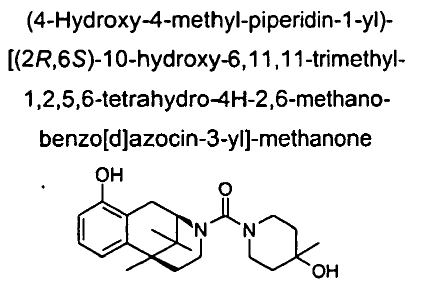 | C | Mass spectrum (ESI⁺): m/z = 373 [M+H]⁺ |
| 28 | 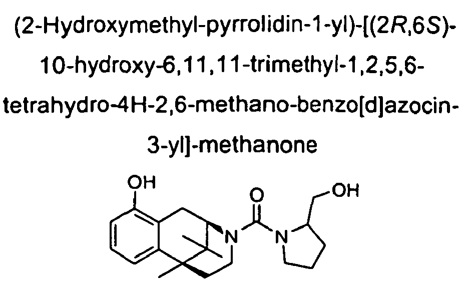 | C | Mass spectrum (ESI⁺): m/z = 359 [M+H]⁺ |
| 29 | 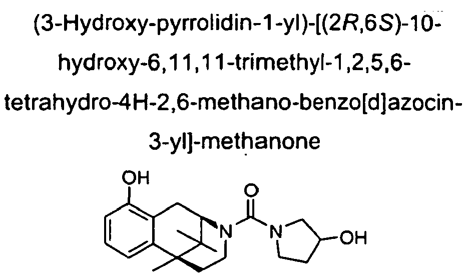 | C | Mass spectrum (ESI⁺): m/z = 345 [M+H]⁺ |
| 30 | 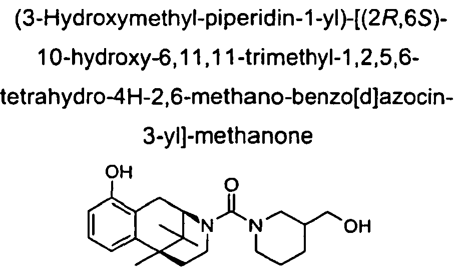 | C | Mass spectrum (ESI⁺): m/z = 373 [M+H]⁺ |
| 31 | 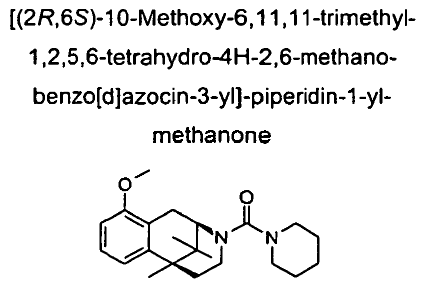 | D | Mass spectrum (ESI⁺): m/z = 357 [M+H]⁺ |
| 32 | 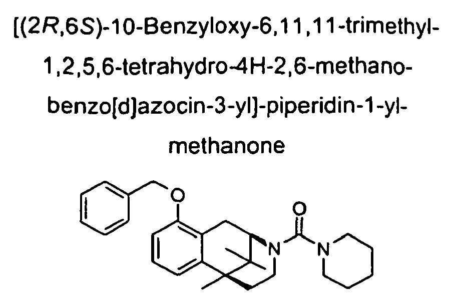 | D | Mass spectrum (ESI⁺): m/z = 433 [M+H]⁺ |
| 33 | 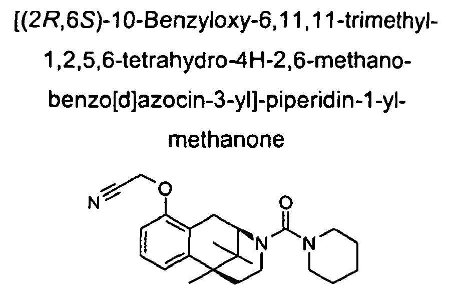 | D | Mass spectrum (ESI⁺): m/z = 382 [M+H]⁺ |
| 34 | 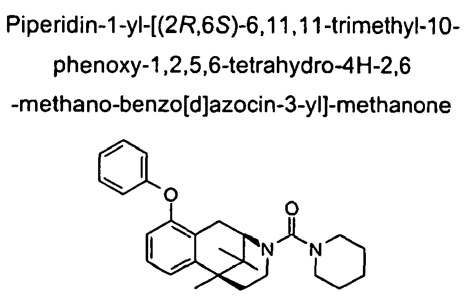 | E | Mass spectrum (ESI⁺): m/z = 419 [M+H]⁺ |
| 35 | 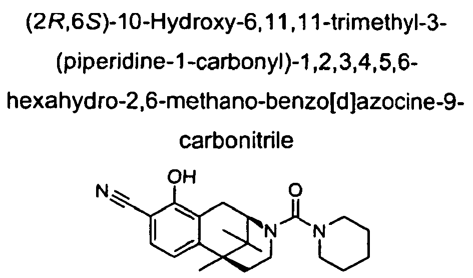 | F | Mass spectrum (ESI⁺): m/z = 368 [M+H]⁺ |
| 36 | 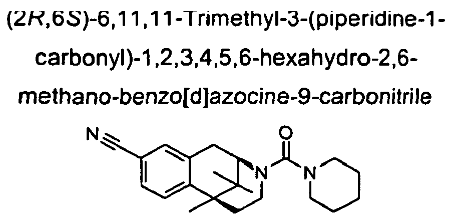 | G | Mass spectrum (ESI⁺): m/z = 352 [M+H]⁺ |
| 37 | 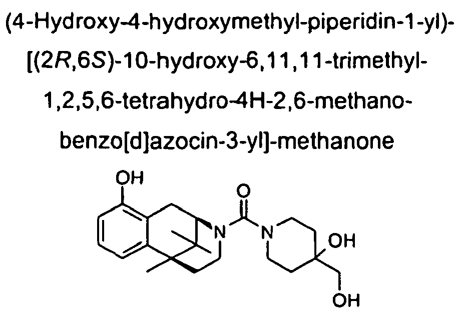 | C | Mass spectrum (ESI⁺): m/z = 389 [M+H]⁺ |
| 38 | 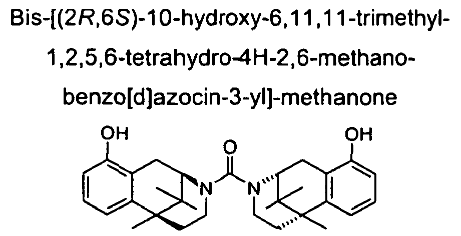 | C | Mass spectrum (ESI⁺): m/z = 489 [M+H]⁺ |
| 39 | 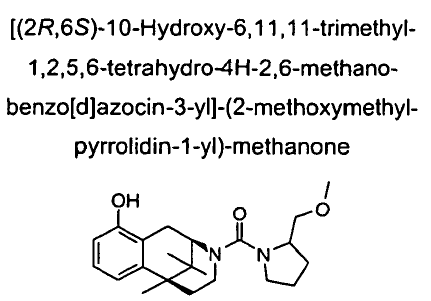 | C | Mass spectrum (ESI⁺): m/z = 373 [M+H]⁺ |
| 40 | 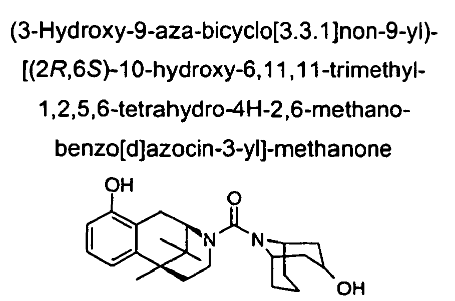 | B | Mass spectrum (ESI⁺): m/z = 399 [M+H]⁺ |
| 41 | 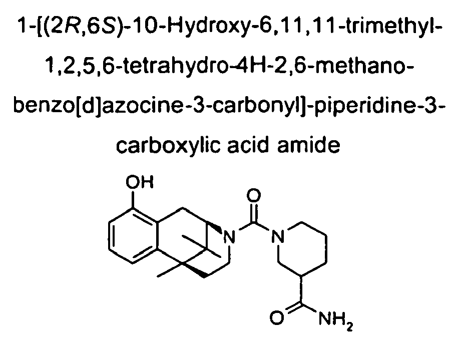 | B | Mass spectrum (ESI⁺): m/z = 386 [M+H]⁺ |
| 42 | 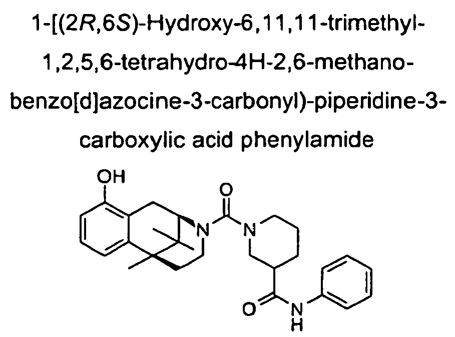 | C | Mass spectrum (ESI⁺): m/z = 462 [M+H]⁺ |
| 43 | 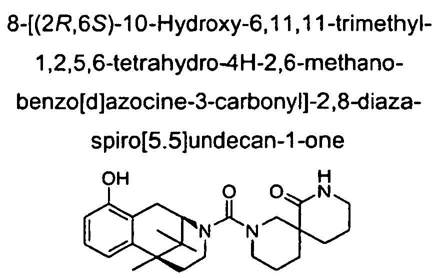 | C | Mass spectrum (ESI⁺): m/z = 426 [M+H]⁺ |
| 44 | 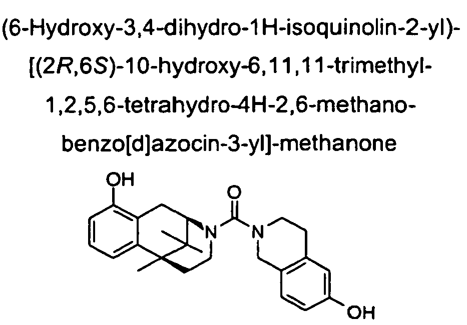 | C | Mass spectrum (ESI⁺): m/z = 407 [M+H]⁺ |
| 45 | 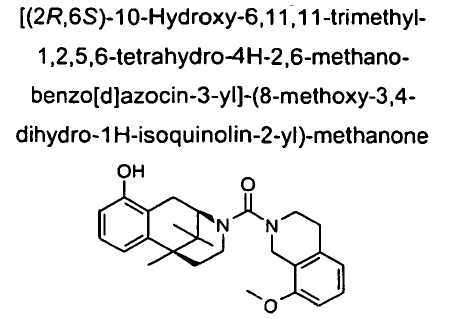 | B | Mass spectrum (ESI⁺): m/z = 421 [M+H]⁺ |
| 46 | 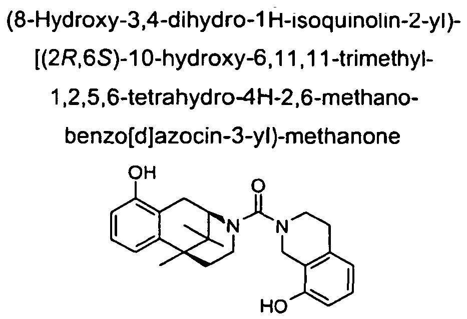 | H | Mass spectrum (ESI⁺): m/z = 407 [M+H]⁺ |
| 47 | 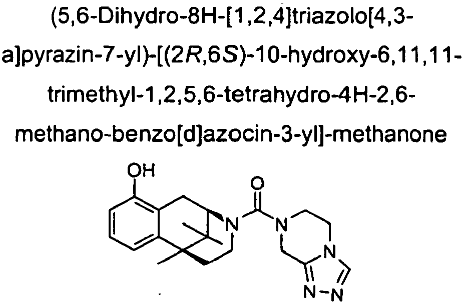 | C | Mass spectrum (ESI⁺): m/z = 382 [M+H]⁺ |
| 48 | 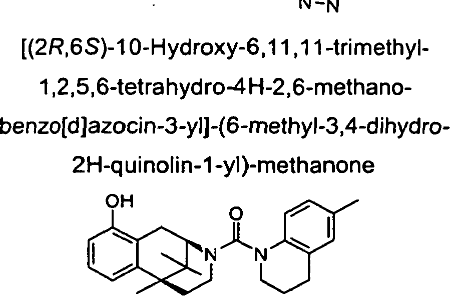 | B | Mass spectrum (ESI⁺): m/z = 405 [M+H]⁺ |
| 49 | 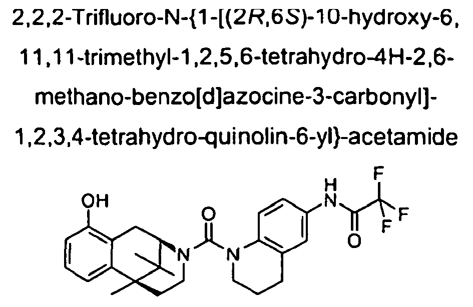 | B | Mass spectrum (ESI⁺): m/z = 502 [M+H]⁺ |
| 50 | 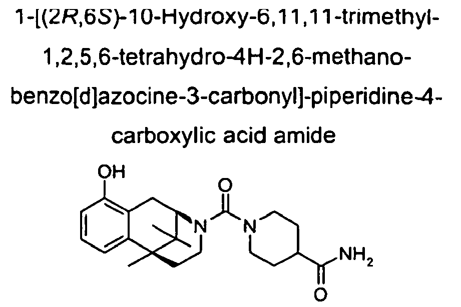 | B | Mass spectrum (ESI⁺): m/z = 386 [M+H]⁺ |
| 51 | 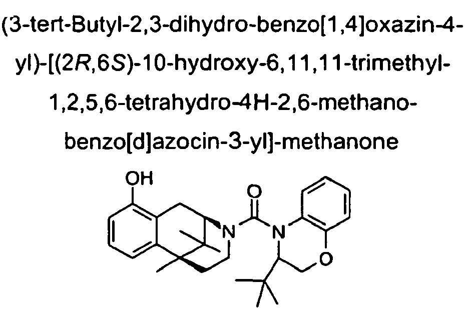 | B | Mass spectrum (ESI⁺): m/z = 449 [M+H]⁺ |
| 52 | 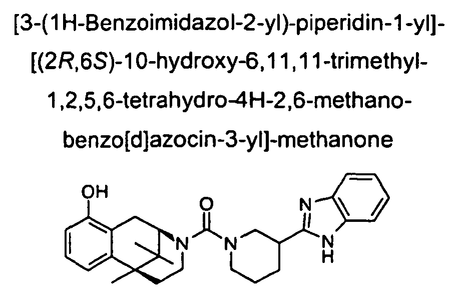 | C | Mass spectrum (ESI⁺): m/z = 459 [M+H]⁺ |
| 53 | 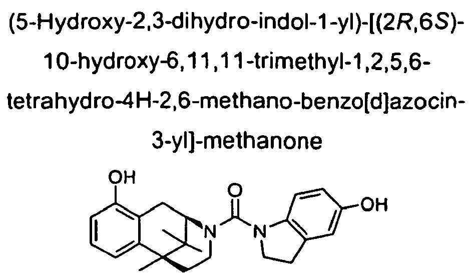 | B | Mass spectrum (ESI⁺): m/z = 393 [M+H]⁺ |
| 54 | 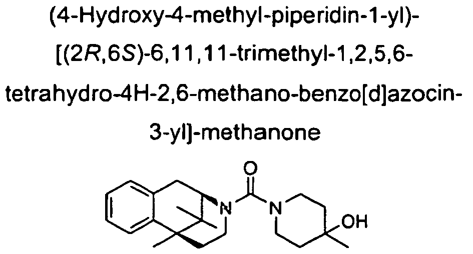 | B | Mass spectrum (ESI⁺): m/z = 357 [M+H]⁺ |
| 55 | 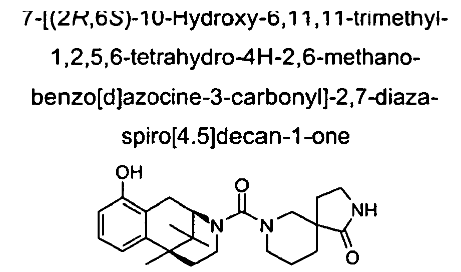 | B | Mass spectrum (ESI⁺): m/z = 412 [M+H]⁺ |
| 56 | 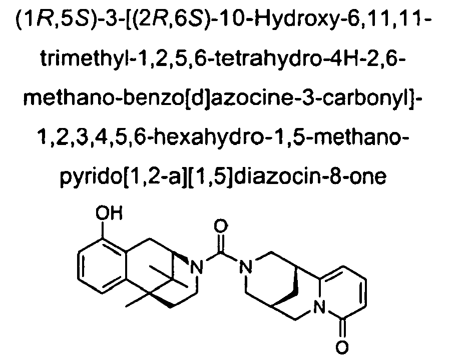 | B | Mass spectrum (ESI⁺): m/z = 412 [M+H]⁺ |
| 57 | 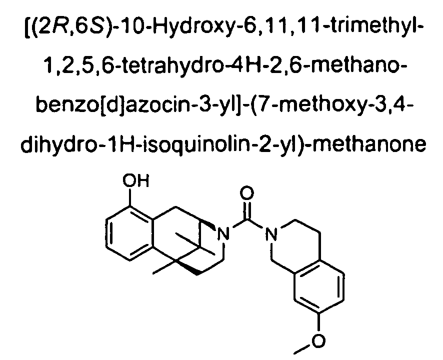 | B | not determined, directly submitted to the production of Example 58 |
| 58 | 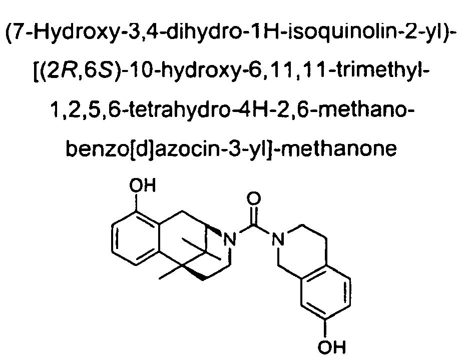 | H | Mass spectrum (ESI⁺): m/z = 407 [M+H]⁺ |
| 59 | 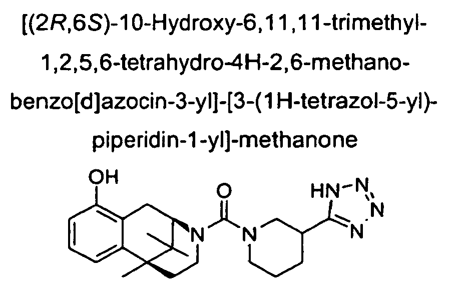 | C | Mass spectrum (ESI⁺): m/z = 411 [M+H]⁺ |
| 60 | 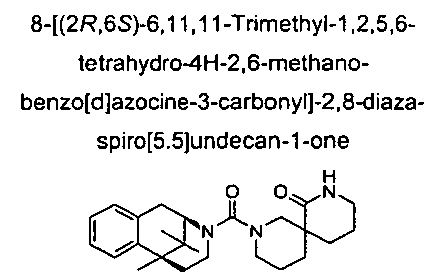 | B | Mass spectrum (ESI⁺): m/z = 410 [M+H]⁺ |
| 61 | 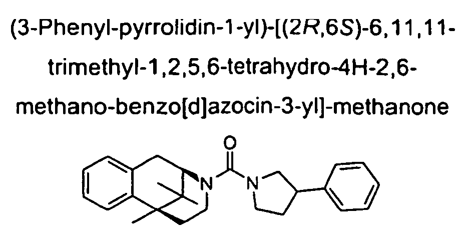 | C | Mass spectrum (ESI⁺): m/z = 389 [M+H]⁺ |
| 62 | 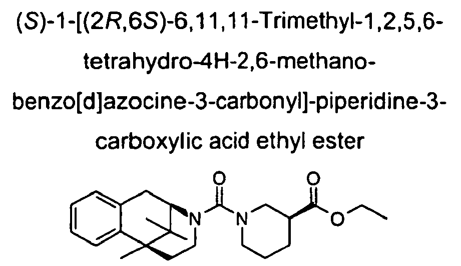 | C | Mass spectrum (ESI⁺): m/z = 399 [M+H]⁺ |
| 63 | 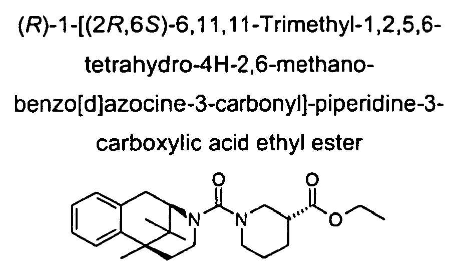 | C | Mass spectrum (ESI⁺): m/z = 399 [M+H]⁺ |
| 64 | 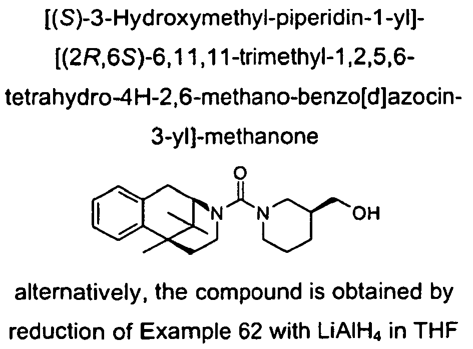 | C | Mass spectrum (ESI⁺): m/z = 357 [M+H]⁺ |
| 65 | 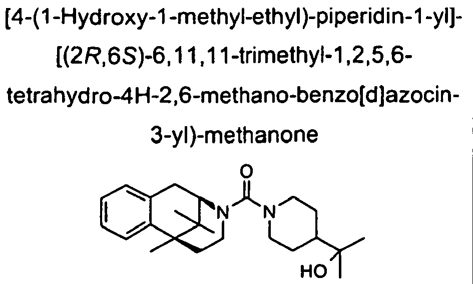 | C | Mass spectrum (ESI⁺): m/z = 385 [M+H]⁺ |
| 66 | 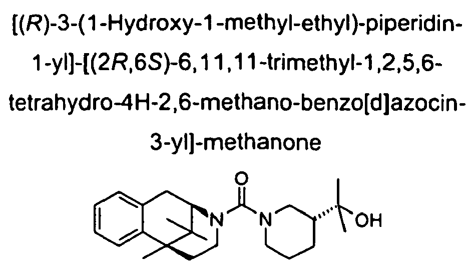 | I | Mass spectrum (ESI⁺): m/z = 385 [M+H]⁺ |
| 67 | 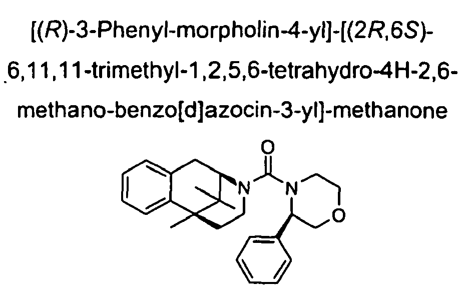 | C | Mass spectrum (ESI⁺): m/z=405 [M+H]⁺ |
| 68 | 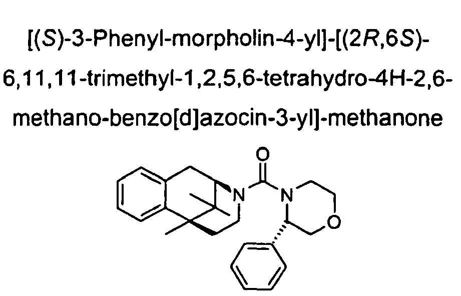 | C | Mass spectrum (ESI⁺): m/z=405 [M+H]⁺ |
| 69 | 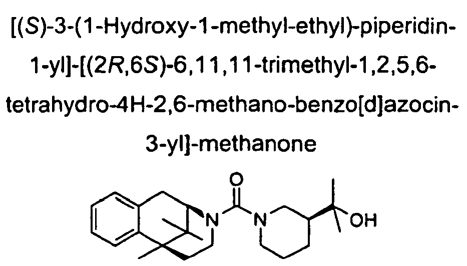 | I | Mass spectrum (ESI⁺): m/z = 385 [M+H]⁺ |
| 70 | 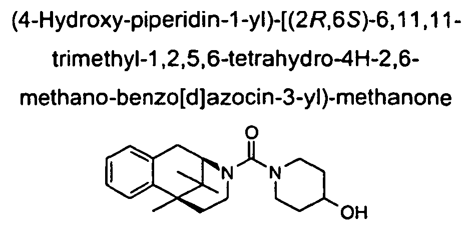 | C | Mass spectrum (ESI⁺):m/z = 343 [M+H]⁺ |
| 71 | 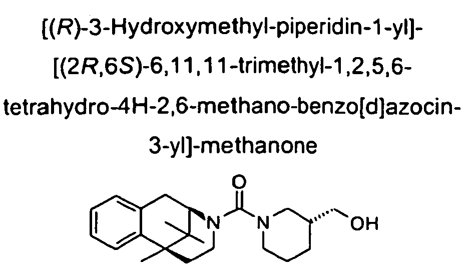 | J | Mass spectrum (ESI⁺): m/z = 357 [M+H]⁺ |
| 72 | 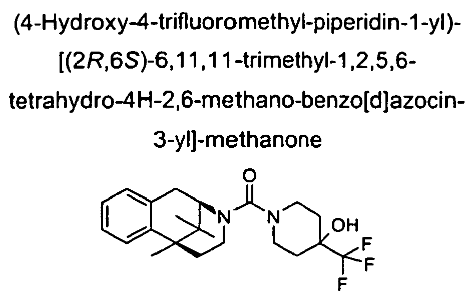 | C | Mass spectrum (ESI⁺): m/z = 411 [M+H]⁺ |
| 73 | 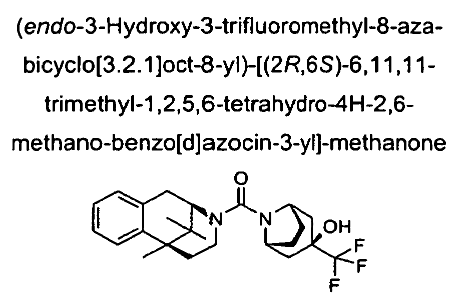 | C | Mass spectrum (ESI⁺): m/z = 437 [M+H]⁺ |
| 74 | 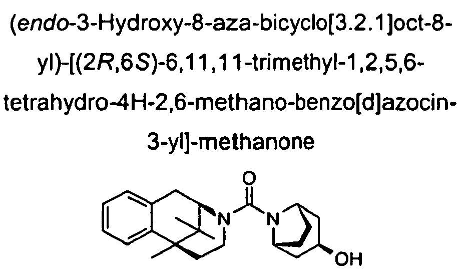 | C | Mass spectrum (ESI⁺): m/z = 369 [M+H]⁺ |
| 75 | 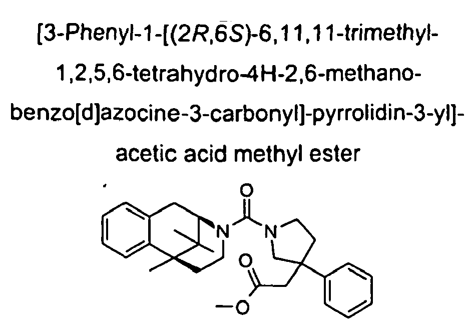 | C | Mass spectrum (ESI⁺): m/z = 461 [M+H]⁺ |
| 76 | 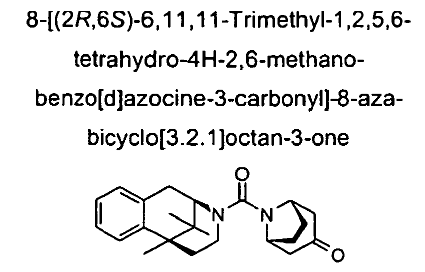 | C | Mass spectrum (ESI⁺): m/z = 367 [M+H]⁺ |
| 77 | 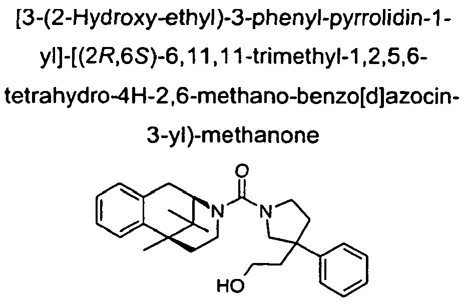 | J | Mass spectrum (ESI⁺): m/z = 433 [M+H]⁺ |
| 78 | 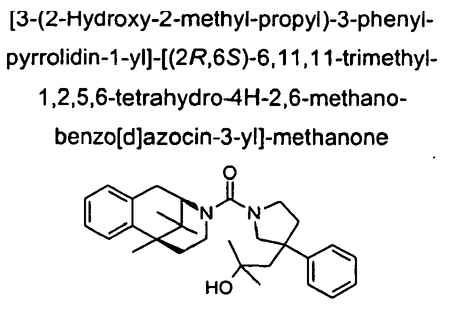 | I | Mass spectrum (ESI⁺): m/z = 461 [M+H]⁺ |
| 79 | 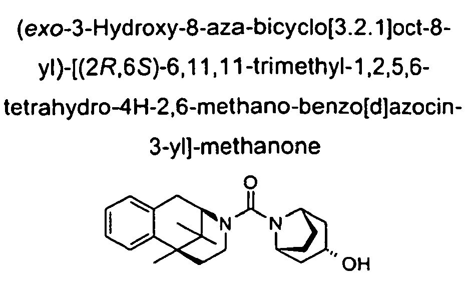 | K | Mass spectrum (ESI⁺): m/z = 369 [M+H]⁺ |
| 80 | 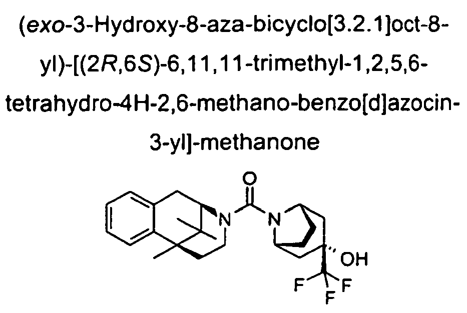 | C | Mass spectrum (ESI⁺): m/z = 437 [M+H]⁺ |

The following compounds are also prepared analogously to the above-mentioned Examples from the respective polycyclic scaffold, preparation of each is described in the section "Preparation of the starting compounds", and piperidine-1-carbonyl chloride or a derivative thereof:

| Example No. | Chemical Name/Structure/Remarks | In analogy to Procedure |
|---|---|---|
| 81 | 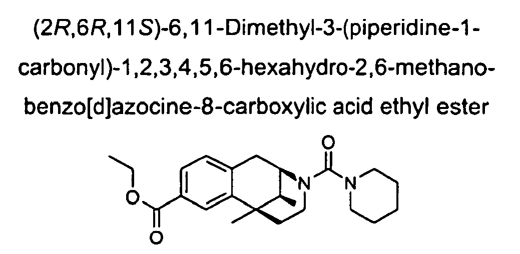 | A |
| 82 | 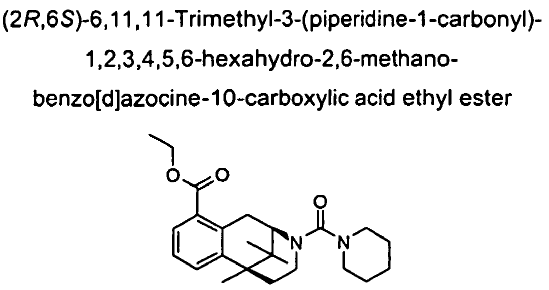 | A |
| 83 | 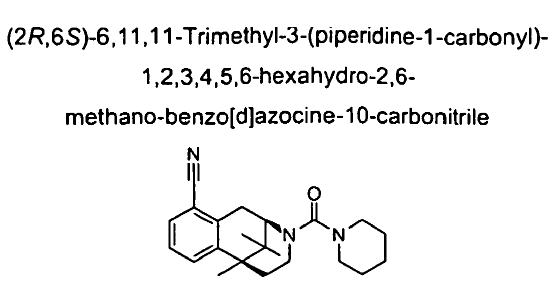 | A |
| 84 | 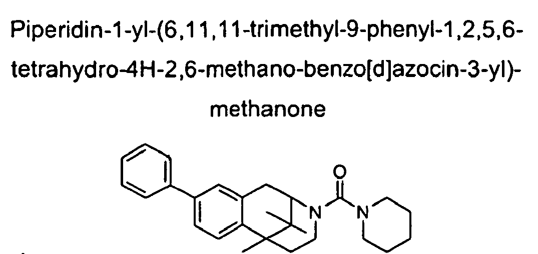 | A |
| 85 | 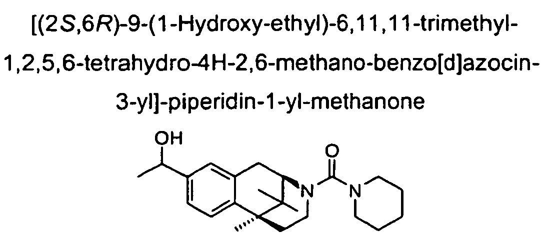 | Procedure A followed by reduction with NaBH₄ in EtOH at room temperature |
| 86 | 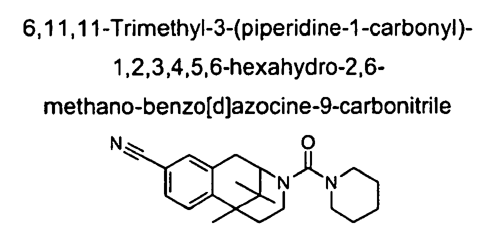 | A |
| 87 | 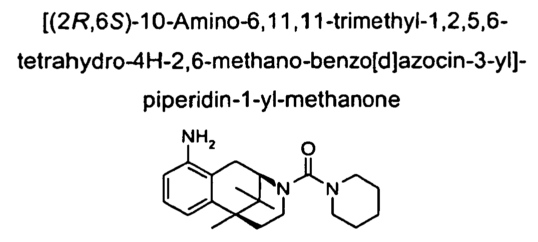 | A |
| 88 | 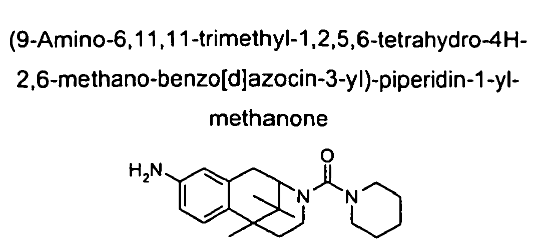 | A |
| 89 | 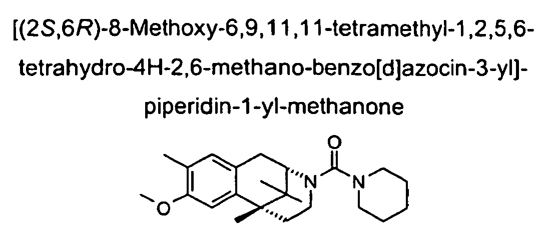 | A |
| 90 | 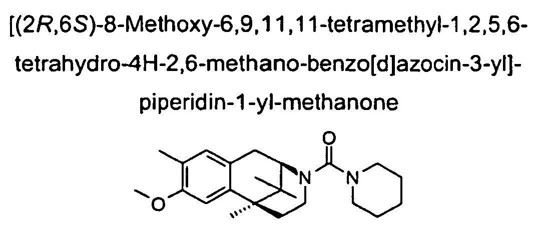 | A |
| 91 | 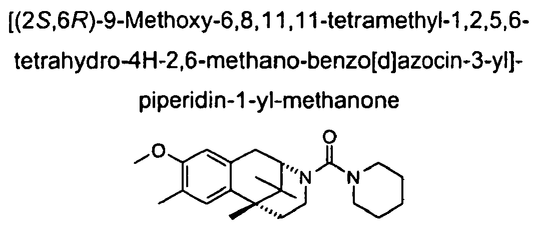 | A |
| 92 | 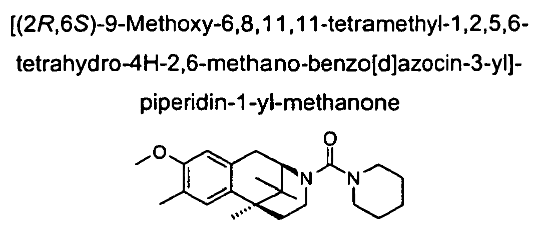 | A |
| 93 | 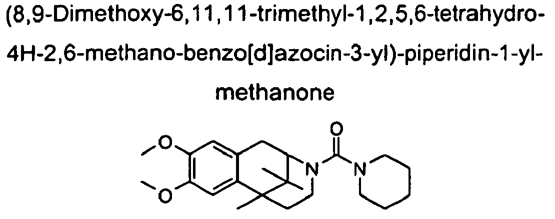 | A |
| 94 | 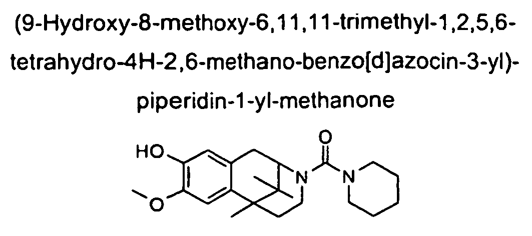 | A |
| 95 | 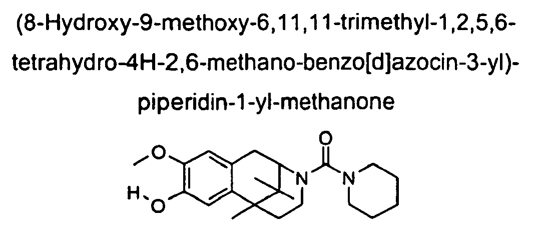 | A |
| 96 | 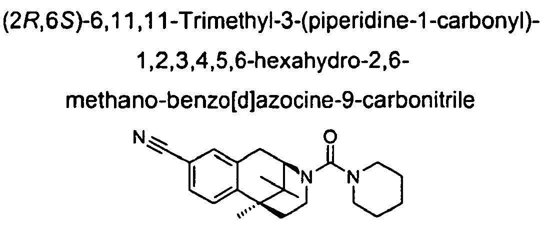 | A |
| 97 | 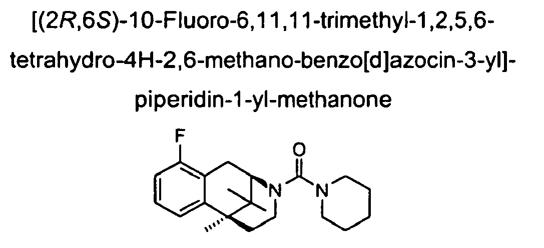 | A |
| 98 | 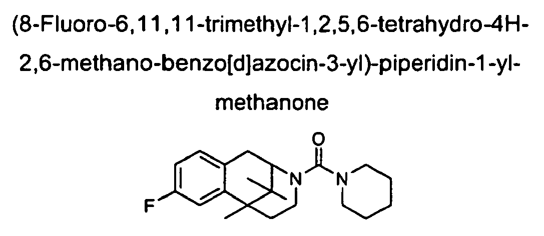 | A |
| 99 | 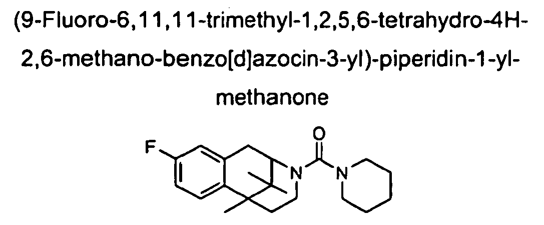 | A |
| 100 | 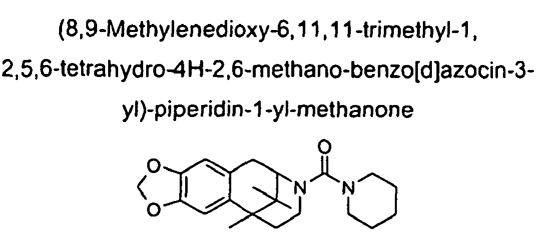 | A |
| 101 | 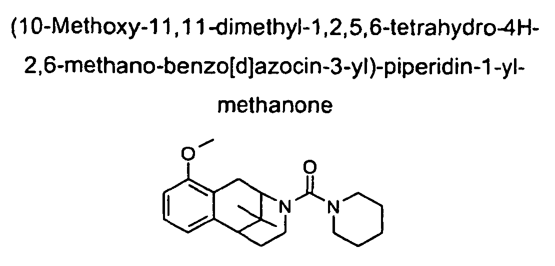 | A |
| 102 | 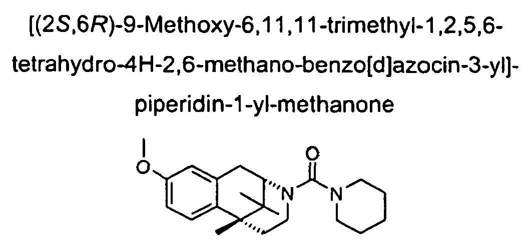 | A |
| 103 | 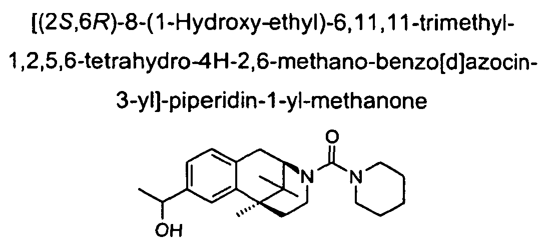 | Procedure A followed by reduction with NaBH₄ in EtOH at room temperature |
| 104 | 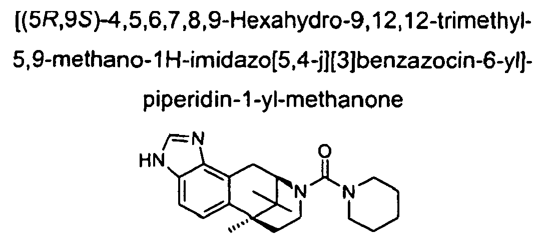 | A |
| 105 | 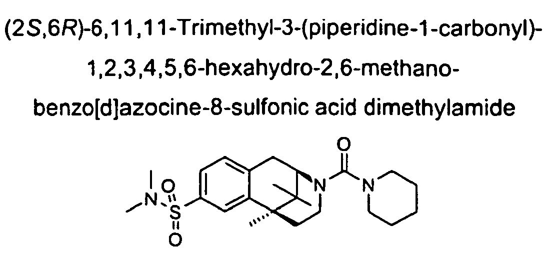 | A |
| | | |
| 106 | 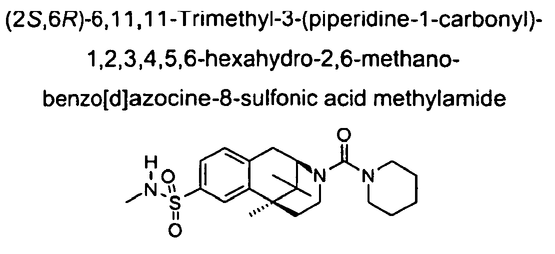 | A |
| 107 | 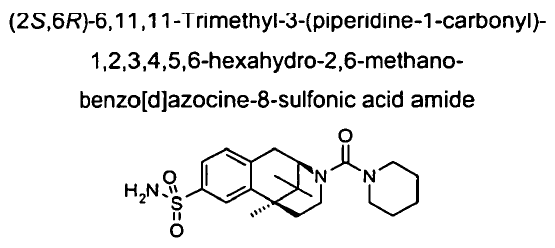 | A |
| 108 | 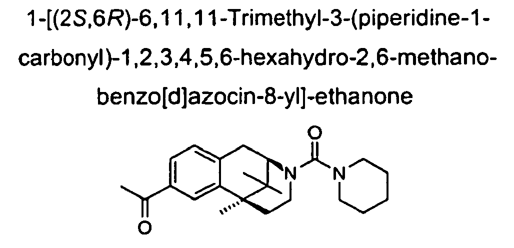 | A |
| 109 | 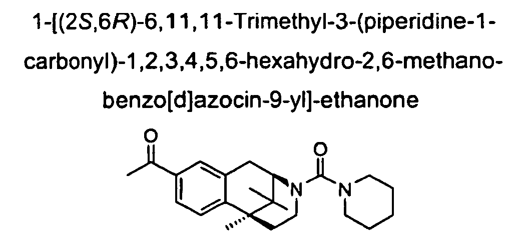 | A |
| 110 | 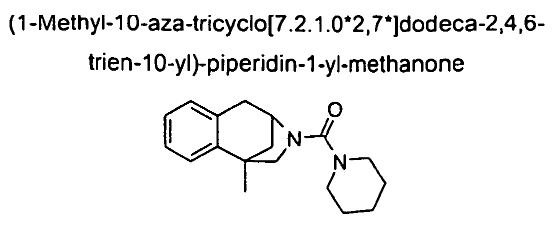 | A |
| 111 | 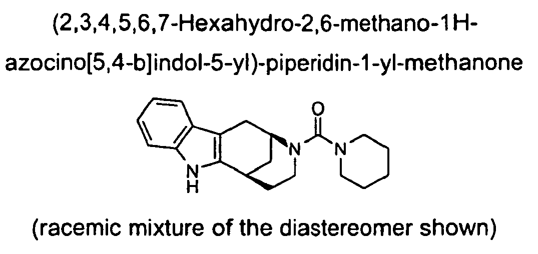 | A |
| 112 | 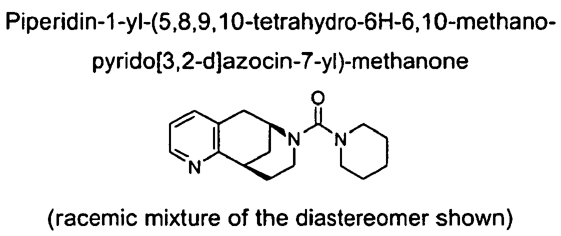 | A |
| 113 | 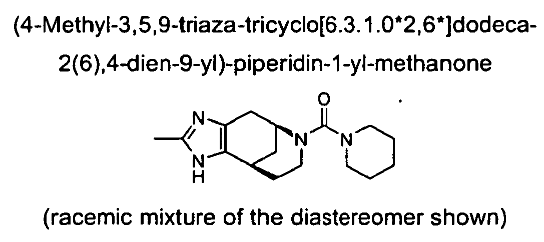 | A |
| 114 | 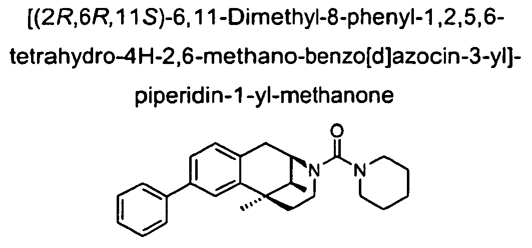 | A |
| 115 | 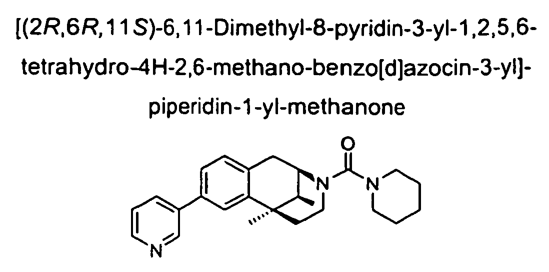 | A |
| 116 | 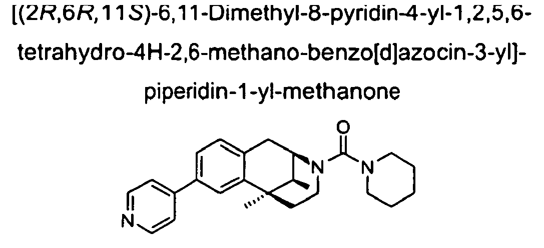 | A |
| 117 | 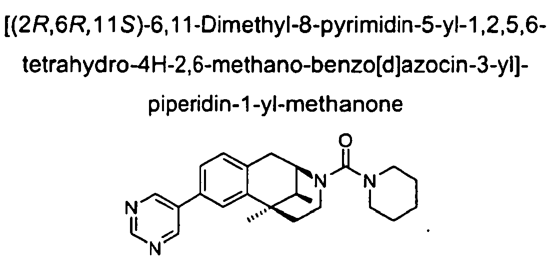 | A |
| 118 | 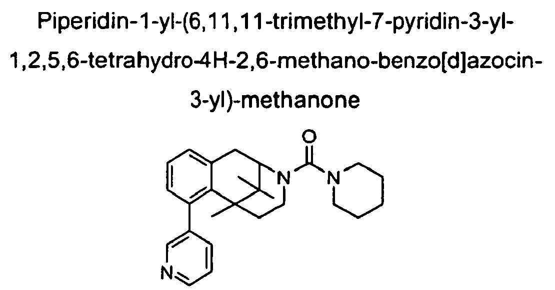 | A |
| 119 | 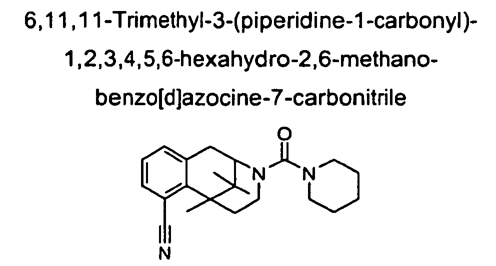 | A |
| 120 | 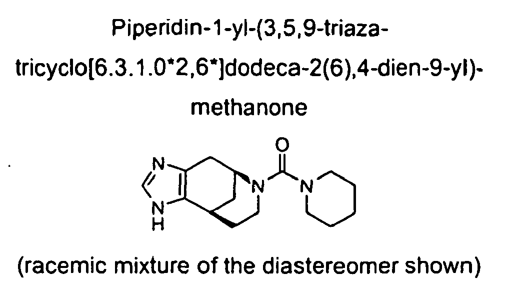 | A |
| 121 | 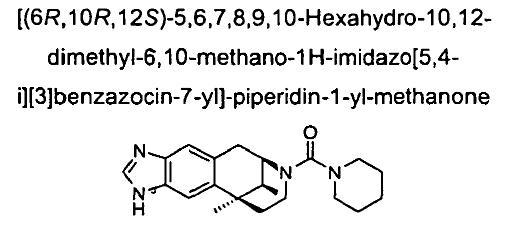 | A |
| 122 | 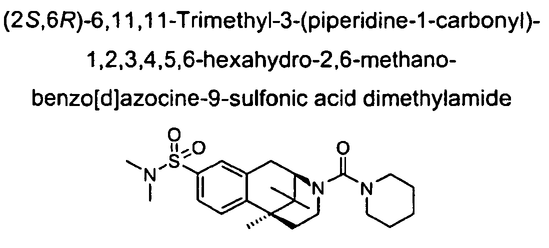 | A |
| 123 | 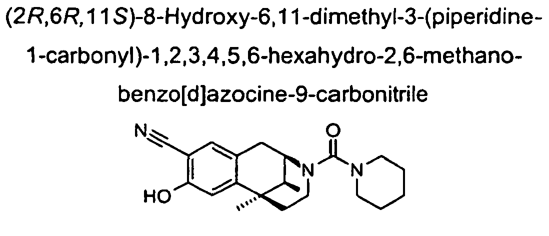 | A |
| 124 | 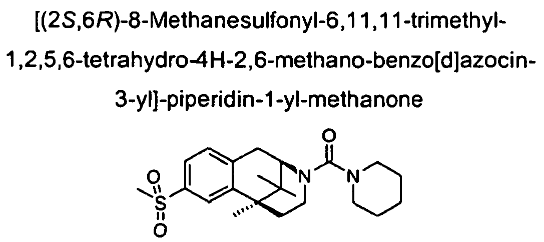 | A |
| 125 | 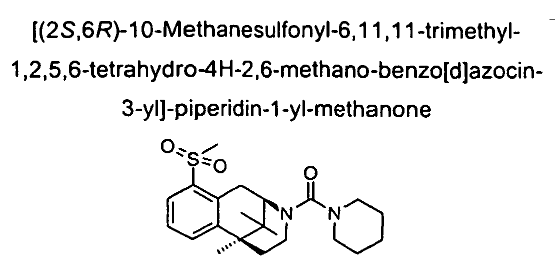 | A |
| 126 | 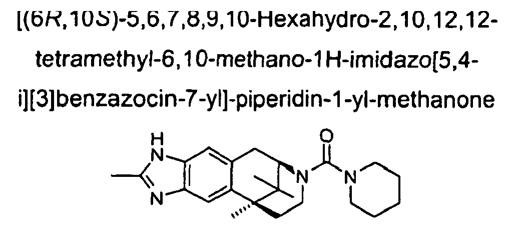 | A |
| 127 | 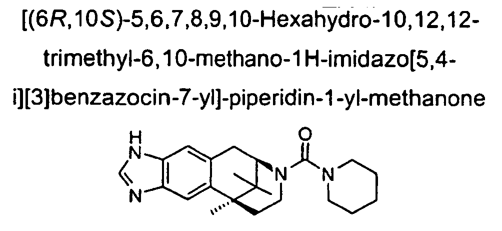 | A |
| 128 | 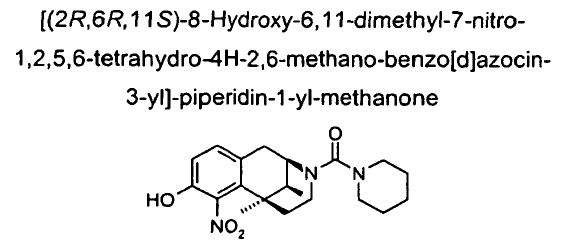 | A |
| 129 | 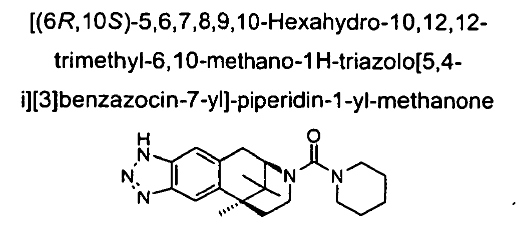 | A |
| 130 | 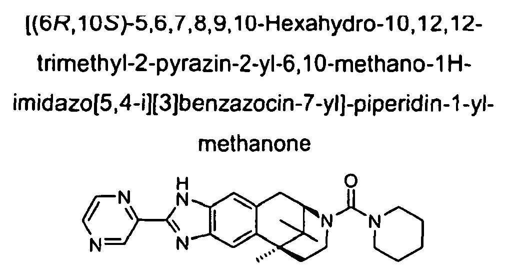 | A |
| 131 | 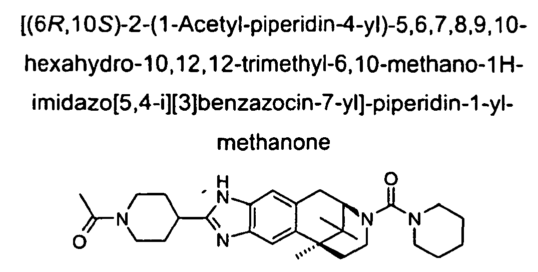 | A |
| 132 | 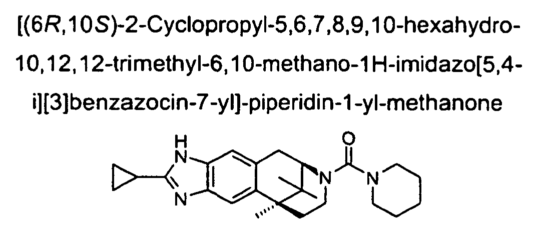 | A |
| 133 | 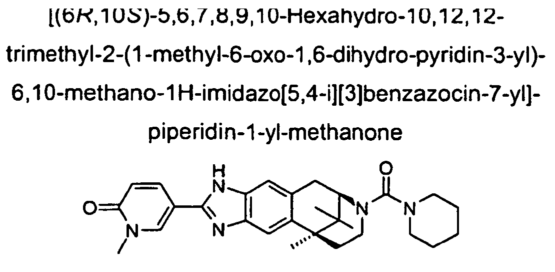 | A |
| 134 | 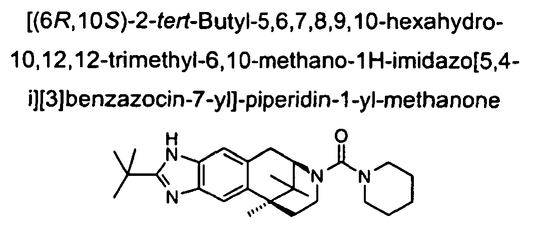 | A |
| 135 | 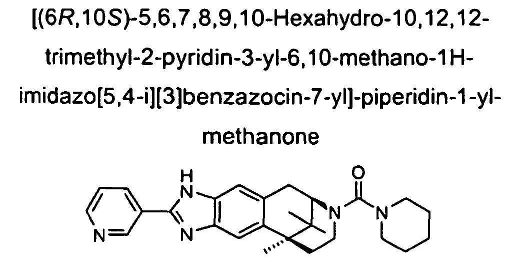 | A |
| 136 | 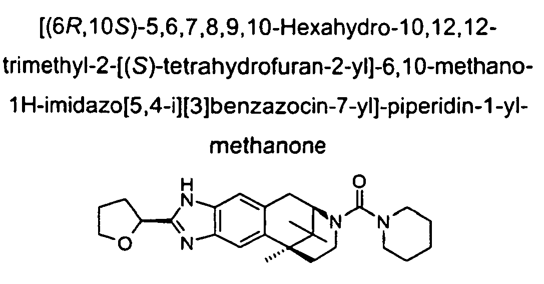 | A |
| 137 | 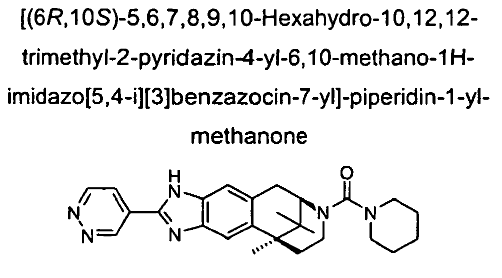 | A |
| 138 | 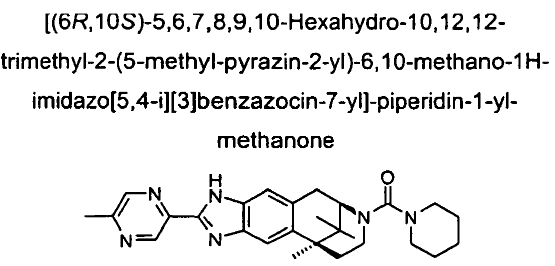 | A |
| 139 | 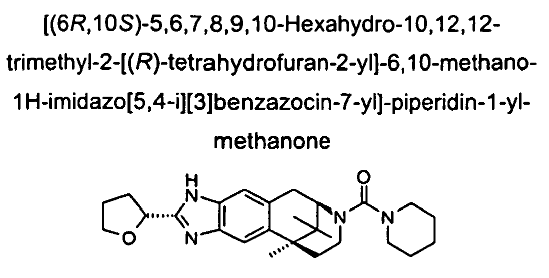 | A |
| 140 | 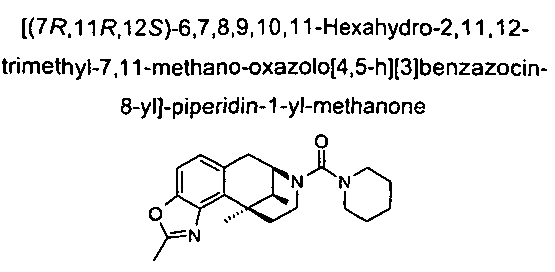 | A |
| 141 | 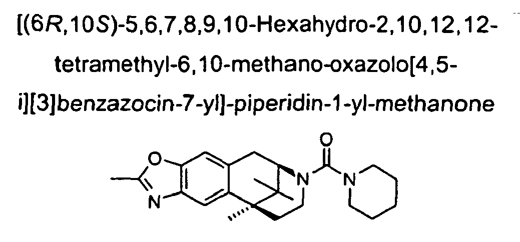 | A |
| 142 | 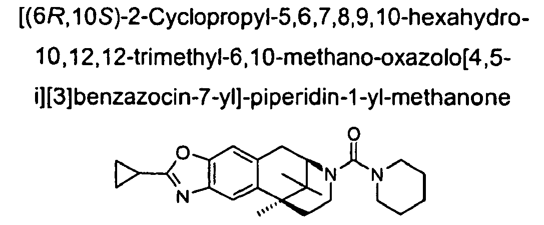 | A |
| 143 | 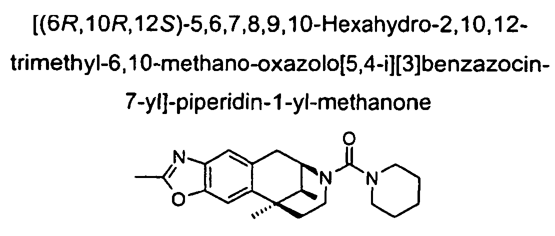 | A |
| 144 | 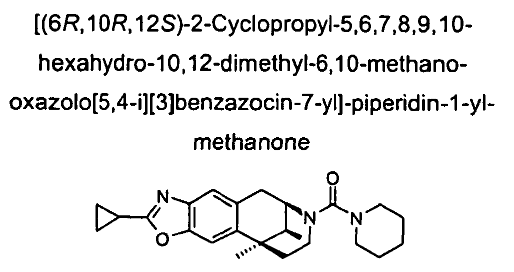 | A |
| 145 | 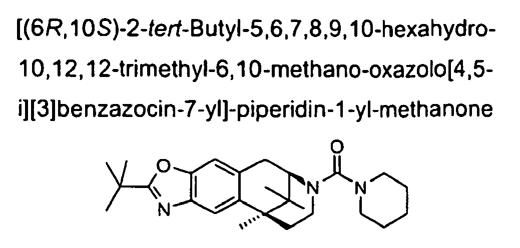 | A |
| 146 | 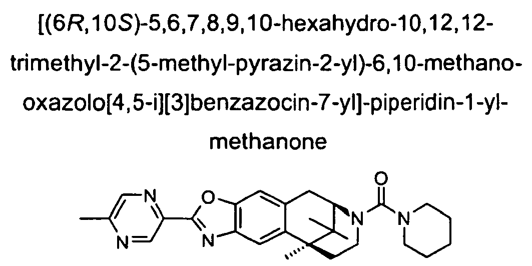 | A |
| 147 | 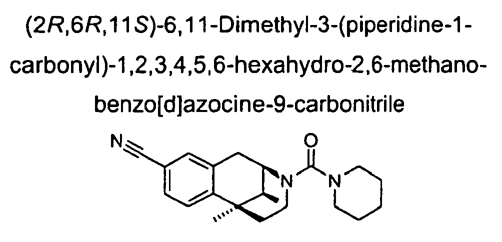 | A |
| 148 | 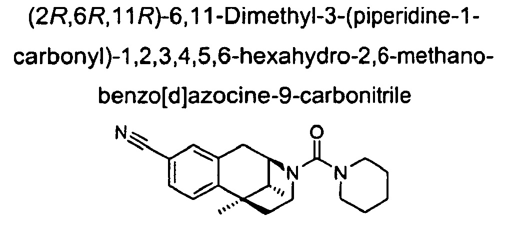 | A |
| 149 | 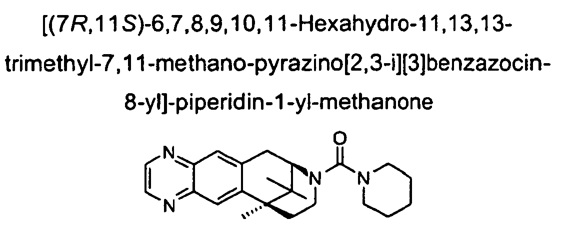 | A |
| 150 | 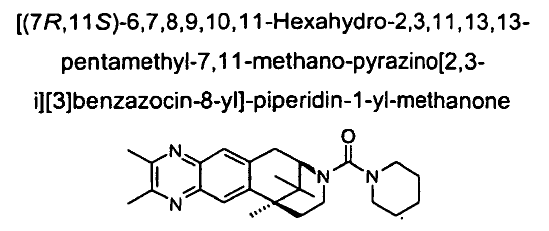 | A |
| 151 | 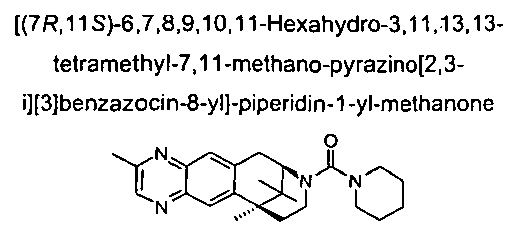 | A |
| 152 | 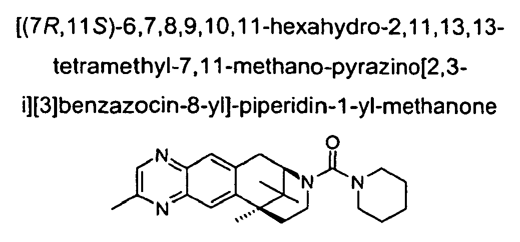 | A |
| 153 | 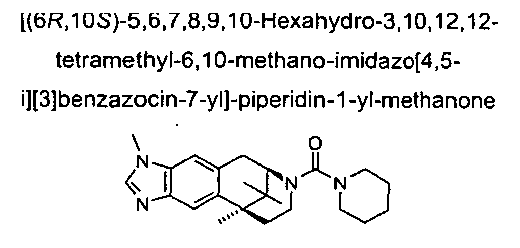 | A |
| 154 | 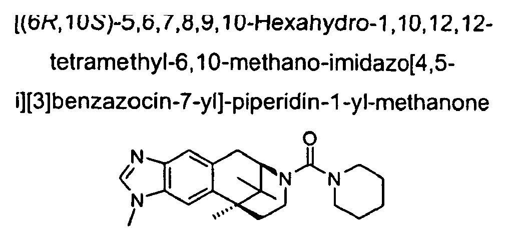 | A |
| 155 | 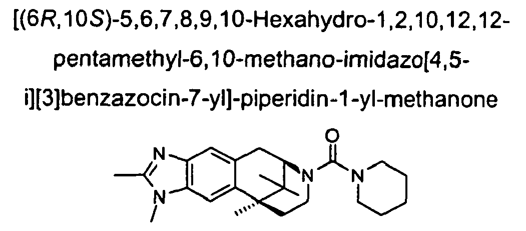 | A |
| 156 | 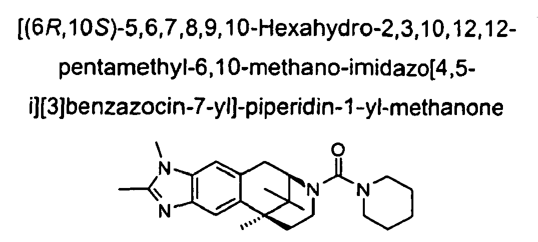 | A |
| 157 | 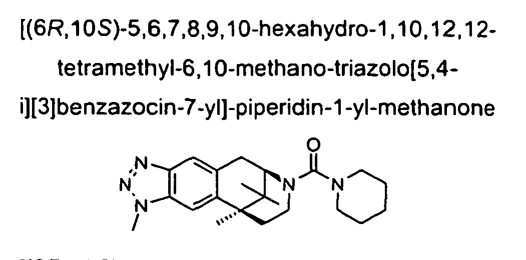 | A |
| 158 | 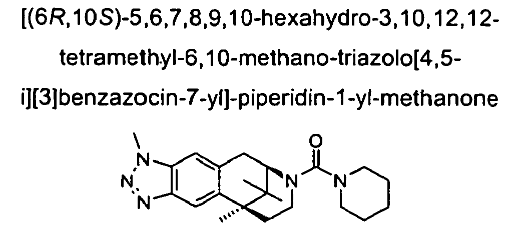 | A |
| 159 | 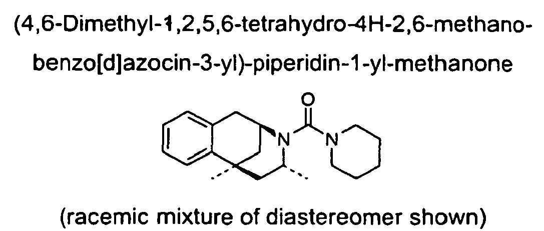 | A |
| 160 | 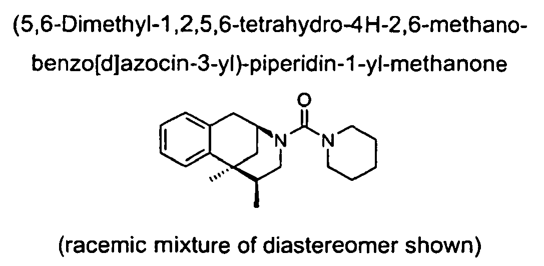 | A |
| 161 | 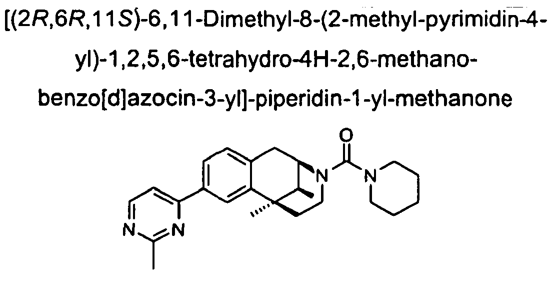 | A |
| 162 | 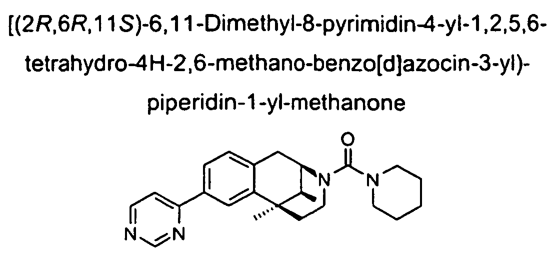 | A |
| 163 | 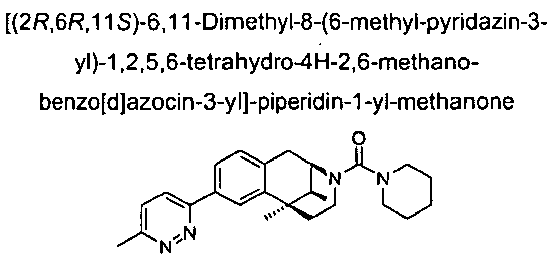 | A |
| 164 | 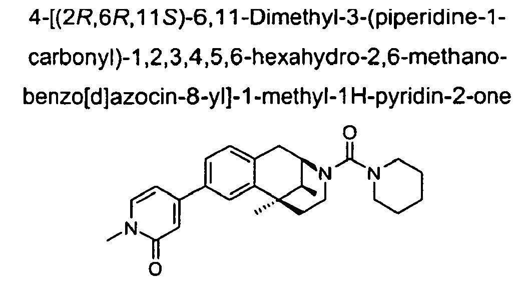 | A |
| 165 | 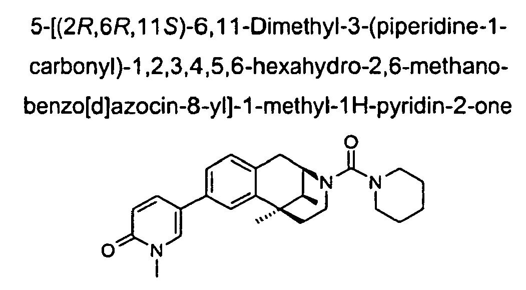 | A |
| 166 | 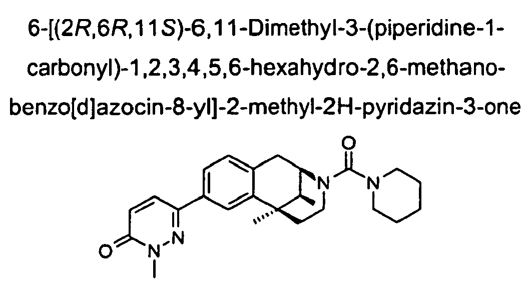 | A |
| 167 | 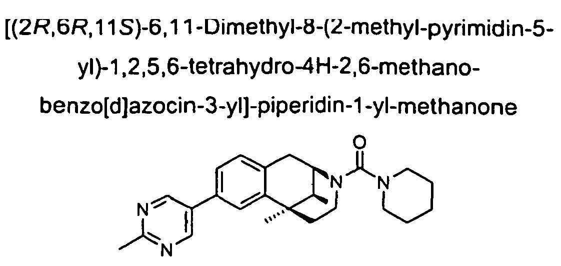 | A |
| 168 | 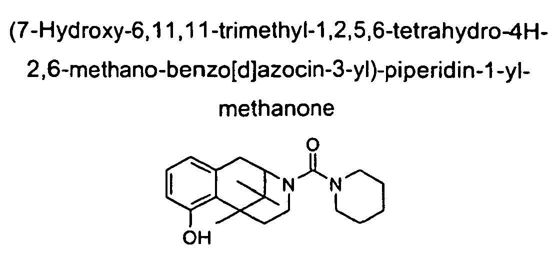 | A |
| 169 | 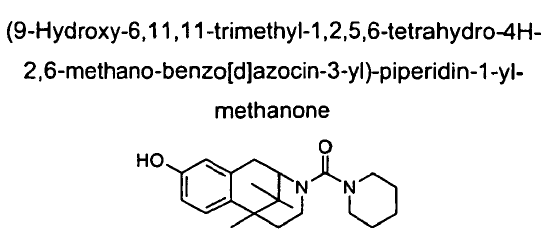 | A |
| 170 | 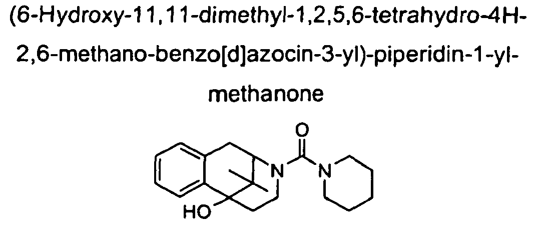 | A |
| 171 | 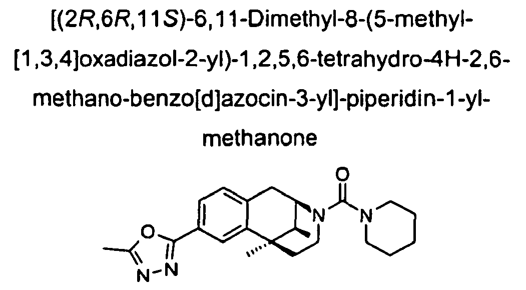 | A |
| 172 | 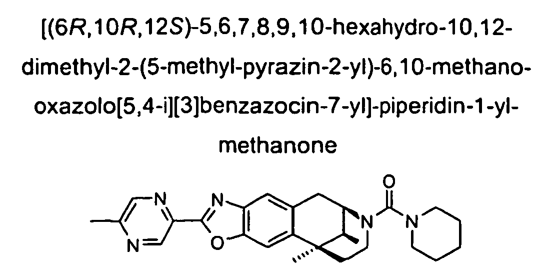 | A |
| 173 | 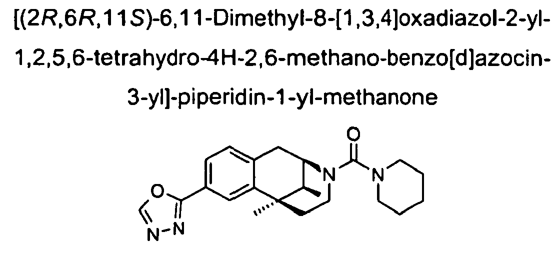 | A |
| 174 | 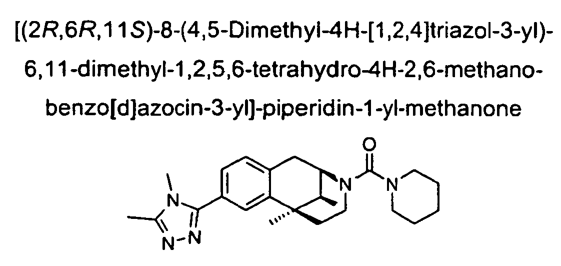 | A |
| 175 | 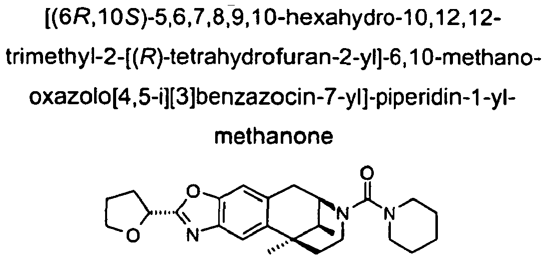 | A |
| 176 | 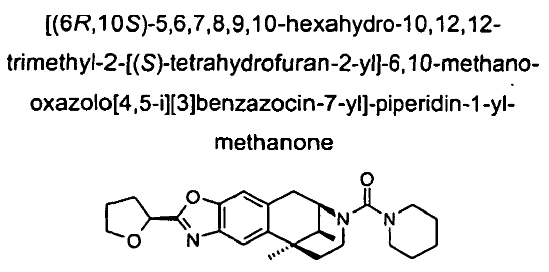 | A |
| 177 | 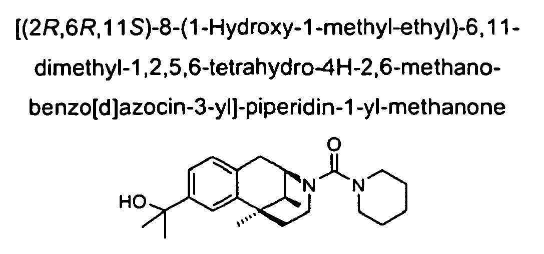 | A |
| 178 | 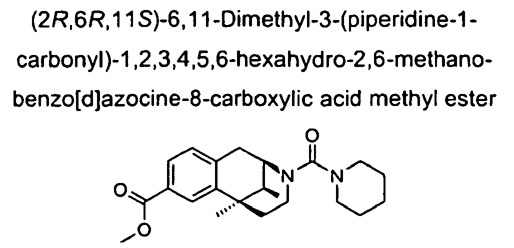 | A |
| 179 | 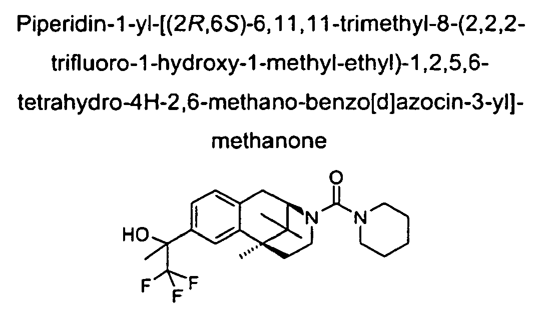 | A |
| 180 | 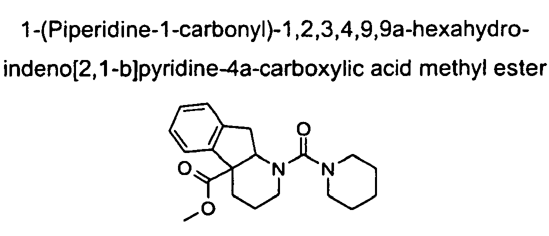 | A |
| 181 | 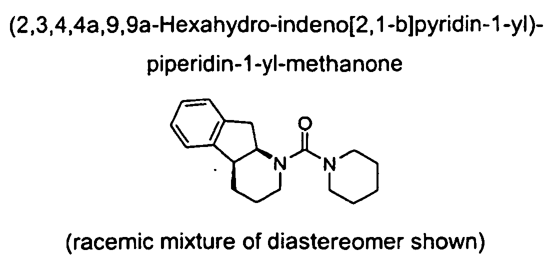 | A |
| 182 | 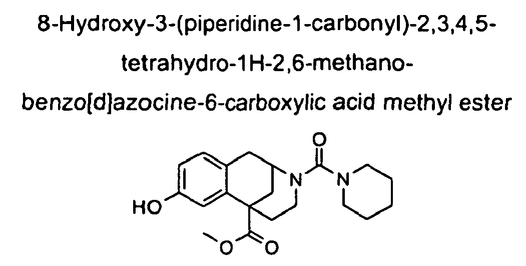 | A |
| 183 | 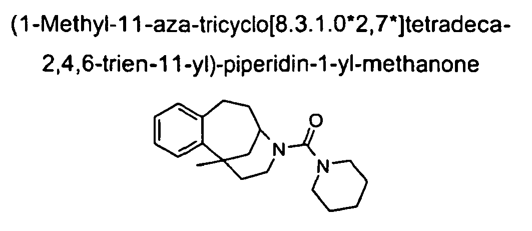 | A |
| 184 | 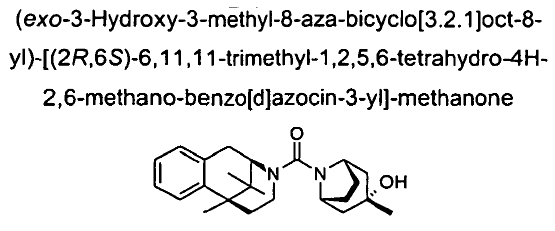 | C |
| 185 | 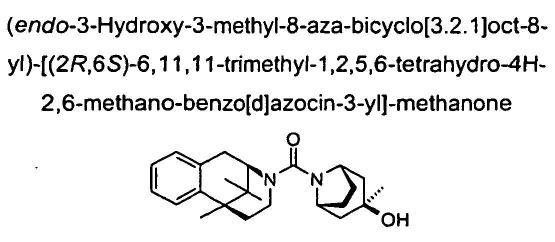 | C |
| 186 | 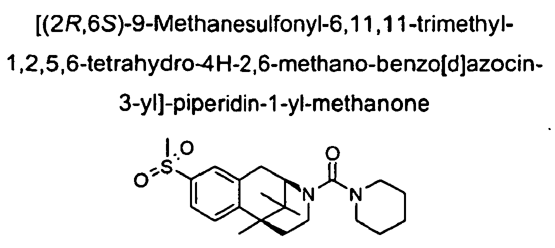 | A |
| 187 | 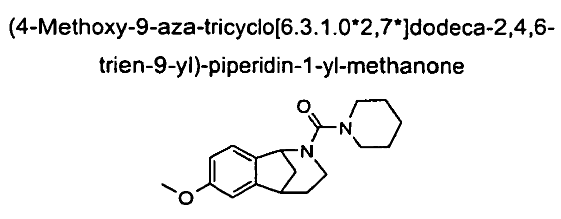 | A |
| 188 | 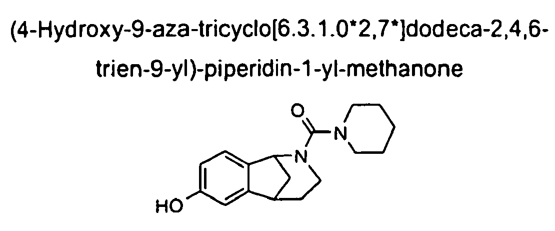 | A |
| 189 | 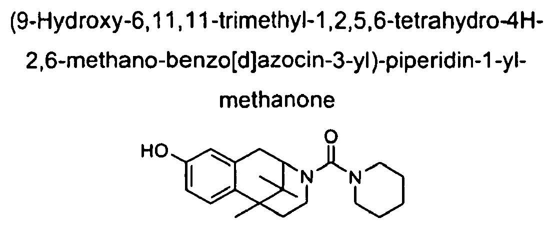 | A |
| 190 | 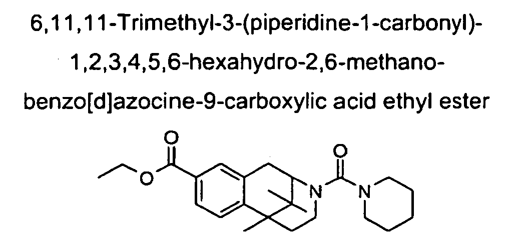 | A |

Some examples of formulations will now be described in which the term "active substance" denotes one or more compounds according to the invention, including the salts thereof. In the case of one of the combinations with one or additional active substances as described previously, the term "active substance" also includes the additional active substances.

### Example A

### Tablets containing 100 mg of active substance

### Composition:

1 tablet contains:

| | |
|---|---|
| active substance | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

### Method of Preparation:

The active substance, lactose and starch are mixed together and uniformly moistened with an aqueous solution of the polyvinylpyrrolidone. After the moist composition has been screened (2.0 mm mesh size) and dried in a rack-type drier at 50°C it is screened again (1.5 mm mesh size) and the lubricant is added. The finished mixture is compressed to form tablets.
Weight of tablet: 220 mg
Diameter: 10 mm, biplanar, facetted on both sides and notched on one side.

### Example B

### Tablets containing 150 mg of active substance

### Composition:

1 tablet contains:

| | |
|---|---|
| active substance | 150.0 mg |
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

### Preparation:

The active substance mixed with lactose, corn starch and silica is moistened with a 20% aqueous polyvinylpyrrolidone solution and passed through a screen with a mesh size of 1.5 mm. The granules, dried at 45°C, are passed through the same screen again and mixed with the specified amount of magnesium stearate. Tablets are pressed from the mixture.

| | |
|---|---|
| Weight of tablet: | 300 mg |
| die: | 10 mm, flat |

### Example C

### Hard gelatine capsules containing 150 mg of active substance

### Composition:

1 capsule contains:

| | | |
|---|---|---|
| active substance | | 150.0 mg |
| corn starch (dried) | approx. | 180.0 mg |
| lactose (powdered) | approx. | 87.0 mg |
| magnesium stearate | | 3.0 mg |
| | approx. | 420.0 mg |

### Preparation:

The active substance is mixed with the excipients, passed through a screen with a mesh size of 0.75 mm and homogeneously mixed using a suitable apparatus. The finished mixture is packed into size 1 hard gelatine capsules.
Capsule filling: approx. 320 mg
Capsule shell: size 1 hard gelatine capsule.

### Example D

### Suppositories containing 150 mg of active substance

### Composition:

1 suppository contains:

| | |
|---|---|
| active substance | 150.0 mg |
| polyethyleneglycol 1500 | 550.0 mg |
| polyethyleneglycol 6000 | 460.0 mg |
| polyoxyethylene sorbitan monostearate | 840.0 mg |
| | 2,000.0 mg |

### Preparation:

After the suppository mass has been melted the active substance is homogeneously distributed therein and the melt is poured into chilled moulds.

### Example E

### Ampoules containing 10 mg active substance

### Composition:

| | | |
|---|---|---|
| active substance | | 10.0 mg |
| 0.01 N hydrochloric acid q.s. | | |
| double-distilled water | ad | 2.0 mL |

### Preparation:

The active substance is dissolved in the necessary amount of 0.01 N HCl, made isotonic with common salt, filtered sterile and transferred into 2 mL ampoules.

### Example F

### Ampoules containing 50 mg of active substance

### Composition:

| | | |
|---|---|---|
| active substance | | 50.0 mg |
| 0.01 N hydrochloric acid q.s. | | |
| double-distilled water | ad | 10.0 mL |

### Preparation:

The active substance is dissolved in the necessary amount of 0.01 N HCl, made isotonic with common salt, filtered sterile and transferred into 10 mL ampoules.

## Claims

1. Compounds of formula I 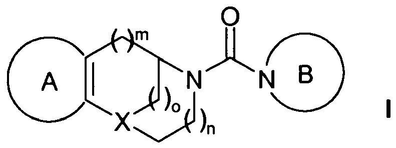 wherein
X denotes CH or N,
m, n, O independently of each other denote 0, 1 or 2,
wherein the C₅₊ₘ₊ₙ-azacydoalkene core structure of the general formula I including the bridging group -(CH₂)ₒ is optionally substituted with 1, 2 or more substituents independently of each other selected from the group consisting of R¹¹ and R¹²,
A denotes a benzo, pyrido, pyrrolo, furo, thieno, pyridazino, pyrimido, or pyrazino ring wherein each of said rings is substituted with one or more substituents independently of each other selected from R¹, and wherein 2 adjacent C-atoms of each of said rings are optionally substituted with R² and R³; or
a pyrazolo, imidazo, oxazolo, thiazolo, isoxazolo, or isothiazolo ring wherein each of said rings is optionally substituted with R¹; or
a 1,2,3-triazolo ring optionally substituted with R^{N}; and
B denotes a 3- to 8-membered monocylic, 7- to 12-membered spirocyclic, 6- to 12-membered bicyclic or 9- to 15-membered tricyclic azacycloalk-1-yl group, which is saturated or partially or fully unsaturated, wherein 1 or 2 -CH₂- groups may be replaced by -NR^{N}-, wherein 1 to 4 -CH₂- groups may be replaced independently of each other by O, S, carbonyl, or sulfonyl,
wherein 1 or 2 -CH= groups may be replaced by -N=, and
wherein said azacycloalkyl group may be substituted with one or more substituents independently of each other selected from L¹,
wherein said azacycloalkyl group may be substituted with 1 or 2 substituents independently of each other selected from L²,
wherein 2 adjacent C-atoms of said azacycloalkyl group may be substituted with L³ and L⁴, and
wherein 2 adjacent C-atoms of said azacycloalkyl group may be substituted with L⁵ and L⁶;
R^{N} independently of each other denotes hydrogen, C₁₋₆-alkyl, C₃₋₆-alkenyl, C₃₋₆-alkynyl, (het)aryl, C₁₋₄-alkylcarbonyl, C₁₋₄-alkylsulfonyl, (het)arylcarbonyl, (het)arylsulfonyl,
wherein each alkyl, alkenyl, and alkynyl group may be mono- or polysubstituted with fluorine, and may be monosubstitued with hydroxy, C₁₋₄-alkoxy, C₁₋₄-alkylsulfanyl, C₁₋₄-alkylsulfinyl, C₁₋₄-alkylsulfonyl, amino, C₁₋₄-alkylamino, di-(C₁₋₄-alkyl)amino, C₁₋₄-alkylcarbonylamino, cyano, carboxy, C₁₋₄-alkoxycarbonyl, aminocarbonyl, C₁₋₄-alkylaminocarbonyl, di-(C₁₋₄-alkyl)aminocarbonyl, or (het)aryl,
R¹ independently of each other denotes fluorine, chlorine, bromine, iodine, cyano, nitro, C₁₋₄-alkyl, difluoromethyl, trifluoromethyl, difluoromethoxy, trifluoromethoxy, trifluoromethyl-hydroxy-C₁₋₂-alkyl, 2,2,2-trifluoro-1-hydroxy-1-(trifluoromethyl)ethyl, C₃₋₆-cycloalkyl, C₃₋₆-cycloalkyloxy, C₃₋₆-cycloalkyl-C₁₋₃-alkyl, C₃₋₆-cycloalkyl-C₁₋₃-alkyloxy, tetrahydrofuran-3-yloxy, tetrahydropyran-3-yloxy, tetrahydropyran-4-yloxy, tetrahydrofuranyl-C₁₋₃-alkyloxy, tetrahydropyranyl-C₁₋₃-alkyloxy, (het)aryl, (het)aryloxy, (het)aryl-C₁₋₃-alkyl, (het)aryl-C₁₋₃-alkyloxy, (het)aryloxy-C₁₋₃-alkyl, C₁₋₃-alkyl-carbonyl, (het)aryl-carbonyl, amino, C₁₋₄-alkylamino, di-(C₁₋₄-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₄-alkyl)-piperazin-1-yl, 4-(C₁₋₄-alkylcarbonyl)-piperazin-1-yl, 4-(C₃₋₆-cycloalkylcarbonyl)-piperazin-1-yl, 4-(C₁₋₄-alkyloxycarbonyl)-piperazin-1-yl, 4-(C₁₋₄-alkylsulfonyl)-piperazin-1-yl, 2-oxo-4-(C₁₋₄-alkyl)-piperazin-1-yl, 3-oxo-4-(C₁₋₄-alkyl)-piperazin-1-yl,
C₁₋₄-alkyl-carbonylamino, (het)aryl-carbonylamino, (het)aryl-C₁₋₄-alkyl-carbonylamino, C₁₋₄-alkyloxy-carbonylamino, aminocarbonylamino, C₁₋₄-alkyl-amino-carbonylamino, di-(C₁₋₄-alkyl)aminocarbonylamino, pyrrolidin-1-yl-carbonyl-amino, piperidin-1-yl-carbonylamino, morpholin-4-yl-carbonylamino, piperazin-1-yl-carbonylamino, 4-(C₁₋₄-alkyl)-piperazin-1-yl-carbonylamino, C₁₋₄-alkyl-sul-fonylamino, aminosulfonylamino, C₁₋₄-alkylamino-sulfonylamino, di-(C₁₋₄-alkyl)-amino-sulfonylamino, pyrrolidin-1-yl-sulfonylamino, piperidin-1-yl-sulfonylamino, morpholin-4-yl-sulfonylamino, piperazin-1-yl-sulfonylamino, 4-(C₁₋₄-alkyl)-piperazin-1-yl-sulfonylamino, (C₁₋₄-alkyloxy-carbonylamino)carbonylamino, (het)arylsulfonylamino, (het)aryl-C₁₋₄-alkyl-sulfonylamino,
N-(C₁₋₄-alkyl)-C₁₋₄-alkyl-carbonylamino. N-(C₁₋₄-alkyl)-(het)arylcarbonylamino, N-(C₁₋₄-alkyl)-(het)aryl-C₁₋₄-alkyl-carbonylamino, N-(C₁₋₄-alkyl)-C₁₋₄-alkyloxy-carbonylamino, N-(aminocarbonyl)-C₁₋₄-alkylamino, N-(C₁₋₄-alkyl-amino-carbonyl)-C₁₋₄-alkylamino, N-[di-(C₁₋₄-alkyl)aminocarbonyl]-C₁-₄-alkylamino,
N-(C₁₋₄-alkyl)-C₁₋₄-alkyl-sulfonylamino, N-(C₁₋₄-alkyl)-(het)arylsulfonylamino, N-(C₁₋₄-alkyl)-(het)aryl-C₁₋₄-alkyl-sulfonylamino,
oxo-imidazolidin-1-yl, 2,4-dioxo-imidazolidin-1-yl, 2,5-dioxo-imidazolidin-1-yl, 2-oxo-hexahydropyrimidin-1-yl, wherein the nitrogen atom in position 3 of the aforementioned groups is optionally substituted with methyl or ethyl,
(hydroxyimino)aminomethyl, (C₁₋₃-alkyloxyimino)aminomethyl, carboxy, C₁₋₄-alkyloxy-carbonyl, aminocarbonyl, C₁₋₄-alkyl-aminocarbonyl, di-(C₁₋₄-alkyl)- aminocarbonyl, pyrrolidin-1-yl-carbonyl, piperidin-1-yl-carbonyl, morpholin-4-yl-carbonyl, piperazin-1-yl-carbonyl, 4-(C₁₋₄-alkyl)-piperazin-1-yl-carbonyl,
carboxy-C₁₋₄-alkyl, C₁₋₄-alkyloxy-carbonyl-C₁₋₄-alkyl, cyano-C₁₋₄-alkyl, aminocarbonyl-C₁₋₄-alkyl, C₁₋₄-alkyl-aminocarbonyl-C₁₋₄-alkyl, di-(C₁₋₄-alkyl)-aminocarbonyl-C₁-₄-alkyl, pyrrolidin-1-yl-carbonyl-C₁₋₄-alkyl, piperidin-1-yl-carbonyl-C₁₋₄-alkyl, morpholin-4-yl-carbonyl-C₁₋₄-alkyl, piperazin-1-yl-carbonyl-C₁₋₄-alkyl, 4-(C₁₋₄-alkyl)-piperazin-1-yl-carbonyl-C₁₋₄-alkyl,
carboxy-C₁₋₄-alkyloxy, C₁₋₄-alkyloxy-carbonyl-C₁₋₄-alkyloxy, cyano-C₁₋₄-alkyl-oxy, aminocarbonyl-C₁₋₄-alkyloxy, C₁₋₄-alkyl-aminocarbonyl-C₁₋₄-alkyloxy, di-(C₁₋₄-alkyl)-aminocarbonyl-C₁₋₄-alkyloxy, pyrrolidin-1-yl-carbonyl-C₁₋₄-alkyl-oxy, piperidin-1-yl-carbonyl-C₁₋₄-alkyloxy, morpholin-4-yl-carbonyl-C₁₋₄-alkyl-oxy, piperazin-1-yl-carbonyl-C₁₋₄-alkyloxy, 4-(C₁₋₄-alkyl)-piperazin-1-yl-carbonyl-C₁₋₄-alkyloxy,
hydroxy-C₁₋₄-alkyl, C₁₋₄-alkyloxy-C₁₋₄-alkyl, amino-C₁₋₄-alkyl, C₁₋₄-alkylamino-C₁-₄-alkyl, di-(C₁₋₄-alkyl)-amino-C₁₋₄-alkyl, pyrrolidin-1-yl-C₁₋₄-alkyl, C₁₋₄-alkyl-carbonyl-amino-C₁₋₄-alkyl, N-(C₁₋₄-alkyl)-C₁₋₄-alkylcarbonyl-amino-C₁₋₄-alkyl, 2-oxo-pyrrolidin-1-yl-C₁₋₄-alkyl, piperidin-1-yl-C₁₋₄-alkyl, 2-oxo-piperidin-1-yl-C₁₋₄-alkyl, morpholin-4-yl-C₁₋₄-alkyl, 3-oxo-morpholin-4-yl-C₁₋₄-alkyl, piperazin-1-yl-C₁₋₄-alkyl, 2-oxo-piperazin-1-yl-C₁₋₄-alkyl, 3-oxo-piperazin-1-yl-C₁-₄-alkyl, 4-(C₁₋₄-alkyl)-piperazin-1-yl-C₁₋₄-alkyl, 2-oxo-4-(C₁₋₄-alkyl)-piperazin-1-yl-C₁₋₄-alkyl, 3-oxo-4-(C₁₋₄-alkyl)-piperazin-1-yl-C₁₋₄-alkyl,
hydroxy-C₁₋₄-alkyloxy, C₁₋₄-alkyloxy-C₁₋₄-alkyloxy, C₁₋₄-alkylsulfanyl-C₁-₄-alkyl-oxy, C₁₋₄-alkylsulfinyl-C₁₋₄-alkyloxy, C₁₋₄-alkylsulfonyl-C₁₋₄-alkyloxy, amino-C₁₋₄-alkyloxy, C₁₋₄-alkylamino-C₁₋₄-alkyloxy, di-(C₁₋₄-alkyl)-amino-C₁₋₄-alkyloxy, pyrrolidin-1-yl-C₁₋₄-alkyloxy, 2-oxo-pyrrolidin-1-yl-C₁₋₄-alkyloxy, piperidin-1-yl-C₁₋₄-alkyloxy, 2-oxo-piperidin-1-yl-C₁₋₄-alkyloxy, morpholin-4-yl-C₁₋₄-alkyloxy, 3-oxo-morpholin-4-yl-C₁₋₄-alkyloxy, piperazin-1-yl-C₁₋₄-alkyloxy, 2-oxo-piperazin-1-yl-C₁₋₄-alkyloxy, 3-oxo-piperazin-1-yl-C₁₋₄-alkyloxy, 4-(C₁₋₄-alkyl)-piperazin-1- yl-C₁₋₄-alkyloxy, 2-oxo-4-(C₁₋₄-alkyl)-piperazin-1-yl-C₁₋₄-alkyloxy, 3-oxo-4-(C₁₋₄-alkyl)-piperazin-1-yl-C₁₋₄-alkyloxy,
C₁₋₄-alkylsulfanyl, C₁₋₄-alkysulfinyl, C₁₋₄-alkylsulfonyl, C₁₋₄-alkylsulfonyloxy, (het)arylsulfonyl, (het)arylsulfonyloxy, trifluoromethylsulfanyl, trifluoromethyl-sulfinyl, trifluoromethylsulfonyl, C₃₋₆-cycloalkylsulfanyl, C₃₋₆-cycloalkylsulfinyl, C₃₋₆-cycloalkylsulfonyl, C₃₋₆-cycloalkyl-C₁₋₃-alkylsulfanyl, C₃₋₆-cycloalkyl-C₁₋₃-alkylsulfinyl, C₃₋₆-cycloalkyl-C₁₋₃-alkylsulfonyl,
aminosulfonyl, C₁₋₄-alkyl-aminosulfonyl, di-(C₁₋₄-alkyl)-aminosulfonyl, pyrrolidin-1-yl-sulfonyl, piperidin-1-yl-sulfonyl, morpholin-4-yl-sulfonyl, piperazin-1-yl-sulfonyl, or 4-(C₁₋₄-alkyl)-piperazin-1-yl-sulfonyl,
wherein the above-mentioned saturated heterocycles and cycloalkyl-rings are optionally substituted with one or two groups independently of each other selected from fluorine, C₁₋₃-alkyl, C₁₋₃-alkoxy, C₁₋₃-alkoxy-C₁₋₃-alkyl, or hydroxy,
R², R³ are linked to each other to form a methylenedioxy, ethylenedioxy or C₃₋₅-alkylene bridging group, which may be mono- or disubstituted with methyl, and which may be mono- or polyfluorinated; or
R², R³ form combined with the carbon atoms to which they are attached a benzo, pyrido, pyrimido, pyrazino, pyridazino, pyrrolo, furano, thieno, pyrazolo, imidazo, triazolo, oxazolo, thiazolo, isoxazolo, or isothiazolo ring, wherein each of said rings may be substituted with one or more substituents independently of each other selected from R^{P};
R^{P} denotes halogen, C₁₋₆-alkyl, hydroxy-C₁₋₄-alkyl, C₁₋₃-alkyloxy-C₁₋₃-alkyl, C₃₋₆-cycloalkyl, hydroxy-C₄₋₆-cycloalkyl, C₁₋₃-alkyloxy-C₃₋₆-cycloalkyl, azetidinyl, 1-(C₁-₃-alkyl)-azetidinyl, 1-(C₁₋₃-alkylcarbonyl)-azetidinyl, pyrrolidinyl, 1-(C₁₋₃-alkyl)-pyrrolidinyl, 1-(C₁₋₃-alkylcarbonyl)-pyrrolidinyl, piperidinyl, 1-(C₁₋₃-alkyl)-piperidinyl, 1-(C₁₋₃-alkylcarbonyl)piperidinyl, tetrahydrofuranyl, tetrahydropyranyl, difluoromethyl, trifluoromethyl, cyano, nitro, amino, C₁₋₃- alkylamino, di-(C₁₋₃-alkyl)amino, C₁₋₃-alkylcarbonylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, aminosulfonyl, methylsulfanyl, methylsulfinyl, methylsulfonyl, hydroxy, C₁₋₃-alkyloxy, difluoromethoxy, trifluoromethoxy, phenyl, or
pyrrolyl, furanyl, thienyl, pyridyl, where in any of these groups 1 or 2 CH are optionally replaced by N atoms, or
1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl,
wherein each of the aromatic or heteroaromatic groups mentioned above is optionally substituted with one or two groups independently selected from fluorine, chlorine, C₁₋₃-alkyl, difluoromethyl, trifluoromethyl, cyano, amino, acetylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, hydroxy, C₁₋₃-alkyloxy, difluoromethoxy, and trifluoromethoxy,
R¹⁰ independently of each other denotes halogen, C₁₋₃-alkyl, difluoromethyl, trifluoromethyl, cyano, nitro, amino, C₁₋₃-alkylamino, di-(C₁₋₃-alkyl)amino, acetylamino, methylsulfonylamino, carboxy, C₁₋₄-alkyloxycarbonyl, aminocarbonyl, C₁₋₃-alkylaminocarbonyl, di-(C₁₋₃-alkyl)-aminocarbonyl, aminosulfonyl, methylsulfanyl, methylsulfinyl, methylsulfonyl, hydroxy, C₁₋₃-alkyloxy, difluoromethoxy, trifluoromethoxy, or phenyl optionally substituted with 1 or 2 substituents independently of each other selected from fluorine, methyl, methoxy, cyano, or hydroxy,
R¹¹ independently of each other denotes fluorine, C₁₋₄-alkyl, (het)aryl, hydroxy, C₁₋₄-alkyloxy, cyano, carboxy, C₁₋₄-alkyloxycarbonyl, aminocarbonyl, C₁₋₄-alkylamino-carbonyl, di-(C₁₋₄-alkyl)-aminocarbonyl, hydroxy-C₁₋₄-alkyl, or C₁₋₃-alkyloxy-C₁₋₄-alkyl,
R¹² independently of each other denotes fluorine or C₁₋₄-alkyl, and
L¹ independently of each other denotes halogen, C₁₋₄-alkyl, trifluoromethyl, hydroxy, C₁₋₄-alkyloxy, or cyano;
L² independently of each other denotes fluorine, chlorine, bromine, iodine, nitro, cyano, hydroxy, C₃₋₆-cycloalkyl , C₃₋₆-cycloalkyloxy, tetrahydrofuran-3-yl-oxy, tetrahydropyran-3-yl-oxy, tetrahydropyran-4-yl-oxy, tetrahydrofuranyl-C₁₋₃-alkyloxy, tetrahydropyranyl-C₁₋₃-alkyloxy, (het)aryl, (het)aryloxy, or
C₁₋₆-alkyl. C₂₋₆-alkenyl, C₂₋₆-alkynyl, C₁₋₄-alkyloxy, wherein in each group one CH₂ group may be replaced by carbonyl or sulfonyl, and wherein each group may be mono or polyfluorinated, and wherein each group may additionally be substituted with
hydroxy, chlorine, C₁₋₃-alkyloxy, amino, C₁₋₃-alkylamino, di-(C₁₋₃-alkyl)-amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₃-alkyl)-piperazin-1-yl, 2-oxo-4-(C₁₋₃-alkyl)-piperazin-1-yl, 3-oxo-4-(C₁₋₃-alkyl)-piperazin-1-yl, carboxy, C₁₋₃-alkyloxy-carbonyl, cyano, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, C₁₋₃-alkylcarbonylamino, arylcarbonylamino, C₁₋₃-alkylsulfanyl, C₁₋₃-alkylsulfinyl, C₁₋₃-alkylsulfonyl, C₃₋₆-cycloalkyl, (het)aryl, or (het)aryloxy;
amino, C₁₋₃-alkylamino, di-(C₁₋₃-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₃-alkyl)- piperazin-1-yl, 4-(C₁₋₄-alkylcarbonyl)-piperazin-1-yl, 4-(C₃₋₆-cycloalkylcarbonyl)-piperazin-1-yl, 4-(C₁₋₄-alkyloxycarbonyl)-piperazin-1-yl, 4-(C₁₋₄-alkylsulfonyl)-piperazin-1-yl, 2-oxo-4-(C₁₋₃-alkyl)-piperazin-1-yl, 3-oxo-4-(C₁₋₃-alkyl)-piperazin-1-yl,
C₁₋₄-alkyl-carbonylamino, (het)aryl-carbonylamino, (het)aryl-C₁₋₃-alkyl-carbonylamino, C₁₋₃-alkyloxy-carbonylamino, aminocarbonylamino, C₁₋₃-alkyl-amino-carbonylamino, di-(C₁₋₃-alkyl)aminocarbonylamino, pyrrolidin-1-yl-carbonyl-amino, piperidin-1-yl-carbonylamino, morpholin-4-yl-carbonylamino, piperazin-1-yl-carbonylamino, 4-(C₁₋₃-alkyl)-piperazin-1-yl-carbonylamino, C₁₋₃-alkyl-sulfonylamino, aminosulfonylamino, C₁₋₃-alkylamino-sulfonylamino, di-(C₁₋₃-alkyl)amino-sulfonylamino, pyrrolidin-1-yl-sulfonylamino, piperidin-1-yl-sulfonylamino, morpholin-4-yl-sulfonylamino, piperazin-1-yl-sulfonylamino, 4-(C₁₋₃-alkyl)-piperazin-1-yl-sulfonylamino, (C₁₋₃-alkyloxy-carbonylamino)carbonylamino, (het)arylsulfonylamino, (het)aryl-C₁₋₃-alkyl-sulfonylamino,
N-(C₁₋₃-alkyl)-C₁₋₃-alkyl-carbonylamino, N-(C₁₋₃-alkyl)-(het)arylcarbonylamino, N-(C₁₋₃-alkyl)-(het)aryl-C₁₋₃-alkyl-carbonylamino, N-(C₁₋₃-alkyl)-C₁₋₃-alkyloxy-carbonylamino, N-(aminocarbonyl)-C₁₋₃-alkylamino, N-(C₁₋₃-alkyl-amino-carbonyl)-C₁₋₃-alkylamino, N-[di-(C₁₋₃-alkyl)aminocarbonyl]-C₁₋₃-alkylamino,
N-(C₁₋₃-alkyl)-C₁₋₃-alkyl-sulfonylamino, N-(C₁₋₃-alkyl)-(het)arylsulfonylamino, N-(C₁₋₃-alkyl)-(het)aryl-C₁₋₃-alkyl-sulfonylamino,
carboxy, C₁-₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, (het)arylaminocarbonyl, N-(C₁₋₃-alkyl)-(het)arylaminocarbonyl, (het)aryl-C₁₋₃-alkylaminocarbonyl, N-(C₁₋₃-alkyl)-(het)aryl-C₁₋₃-alkylaminocarbonyl,
C₁₋₃-alkylsulfanyl, C₁₋₃-alkysulfinyl, (het)arylsulfonyl, trifluoromethylsulfanyl, trifluoromethylsulfinyl, aminosulfonyl, C₁₋₃-alkyl-aminosulfonyl, di-(C₁₋₃-alkyl)-aminosulfonyl, pyrroli-din-1-yl-sulfonyl, piperidin-1-yl-sulfonyl, morpholin-4-yl-sulfonyl, piperazin-1-yl-sulfonyl, 4-(C₁₋₃-alkyl)-piperazin-1-yl-sulfonyl,
wherein the above-mentioned saturated heterocyclic- and cycloalkyl-rings are optionally substituted with one or two groups selected from fluorine, C₁₋₃-alkyl, C₁₋₃-alkoxy, C₁₋₃-alkoxy-C₁₋₃-alkyl, or hydroxy, and
L³ and L⁴ are linked to each other, and
L⁵ and L⁶ are linked to each other, such that independently of each other and in each case together with the 2 adjacent C-atoms to which they are attached an aryl- or heteroaryl-group is formed which is fused to the cyclic group B, and which aryl- or heteroaryl-group is optionally substituted with 1, 2 or 3 identical or different R¹⁰,
while by aryl is meant phenyl or naphthtyl and by heteroaryl is meant pyrrolyl, furanyl, thienyl, pyridyl, indolyl, benzofuranyl, benzothiophenyl, quinolinyl, isoquinolinyl, or pyrrolyl, furanyl, thienyl, pyridyl wherein in said pyrrolyl, furanyl, thienyl, pyridyl 1 or 2 CH groups are each replaced by N, or indolyl, benzofuranyl, benzothiophenyl, quinolinyl, isoquinolinyl wherein in said indolyl, benzofuranyl, benzothiophenyl, quinolinyl, isoquinolinyl 1 to 3 CH groups are each replaced by N, or tetrazolyl,
while the above-mentioned (het)aryl is an aryl group as defined hereinbefore, or a heteroaryl group as defined hereinbefore, or a ring selected from the group consisting of 1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl, 1,2,3,4-tetrahydro-2,3-dioxo-pyrazinyl, 2,3-dihydro-2-oxo-indolyl, 2,3-dihydrobenzofuranyl, 2,3-dihydro-2-oxo-1*H*-benzimidazolyl, 2,3-dihydro-2-oxo-benzoxazolyl,1,2-dihydro-2-oxo-quinolinyl,1,4-dihydro-4-oxo-quinolinyl,1,2-dihydro-1-oxo-isoquinolinyl, 1,4-dihydro-4-oxo-cinnolinyl, 1,2-dihydro-2-oxo-quinazolinyl, 1,4-dihydro-4-oxo-quinazolinyl, 1,2,3,4-tetrahydro-2,4-dioxo-quinazolinyl, 1,2-dihydro-2-oxoquinoxalinyl, 1,2,3,4-tetrahydro-3-oxo-quinoxalinyl, 1,2,3,4-tetrahydro-2,3-dioxo-quinoxalinyl, 1,2-dihydro-1-oxo-phthalazinyl, 1,2,3,4-tetrahydro-1,4-dioxo-phthalazinyl, chromanyl, coumarinyl, 2,3-dihydro-benzo[1,4]dioxinyl and 3,4-dihydro-3-oxo-2*H*-benzo[1,4]oxazinyl, wherein each of said rings is optionally substituted with 1, 2 or 3 substituents independently of each other selected from R¹⁰,
whilst each of the above-mentioned alkyl or alkylene moieties may be branched or unbranched,
the tautomers, the stereoisomers thereof, the mixtures thereof, and the salts thereof, while the following compounds (M1) to (M4) are excluded,

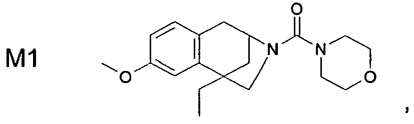

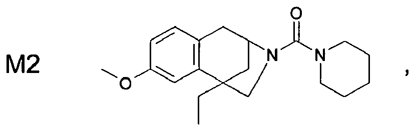

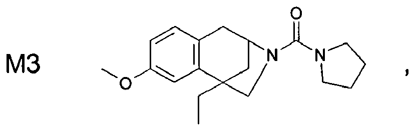

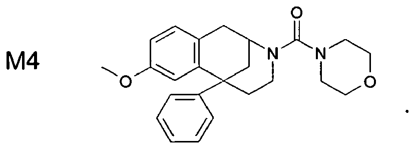

2. Compounds according to claim 1, **characterized by** a general formula selected from one or more of the formulae I.1 to I.10 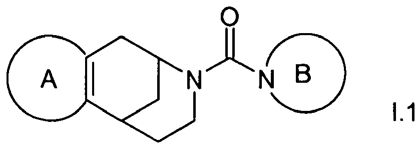 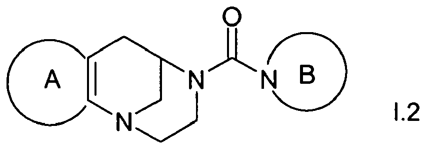 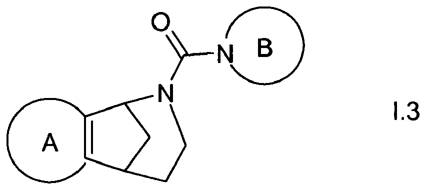 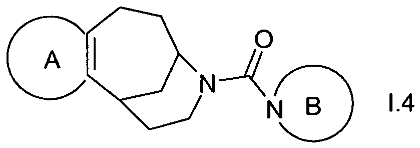 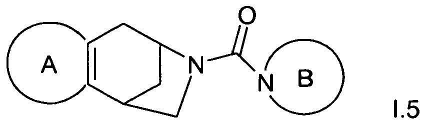 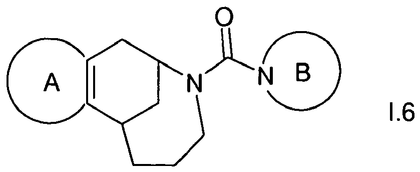 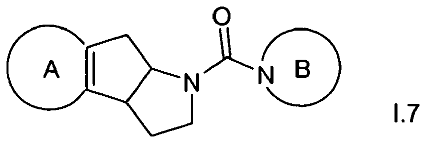 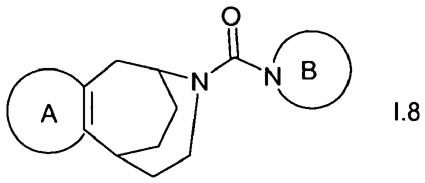 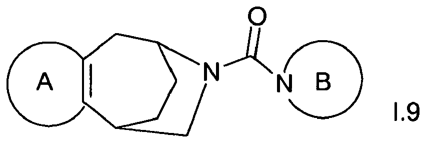 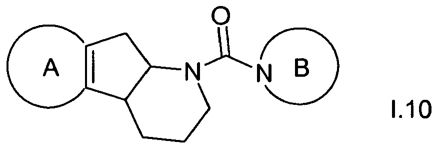 wherein the C₅₊ₘ₊ₙ-azacydoalkene core structure of general formulae I.1 to I.10 including the bridging group -(CH₂)ₒ is optionally substituted with 1, 2 or more substituents independently of each other selected from the group consisting of R¹¹ and R¹², and
wherein the rings A and B and m, n, o, R¹¹, R¹² are defined as in claim 1, their tautomers, their stereoisomers, mixtures thereof and the salts thereof, while the compounds (M1), (M2), (M3) and (M4) as defined in claim 1, are excluded.

3. Compounds according to claim 1 or 2, wherein the nitrogen containing ring B denotes azetidin-1-yl, pyrrolidin-1-yl, piperidin-1-yl, azepan-1-yl, azocan-1-yl,
azetidin-1-yl, wherein one -CH₂- group is replaced by O, S, NR^{N}, carbonyl, or sulfonyl, or
pyrrolidin-1-yl, piperidin-1-yl, azepan-1-yl, azocan-1-yl, which may be partially or fully unsaturated and wherein one or two -CH₂- groups are independently of each other replaced by O, S, carbonyl, or sulfonyl, and wherein one -CH₂- group may be replaced by -NR^{N}-,
aza-bicyclohept-N-yl, aza-bicyclooct-N-yl, aza-bicyclonon-N-yl, aza-bicyclodec-N-yl, bicycloundec-N-yl, bicyclododec-N-yl, each of which may be partially unsaturated, and in each of which one or two -CH₂- groups may be replaced independently of each other by O, S, -NR^{N}-, carbonyl, or sulfonyl, and in each of which one -CH= group may be replaced by -N=,
aza-tricyclonon-N-yl, aza-tricyclodec-N-yl, aza-tricycloundec-N-yl, aza-tricyclododec-N-yl, aza-tricyclotridec-N-yl, aza-tricyclotetradec-N-yl, in each of which one or two -CH₂- groups may be replaced independently of each other by O, S, NR^{N}, carbonyl, or sulfonyl, and in each of which one -CH= group may be replaced by -N=,
wherein each of the above rings B may be substituted with one or more substituents independently of each other selected from L¹,
wherein each of the above rings B may be substituted with 1 or 2 substituents independently of each other selected from L²,
wherein 2 adjacent C-atoms of each of the above rings B may be substituted with L³ and L⁴, and
wherein 2 adjacent C-atoms of each of the above rings B may be substituted with L⁵ and L⁶;
wherein R^{N}, L¹, L², L³, L⁴, L⁵, L⁶ are defined as in claim 1.

4. Compounds according to claim 1, 2 or 3, wherein the ring A denotes a benzo, pyrido, pyrrolo, furo, thieno, pyridazino, pyrimido, or pyrazino ring wherein each of said rings is substituted with one or more substituents independently of each other selected from R¹, and wherein 2 adjacent C-atoms of each of said rings are optionally substituted with R² and R³; or
a pyrazolo, oxazolo, thiazolo, or imidazo ring each of which is optionally substituted with R¹; wherein R¹, R² and R³ are defined as in claim 1.

5. Physiologically acceptable salts of the compounds according to at least one of claims 1 to 4 with inorganic or organic acids or bases.

6. Pharmaceutical compositions containing a compound according to at least one of claims 1 to 4 or a physiologically acceptable salt according to claim 5 optionally together with one or more inert carriers and/or diluents.

7. Compounds of formula I wherein
A denotes a benzo, pyrido, pyrrolo, furo, thieno, pyridazino, pyrimido, or pyrazino ring wherein each of said rings is optionally substituted with one or more substituents independently of each other selected from R¹, and wherein 2 adjacent C-atoms of each of said rings are optionally substituted with R² and R³; or
a pyrazolo, imidazo, oxazolo, thiazolo, isoxazolo, or isothiazolo ring wherein each of said rings is optionally substituted with R¹; or
a 1,2,3-triazolo ring optionally substituted with R^{N}; and
R¹ independently of each other denotes fluorine, chlorine, bromine, iodine, cyano, nitro, C₁₋₄-alkyl, hydroxy, C₁₋₄-alkyloxy, difluoromethyl, trifluoromethyl, difluoromethoxy, trifluoromethoxy, trifluoromethyl-hydroxy-C₁₋₂-alkyl, 2,2,2- trifluoro-1-hydroxy-1-(trifluoromethyl)ethyl, C₃₋₆-cycloalkyl, C₃₋₆-cycloalkyloxy, C₃₋₆-cycloalkyl-C₁-₃-alkyl, C₃₋₆-cycloalkyl-C₁₋₃-alkyloxy, tetrahydrofuran-3-yloxy, tetrahydropyran-3-yloxy, tetrahydropyran-4-yloxy, tetrahydrofuranyl-C₁₋₃-alkyloxy, tetrahydropyranyl-C₁₋₃-alkyloxy, (het)aryl, (het)aryloxy, (het)aryl-C₁₋₃-alkyl, (het)aryl-C₁₋₃-alkyloxy, (het)aryloxy-C₁₋₃-alkyl, C₁₋₃-alkyl-carbonyl, (het)aryl-carbonyl,
amino, C₁₋₄-alkylamino, di-(C₁₋₄-alkyl)amino, pyrrolidin-1-yl, 2-oxo-pyrrolidin-1-yl, piperidin-1-yl, 2-oxo-piperidin-1-yl, morpholin-4-yl, 3-oxo-morpholin-4-yl, piperazin-1-yl, 2-oxo-piperazin-1-yl, 3-oxo-piperazin-1-yl, 4-(C₁₋₄-alkyl)-piperazin-1-yl, 4-(C₁₋₄-alkylcarbonyl)-piperazin-1-yl, 4-(C₃₋₆-cycloalkylcarbonyl)-piperazin-1-yl, 4-(C₁₋₄-alkyloxycarbonyl)-piperazin-1-yl, 4-(C₁₋₄-alkylsulfonyl)-piperazin-1-yl, 2-oxo-4-(C₁₋₄-alkyl)-piperazin-1-yl, 3-oxo-4-(C₁₋₄-alkyl)-piperazin-1-yl,
C₁₋₄-alkyl-carbonylamino, (het)aryl-carbonylamino, (het)aryl-C₁₋₄-alkyl-carbonylamino, C₁₋₄-alkyloxy-carbonylamino, aminocarbonylamino, C₁₋₄-alkyl-amino-carbonylamino, di-(C₁₋₄-alkyl)aminocarbonylamino, pyrrolidin-1-yl-carbonyl-amino, piperidin-1-yl-carbonylamino, morpholin-4-yl-carbonylamino, piperazin-1-yl-carbonylamino, 4-(C₁₋₄-alkyl)-piperazin-1-yl-carbonylamino, C₁₋₄-alkyl-sulfonylamino, aminosulfonylamino, C₁₋₄-alkylamino-sulfonylamino, di-(C₁₋₄-alkyl)-amino-sulfonylamino, pyrrolidin-1-yl-sulfonylamino, piperidin-1-yl-sulfonylamino, morpholin-4-yl-sulfonylamino, piperazin-1-yl-sulfonylamino, 4-(C₁-₄-alkyl)-piperazin-1-yl-sulfonylamino, (C₁₋₄-alkyloxy-carbonylamino)carbonylamino, (het)arylsulfonylamino, (het)aryl-C₁₋₄-alkyl-sulfonylamino,
N-(C₁₋₄-alkyl)-C₁₋₄-alkyl-carbonylamino, N-(C,₄-alkyl)-(het)arylcarbonylamino, N-(C₁₋₄-alkyl)-(het)aryl-C₁₋₄-alkyl-carbonylamino, N-(C₁₋₄-alkyl)-C₁₋₄-alkyloxy-carbonylamino, N-(aminocarbonyl)-C₁₋₄-alkylamino, N-(C₁₋₄-alkyl-amino-carbonyl)-C₁₋₄-alkylamino, N-[di-(C₁₋₄-alkyl)aminocarbonyl]-C₁₋₄-alkylamino,
N-(C₁₋₄-alkyl)-C₁₋₄-alkyl-sulfonylamino, N-(C₁₋₄-alkyl)-(het)arylsulfonylamino, N-(C₁₋₄-alkyl)-(het)aryl-C₁₋₄-alkyl-sulfonylamino, oxo-imidazolidin-1-yl, 2,4-dioxo-imidazolidin-1-yl, 2,5-dioxo-imidazolidin-1-yl, 2-oxo-hexahydropyrimidin-1-yl, wherein the nitrogen atom in position 3 of the aforementioned groups is optionally substituted with methyl or ethyl,
(hydroxyimino)aminomethyl, (C₁₋₃-alkyloxyimino)aminomethyl, carboxy, C₁₋₄-alkyloxy-carbonyl, aminocarbonyl, C₁₋₄-alkyl-aminocarbonyl, di-(C₁₋₄-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₄-alkyl)-piperazin-1-yl-carbonyl,
carboxy-C₁₋₄-alkyl, C₁₋₄-alkyloxy-carbonyl-C₁₋₄-alkyl, cyano-C₁₋₄-alkyl, aminocarbonyl-C₁₋₄-alkyl, C₁₋₄-alkyl-aminocarbonyl-C₁₋₄-alkyl, di-(C₁₋₄-alkyl)-aminocarbonyl-C₁₋₄-alkyl, pyrrolidin-1-yl-carbonyl-C₁₋₄-alkyl, piperidin-1-yl-carbonyl-C₁₋₄-alkyl, morpholin-4-yl-carbonyl-C₁₋₄-alkyl, piperazin-1-yl-carbonyl-C₁₋₄-alkyl, 4-(C₁₋₄-alkyl)-piperazin-1-yl-carbonyl-C₁₋₄-alkyl,
carboxy-C₁₋₄-alkyloxy, C₁₋₄-alkyloxy-carbonyl-C₁₋₄-alkyloxy, cyano-C₁₋₄-alkyl-oxy, aminocarbonyl-C₁₋₄-alkyloxy, C₁₋₄-alkyl-aminocarbonyl-C₁₋₄-alkyloxy, di-(C₁₋₄-alkyl)-aminocarbonyl-C₁₋₄-alkyloxy, pyrrolidin-1-yl-carbonyl-C₁₋₄-alkyloxy, piperidin-1-yl-carbonyl-C₁₋₄-alkyloxy, morpholin-4-yl-carbonyl-C₁₋₄-alkyl-oxy, piperazin-1-yl-carbonyl-C₁₋₄-alkyloxy, 4-(C₁₋₄-alkyl)-piperazin-1-yl-carbonyl-C₁₋₄-alkyloxy,
hydroxy-C₁₋₄-alkyl, C₁₋₄-alkyloxy-C₁₋₄-alkyl, amino-C₁₋₄-alkyl, C₁₋₄-alkylamino-C₁₋₄-alkyl, di-(C₁₋₄-alkyl)-amino-C₁₋₄-alkyl, pyrrolidin-1-yl-C₁₋₄-alkyl, C₁₋₄-alkyl-carbonyl-amino-C₁₋₄-alkyl, N-(C₁₋₄-alkyl)-C₁₋₄-alkylcarbonyl-amino-C₁₋₄-alkyl, 2-oxo-pyrrolidin-1-yl-C₁₋₄-alkyl, piperidin-1-yl-C₁₋₄-alkyl, 2-oxo-piperidin-1-yl-C₁₋₄-alkyl, morpholin-4-yl-C₁₋₄-alkyl, 3-oxo-morpholin-4-yl-C₁₋₄-alkyl, piperazin-1-yl-C₁₋₄-alkyl, 2-oxo-piperazin-1-yl-C₁₋₄-alkyl, 3-oxo-piperazin-1-yl-C₁₋₄-alkyl, 4-(C₁₋₄-alkyl)-piperazin-1-yl-C₁₋₄-alkyl, 2-oxo-4-(C₁₋₄-alkyl)-piperazin-1-yl-C₁₋₄-alkyl, 3-oxo-4-(C₁₋₄-alkyl)-piperazin-1-yl-C₁₋₄-alkyl, hydroxy-C₁₋₄-alkyloxy, C₁₋₄-alkyloxy-C₁₋₄-al kyloxy, C₁₋₄-alkylsulfanyl-C₁₋₄-alkyl-oxy, C₁₋₄-alkylsulfinyl-C₁₋₄-alkyloxy, C₁₋₄-alkylsulfonyl-C₁₋₄-alkyloxy, amino-C₁₋₄-alkyloxy, C₁₋₄-alkylamino-C₁₋₄-alkyloxy, di-(C₁₋₄-alkyl)-amino-C₁₋₄-alkyloxy, pyrrolidin-1-yl-C₁₋₄-alkyloxy, 2-oxo-pyrrolidin-1-yl-C₁₋₄-alkyloxy, piperidin-1-yl-C₁₋₄-alkyloxy, 2-oxo-piperidin-1-yl-C₁₋₄-alkyloxy, morpholin-4-yl-C₁₋₄-alkyloxy, 3-oxo-morpholin-4-yl-C₁₋₄-alkyloxy, piperazin-1-yl-C₁₋₄-alkyloxy, 2-oxo-piperazin-1-yl-C₁₋₄-alkyloxy, 3-oxo-piperazin-1-yl-C₁₋₄-alkyloxy, 4-(C₁₋₄-alkyl)-piperazin-1-yl-C₁₋₄-alkyloxy, 2-oxo-4-(C₁₋₄-alkyl)-piperazin-1-yl-C₁₋₄-alkyloxy, 3-oxo-4-(C₁₋₄-alkyl)-piperazin-1-yl-C₁₋₄-alkyloxy,
C₁₋₄-alkylsulfanyl, C₁₋₄-alkysulfinyl, C₁₋₄-alkylsulfonyl, C₁₋₄-alkylsulfonyloxy, (het)arylsulfonyl, (het)arylsulfonyloxy, trifluoromethylsulfanyl, trifluoromethyl-sulfinyl, trifluoromethylsulfonyl, C₃₋₆-cycloalkylsulfanyl, C₃₋₆-cycloalkylsulfinyl, C₃-₆-cycloalkylsulfonyl, C₃₋₆-cycloalkyl-C₁₋₃-alkylsulfanyl, C₃₋₆-cycloalkyl-C₁₋₃-alkylsulfinyl, C₃₋₆-cycloalkyl-C₁₋₃-alkylsulfonyl,
aminosulfonyl, C₁₋₄-alkyl-aminosulfonyl, di-(C₁₋₄-alkyl)-aminosulfonyl, pyrrolidin-1-yl-sulfonyl, piperidin-1-yl-sulfonyl, morpholin-4-yl-sulfonyl, piperazin-1-yl-sulfonyl, or 4-(C₁₋₄-alkyl)-piperazin-1-yl-sulfonyl,
wherein the above-mentioned saturated heterocycles and cycloalkyl-rings are optionally substituted with one or two groups independently of each other selected from fluorine, C₁₋₃-alkyl, C₁₋₃-alkoxy, C₁₋₃-alkoxy-C₁₋₃-alkyl, or hydroxy, or a physiologically acceptable salt thereof,
including the compounds disclaimed in claim 1, for treatment or prevention of diseases or conditions selected from metabolic disorders, type 1 diabetes mellitus, type 2 diabetes mellitus, complications of diabetes (such as retinopathy, nephropathy or neuropathies, diabetic foot, ulcers, macroangiopathies, slow or poor wound healing), metabolic acidosis, metabolic ketosis, reactive hypoglycaemia, hyperinsulinaemia, glucose metabolic disorder, insulin resistance, metabolic syndrome, dyslipidaemias of different origins, atherosclerosis, obesity, high blood pressure, chronic heart failure, edema, hyperuricaemia, beta-cell degeneration (apoptosis or necrosis of pancreatic beta cells), improvement or restoring the functionality of pancreatic cells, increase of the number and size of pancreatic beta cells, acute renal failure, ocular hypertension, glaucoma, osteoporosis, amelioration or prevention of cognitive impairment, anxiety, depression, tuberculosis, leprosy, and psoriasis.

8. Use of at least one compound as defined in claim 7, for preparing a pharmaceutical composition which is suitable for the treatment or prevention or diseases or conditions selected from metabolic disorders, type 1 diabetes mellitus, type 2 diabetes mellitus, complications of diabetes (such as retinopathy, nephropathy or neuropathies, diabetic foot, ulcers, macroangiopathies, slow or poor wound healing), metabolic acidosis, metabolic ketosis, reactive hypoglycaemia, hyperinsulinaemia, glucose metabolic disorder, insulin resistance, metabolic syndrome, dyslipidaemias of different origins, atherosclerosis, obesity, high blood pressure, chronic heart failure, edema, hyperuricaemia, beta-cell degeneration (apoptosis or necrosis of pancreatic beta cells), improvement or restoring the functionality of pancreatic cells, increase of the number and size of pancreatic beta cells, acute renal failure, ocular hypertension, glaucoma, osteoporosis, amelioration or prevention of cognitive impairment, anxiety, depression, tuberculosis, leprosy, and psoriasis.

9. Process for preparing a compound of the general formula I according to any of claims 1 to 4 or a physiologically acceptable salt according to claim 5, **characterized in that**
one of the amines of general formula II 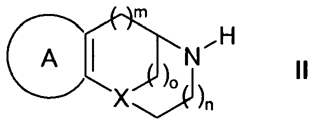 wherein the groups A, X, m, n and o are defined as in claim 1,
or of general formula III 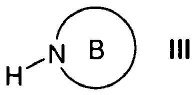 wherein B is definded as in claim 1,
is reacted with a carbonic acid derivative Y-CO-Y yielding a compound either of general formula IV or V as intermediate 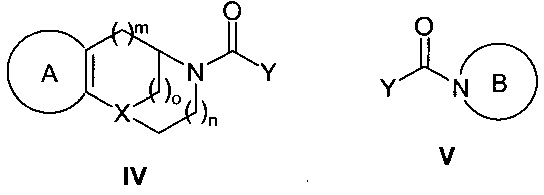 which is optionally isolated and optionally purified,
and the intermediate of the formula IV or V is reacted with the other amine of the formula III or II to yield the compound of the formula I,
wherein Y is a leaving group and in particular denotes fluorine, chlorine, bromine, cyano, C₁₋₄-alkoxy, C₂₋₄-alkenyloxy, C₂₋₄-alkynyloxy, partially or fully fluorinated C₂₋₁₀-alkoxy, oxyarylotriazol, oxyheteroarylotriazol, heteroar-N-yl, 3-methyl-imidazol-1-yl, succinyl-N-oxy, di-(C₁₋₄-alkyl)aminocarbonyloxy, pyrrolylcarbonyloxy, piperidinylcarbonyloxy, morpholinylcarbonyloxy, aryloxy, or heteroaryloxy,
while the alkyl, alkenyl, and alkynyl groups mentioned in the definition of the above groups, either alone or as part of another group, may be substituted with one or more substituents independently of each other selected from fluorine, chlorine, C₁₋₃-alkyl, and C₁₋₃-alkoxy,
while the aryl groups mentioned in the definition of the above groups, either alone or as part of another group, denote phenyl or naphthyl and the heteroaryl groups mentioned in the definition of the above groups, either alone or as part of another group, denote pyridinyl, pyrimidinyl, triazinyl, imidazolyl, pyrazolyl, triazolyl, tetrazolyl, whilst both the aryl and heteroaryl groups optionally are substituted with one or more substituents independently of each other selected from fluorine, chlorine, bromine, C₁₋₃-alkyl, C₁₋₃-alkyloxy, nitro, cyano, and di-(C₁₋₃-alkyl)amino groups,
while the two Y in Y-CO-Y may be identical or different,
while the second Y to be replaced may also be transformed to a more reactive Y after the first Y is replaced with one of the two amines,
optionally in the presence of an organic base or another additive;
and, if necessary any protective group used in the reactions described above is cleaved concurrently or subsequently;
if desired a compound of general formula I thus obtained is resolved into its stereoisomers;
if desired a compound of general formula I thus obtained is converted into the salts thereof, particularly for pharmaceutical use into the physiologically acceptable salts thereof.

## Patentansprüche

1. Verbindungen der Formel 1 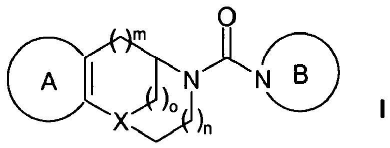 worin
X CH oder N bezeichnet,
m, n, o bezeichnen unabhängig voneinander 0, 1 oder 2,
wobei die C₅₊ₘ₊ₙ-Azacycloalken-Kernstruktur der allgemeinen Formel I, einschließlich der Brückengruppe -(CH₂)ₒ-, gegebenenfalls substituiert ist mit 1, 2 oder mehr Substituenten, unabhängig voneinander ausgewählt aus der Gruppe, bestehend aus R¹¹ und R¹²,
A bezeichnet einen Benzo-, Pyrido-, Pyrrolo-, Furo-, Thieno-, Pyridazino-, Pyrimido- oder Pyrazinoring, wobei jeder der Ringe mit ein oder mehr Substituenten substituiert ist, unabhängig voneinander ausgewählt aus R¹, und wobei zwei benachbarte C-Atome von jedem der Ringe gegebenenfalls substituiert sind mit R² und R³; oder
einen Pyrazolo-, Imidazo-, Oxazolo-, Thiazolo-, Isoxazolo- oder Isothiazoloring, wobei jeder der Ringe gegebenenfalls substituiert ist mit R¹; oder
einen 1,2,3-Triazoloring, gegebenenfalls substituiert mit R^{N}; und
B bezeichnet eine 3- bis 8-gliedrige monocyclische, 7- bis 12-gliedrige spirocyclische, 6- bis 12-gliedrige bicyclische oder 9- bis 15-gliedrige tricyclische Azacycloalk-1-yl-Gruppe, die gesättigt oder teilweise oder vollständig ungesättigt ist,
wobei 1 oder 2 -CH₂-Gruppen ersetzt sein können durch -NR^{N}-,
wobei 1 bis 4 -CH₂-Gruppen unabhängig voneinander ersetzt sein können durch O, S, Carbonyl oder Sulfonyl,
wobei 1 oder 2 -CH=-Gruppen ersetzt sein können durch -N=, und
wobei die Azacycloalkyl-Gruppe substituiert sein kann mit ein oder mehr Substituenten, unabhängig voneinander ausgewählt aus L¹,
wobei die Azacycloalkyl-Gruppe substituiert sein kann mit ein oder zwei Substituenten, unabhängig voneinander ausgewählt aus L²,
wobei zwei benachbarte C-Atome der Azacycloalkyl-Gruppe mit L³ und L⁴ substituiert sein können, und
wobei zwei benachbarte C-Atome der Azacycloalkyl-Gruppe mit L⁵ und L⁶ substituiert sein können;
R^{N} bezeichnet unabhängig voneinander Wasserstoff, C₁₋₆-Alkyl, C₃₋₆-Alkenyl, C₃₋₆-Alkinyl, (Het)Aryl, C₁₋₄-Alkylcarbonyl, C₁₋₄-Alkylsulfonyl, (Het)Arylcarbonyl, (Het)Arylsulfonyl,
wobei jede Alkyl-, Alkenyl- und Alkinylgruppe mono- oder polysubstituiert sein kann mit Fluor, und monosubstituiert sein kann mit Hydroxy, C₁₋₄-Alkoxy, C₁₋₄-Alkylsulfanyl, C₁₋₄-Alkylsulfinyl, C₁₋₄-Alkyl-sulfonyl, Amino, C₁₋₄-Alkylamino, Di-(C₁₋₄-Alkyl)amino, C₁₋₄-Alkylcarbonylamino, Cyano, Carboxy, C₁₋₄-Alkoxycarbonyl, Amino-carbonyl, C₁₋₄-Alkylaminocarbonyl, Di(C₁₋₄-Alkyl)-aminocarbonyl oder (Het)Aryl,
R¹ bezeichnet unabhängig voneinander Fluor, Chlor, Brom, Iod, Cyano, Nitro, C₁₋₄-Alkyl, Difluormethyl, Trifluormethyl, Difluormethoxy, Trifluormethoxy, Trifluormethyl-hydroxy-C₁₋₂-alkyl, 2,2,2-Trifluor-1-hydroxy-1-(trifluormethyl)-ethyl, C₃₋₆-Cycloalkyl, C₃₋₆-Cycloalkyloxy, C₃₋₆-Cycloalkyl-C₁₋₃-Alkyl, C₃₋₆-Cycloalkyl-C₁₋₃-alkyloxy, Tetrahydrofuran-3-yloxy, Tetrahydropyran-3-yloxy, Tetrahydropyran-4-yloxy, Tetrahydrofuranyl-C₁₋₃-alkyloxy, Tetrahydropyranyl- C₁₋₃-alkyloxy, (Het)Aryl, (Het)Aryloxy, (Het)Aryl-C₁₋₃-alkyl, (Het)Aryl-C₁₋₃-alkyloxy, (Het)Aryloxy-C₁₋₃-alkyl, C₁₋₃-Alkylcarbonyl, (Het)Arylcarbonyl, Amino, C₁₋₄-Alkylamino, Di-(C₁₋₄-alkyl)amino, Pyrrolidin-1-yl, 2-Oxo-pyrrolidin-1-yl, Piperidin-1-yl, 2-Oxo-piperidin-1-yl, Morpholin-4-yl, 3-Oxo-morpholin-4-yl, Piperazin-1-yl, 2-Oxopiperazin-1-yl, 3-Oxopiperazin-1-yl, 4-(C₁₋₄-Alkyl)piperazin-1-yl, 4-(C₁₋₄-Alkylcarbonyl)piperazin-1-yl, 4-(C₃₋₆-Cyclo-alkylcarbonyl)piperazin-1-yl, 4-(C₁₋₄-Alkoxycarbonyl)piperazin-1-yl, 4-(C₁₋₄-Alkylsulfonyl)piperazin-1-yl, 2-Oxo-4-(C₁₋₄-alkyl)piperazin-1-yl, 3-Oxo-4-(C₁₋₄-alkyl)piperazin-1-yl,
C₁₋₄-Alkylcarbonylamino, (Het)Arylcarbonylamino, (Het)Aryl-C₁₋₄-Alkylcarbonylamino, C₁₋₄-Alkyloxycarbonylamino, Aminocarbonylamino, C₁₋₄-Alkylaminocarbonylamino, Di-(C₁₋₄-Alkyl)aminocarbonylamino, Pyrrolidin-1-yl-carbonylamino, Piperidin-1-yl-carbonylamino, Morpholin-4-yl-carbonyl-amino, Piperazin-1-yl-carbonylamino, 4-(C₁₋₄-Alkyl)piperazin-1-yl-carbonyl-amino, C₁₋₄-Alkylsulfonylamino, Aminosulfonylamino, C₁₋₄-Alkyl-aminosulfonylamino, Di-(C₁₋₄-Alkyl)aminosulfonylamino, Pyrrolidin-1-yl-sulfonylamino, Piperidin-1-yl-sulfonylamino, Morpholin-4-yl-sulfonylamino, Piperazin-1-yl-sulfonylamino, 4-(C₁₋₄-Alkyl)piperazin-1-yl-sulfonylamino, (C₁₋₄-Alkoxycarbonylamino)carbonylamino, (Het)Arylsulfonylamino, (Het)Aryl-C₁₋₄-Alkylsulfonylamino,
N-(C₁₋₄-Alkyl)-C₁₋₄-alkylcarbonylamino, N-(C₁₋₄-Alkyl)-(het)arylcarbonylamino, N-(C₁₋₄-Alkyl)-(het)aryl-C₁₋₄-alkylcarbonylamino, N-(C₁₋₄-Alkyl)-C₁₋₄-alkyl-oxycarbonylamino, N-(Aminocarbonyl)-C₁₋₄-alkylamino, N-(C₁₋₄-Alkylaminocarbonyl)-C₁₋₄-alkylamino, N-[Di-(C₁₋₄-Alkyl)aminocarbonyl]-C₁₋₄-alkylamino,
N-(C₁₋₄-Alkyl)-C₁₋₄-alkylsulfonylamino, N-(C₁₋₄-Alkyl)-(het)arylsulfonylamino, N-(C₁₋₄-Alkyl)-(het)aryl-C₁₋₄-alkylsulfonylamino,
Oxo-imidazolidin-1-yl, 2,4-Dioxo-imidazolidin-1-yl, 2,5-Dioxo-imidazolidin-1-yl, 2-Oxo-hexahydropyrimidin-1-yl, wobei das Stickstoffatom an Position 3 der vorgenannten Gruppen gegebenenfalls mit Methyl oder Ethyl substituiert ist,
(Hydroxyimino)aminomethyl, (C₁₋₃-Alkyloxyimino)aminomethyl, Carboxy, C₁₋₄-Alkyloxycarbonyl, Aminocarbonyl, C₁₋₄-Alkylaminocarbonyl, Di-(C₁₋₄-alkyl)-aminocarbonyl, Pyrrolidin-1-yl-carbonyl, Piperidin-1-yl-carbonyl, Morpholin-4-yl-carbonyl, Piperazin-1-yl-carbonyl, 4-(C₁₋₄-Alkyl)piperazin-1-yl-carbonyl,
Carboxy-C₁₋₄-alkyl, C₁₋₄-Alkyloxycarbonyl-C₁₋₄-alkyl, Cyano-C₁₋₄-alkyl, Amino-carbonyl-C₁₋₄-alkyl, C₁₋₄-Alkyl-aminocarbonyl-C₁₋₄-alkyl, Di-(C₁₋₄-alkyl)-aminocarbonyl-C₁₋₄-alkyl, Pyrrolidin-1-yl-carbonyl-C₁₋₄-alkyl, Piperidin-1-yl-carbonyl-C₁₋₄-alkyl, Morpholin-4-yl-carbonyl-C₁₋₄-alkyl, Piperazin-1-yl-carbonyl-C₁₋₄-alkyl, 4-(C₁₋₄-Alkyl)-piperazin-1-yl-carbonyl-C₁₋₄-alkyl,
Carboxy-C₁₋₄-alkyloxy, C₁₋₄-Alkyloxycarbonyl-C₁₋₄-alkyloxy, Cyano-C₁₋₄-alkyl-oxy, Aminocarbonyl-C₁₋₄-alkyloxy, C₁₋₄-Alkyl-aminocarbonyl-C₁₋₄-alkyloxy, Di-(C₁₋₄-alkyl)-aminocarbonyl-C₁₋₄-alkyloxy, Pyrrolidin-1-yl-carbonyl-C₁₋₄-alkyl-oxy, Piperidin-1-yl-carbonyl-C₁₋₄-alkyloxy, Morpholin-4-yl-carbonyl-C₁₋₄-alkyl-oxy, Piperazin-1-yl-carbonyl-C₁₋₄-alkyloxy, 4-(C₁₋₄-Alkyl)-piperazin-1-yl-carbonyl-C₁₋₄-alkyloxy,
Hydroxy-C₁₋₄-alkyl, C₁₋₄-Alkyloxy-C₁₋₄-alkyl, Amino-C₁₋₄-alkyl, C₁₋₄-Alkylamino-C₁₋₄-alkyl, Di(C₁₋₄-alkyl)-amino-C₁₋₄-alkyl, Pyrrolidin-1-yl-C₁₋₄-alkyl, C₁₋₄-Alkylcarbonylamino-C₁₋₄-alkyl, N-(C₁₋₄-Alkyl)-C₁₋₄-alkylcarbonylamino-C₁₋₄-alkyl, 2-Oxo-pyrrolidin-1-yl-C₁₋₄-alkyl, Piperidin- 1-yl-C₁₋₄-alkyl, 2-Oxo-piperidin-1-yl-C₁₋₄-alkyl, Morpholin-4-yl-C₁₋₄-alkyl, 3-Oxo-morpholin-4-yl-C₁₋₄-alkyl, Piperazin-1-yl-C₁₋₄-alkyl, 2-Oxo-piperazin-1-yl-C₁₋₄-alkyl, 3-Oxo-piperazin-1-yl-C₁₋₄-alkyl, 4-(C₁₋₄-Alkyl)-piperazin-1-yl-C₁₋₄-alkyl, 2-Oxo-4-(C₁₋₄-alkyl)-piperazin-1-yl-C₁₋₄-alkyl, 3-Oxo-4-(C₁₋₄-alkyl)-piperazin-1-yl-C₁₋₄-alkyl,
Hydroxy-C₁₋₄-alkyloxy, C₁₋₄-Alkyloxy-C₁₋₄-alkyloxy, C₁₋₄-Alkylsulfanyl-C₁₋₄-alkyloxy, C₁₋₄-Alkylsulfinyl-C₁₋₄-alkyloxy, C₁₋₄-Alkylsulfonyl-C₁₋₄-alkyloxy, Amino-C₁₋₄-alkyloxy, C₁₋₄-Alkylamino-C₁₋₄-alkyloxy, Di-(C₁₋₄-alkyl)-amino-C_{1- 4}-alkyloxy, Pyrrolidin-1-yl-C₁₋₄-alkyloxy, 2-Oxo-pyrrolidin-1-yl-C₁₋₄-alkyloxy, Piperidin-1-yl-C₁₋₄-alkyloxy, 2-Oxo-piperidin-1-yl-C₁₋₄-alkyloxy, Morpholin-4-yl-C₁₋₄-alkyloxy, 3-Oxo-morpholin-4-yl-C₁₋₄-alkyloxy, Piperazin-1-yl-C₁₋₄-alkyl-oxy, 2-Oxo-piperazin-1-yl-C₁₋₄-alkyloxy, 3-Oxo-piperazin-1-yl-C₁₋₄-alkyloxy, 4-(C₁₋₄-Alkyl)-piperazin-1-yl-C₁₋₄-alkyloxy, 2-Oxo-4-(C₁₋₄-alkyl)-piperazin-1-yl-C₁₋₄-alkyloxy, 3-Oxo-4-(C₁₋₄-alkyl)-piperazin-1-yl-C₁₋₄-alkyloxy,
C₁₋₄-Alkylsulfanyl, C₁₋₄-Alkylsulfinyl, C₁₋₄-Alkylsulfonyl, C₁₋₄-Alkylsulfonyloxy, (Het)Arylsulfonyl, (Het)Arylsulfonyloxy, Trifluormethylsulfanyl, Trifluormethylsulfinyl, Trifluormethylsulfonyl, C₃₋₆-Cycloalkylsulfanyl, C₃₋₆-Cycloalkylsulfinyl, C₃₋₆-Cycloalkylsulfonyl, C₃₋₆-Cycloalkyl-C₁₋₃-alkylsulfanyl, C₃₋₆-Cycloalkyl-C₁₋₃-alkylsulfinyl, C₃₋₆-Cycloalkyl-C₁₋₃-alkylsulfonyl,
Aminosulfonyl, C₁₋₄-Alkyl-aminosulfonyl, Di-(C₁₋₄-alkyl)-aminosulfonyl, Pyrrolidin-1-yl-sulfonyl, Piperidin-1-yl-sulfonyl, Morpholin-4-yl-sulfonyl, Piperazin-1-yl-sulfonyl oder 4-(C₁₋₄-Alkyl)-piperazin-1-yl-sulfonyl,
wobei die oben genannten gesättigten Heterocyclen und Cycloalkylringe gegebenenfalls substituiert sind mit ein oder zwei Gruppen, unabhängig voneinander ausgewählt aus Fluor, C₁₋₃-Alkyl, C₁₋₃-Alkoxy, C₁₋₃-Alkoxy-C₁₋₃-alkyl oder Hydroxy,
R², R³ sind miteinander verknüpft, um eine Methylendioxy-, Ethylendioxy- oder C₃₋₅-Alkylenbrükengruppe zu bilden, die mono- oder disubstituiert sein kann mit Methyl und die mono- oder polyfluoriert sein kann; oder
R², R³ bilden zusammen mit den Kohlenstoffatomen, mit denen sie verbunden sind, einen Benzo-, Pyrido-, Pyrimido-, Pyrazino-, Pyridazino-, Pyrrolo-, Furano-, Thieno-, Pyrazolo-, Imidazo-, Triazolo-, Oxazolo-, Thiazolo-, Isoxazolo- oder Isothiazolo-Ring, wobei jeder der Ringe mit ein oder mehr Substituenten substituiert sein kann, unabhängig voneinander ausgewählt aus R^{P};
R^{P} bezeichnet Halogen, C₁₋₆-Alkyl, Hydroxy-C₁₋₄-alkyl, C₁₋₃-Alkyloxy-C₁₋₃-alkyl, C₃₋₆-Cycloalkyl, Hydroxy-C₄₋₆-cycloalkyl, C₁₋₃-Alkyloxy-C₃₋₆-cycloalkyl, Azetidinyl, 1-(C₁₋₃-Alkyl)-azetidinyl, 1-(C₁₋₃-Alkylcarbonyl)-azetidinyl, Pyrrolidinyl, 1-(C₁₋₃-Alkyl)-pyrrolidinyl, 1-(C₁₋₃-Alkylcarbonyl)-pyrrolidinyl, Piperidinyl, 1-(C₁₋₃-Alkyl)-piperidinyl, 1-(C₁₋₃-Alkylcarbonyl)-piperidinyl, Tetrahydrofuranyl, Tetrahydropyranyl, Difluormethyl, Trifluormethyl, Cyano, Nitro, Amino, C₁₋₃-Alkylamino, Di-(C₁₋₃-Alkyl)-amino, C₁₋₃-Alkylcarbonylamino, Methylsulfonylamino, Carboxy, C₁₋₄-Alkyloxycarbonyl, Aminocarbonyl, C₁₋₃-Alkylaminocarbonyl, Di-(C₁₋₃-Alkyl)-aminocarbonyl, Aminosulfonyl, Methylsulfanyl, Methylsulfinyl, Methylsulfonyl, Hydroxy, C₁₋₃-Alkyloxy, Difluormethoxy, Trifluormethoxy, Phenyl, oder
Pyrrolyl, Furanyl, Thienyl, Pyridyl, wobei in irgendeiner dieser Gruppen 1 oder 2 CH gegebenenfalls ersetzt sind durch N-Atome, oder
1,2-Dihydro-2-oxo-pyridinyl, 1,4-Dihydro-4-oxo-pyridinyl, 2,3-Dihydro-3-oxo-pyridazinyl, 1,2,3,6-Tetrahydro-3,6-dioxo-pyridazinyl, 1,2-Dihydro-2-oxo-pyrimidinyl, 3,4-Dihydro-4-oxo-pyrimidinyl, 1,2,3,4-Tetrahydro-2,4-dioxopyrimidinyl, 1,2-Dihydro-2-oxo-pyrazinyl,
wobei jede der oben genannten aromatischen oder heteroaromatischen Gruppen gegebenenfalls substituiert ist mit ein oder zwei Gruppen, unabhängig ausgewählt aus Fluor, Chlor, C₁₋₃-Alkyl, Difluormethyl, Trifluormethyl, Cyano, Amino, Acetylamino, Methylsulfonylamino, Carboxy, C₁₋₄-Alkyloxycarbonyl, Aminocarbonyl, C₁₋₃-Alkylaminocarbonyl, Di-(C₁₋₃-alkyl)-aminocarbonyl, Hydroxy, C₁₋₃-Alkyloxy, Difluormethoxy und Trifluormethoxy,
R¹⁰ bezeichnet jeweils voneinander unabhängig Halogen, C₁₋₃-Alkyl, Difluormethyl, Trifluormethyl, Cyano, Nitro, Amino, C₁₋₃-Alkylamino, Di-(C₁₋₃-alkyl)amino, Acetylamino, Methylsulfonylamino, Carboxy, C₁₋₄-Alkyloxycarbonyl, Amino-carbonyl, C₁₋₃-Alkylaminocarbonyl, Di-(C₁₋₃-alkyl)-aminocarbonyl, Aminosulfonyl, Methylsulfanyl, Methylsulfinyl, Methylsulfonyl, Hydroxy, C₁₋₃-Alkyloxy, Difluormethoxy, Trifluormethoxy oder Phenyl, gegebenenfalls substituiert mit 1 oder 2 Substituenten, jeweils unabhängig voneinander ausgewählt aus Fluor, Methyl, Methoxy, Cyano oder Hydroxy,
R¹¹ bezeichnet unabhängig voneinander Fluor, C₁₋₄-Alkyl, (Het)Aryl, Hydroxy, C₁₋₄-Alkyloxy, Cyano, Carboxy, C₁₋₄-Alkyloxycarbonyl, Aminocarbonyl, C₁₋₄-Alkylaminocarbonyl, Di-(C₁₋₄-alkyl)-aminocarbonyl, Hydroxy-C₁₋₄-alkyl oder C₁₋₃-Alkyloxy-C₁₋₄-Alkyl,
R¹² bezeichnet unabhängig voneinander Fluor oder C₁₋₄-Alkyl, und
L¹ bezeichnet unabhängig voneinander Halogen, C₁₋₄-Alkyl, Trifluormethyl, Hydroxy, C₁₋₄-Alkyloxy oder Cyano,
L² bezeichnet unabhängig voneinander Fluor, Chlor, Brom, Iod, Nitro, Cyano, Hydroxy, C₃₋₆-Cycloalkyl, C₃₋₆-Cycloalkyloxy, Tetrahydrofuran-3-yl-oxy, Tetrahydropyran-3-yl-oxy, Tetrahydropyran-4-yl-oxy, Tetrahydrofuranyl-C₁₋₃-alkyloxy, Tetrahydropyranyl-C₁₋₃-Alkyloxy, (Het)Aryl, (Het)Aryloxy, oder
C₁₋₆-Alkyl, C₂₋₆-Alkenyl, C₂₋₆-Alkinyl, C₁₋₄-Alkyloxy, wobei in jeder dieser Gruppen eine CH₂-Gruppe ersetzt sein kann durch Carbonyl oder Sulfonyl, und wobei jede Gruppe mono- oder polyfluoriert sein kann, und worin jede Gruppe zusätzlich substituiert sein kann mit
Hydroxy, Chlor, C₁₋₃-Alkyloxy, Amino, C₁₋₃-Alkylamino, Di-(C₁₋₃-alkyl)amino, Pyrrolidin-1-yl, 2-Oxo-pyrrolidin-1-yl, Piperidin-1-yl, 2-Oxo-piperidin-1-yl, Morpholin-4-yl, 3-Oxo-morpholin-4-yl, Piperazin-1-yl, 2-Oxo-piperazin-1-yl, 3-Oxo-piperazin-1-yl, 4-(C₁₋₃-Alkyl)-piperazin-1-yl, 2-Oxo-4-(C₁₋₃-alkyl)-piperazin-1-yl, 3-Oxo-4-(C₁₋₃-alkyl)-piperazin-1-yl, Carboxy, C₁₋₃-Alkyloxycarbonyl, Cyano, Aminocarbonyl, C₁₋₃-Alkylaminocarbonyl, Di-(C₁₋₃-alkyl)-aminocarbonyl, Pyrrolidin-1-yl-carbonyl, Piperidin-1-yl-carbonyl, Morpholin-4-yl-carbonyl, Piperazin-1- yl-carbonyl, 4-(C₁₋₃-Alkyl)-piperazin-1-yl-carbonyl, C₁₋₃-Alkylcarbonyl-amino, Arylcarbonylamino, C₁₋₃-Alkylsulfanyl, C₁₋₃-Alkylsulfinyl, C₁₋₃-Alkylsulfonyl, C₃₋₆-Cycloalkyl, (Het)Aryl oder (Het)Aryloxy;
Amino, C₁₋₃-Alkylamino, Di-(C₁₋₃-alkyl)amino, Pyrrolidin-1-yl, 2-Oxo-pyrrolidin-1-yl, Piperidin-1-yl, 2-Oxo-piperidin-1-yl, Morpholin-4-yl, 3-Oxo-morpholin-4-yl, Piperazin-1-yl, 2-Oxo-piperazin-1-yl, 3-Oxo-piperazin-1-yl, 4-(C₁₋₃-Alkyl)-piperazin-1-yl, 4-(C₁₋₄-Alkylcarbonyl)-piperazin-1-yl, 4-(C₃₋₆-Cycloalkylcarbonyl)-piperazin-1-yl, 4-(C₁₋₄-Alkyloxycarbonyl)-piperazin-1-yl, 4-(C₁₋₄-Alkylsulfonyl)-piperazin-1-yl, 2-Oxo-4-(C₁₋₃-alkyl)-piperazin-1-yl, 3-Oxo-4-(C₁₋₃-alkyl)-piperazin-1-yl,
C₁₋₄-Alkyl-carbonylamino, (Het)Aryl-carbonylamino, (Het)Aryl-C₁₋₃-alkyl-carbonylamino, C₁₋₃-Alkyloxy-carbonylamino, Aminocarbonylamino, C₁₋₃-Alkylamino-carbonylamino, Di-(C₁₋₃-alkyl)-aminocarbonylamino, Pyrrolidin-1-yl-carbonylamino, Piperidin-1-yl-carbonylamino, Morpholin-4-yl-carbonylamino, Piperazin-1-yl-carbonylamino, 4-(C₁₋₃-Alkyl)-piperazin-1-yl-carbonylamino, C₁₋₃-Alkylsulfonylamino, Aminosulfonylamino, C₁₋₃-Alkyl-amino-sulfonylamino, Di-(C₁₋₃-alkyl)-amino-sulfonylamino, Pyrrolidin-1-yl-sulfonylamino, Piperidin-1-yl-sulfonylamino, Morpholin-4-yl-sulfonylamino, Piperazin-1-yl-sulfonyl-amino, 4-(C₁₋₃-Alkyl)-piperazinin-1-yl-sulfonylamino, (C₁₋₃-Alkyloxy-carbonyl-amino)-carbonylamino, (Het)Arylsulfonylamino, (Het)Aryl-C₁₋₃-alkylsulfonyl-amino,
N-(C₁₋₃-Alkyl)-C₁₋₃-alkyl-carbonylamino, N-(C₁₋₃-Alkyl)-(het)aryl-carbonylamino, N-(C₁₋₃-Alkyl)-(het)aryl-C₁₋₃-alkyl-carbonylamino, N-(C₁₋₃-Alkyl)-C₁₋₃-alkyloxy-carbonylamino, N-(Aminocarbonyl)-C₁₋₃-alkylamino, N-(C₁₋₃-Alkyl-aminocarbonyl)-C₁₋₃-alkylamino, N-[Di-(C₁₋₃-alkyl)-aminocarbonyl]-C₁₋₃-alkyl-amino,
N-(C₁₋₃-Alkyl)-C₁₋₃-alkyl-sulfonylamino, N-(C₁₋₃-Alkyl)-(het)aryl-sulfonylamino, N-(C₁₋₃-Alkyl)-(het)aryl-C₁₋₃-alkyl-sulfonylamino, Carboxy, C₁₋₃-Alkyloxy-carbonyl, Aminocarbonyl, C₁₋₃-Alkyl-aminocarbonyl, Di-(C₁₋₃-alkyl)-aminocarbonyl, Pyrrolidin-1-yl-carbonyl, Piperidin-1-yl-carbonyl, Morpholin-4-yl-carbonyl, Piperazin-1-yl-carbonyl, 4-(C₁₋₃-Alkyl)-piperazin-1-yl-carbonyl, (Het)Aryl-aminocarbonyl, N-(C₁₋₃-Alkyl)-(het)arylaminocarbonyl, (Het)Aryl-C₁₋₃-alkyl-aminocarbonyl, N-(C₁₋₃-Alkyl)-(het)aryl-C₁₋₃-alkyl-aminocarbonyl,
C₁₋₃-Alkylsulfanyl, C₁₋₃-Alkylsulfinyl, (Het)Arylsulfonyl, Trifluormethylsulfanyl, Trifluormethylsulfinyl,
Aminosulfonyl, C₁₋₃-Alkylaminosulfonyl, Di-(C₁₋₃-Alkyl)aminosulfonyl, Pyrrolidin-1-yl-sulfonyl, Piperidin-1-yl-sulfonyl, Morpholin-4-yl-sulfonyl, Piperazin-1-yl-sulfonyl, 4-(C₁₋₃-Alkyl)-piperazin-1-yl-sulfonyl,
wobei die oben genannten gesättigten heterocyclischen und Cycloalkylringe gegebenenfalls substituiert sind mit ein oder zwei Gruppen, ausgewählt aus Fluor, C₁₋₃-Alkyl, C₁₋₃-Alkoxy, C₁₋₃-Alkoxy-C₁₋₃-Alkyl oder Hydroxy, und
L³ und L⁴ sind miteinander verknüpft, und
L⁵ und L⁶ sind miteinander verknüpft, so dass unabhängig voneinander und in jedem Fall zusammen mit den 2 benachbarten C-Atomen, mit denen sie verknüpft sind, eine Aryl- oder Heteroarylgruppe gebildet wird, die mit der cyclischen Gruppe B kondensiert ist und die Aryl- oder Heteroarylgruppe ist gegebenenfalls substituiert mit 1, 2 oder 3 identischen oder unterschiedlichen R¹⁰,
wobei mit Aryl Phenyl oder Naphthyl gemeint ist, und mit Heteroaryl Pyrrolyl, Furanyl, Thienyl, Pyridyl, Indolyl, Benzofuranyl, Benzothiophenyl, Chinolinyl, Isochinolinyl oder Pyrrolyl, Furanyl, Thienyl, Pyridyl gemeint ist, wobei in dem Pyrrolyl, Furanyl, Thienyl, Pyridyl 1 oder 2 CH-Gruppen jeweils ersetzt sind durch N, oder Indolyl, Benzofuranyl, Benzothiophenyl, Chinolinyl, Isochinolinyl, wobei in dem Indolyl, Benzofuranyl, Benzothiophenyl, Chinolinyl, Isochinolinyl 1 bis 3 CH-Gruppen jeweils ersetzt sind durch N, oder Tetrazolyl,
wobei das oben genannte (Het)Aryl eine Arylgruppe darstellt wie hier zuvor definiert, oder eine Heteroarylgruppe wie hier zuvor definiert, oder einen Ring, ausgewählt aus der Gruppe, bestehend aus 1,2-Dihydro-2-oxo-pyridinyl, 1,4-Dihydro-4-oxo-pyridinyl, 2,3-Dihydro-3-oxo-pyridazinyl, 1,2,3,6-Tetrahydro-3,6-dioxo-pyridazinyl, 1,2-Dihydro-2-oxo-pyrimidinyl, 3,4-Dihydro-4-oxo-pyrimidinyl, 1,2,3,4-Tetrahydro-2,4-dioxo-pyrimidinyl, 1,2-Dihydro-2-oxo-pyrazinyl, 1,2,3,4-Tetrahydro-2,3-dioxo-pyrazinyl, 2,3-Dihydro-2-oxo-indolyl, 2,3-Dihydro-benzofuranyl, 2,3-Dihydro-2-oxo-1H-benzimidazolyl, 2,3-Dihydro-2-oxo-benzoxazolyl, 1,2-Dihydro-2-oxo-chinolinyl, 1,4-Dihydro-4-oxo-chinolinyl, 1,2-Dihydro-1-oxo-isochinolinyl, 1,4-Dihydro-4-oxo-cinnolinyl, 1,2-Dihydro-2-oxo-chinazolinyl, 1,4-Dihydro-4-oxo-chinazolinyl, 1,2,3,4-Tetrahydro-2,4-dioxo-chinazolinyl, 1,2-Dihydro-2-oxo-chinoxalinyl, 1,2,3,4-Tetrahydro-3-oxo-chinoxalinyl, 1,2,3,4-Tetrahydro-2,3-dioxo-chinoxalinyl, 1,2-Dihydro-1-oxo-phthalazinyl, 1,2,3,4-Tetrahydro-1,4-dioxo-phthalazinyl, Chromanyl, Cumarinyl, 2,3-Dihydro-benzo[1,4]dioxinyl und 3,4-Dihydro-3-oxo-2H-benzo[1,4]oxazinyl, wobei jeder der Ringe gegebenenfalls substituiert ist mit 1, 2 oder 3 Substituenten, jeweils unabhängig voneinander ausgewählt aus R¹⁰,
wobei jeder der oben genannten Alkyl- oder Alkylen-Reste verzweigt oder unverzweigt sein kann,
die Tautomeren, die Stereoisomeren hiervon, die Mischungen hiervon und die Salze hiervon,
wobei die folgenden Verbindungen (M1) bis (M4) ausgenommen sind:

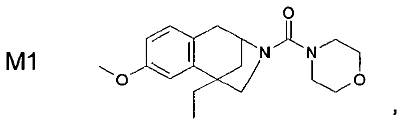

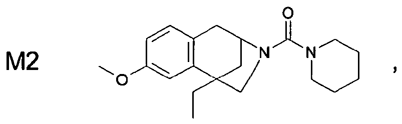

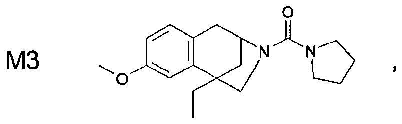

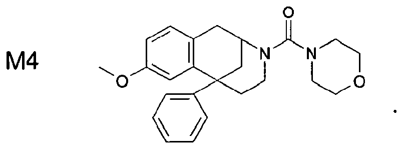

2. Verbindungen nach Anspruch 1, **gekennzeichnet durch** eine allgemeine Formel, ausgewählt aus ein oder mehr der Formeln I.1 bis I.10 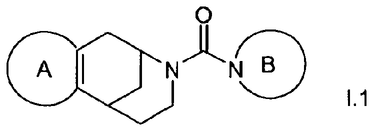 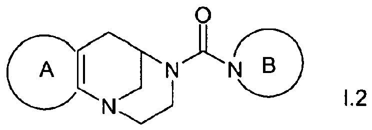 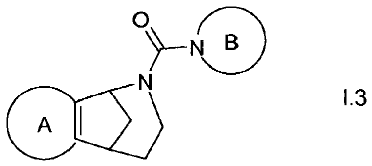 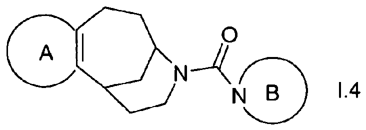 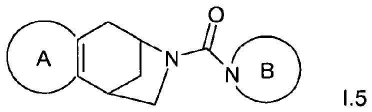 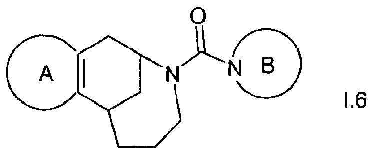 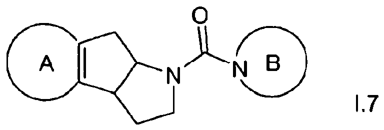 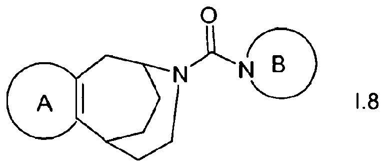 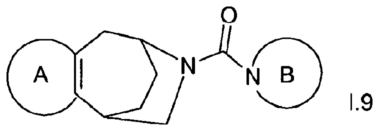 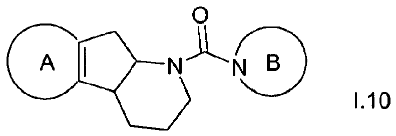 wobei die C₅₊ₘ₊ₙ-Azacycloalken-Kernstruktur der allgemeinen Formeln I.1 bis I.10 einschließlich der Brückengruppe -(CH₂)ₒ- gegebenenfalls substituiert ist mit 1, 2 oder mehr Substituenten, unabhängig voneinander ausgewählt aus der Gruppe, bestehend aus R¹¹ und R¹², und
wobei die Ringe A und B und m, n, o, R¹¹, R¹² wie in Anspruch 1 definiert sind, ihre Tautomeren, ihre Stereoisomeren, Mischungen hiervon und die Salze hiervon, wobei die Verbindungen (M1), (M2), (M3) und (M4), wie in Anspruch 1 definiert, ausgenommen sind.

3. Verbindungen nach Anspruch 1 oder 2, worin der Stickstoff enthaltende Ring B bezeichnet: Azetidin-1-yl, Pyrrolidin-1-yl, Piperidin-1-yl, Azepan-1-yl, Azocan-1-yl,
Azetidin-1-yl, worin eine -CH₂-Gruppe ersetzt ist durch O, S, NR^{N}, Carbonyl oder Sulfonyl, oder
Pyrrolidin-1-yl, Piperidin-1-yl, Azepan-1-yl, Azocan-1-yl, die ganz oder teilweise ungesättigt sein können und worin ein oder zwei -CH₂-Gruppen unabhängig voneinander ersetzt sind durch O, S, Carbonyl oder Sulfonyl, und worin eine -CH₂-Gruppe ersetzt sein kann durch -NR^{N}-,
Aza-bicyclohept-N-yl, Aza-bicyclooct-N-yl, Aza-bicyclonon-N-yl, Aza-bicyclodec-N-yl, Bicycloundec-N-yl, Bicyclododec-N-yl, von denen jedes teilweise ungesättigt sein kann und in jedem davon können ein oder zwei -CH₂-Gruppe unabhängig voneinander ersetzt sein durch O, S, -NR^{N}-, Carbonyl oder Sulfonyl, und in jedem davon kann eine -CH=-Gruppe ersetzt sein durch -N=,
Aza-tricyclonon-N-yl, Aza-tricyclodec-N-yl, Aza-tricycloundec-N-yl, Aza-tricyclododec-N-yl, Aza-tricyclotridec-N-yl, Aza-tricyclotetradec-N-yl, wobei in jedem hiervon ein oder zwei -CH₂-Gruppe unabhängig voneinander ersetzt sein können durch O, S, NR^{N}, Carbonyl oder Sulfonyl, und in jedem davon kann eine -CH=-Gruppe ersetzt sein durch -N=,
wobei jeder der obigen Ringe B mit ein oder mehr Substituenten substituiert sein kann, unabhängig voneinander ausgewählt aus L¹,
wobei jeder der obigen Ringe B mit ein oder zwei Substituenten substituiert sein kann, unabhängig voneinander ausgewählt aus L²,
wobei zwei benachbarte C-Atome in jedem der obigen Ringe B substituiert sein können mit L³ und L⁴, und
wobei zwei benachbarte C-Atome in jedem der obigen Ringe B substituiert sein können mit L⁵ und L⁶
wobei R^{N}, L¹, L², L³, L⁴, L⁵, L⁶ wie in Anspruch 1 definiert sind.

4. Verbindungen nach Anspruch 1, 2 oder 3, wobei der Ring A einen Benzo-, Pyrido-, Pyrrolo-, Furo-, Thieno-, Pyridazino-, Pyrimido- oder Pyrazino-Ring bezeichnet, wobei jeder der Ringe mit ein oder mehr Substituenten substituiert ist, unabhängig voneinander ausgewählt aus R¹, und wobei zwei benachbarte C-Atome in jedem der Ringe gegebenenfalls substituiert sind mit R² und R³, oder
einen Pyrazolo-, Oxazolo-, Thiazolo- oder Imidazo-Ring, wobei jeder hiervon gegebenenfalls substituiert ist mit R¹; wobei R¹, R² und R³ wie in Anspruch 1 definiert sind.

5. Physiologisch akzeptable Salze der Verbindungen nach mindestens einem der Ansprüche 1 bis 4 mit organischen oder anorganischen Säuren oder Basen.

6. Pharmazeutische Zusammensetzungen, enthaltend eine Verbindung nach mindestens einem der Ansprüche 1 bis 4 oder ein physiologisch akzeptables Salz nach Anspruch 5, gegebenenfalls zusammen mit ein oder mehr inerten Trägern und/oder Verdünnungsmitteln.

7. Verbindungen der Formel I, worin
A einen Benzo-, Pyrido-, Pyrrolo-, Furo-, Thieno-, Pyridazino-, Pyrimido- oder Pyrazinoring bezeichnet, wobei jeder der Ringe gegebenenfalls mit ein oder mehr Substituenten substituiert ist, unabhängig voneinander ausgewählt aus R¹, und wobei zwei benachbarte C-Atome von jedem der Ringe gegebenenfalls substituiert sind mit R² und R³; oder
einen Pyrazolo-, Imidazo-, Oxazolo-, Thiazolo-, Isoxazolo- oder Isothiazoloring, wobei jeder der Ringe gegebenenfalls substituiert ist mit R¹; oder einen 1,2,3-Triazoloring, gegebenenfalls substituiert mit R^{N}; und
R¹ bezeichnet unabhängig voneinander Fluor, Chlor, Brom, Iod, Cyano, Nitro, C₁₋₄-Alkyl, Hydroxy, C₁₋₄-Alkyloxy, Difluormethyl, Trifluormethyl, Difluormethoxy, Trifluormethoxy, Trifluormethyl-hydroxy-C₁₋₂-alkyl, 2,2,2-Trifluor-1-hydroxy-1-(trifluormethyl)ethyl, C₃₋₆-Cycloalkyl, C₃₋₆-Cycloalkyloxy, C₃₋₆-Cyloalkyl-C₁₋₃-alkyl, C₃₋₆-Cycloalkyl-C₁₋₃-alkyloxy, Tetrahydrofuran-3-yloxy, Tetrahydropyran-3-yloxy, Tetrahydropyran-4-yloxy, Tetrahydrofuranyl-C₁₋₃-alkyloxy, Tetrahydropyranyl-C₁₋₃-alkyloxy, (Het)Aryl, (Het)Aryloxy, (Het)Aryl-C₁₋₃-alkyl, (Het)Aryl-C₁₋₃-alkyloxy, (Het)Aryloxy-C₁₋₃-alkyl, C₁₋₃-Alkylcarbonyl, (Het)Arylcarbonyl,
Amino, C₁₋₄-Alkylamino, Di-(C₁₋₄-alkyl)amino, Pyrrolidin-1-yl, 2-Oxo-pyrrolidin-1-yl, Piperidin-1-yl, 2-Oxo-piperidin-1-yl, Morpholin-4-yl, 3-Oxo-morpholin-4-yl, Piperazin-1-yl, 2-Oxo-piperazin-1-yl, 3-Oxopiperazin-1-yl, 4-(C₁₋₄-Alkyl)piperazin-1-yl, 4-(C₁₋₄-Alkylcarbonyl)piperazin-1-yl, 4-(C₃₋₆-Cycloalkyl-carbonyl)piperazin-1-yl, 4-(C₁₋₄-Alkyloxycarbonyl)piperazin-1-y 1, 4-(C₁₋₄-Alkylsulfonyl)piperazin-1-yl, 2-Oxo-4-(C₁₋₄-alkyl)piperazin-1-yl, 3-Oxo-4-(C₁₋₄-alkyl)piperazin-1-yl,
C₁₋₄-Alkylcarbonylamino, (Het)Arylcarbonylamino, (Het)Aryl-C₁₋₄-alkylcarbonylamino, C₁₋₄-Alkyloxycarbonylamino, Aminocarbonylamino, C₁₋₄-Alkylaminocarbonylamino, Di-(C₁₋₄-alkyl)aminocarbonylamino, Pyrrolidin-1-yl-carbonylamino, Piperidin-1-yl-carbonylamino, Morpholin-4-yl-carbonylamino, Piperazin-1-yl-carbonylamino, 4-(C₁₋₄-Alkyl)piperazin-1-yl-carbonylamino, C₁₋₄-Alkylsulfonylamino, Aminosulfonylamino, C₁₋₄-Alkylaminosulfonylamino, Di-(C₁₋₄-alkyl)aminosulfonylamino, Pyrrolidin-1-yl-sulfonylamino, Piperidin-1-yl-sulfonylamino, Morpholin-4-yl-sulfonylamino, Piperazin-1-yl-sulfonylamino, 4-(C₁₋₄-Alkyl)piperazin-1-yl-sulfonylamino, (C₁₋₄-Alkoxycarbonylamino)carbonylamino, (Het)Arylsulfonylamino, (Het)Aryl-C₁₋₄-alkylsulfonylamino,
N-(C₁₋₄-Alkyl)-C₁₋₄-alkylcarbonylamino, N-(C₁₋₄-Alkyl)-(het)arylcarbonylami- no, N-(C₁₋₄-Alkyl)-(het)aryl-C₁₋₄-alkylcarbonylamino, N-(C₁₋₄-Alkyl)-C₁₋₄-alkyl-oxycarbonylamino, N-(Aminocarbonyl)-C₁₋₄-alkylamino, N-(C₁₋₄-Alkylamino-carbonyl)-C₁₋₄-alkylamino, N-[Di-(C₁₋₄-alkyl)aminocarbonyl]-C₁₋₄-alkylamino,
N-(C₁₋₄-Alkyl)-C₁₋₄-alkylsulfonylamino, N-(C₁₋₄-Alkyl)-(het)arylsulfonylamino, N-(C₁₋₄-Alkyl)-(het)aryl-C₁₋₄-alkylsulfonylamino,
Oxo-imidazolidin-1-yl, 2,4-Dioxo-imidazolidin-1-yl, 2,5-Dioxo-imidazolidin-1-yl, 2-Oxo-hexahydropyrimidin-1-yl, wobei das Stickstoffatom an Position 3 der vorgenannten Gruppen gegebenenfalls mit Methyl oder Ethyl substituiert ist,
(Hydroxyimino)aminomethyl, (C₁₋₃-Alkyloxyimino)aminomethyl, Carboxy, C₁₋₄-Alkyloxycarbonyl, Aminocarbonyl, C₁₋₄-Alkylaminocarbonyl, Di-(C₁₋₄-alkyl)-aminocarbonyl, Pyrrolidin-1-yl-carbonyl, Piperidin-1-yl-carbonyl, Morpholin-4-yl-carbonyl, Piperazin-1-yl-carbonyl, 4-(C₁₋₄-Alkyl)piperazin-1-yl-carbonyl,
Carboxy-C₁₋₄-alkyl, C₁₋₄-Alkyloxycarbonyl-C₁₋₄-alkyl, Cyano-C₁₋₄-alkyl, Aminocarbonyl-C₁₋₄-alkyl, C₁₋₄-Alkyl-aminocarbonyl-C₁₋₄-alkyl, Di-(C₁₋₄-alkyl)-aminocarbonyl-C₁₋₄-alkyl, Pyrrolidin-1-yl-carbonyl-C₁₋₄-alkyl, Piperidin-1-yl-carbonyl-C₁₋₄-alkyl, Morpholin-4-yl-carbonyl-C₁₋₄-alkyl, Piperazin-1-yl-carbonyl-C₁₋₄-alkyl, 4-(C₁₋₄-Alkyl)-piperazin-1-yl-carbonyl-C₁₋₄-alkyl,
Carboxy-C₁₋₄-alkyloxy, C₁₋₄-Alkyloxycarbonyl-C₁₋₄-alkyloxy, Cyano-C₁₋₄-alkyloxy, Aminocarbonyl-C₁₋₄-alkyloxy, C₁₋₄-Alkyl-aminocarbonyl-C₁₋₄-alkyloxy, Di-(C₁₋₄-alkyl)-aminocarbonyl-C₁₋₄-alkyloxy, Pyrrolidin-1-yl-carbonyl-C₁₋₄-alkyl-oxy, Piperidin-1-yl-carbonyl-C₁₋₄-alkyloxy, Morpholin-4-yl-carbonyl-C₁₋₄-alkyloxy, Piperazin-1-yl-carbonyl-C₁₋₄-alkyloxy, 4-(C₁₋₄-Alkyl)-piperazin-1-yl-carbonyl-C₁₋₄-alkyloxy,
Hydroxy-C₁₋₄-alkyl, C₁₋₄-Alkyloxy-C₁₋₄-alkyl, Amino-C₁₋₄-alkyl, C₁₋₄-Alkylamino-C₁₋₄-alkyl, Di(C₁₋₄-alkyl)-amino-C₁₋₄-alkyl, Pyrrolidin-1-yl-C₁₋₄-alkyl, C₁₋₄-Alkylcarbonylamino-C₁₋₄-alkyl, N-(C₁₋₄-Alkyl)-C₁₋₄-alkylcarbonylamino-C₁₋₄- alkyl, 2-Oxo-pyrrolidin-1-yl-C₁₋₄-alkyl, Piperidin-1-yl-C₁₋₄-alkyl, 2-Oxo-piperidin-1-yl-C₁₋₄-alkyl, Morpholin-4-yl-C₁₋₄-alkyl, 3-Oxo-morpholin-4-yl-C₁₋₄-alkyl, Piperazin-1-yl-C₁₋₄-alkyl, 2-Oxo-piperazin-1-yl-C₁₋₄-alkyl, 3-Oxo-piperazin-1-yl-C₁₋₄-alkyl, 4-(C₁₋₄-Alkyl)-piperazin-1-yl-C₁₋₄alkyl, 2-Oxo-4-(C₁₋₄-alkyl)-piperazin-1-yl-C₁₋₄-alkyl, 3-Oxo-4-(C₁₋₄-alkyl)-piperazin-1-yl-C₁₋₄-alkyl,
Hydroxy-C₁₋₄-alkyloxy, C₁₋₄-Alkyloxy-C₁₋₄-alkyloxy, C₁₋₄-Alkylsulfanyl-C₁₋₄-alkyloxy, C₁₋₄-Alkylsulfinyl-C₁₋₄-alkyloxy, C₁₋₄-Alkylsulfonyl-C₁₋₄-alkyloxy, Amino-C₁₋₄-alkyloxy, C₁₋₄-Alkylamino-C₁₋₄-alkyloxy, Di-(C₁₋₄-alkyl)-amino-C₁₋₄-alkyloxy, Pyrrolidin-1-yl-C₁₋₄-alkyloxy, 2-Oxo-pyrrolidin-1-yl-C₁₋₄-alkyloxy, Piperidin-1-yl-C₁₋₄-alkyloxy, 2-Oxo-piperidin-1-yl-C₁₋₄-alkyloxy, Morpholin-4-yl-C₁₋₄-alkyloxy, 3-Oxo-morpholin-4-yl-C₁₋₄-alkyloxy, Piperazin-1-yl-C₁₋₄-alkyl-oxy, 2-Oxo-piperazin-1-yl-C₁₋₄-alkyloxy, 3-Oxo-piperazin-1-yl-C₁₋₄-alkyloxy, 4-(C₁₋₄-Alkyl)-piperazin-1-yl-C₁₋₄-alkyloxy, 2-Oxo-4-(C₁₋₄-alkyl)-piperazin-1-yl-C₁₋₄-alkyloxy, 3-Oxo-4-(C₁₋₄-alkyl)-piperazin-1-yl-C₁₋₄-alkyloxy,
C₁₋₄-Alkylsulfanyl, C₁₋₄-Alkylsulfinyl, C₁₋₄-Alkylsulfonyl, C₁₋₄-Alkylsulfonyloxy, (Het)Arylsulfonyl, (Het)Arylsulfonyloxy, Trifluormethylsulfanyl, Trifluor-methylsulfinyl, Trifluormethylsulfonyl, C₃₋₆-Cycloalkylsulfanyl, C₃₋₆-Cycloalkylsulfinyl, C₃₋₆-Cycloalkylsulfonyl, C₃₋₆-Cycloalkyl-C₁₋₃-alkylsulfanyl, C₃₋₆-Cycloalkyl-C₁₋₃-alkylsulfinyl, C₃₋₆-Cycloalkyl-C₁₋₃-alkylsulfonyl,
Aminosulfonyl, C₁₋₄-Alkyl-aminosulfonyl, Di-(C₁₋₄-alkyl)-aminosulfonyl, Pyrrolidin-1-yl-sulfonyl, Piperidin-1-yl-sulfonyl, Morpholin-4-yl-sulfonyl, Piperazin-1-yl-sulfonyl oder 4-(C₁₋₄-Alkyl)-piperazin-1-yl-sulfonyl,
wobei die oben genannten gesättigten Heterocyclen und Cycloalkylringe gegebenenfalls substituiert sind mit ein oder zwei Gruppen, unabhängig voneinander ausgewählt aus Fluor, C₁₋₃-Alkyl, C₁₋₃-Alkoxy, C₁₋₃-Alkoxy-C₁₋₃-alkyl oder Hydroxy, oder einem physiologisch akzeptables Salz davon,
einschließlich der in Anspruch 1 ausgenommenen Verbindungen, zur Behandlung oder
Vorbeugung von Krankheiten und Zuständen, ausgewählt aus metabolischen Störungen, Diabetes mellitus Typ 1, Diabetes mellitus Typ 2, Komplikationen bei Diabetes (wie Retinopathie, Nephropathie oder Neuropathien, diabetischem Fuß, Magengeschwüren, Makroangiopathien, langsame oder schlechte Wundheilung), metabolischer Acidose, metabolischer Ketose, reaktiver Hypoglykämie, Hyperinsulinämie, metabolischer Glucosestörung, Insulinresistenz, metabolischem Syndrom, Dyslipidämien verschiedenen Ursprungs, Arteriosklerose, Adipositas, Bluthochdruck, chronischer Herzschwäche, Ödemen, Hyperurikämie, beta-Zelldegeneration (Apoptose oder Nekrose der Pankreas-beta-Zellen), Verbesserung oder Wiederherstellung der Funktionalität der Pankreaszellen, Erhöhung der Zahl und der Größe der Pankreas-beta-Zellen, akutem Nierenversagen, Augeninnenüberdruck, Glaukom, Osteoporose, Verbesserung oder Vorbeugung kognitiver Beeinträchtigungen, Angstzustände, Depression, Tuberkulose, Lepra und Psoriasis.

8. Verwendung mindestens einer Verbindung, wie definiert in Anspruch 7, zur Herstellung einer pharmazeutischen Zusammensetzung, die geeignet ist für die Behandlung oder Vorbeugung von Erkrankungen oder Zuständen, ausgewählt aus metabolischen Störungen, Diabetes mellitus Typ 1, Diabetes mellitus Typ 2, Komplikationen bei Diabetes (wie Retinopathie, Nephropathie oder Neuropathien, diabetischem Fuß, Magengeschwüren, Makroangiopathien, langsame oder schlechte Wundheilung), metabolischer Acidose, metabolischer Ketose, reaktiver Hypoglykämie, Hyperinsulinämie, metabolischer Glucosestörung, Insulinresistenz, metabolischem Syndrom, Dyslipidämien verschiedenen Ursprungs, Arteriosklerose, Adipositas, Bluthochdruck, chronischer Herzschwäche, Ödemen, Hyperurikämie, beta-Zelldegeneration (Apoptose oder Nekrose der Pankreas-beta-Zellen), Verbesserung oder Wiederherstellung der Funktionalität der Pankreaszellen, Erhöhung der Zahl und der Größe der Pankreas-beta-Zellen, akutem Nierenversagen, Augeninnenüberdruck, Glaukom, Osteoporose, Verbesserung oder Vorbeugung kognitiver Beeinträchtigungen, Angstzustände, Depression, Tuberkulose, Lepra und Psoriasis.

9. Verfahren zur Herstellung einer Verbindung der allgemeinen Formel I nach irgendeinem der Ansprüche 1 bis 4 oder eines physiologisch akzeptablen Salzes nach Anspruch 5, **dadurch gekennzeichnet, dass** eines der Amine der allgemeinen Formel II 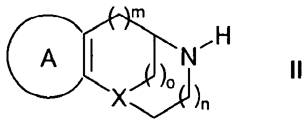 worin die Gruppen A, X, m, n und o wie in Anspruch 1 definiert sind, oder der allgemeinen Formel III 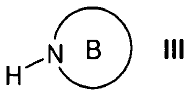 worin B wie in Anspruch 1 definiert ist,
umgesetzt wird mit einem Carbonsäurederivat Y-CO-Y, was eine Verbindung entweder der allgemeinen Formel IV oder der allgemeinen Formel V als Zwischenprodukt liefert 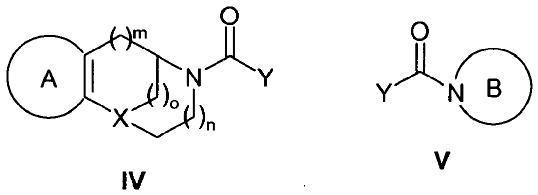 die gegebenenfalls isoliert und gegebenenfalls gereinigt wird,
und das Zwischenprodukt der Formel IV oder V wird umgesetzt mit dem anderen Amin der Formel III oder II, um die Verbindung der Formel I zu ergeben,
wobei Y eine Abgangsgruppe darstellt und insbesondere Fluor, Chlor, Brom, Cyano, C₁₋₄-Alkyloxy, C₂₋₄-Alkenyloxy, C₂₋₄-Alkinyloxy, teilweise oder vollständig fluoriertes C₂₋₁₀-Alkoxy, Oxyarylotriazol, Oxyheteroarylotriazol, Heteroar-N-yl, 3-Methylimidazol-1-yl, Succinyl-N-oxy, Di-(C₁₋₄-Alkyl)aminocarbonyloxy, Pyrrolylcarbonyloxy, Piperidinylcarbonyloxy, Morpholinylcarbonyloxy, Aryloxy oder Heteroaryloxy darstellt,
wobei die bei der Definition der oben genannten Gruppen erwähnten Alkyl-, Alkenyl- und Alkinylgruppen, entweder allein oder als Teil anderer Gruppen,
substituiert sein können mit ein oder mehr Substituenten, unabhängig voneinander ausgewählt aus Fluor, Chlor, C₁₋₃-Alkyl und C₁₋₃-Alkoxy,
wobei die bei der Definition der oben genannten Gruppen erwähnten Arylgruppen, entweder allein oder als Teil anderer Gruppen, Phenyl oder Naphthyl bezeichnen und die bei der Definition der oben genannten Gruppen erwähnten Heteroarylgruppen, entweder allein oder als Teil einer anderen Gruppe, Pyridinyl, Pyrimidinyl, Triazinyl, Imidazolyl, Pyrazolyl, Triazolyl, Tetrazolyl bezeichnen, wobei sowohl die Aryl- als auch die Heteroarylgruppen gegebenenfalls substituiert sind mit ein oder mehr Substituenten, unabhängig voneinander ausgewählt aus Fluor-, Chlor-, Brom-, C₁₋₃-Alkyl-, C₁₋₃-Alkyloxy-, Nitro-, Cyano- und Di-(C₁₋₃-alkyl)amino-Gruppen,
wobei die zwei Y in Y-CO-Y gleich oder verschieden sein können,
wobei das zweite zu ersetzende Y auch in ein reaktiveres Y umgewandelt werden kann, nachdem das erste Y durch eines der beiden Amine ersetzt wurde,
gegebenenfalls in Gegenwart einer organischen Base oder einem anderen Additiv;
und, wenn notwendig, wird jedwede Schutzgruppe, die in den oben beschriebenen Reaktionen verwendet wird, gleichzeitig oder nacheinander abgespalten;
wenn gewünscht wird eine so erhaltene Verbindung der Formel I in ihre Stereoisomeren aufgetrennt;
wenn gewünscht wird eine so erhaltene Verbindung der Formel I in die Salze davon umgewandelt, insbesondere für die pharmazeutische Verwendung in die physiologisch akzeptablen Salze davon.

## Revendications

1. Composés de formule I 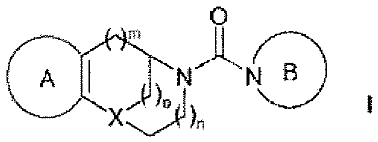 dans laquelle
X désigne CH ou N,
m, n, o désigne indépendamment les uns des autres 0, 1 ou 2,
où la structure de noyau azacycloalcène en C₅₊ₘ₊ₙ de formule générale I incluant le groupe de pontage -(CH₂)ₒ- est facultativement substituée par 1, 2 ou plusieurs substituants choisis indépendamment les uns des autres dans le groupe consistant en R¹¹ et R¹²,
A désigne une cycle benzo, pyrido, pyrrolo, furo, thiéno, pyridazino, pyrimido, ou pyrazino où chacun desdits cycles est substitué par un ou plusieurs substituants choisis indépendamment les uns des autres parmi R¹, et où 2 atomes C adjacents de chacun desdits cycles sont facultativement substitués par R² et R³ ; ou
un cycle pyrazolo, imidazo, oxazolo, thiazolo, isoxazolo, ou isothiazolo dans lequel chacun desdits cycles est facultativement substitué par R¹ ; ou
un cycle 1,2,3-triazolo facultativement substitué par R^{N} ; et
B désigne un groupe azacycloalk-1-yle monocyclique à 3 à 8 chaînons, spirocyclique à 7 à 12 chaînons, bicyclique à 6 à 12 chaînons ou tricyclique à 9 à 15 chaînon, qui est saturé ou partiellement ou totalement insaturé,
où 1 ou 2 groupes -CH₂- peuvent être remplacés par -NR^{N}-,
où 1 à 4 groupes -CH₂- peuvent être remplacés indépendamment les uns des autres par O, S, un groupe carbonyle ou sulfonyle,
où 1 ou 2 groupes -CH= peut être remplacé par -N=, et
où ledit groupe azacycloalkyle peut être substitué par un ou plusieurs substituants choisis indépendamment les uns des autres parmi L¹,
où ledit groupe azacycloalkyle peut être substitué par 1 ou 2 substituants choisis indépendamment l'un de l'autre parmi L²,
où 2 atomes C adjacents dudit groupe azacycloalkyle peuvent être substitués par L³ et L⁴, et
où 2 atomes C adjacents dudit groupe azacycloalkyle peuvent être substitués par L⁵ et L⁶ ;
R^{N} indépendamment des autres désignent un atome d'hydrogène, un groupe alkyle en C₁ à ₆, alcényle en C₃ à ₆, alcynyle en C₃ à ₆, (het)aryle, alkylcarbonyle en C₁ à ₄, alkylsulfonyle en C₁ à ₄, (het)arylcarbonyle, (het)arylsulfonyle,
où chaque groupe alkyl, alcényle, et alcynyle peut être mono- ou poly-substitué par un atome de fluor, et peut être monosubstitué par un groupe hydroxy, alcoxy en C_{1 à 4}, alkylsulfanyle en C₁ à ₄, alkylsulfinyle en C₁ à ₄, alkylsulfonyle en C₁ à ₄, amino, alkylamino en C₁ à ₄, di-(alkyl en C₁ à ₄)-amino, alkylcarbonylamino en C₁ à ₄, cyano, carboxy, alcoxycarbonyle en C₁ à ₄, aminocarbonyle, alkylaminocarbonyle en C₁ à ₄, di-(alkyl en C₁ à ₄) aminocarbonyle, ou (het) aryle,
R¹ indépendamment des autres désigne un atome de fluor, chlore, brome, iode, un groupe cyano, nitro, alkyl en C₁ à ₄, difluorométhyle, trifluorométhyle, difluorométhoxy, trifluorométhoxy, trifluorométhylhydroxy- alkyle en C₁ à ₂, 2,2,2-triflucro-1-hydroxy-1-(trifluorométhyl)éthyle, cycloalkyle en C₃ à ₆, cycloalkyloxy en C₃ à ₆, cycloalkyl, en C₃ à ₆-alkyle en C_{1 à 3}, cycloalkyl en C₃ à ₆-alkyloxy en C_{1 à} 3, tétrahydrofuran-3-yloxy, Létrahydropyran-3-yloxy, tétrahydropyran-4-yloxy, tétrahydrofuranyl-alkyloxy en C₁ à ₃, tétrahydropyranyl-alkyloxy en C₁ à ₃, (het)aryl, (het)aryloxy, (het)aryl-alkyle en C₁ à ₃, (het)aryl-alkyloxy, en C₁ à ₃, (het)aryloxy-alkyle en C₁ à ₃, alkyl en C₁ à ₃-carbonyle, (het)aryl-carbonyle,
amino, alkylamino en C₁ à ₄, di-(alkyl en C₁ à ₄)aminoe, pyrrolidin-1-yle, 2-oxo-pyrrolidin-1-yle, pipéridin-1-yle, 2-oxo-pipéridin-1-yle, morpholin-4-yle, 3-oxo-morpholin-4-yle, pipérazin-1-ylc, 2-oxo-pipérazin-1-yle, 3-oxo-pipérazin-1-yle, 4-(alkyl en C₁ à ₄)-pipérazin-1-yle, 4-(alkylcarbonyl en C_{1 à 4})-pipérazin-1-yl, 4-(cycloalkylcarbonyl en C_{3 à 6})-pipérazin-₁-yle, 4-(alkyloxycarbonyl en C_{1 à 4})-pipérazin-1-yle, 4-(alkylsulfonyl en C_{1 à 4})-pipérazin-1-yle, 2-oxo-4-(alkyl en C_{1 à 4})-pipérazin-1-yle, 3-oxo-4-(alkyl en C_{1 à 4})-pipérazin-1-yle,
alkyl en C_{1 à 4}-carbonylamino, (het)arylcarbonylamino, (het)aryl-alkyl en C_{1 à 4}-carbonylamino, alkyloxy en C_{1 à 4}-carbonylamino, aminocarbonylamino, alkyl en C1 à 4-amino-carbonylamino, di-(alkyl en C_{1 à 4})aminocarbonylamino, pyrrolidin-1-yl-carbonylamino, pipéridin-1-yl-carbonylamino, morpholin-4-yl-carbonylamino, pipérazin-1-yl-carbonylamino, 4-(alkyl, en C_{1 à 4})-pipérazin-1-yl-carbonylamino, alkyl en C_{1 à 4}-sulfonylamino, aminosulfonylamino, alkylamino en C_{1 à 4}-sulfonylamino, di-(alkyl en C_{1 à 4})-amino-sulfonylamino, pyrrolidin-1-yl-sulfonylamino, pipéridin-1-yl-sulfonylamino, morpholin-4-yl-sulfonylamino, pipérazin-1-yl-sulfonylamino, 4-(alkyl en C_{1 à 4})-pipérazin-1-yl-sulfonylamino, (alkyloxy en C_{1 à 4}-carbonylamino)carbonylamino, (het)arylsulfonylamino, (het)aryl-alkyl en C_{1 à 4}-sulfonylamino,
N-(alkyl en C_{1 à 4})-alkyl en C_{1 à 4}-carbonylamino, N-(alkyl C_{1 à 4})-(het)arylcarbonylamino, N-(alkyl en C_{1 à 4})-(het)aryl-alkyl en C_{1 à 4}-carbonylamino, N-(alkyl en C_{1 à 4})-alkyloxy en C_{1 à 4}-carbonylamino, N-(aminocarbonyl)-alkylamino en C_{1 à 4}, N-(alkyl en C_{1 à 4}-aminocarbonyl)-alkylamino en C_{1 à 4}, N-[di-(alkyl en C_{1 à 4})aminocarbonyl]-alkylamino en C_{1 à 4},
N-(alkyl en C_{1 à 4})-alkyl en C_{1 à 4}-sulfonylamino, N-(alkyl en C_{1 à 4})-(het)arylsulfonylamino, N-(alkyl en C_{1 à 4}) - (het)aryl-alkyl en C_{1 à 4}-sulfonylamino,
oxo-1-midazolidin-1-yle, 2,4-dioxo-imidazolidin-1-yle, 2,5-dioxo-imidazolidin-1-yle, 2-oxo-hexahydropyrimidin-1-yle, où l'atome d'azote dans la position 3 des groupes susmentionnés est facultativement substitué par un groupe méthyle ou éthyle,
(hydroxyimino)aminométhyl, (alkyloxyimino en C_{1 à 3})-aminaméthyle, carboxy, alkyloxy en C_{1 à 4}-carbonyle, aminocarbonyle, alkyl en C_{1 à 4}-aminocarbonyle, di-(alkyl en C_{1 à 4})-aminocarbonyle, pyrrolidin-1-yl-carbonyle, pipéridin-1-yl-carbonyle, morpholin-4-yl-carbonyle, pipérazin-1-yl-carbonyle, 4-(alkyl en C_{1 à 4})-pipérazin-1-yl-carbonyle,
carboxy-alkyle en C_{1 à 4}, alkyloxy en C_{1 à 4}-carbonyl-alkyle en C_{1 à 4}, cyano-alkyle en C_{1 à 4}, aminocarbonyl-alkyle en C_{1 à 4}, alkyl en C_{1 à 4}-aminocarbonyl-alkyle en C_{1 à 4}, di-(alkyl en C_{1 à 4})-aminocarbonyl-alkyle C_{1 à 4}, pyrrolidin-1-yl-carbonyl-alkyle en C_{1 à 4}, pipéridin-1-yl-carbonyl-alkyle C_{1 à 4}, morpholin-4-yl-carbonyl-alkyle en C_{1 à 4}, pipérazin-1-yl-carbonyl-alkyle en C_{1 à 4}, 4-(alkyl en C_{1 à 4})-pipérazin-1-yl-carbanyl-alkyle en C_{1 à 4},
carboxy-alkyloxy en C_{1 à 4}, alkylaxy en C_{1 à 4}-carbonyl-alkyloxy en C_{1 à 4}, cyano- alkyl en C_{1 à 4}-oxy, aminocarbonyl-alkyloxy en C_{1 à 4}, alkyl en C_{1 à 4}-aminocarbonyl-alkyloxy en C_{1 à 4}, di-(alkyl en C_{1 à 4})-aminocarbonyl-alkyloxy en C_{1 à 4}, pyrrolidin-1-yl-carbonyl-alkyl en C_{1 à 4}-oxy, pipéridin-1-yl-carbonyl-alkyloxy en C_{1 à 4}, morpholin-4-yl-carbonyl-alkyl en C_{1 à 4}-oxy, pipérazin-1-yl-carbonyl-alkyloxy en C_{1 à 4}, 4-(alkyl en C_{1 à 4})-pipérazin-1-yl-carbonyl-alkyloxy en C_{1 à 4},
hydroxy-alkyle en C_{1 à 4}, alkyloxy en C_{1 à 4}-alkyle en C_{1 à 4}, amino-alkyle en C_{1 à 4}, alkylamino, en C_{1 à 4}-alkyle en C_{1 à 4}, di-(alkyl en C_{1 à 4})-amino-alkyle en C_{1 à 4}, pyrrolidin-1-yl-alkyle en C_{1 à 4}, alkyl en C_{1 à 4}-carbonyl-amino-alkyle en C_{1 à 4}, N-(alkyl en C_{1 à 4})-alkylcarbonyl en C_{1 à 4}-amino-alkyle en C_{1 à 4}, 2-oxo-pyrrolzdin-1-yl-alkyl e en C_{1 à 4}, plpéridin-1-yl-alkyle en C_{1 à 4}, 2-oxo-pipéridin-1-yl-alkyle en C_{1 à 4}, morpholin-4-yl-alkyle en C_{1 à 4}, 3-oxo-morpholin-4-yl-alkyle en C_{1 à 4}, pipérazin-1-yl-alkyle en C_{1 à 4}, 2-oxo-pipérazin-1-yl-alkyle en C_{1 à 4}, 3-oxo-pipérazin-1-yl-alkyle en C_{1 à 4}, 4-(alkyl en C_{1 à 4})-pipérazin-1-yl-alkyle en C_{1 à 4}, 2-oxo-4-(alkyl en C_{1 à 4})-pipérazin-1-yl-alkyle en C_{1 à 4}, 3-oxo-4-(alkyl en C_{1 à 4})-piperazin-1-yl-alkyl en C_{1 à 4},
hydroxy-alkyloxy en C_{1 à 4}, alkyloxy en C_{1 à 4}-alkyloxy, en C_{1 à 4}, alkylsulfanyl en C_{1 à 4}-alkylozy en C_{1 à 4}, alkylsulfinyl en C_{1 à 4}-alkyloxy en C_{1 à 4}, alkylsulfonyl en C_{1 à 4}-alkyloxy en C_{1 à 4}, amino-alkyloxy en C_{1 à 4}, alkylamino, en C_{1 à 4}-alkyloxy en C_{1 à 4}, di-(alkyl en C_{1 à 4})-amino-alkylozy en C_{1 à 4}, pyrrolidin-1-yl-alkyloxy en C_{1 à 4}, 2-oxo-pyrrolidin-1-yl-alkyloxy en C_{1 à 4}, pipéridin-1-yl-alkyloxy en C_{1 à 4}, 2-oxo-pipéridin-1-yl-alkyloxy en C_{1 à 4}, morpholin-4-yl-alkyloxy en C_{1 à 4}, 3-oxo-morpholin-4-yl-alkyloxy en C_{1 à 4}, pipérazin-1-yl-alkyloxy en C_{1 à 4}, 2-oxo-pipérazin-1-yl-alkyloxy en C_{1 à 4}, 3-oxo-pipérazin-1-yl-alkyloxy en C_{1 à 4}, 4-(alkyl en C_{1 à 4})-pipérazin-1-yl-alkyloxy en C_{1 à 4}, 2-oxo-4-(alkyl en C_{1 à 4})-pipérazin-1-yl-alkyloxy en C_{1 à 4}, 3-oxo-4-(alkyl en C_{1 à 4})-pipérazin-1-yl-alkyloxy en C_{1 à 4},
alkylsulfanyle en C_{1 à 4}, alkysulfinyle en C_{1 à 4}, alkylsulfonyle en C_{1 à 4}, alkylsulfonyloxy en C_{1 à 4}, (het)arylsulfonyle, (het)arylsulfonyloxy, trifluorométhylsulfanyle, trifluorométhylsulfinyle, trifluorométhylsulfonyle, cycloalkylsulfanyle en C_{3 à 6}, cycloalkylsulfinyle en C_{3 à 6}, cycloalkylsulfonyle en C_{3 à 6}, cycloalkyl en C_{3 à 6}-alkylsulfanyle en C_{1 à 3}, cycloalkyl en C_{3 à 6}-alkylsulfinyle en C_{1 à 3}, cycloalkyl en C_{3 à 6}-alkylsulfonyle en C_{1 à 3},
aminosulfonyle, alkyl en C_{1 à 4}-aminosulfonyle, di-(alkyle en C_{1 à 4})-aminosulfonyle, pyrrolidin-1-yl-sulfonyl, pipéridin-1-yl-sulfonyle, morpholin-4-yl-sulfonyl, pipérazin-1-yl-sulfonyle, ou 4-(alkyle C_{1 à 4})-pipérazin-1-yl-sulfonyle,
où les hétérocycles et cycles cycloalkyle saturés susmentionnés sont facultativement substitués par un ou deux groupes choisis indépendamment l'un de l'autre parmi un atome de fluor, un groupe alkyle en C_{1 à 3}, alcoxy en C_{1 à 3}, alcoxy en C_{1 à 3}-alkyle en C_{1 à 3}, ou hydroxy,
R², R³ sont liés l'un à l'autre pour former un groupe de pontage méthylènedioxy, éthylénedioxy ou alkylène en C_{3 à 5}, qui peut être mono- ou di-substitué par un groupe méthyle, et qui peut être mono- ou poly-fluoré ; ou
R², R³ forment en combinaison avec les atomes de carbone auxquels ils sont attachés un cycle benzo, pyrido, pyrimido, pyrazino, pyridazino, pyrrolo, furano, thiéno, pyrazolo, imidazo, triazolo, oxazolo, thiazolo, isoxazolo, ou isothiazolo, où chacun desdits cycles peut être substitué par un ou plusieurs substituants choisis indépendamment les uns des autres parmi R^{P};
R^{P} désigne un atome d'halogène, un groupe alkyle en C_{1 à 6}, hydroxy-alkyle en C_{1 à 4}, alkyloxy en C_{1 à 3}-alkyle en C_{1 à 3}, cycloalkyl, en C_{3 à 6}, hydroxy-cycloalkyle en C_{4 à 6}, alkyloxy C_{1 à 3}-cycloalkyle en C_{3 à 6}, azétidinyle, 1-(alkyl en C_{1 à 3})-azétidinyle, 1-(alkylcarbonyl en C_{1 à 3})-azétidinyle, pyrrolidinyle, 1-(alkyl en C_{1 à 3})-pyrrolidinyle, 1-(alkylcarbonyl en C_{1 à 3})-pyrrolidinyle, pipéridinyle, 1-(alkyl en C_{1 à 3})-pipéridinyle, 1-(alkylcarbonyl en C_{1 à 3})-pipéridinyle, tétrahydrofuranyle, tétrahydropyranyle, difluorométhyle, trifluorométhyle, cyano, nitro, amino, alkylamino en C_{1 à 3}, di-(alkyl en C_{1 à 3})amino, alkylcarbonylamino en C_{1 à 3}, méthylsulfonylamino, carboxy, alkyloxycarbonyle en C_{1 à 4}, aminocarbonyle, alkylaminocarbonyle en C_{1 à 3}, di-(alkyl en C_{1 à 3})-aminocarbonyle, aminosulfonyle, méthylsulfanyle, méthylsulfinyle, méthylsulfonyle, hydroxy, alkyloxy, en C_{1 à 3}, difluorométhoxy, trifluorométhoxy, phényle, ou
pyrrolyle, furanyle, thiényle, pyridyle, où dans l'un quelconque de ces groupes, 1 ou 2 CH sont facultativement remplacés par des atomes N, ou
1,2-dihydro-2-oxo-pyridinyle, 1,4-dihydro-4-oxo-pyridinyle, 2,3-dihydro-3-oxo-pyridazinyle, 1,2,3,6-tétrahydro-3,6-dioxo-pyridazinyle, 1,2-dihydro-2-oxo-pyrimidinyle, 3,4-dihydro-4-oxo-pyrimidinyle, 1,2,3,4-tétrahydro-2,4-dioxo-pyrimidinyle, 1,2-dihydro-2-oxo-pyrazinyle,
où chacun des groupes aromatiques ou hétéroaromatiques mentionnés ci-dessus est facultativement substitué par un ou deux groupes indépendamment choisis parmi un atome de fluor, de chlore, un groupe alkyle en C_{1 à 3}, difluorométhyle, trifluorométhyle, cyano, amino, acétylamino, méthylsulfonylamino, carboxy, alkyloxycarbonyle en C_{1 à 4}, aminocarbonyle, alkylaminocarbonyl en C_{1 à 3}, di-(alkyl en C_{1 à 3})-aminocarbonyle, hydroxy, alkyloxy en C_{1 à 3}, difluorométhoxy, et trifluorométhoxy,
R¹⁰ indépendamment des autres désigne un atome d'halogène, un groupe alkyle en C_{1 à 3}, difluorométhyle, trifluorométhyle, cyano, nitro, amine, alkylamino en C_{1 à 3}, di-(alkyl en C_{1 à 3})amino, acétylamino, méthylsulfonylamino, carboxy, alkyloxycarbonyl en C_{1 à 4}, aminocarbonyl, alkylaminocarbonyle en C_{1 à 3}, di-(alkyl en C_{1 à 3})-aminocarbonyle, aminosulfonyle, méthylsulfanyle, méthylsulfinyle, méthylsulfonyle, hydroxy, alkyloxy en C_{1 à 3}, difluorométhoxy, trifluorométhoxy, ou phényle facultativement substitué par 1 ou 2 substituants choisis indépendamment de l'autre parmi un atome de fluor, un groupe méthyle, méthoxy, cyano, ou hydroxy,
R¹¹ indépendamment des autres désigne un atome de fluor, un groupe alkyle en C_{1 à 4}, (het)aryle, hydroxy, alkyloxy en C_{1 à 4}, cyano, carboxy, alkyloxycarbonyle en C_{1 à 4}, aminocarbonyle, alkylamino en C_{1 à 4}-carbonyle, di-(alkyl en C_{1 à 4})-aminocarbonyle, hydroxy-alkyle en C_{1 à 4}, ou alkyloxy en C_{1 à 3}-alkyle en C_{1 à 4},
R¹² désigne indépendamment des autres un atome de fluor ou un groupe alkyle en C_{1 à 4} ; et
L¹ désigne indépendamment des autres un atome d'halogène, un groupe alkyle en C_{1 à 4}, trifluorométhyle, hydroxy, alkyloxy en C_{1 à 4}, ou cyano ;
L² désigne indépendamment des autres un atome de fluor, de chlore, de brome, d'iode, un groupe nitro, cyano, hydroxy, cycloalkyle en C_{3 à 6}, cycloalkyloxy en C_{3 à 6}, tétrahydrofuran-3-yl-oxy, tétrahydropyran-3-yl-oxy, tétrahydropyran-4-yl-oxy, tétrahydrofuranyl-alkyloxy en C_{1 à 3}, tétrahydropyranyl-alkyloxy en C_{1 à 3}, (het)aryle, (het)aryloxy, ou
alkyle en C_{1 à 6}, alcényle en C_{2 à 6}, alcynyl en C_{2 à 6}, alkyloxy en C_{1 à 4}, où dans chaque groupe, un groupe CH₂ peut être remplacé par un groupe carbonyle ou sulfonyle, et où chaque groupe peut être mono- ou poly-fluoré, et où chaque groupe peut en outre être substitué par
un groupe hydroxy, un atome de chlore, un groupe alkyloxy en C_{1 à 3}, amino, alkylamino en C_{1 à 3}, di-(alkyl en C_{1 à 3})-amino, pyrrolidin-1-yle, 2-oxo-pyrrolidin-1-yle, pipéridin-1-yle, 2-oxo-pipéridin-1-yle, morpholin-4-yle, 3-oxo-morpholin-4-yle, pipérazin-1-yle, 2-oxo-pipérazin-1-yle, 3-oxo-pipérazin-1-yle, 4-(alkyl en C_{1 à 3})-pipérazin-1-yle, 2-oxo-4-(alkyl en C_{1 à 3})-pipérazin-1-yle, 3-oxo-4-(alkyl en C_{1 à 3})-pipérazin-1-yle, carboxy, alkyloxy en C_{1 à 3}-carbonyle, cyano, aminocarbonyle, alkyle en C_{1 à 3}-aminocarbonyle, di-(alkyl en C_{1 à 3})-aminocarbonyle, pyrrolidin-1-yl-carbonyle, pipéridin-1-yl-carbonyle, morpholin-4-yl-carbonyle, pipérazin-1-yl-carbonyle, 4-(alkyle en C_{1 à 3})-pipérazin-1-yl-carbonyle, alkylcarbonylamino en C_{1 à 3}, arylcarbonylamino, alkylsulfanyle en C_{1 à 3}, alkylsulfinyle en C_{1 à 3}, alkylsulfonyle en C_{1 à 3}, cycloalkyle en C_{3 à 6}, (het)aryl, ou (het)aryloxy ;
amino, alkylamino en C_{1 à 3}, di-(alkyl en C_{1 à 3})amino, pyrrolidin-1-yle, 2-ozo-pyrrolidin-1-yle, pipéridin-1-yle, 2-oxo-pipéridin-1-yle, morpholin-4-yle, 3-oxo-morpholin-4-yle, pipérazin-1-yle, 2-oxo-pipérazin-1-yle, 3-ozo-pipérazin-1-yle, 4-(alkyl en C_{1 à 3})-pipérazin-1-yle, 4-(alkylcarbonyl en C_{1 à 4})-pipérazin-1-yle, 4-(cycloalkylcarbonyl- C_{3 à 6})-pipérazin-1-yle, 4-(alkyloxycarbonyl en C_{1 à 4})-pipérazin-1-yle, 4-(alkylsulfonyl en C_{1 à 4})-pipérazin-1-yle, 2-oxo-4-(alkyl en C_{1à 3})-pipérazin-1-yle, 3-oxo-4-(alkyl en C_{1 à 3})-pipérazin-1-yle,
alkyl en C_{1 à 4}-carbonylamino, (het)arylcarbonylamino, (het)aryl-alkyl en C_{1 à 3}-carbonylamino, alkyloxy en C_{1 à 3}-carbonylamino, aminocarbonylamino, alkyl en C_{1 à 3}-aminocarbonylamino, di-(alkyl en C_{1 à 3})aminocarbonylamino, pyrrolidin-1-yl-carbonylamino, pipéridin-1-yl-carbonylamino, morpholin-4-yl-carbonylamino, pipérazin-1-yl-carbonylamino, 4-(alkyl, en C_{1 à 3})-pipérazin-1-yl-carbonylamino, alkyl en C_{1 à 3}-sulfonylamino, aminosulfonylamino, alkylamino en C_{1 à 3}-sulfonylamino, di-(alkylamino en C_{1 à 3})-sulfonylamino, pyrrolidin-1-yl-sulfonylamino, pipéridin-1-yl-sulfonylamino, morpholin-4-yl-sulfonylamino, pipérazin-1-yl-sulfonylamino, 4-(alkyl en C_{1 à 3})-pipérazin-1-yl-sulfonylamino, (alkyloxy en C_{1 à 3}-carbonylamino)carbonylamino, (het)arylsulfonylamino, (het)aryl-alkyl en C_{1 à 3}-sulfonylamino,
N-(alkyl en C_{1 à 3})-alkyl en C_{1 à 3}-carbonylamino, N-(alkyl en C_{1 à 3})-(het)arylcarbonylamino, N-(alkyl en C_{1 à 3})-(het)aryl- alkyl en C_{1 à 3}-carbonylamino, N-(alkyl en C_{1 à 3})-alkyloxy en C_{1 à 3}-carbonylamino, N-(aminocarbonyl)-alkylamino en C_{1 à 3}, N-(alkyl en C_{1 à 3}-aminocarbonyl)-alkylamino en C_{1 à 3}, N-di-(alkyl en C_{1 à 3})aminocarbonyl]-alkylamino en C_{1 à 3},
N-(alkyl en C_{1 à 3})-alkyl en C_{1 à 3}-sulfonylamino, N-(alkyl en C_{1 à 3})-(het)arylsulfonylamino, N- (alkyl en C_{1 à 3}) - (het) aryl-alkyl en C_{1 à 3}-sulfonylamino,
carboxy, alkyloxy en C_{1 à 3}-carbonyle, aminocarbonyle, alkyl en C_{1 à 3}-aminocarbonyle, di-(alkyl en C₁ à ₃)-aminocarbonyle, pyrrolidin-1-yl-carbonyle, pipéridin-1-yl-carbonyle, morpholin-4-yl-carbonyle, pipérazin-1-yl-carbonyle, 4-(alkyl en C_{1 à 3})-pipérazin-1-yl-carbonyle, (het)arylaminocarbonyle, N-(alkyl en C_{1 à 3})-(het)arylaminocarbonyle, (het)aryl-alkylaminocarbonyle en C_{1 à 3}, N-(alkyl en C_{1 à 3})-(het)aryl-alkylaminocarbonyl en C_{1 à 3},
alkylsulfanyle en C_{1 à 3}, alkysulfinyle en C_{1 à 3}, (het)arylsulfonyle, trifluorométhylsulfanyle, trifluorométhylsulfinyle,
aminosulfonyle, alkyl en C_{1 à 3}-aminosulfonyle, di-(alkyl en C_{1 à 3})-aminosulfonyle, pyrrolidin-1-yl-sulfonyle, pipéridin-1-yl-sulfonyle, morpholin-4-yl-sulfonyle, pipérazin-1-yl-sulfonyle, 4-(alkyl en C_{1 à 3})-pipérazin-1-yl-sulfonyle,
où les cycles hétérocyclique et cycloalkyle saturés décrits ci-dessus sont facultativement substitués par un ou deux groupes choisis parmi un atome de fluor, un groupe alkyle en C_{1 à 3}, alcoxy en C_{1 à 3}, alcoxy en C_{1 à 3}-alkyle en C_{1 à 3}, ou hydroxy, et
L³ et L⁴ sont liés l'un à l'autre, et
L⁵ et L⁶ sont liés l'un à l'autre, de telle sorte qu'indépendamment l'un de l'autre et dans chaque cas, conjointement avec les 2 atomes C adjacents auxquels ils sont attachés, un groupe aryle ou hétéroaryle est formé qui est condensé au groupe cyclique B, et lequel groupe aryle ou hétéroaryle est facultativement substitué par 1, 2 ou 3 R¹⁰ identiques ou différents,
où l'on entend par aryle un groupe phényle ou naphtyle et par hétéroaryle, un groupe pyrrolyle, furanyle, thiényle, pyridyle, indolyle, benzofuranyle, benzothiophényle, quinoléinyle, isoquinoléinyle, ou pyrrolyle, furanyle, thiényle, pyridyle où dans lesdits groupes pyrrolyle, furanyle, thiényle, pyridyle 1 ou 2 groupes CH sont chacun remplacés par N, ou indolyle, benzofuranyle, benzothiophényle, quinoléinyle, isoquinoléinyle où dans lesdits groupes indolyle, benzofuranyle, benzothiophényle, quinoléinyle, isoquinoléinyle, 1 à 3 groupes CH sont chacun remplacés par N ou un groupe tétrazolyle,
tandis que l'(het)aryle susmentionné est un groupe aryle tel que défini ci-avant, ou un groupe hétéroaryle tel que défini ci-avant, ou un cycle choisi dans le groupe consistant en un groupe 1,2-dihydro-2-oxo-pyridinyle, 1,4-dihydro-4-oxo-pyridinyle, 2,3-dihydro-3-oxo-pyridazinyle, 1,2,3,6-tétrahydro-3,6-dioxo-pyridazinyle, 1,2-dihydro-2-oxo-pyrimidinyle, 3,4-dihydro-4-oxo-pyrimidinyle, 1,2,3,4-tétrahydro-2,4-dioxo-pyrimidinyle, 1,2-dihydro-2-oxo-pyrazinyle, 1,2,3,4-tétrahydro-2,3-dioxo-pyrazinyle, 2,3-dihydro-2-oxo-indolyle, 2,3-dihydrobenzofuranyle, 2,3-dihydro-2-oxo-1H-benzimidazolyle, 2,3-dihydro-2-oxo-benzoxazolyle, 1,2-dihydro-2-oxo-quinoléinyle, 1,4-dihydro-4-oxo-quinoléinyle, 1,2-dihydro-1-oxo-isoquinoléinyle, 1,4-dihydro-4-oxo-cinnolinyle, 1,2-dihydro-2-oxo-quinazoléinyle, 1,4-dihydro-4-oxo-quinazoléinyle, 1,2,3,4-tétrahydro-2,4-dioxo-quinazoléinyle, 1,2-dihydro-2-oxoquinoxaléinyle, 1,2,3,4-tétrahydro-3-oxo-quinoxalinyle, 1,2,3,4-tétrahydro-2,3-dioxo-quinoxaléinyle, 1,2-dihydro-1-oxo-phtalazinyle, 1,2,3,4-tétrahydro-1,4-dioxo-phtalazinyle, chromanyle, coumarinyle, 2,3-dihydro-benzo[1,4]dioxinyle et 3,4-dihydro-3-oxo-2H-benzo[1,4]oxazinyle, où chacun desdits cycles est facultativement substitué par 1, 2 ou 3 substituants choisis indépendamment les uns des autres parmi R¹⁰,
tandis que chacun des fragments alkyle ou alkylène susmentionné peut être ramifié ou non ramifié,
leurs tautomères, leurs stéréoisomères, leurs mélanges et leurs sels,
alors que les composés (M1) à (M4) suivants sont exclus,

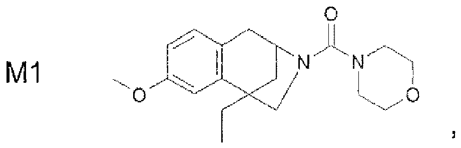

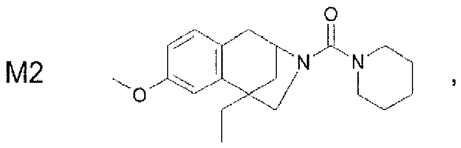

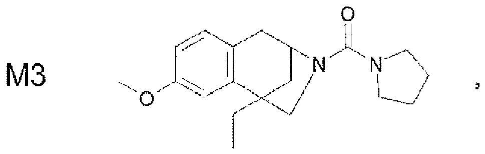

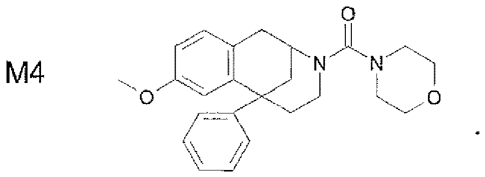

2. Composés selon la revendication 1, **caractérisé par** une formule générale choisie parmi une ou plusieurs des formules I.1 à I.10 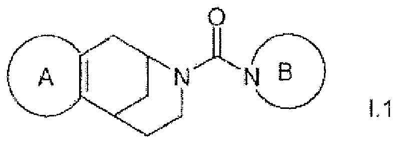 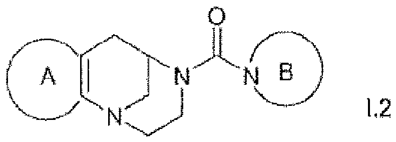 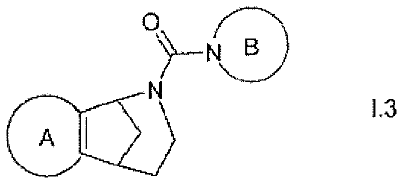 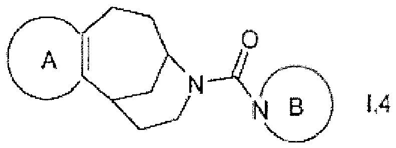 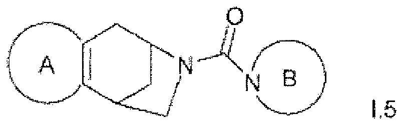 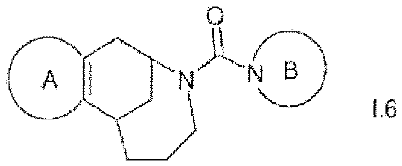 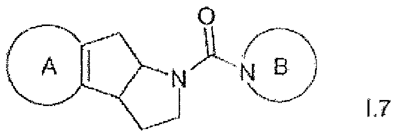 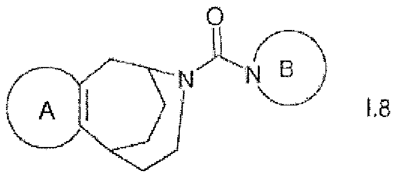 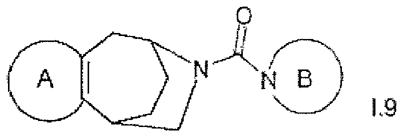 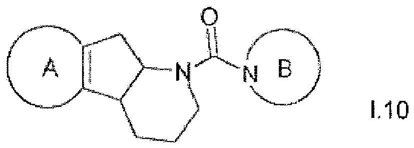 où la structure de noyau azacycloalcène en C₅₊ₘ₊ₙ des formules générales I.1 à I.10 incluant le groupe de pontage -(CH₂)ₒ- est facultativement substituée par 1, 2 ou plusieurs substituants choisis indépendamment les uns des autres dans le groupe consistant en R¹¹ et R¹², et
où les cycles A et B et m, n, o, R¹¹, R¹² sont tels que définis dans la revendication 1, leurs tautomères, leurs stéréoisomères, leurs mélangeas et leurs sels, alors que les composés (M1), (M2), (M3) et (M4) tels que définis dans la revendication 1, sont exclus.

3. Composés selon la revendication 1 ou 2, dans lesquels le cycle B contenant de l'azote désigne l'azétidin-1-yle, le pyrrolidin-1-yle, le pipéridin-1-yle, l'azépan-1-yle, l'azocan-1-yle,
l'azétidin-1-yle, où un groupe -CH₂- est remplacé par O, S, NR^{N}, un groupe carbonyle, ou sulfonyle, ou
pyrrolidin-1-yle, pipéridin-1-yle, azépan-1-yle, azocan-1-yle, qui peut être partiellement ou totalement insaturé et où un ou deux groupes -CH₂- sont remplacés indépendamment l'un de l'autre par 0, S, un groupe carbonyle ou sulfonyle, et où un groupe -CH₂- peut être remplacé par -NR^{N}-,
aza-bicyclohept-N-yle, aza-bicyclooct-N-yle, aza-bicyclonon-N-yle, aza-bicyclodéc-N-yle, bicycloundéc-N-yle, bicyclododéc-N-yle, dont chacun peut être partiellement insaturé, et dans chacun desquels un ou deux groupes -CH₂- peut être remplacé indépendamment des autres par O, S, -NR^{N}-, un groupe carbonyle ou sulfonyle, et dans chacun desquels un groupe -CH= peut être remplacé par -N=,
aza-tricyclonon-N-yle, aza-tricyclodéc-N-yle, aza-tricycloundéc-N-yle, aza-tricyclododéc-N-yle, aza-tricyclotridéc-N-yle, aza-tricyclotétradéc-N-yle, dans chacun desquels un ou deux groupe -CH₂- peut être remplacé indépendamment des autres par O, S, NR^{N}, un groupe carbonyle ou sulfonyle, et dans chacun desquels un groupe -CH= peut être remplacé par -N=,
où chacun des cycles B ci-dessus peut être substitué par un ou plusieurs substituants choisis indépendamment des autres parmi L¹,
où chacun des cycles B peut être substitué par 1 ou 2 substituants choisis indépendamment des autres parmi L²,
où 2 atomes C adjacents de chacun des cycles B ci-dessus peuvent être substitués par L³ et L⁴, et
où 2 atomes C adjacents de chacun des cycles B ci-dessus peuvent être substitués par L⁵ et L^{6 ;}
où R^{N,} L^{1,} L^{2,} L^{3,} L^{4,} L^{5,} L⁶ sont tels que définis dans la revendication 1.

4. Composés selon la revendication 1, 2 ou 3, dans lesquels le cycle A désigne un cycle benzo, pyrido, pyrrolo, furo, thiéno, pyridazino, pyrimido, ou pyrazino où chacun desdits cycles est substitué par un ou plusieurs substituants choisis indépendamment les uns des autres parmi R¹, et où 2 atomes C adjacents de chacun desdits cycles sont facultativement substitués par R² et R³ ou
un cycle pyrazolo, oxazolo, thiazolo, ou imidazo dont chacun est facultativement substitué par R^{1 ;} où R¹, R² et R³ sont tels que définis dans la revendication 1.

5. Sels physiologiquement acceptables des composés selon au moins l'une des revendications 1 à 4, avec des acides ou bases inorganiques ou organiques.

6. Compositions pharmaceutiques Contenant un composé selon au moins l'une des revendications 1 à 4, ou un sel physiologiquement acceptable selon la revendication 5, éventuellement conjointement avec un ou plusieurs supports et/ou diluants inertes.

7. Composés dans lesquels
A désigne un cycle benzo, pyrido, pyrrolo, furo, thiéno, pyridazino, pyrimido, ou pyrazino où chacun desdits cycles est facultativement substitué par un ou plusieurs substituant choisis indépendamment les uns des autres parmi R¹, et où 2 atomes C adjacents de chacun desdits cycles sont facultativement substitués par R² et R^{3 ;} ou
un cycle pyrazolo, imidazo, oxazolo, thiazolo, isoxazolo, ou isothiazolo, dans lequel chacun desdits cycles est facultativement substitué par R^{1 ;} ou
un cycle 1,2,3-triazolo facultativement substitué par R^{N} ; et
R¹ indépendamment des autres désigne un atome de fluor, chlore, brome, iode, un groupe cyano, nitro, alkyle en C_{1 à 4}, difluorométhyle, trifluorométhyle, difluorométhoxy, trifluorométhoxy, trifluorométhyl-hydroxy- alkyle en C_{1 à 2}, 2,2,2-trifluoro-1-hydroxy-1-(trifluorométhyl)éthyle, cycloalkyle en C_{3 à 6}, cycloalkyloxy en C_{3 à 6}, cycloalkyl en C_{3 à 6}-alkyle en C_{1 à 3}, cycloalkyl en C_{3 à 6}-alkyloxy en C_{1 à 3}, tétrahydrofuran-3-yloxy, tétrahydropyran-3-yloxy, tétrahydropyran-4-yloxy, tétrahydrofuranyl-alkyloxy en C_{1 à 3}, tétrahydropyranyl-alkyloxy en C_{1 à 3}, (het)aryl, (het)aryloxy, (het)aryl-alkyle en C_{1 à 3}, (het)aryl-alkyloxy, en C_{1 à 3}, (het)aryloxy-alkyle en C_{1 à 3}, alkyl en C_{1 à 3}-carbonyle, (het)aryl-carbonyle,
amino, alkylamino en C_{1 à 4}, di-(alkyl en C_{1 à 4})amino, pyrrolidin-1-yle, 2-oxo-pyrrolidin-1-yle, pipéridin-1-yle, 2-oxo-pipéridin-1-yle, morpholin-4-yle, 3-oxo-morpholin-4-yle, pipérazin-1-yle, 2-oxo-pipérazin-1-yle, 3-exo-pipérazin-1-yle, 4-(alkyl en C_{1 à 4})-pipérazin-1-yle, 4-(alkylcarbonyl en C_{1 à 4})-pipérazin-1-yl, 4-(cycloalkylcarbonyl en C_{3 à 6})-pipérazin-1-yle, 4-(alkyloxycarbonyl en C_{1 à 4}) - pipérazin-1-yle, 4-(alkylsulfonyl en C_{1 à 4})-pipérazin-1-yle, 2-oxo-9- (alkyl en C_{1 à 4})-pipérazin-1-yle, 3-oxo-4-(alkyl en C_{1 à 4})-pipérazin-1-yle,
alkyl en C_{1 à 4}-carbonylamino, (het)arylcarbonylamino, (het)aryl-alkyl en C_{1 à 4}-carbonylamino, alkyloxy en C_{1 à 4}-carbonylamino, aminocarbonylamino, alkyl en C₁ à 4-amino-carbonylamino, di-(alkyl en C_{1 à 4})aminocarbonylamino, pyrrolidin-1-yl-carbonylamino, pipéridin-1-yl-carbonylamino, morpholin-4-yl-carbonylamino, pipérazin-1-yl-carbonylamino, 4-(alkyl en C_{1 à 4})-pipérazin-1-yl-carbonylamino, alkyl en C_{1 à 4}-sulfonylamino, aminosulfonylamino, alkylamino en C_{1 à 4}-sulfonylamino, di-(alkyl en C_{1 à 4})-amino-sulfonylamino, pyrrolidin-1-yl-sulfonylamino, pipéridin-1-yl-sulfonylamino, morpholin-4-yl-sulfonylamino, pipérazin-1-yl-sulfonylamino, 4-(alkyl en C_{1 à 4})-pipérazin-1-yl-sulfonylamino, (alkyloxy en C_{1 à 4}-carbonylamino)carbonylamino, (het)arylsulfonylamino, (het) aryl-alkyl en C_{1 à 4}-sulfonylamino,
N-(alkyl en C_{1 4})-alkyl en C_{1 à 4}-carbonylamino, N-(alkyl C_{1 à 4})-(het)arylcarbonylamino, N-(alkyl en C_{1 à 4})-(het)aryl-alkyl en C_{1 à 4}-carbonylamino, N-(alkyl en C_{1 à 4})-alkyloxy en C_{1 à 4}-carbonylamino, N-(aminocarbonyl)-alkylamino en C_{1 à 4}, N-(alkyl en C_{1 à 4}-aminocarbonyl)-alkylamino en C_{1 à 4}, N-[di- (alkyl en C_{1 à 4})aminocarbonyl]-alkylamino en C_{1 à 4},
N-(alkyl en C_{1 à 4})-alkyl en C_{1 à 4}-sulfonylamino, N-(alkyl en C_{1 à 4})-(het)arylsulfonylamino, N-(alkyl en C₁ à ₄)- (het)aryl-alkyl en C_{1 à 4}-sulfonylamino,
oxo-imidazolidin-1-yle, 2,4-dioxo-imidazolidin-1-yle, 2,5-dioxo-imidazolidin-1-yle, 2-oxo-hexahydropyrimidin-1-yle, où l'atome d'azote en position 3 des groupes susmentionnés est facultativement substitué par un groupe méthyle ou éthyle,
(hydroxyimino)aminométhyl, (alkyloxyimino en C_{1 à 3})-aminométhyle, carboxy, alkyloxy en C_{1 à 4}-carbonyle, aminocarbonyle, alkyl en C_{1 à 4}-aminocarbonyle, di-(alkyl en C_{1 à 4})-aminocarbonyle, pyrrolidin-1-yl-carbonyle, pipéridin-1-yl-carbonyle, morpholin-4-yl-carbonyle, pipérazin-1-yl-carbonyle, 4-(alkyl en C_{1 à 4})-pipérazin-1-yl-carbonyle,
carboxy-alkyle en C_{1 à 4}, alkyloxy en C_{1 à 4}-carbonyl-alkyle en C_{1 à 4}, cyano-alkyle en C_{1 à 4}, aminocarbonyl-alkyle en C_{1 à 4}, alkyl en C_{1 à 4}-aminocarbonyl-alkyle en C_{1 à 4}, di-(alkyl en C_{1 à 4})-aminocarbonyl-alkyle en C_{1 à 4}, pyrrolidin-1-yl-carbonyl-alkyle en C_{1 à 4}, pipéridin-1-yl-carbonyl-alkyle en C_{1 à 4}, morpholin-4-yl-carbonyl-alkyle en C_{1 à 4}, pipérazin-1-yl-carbonyl-alkyle en C_{1 à 4}, 4-(alkyl en C_{1 à 4})-pipérazin-1-yl-carbonyl-alkyle en C_{1 à 4},
carboxy-alkyloxy en C_{1 à 4}, alkyloxy en C_{1 à 4}-carbonyl-alkyloxy en C_{1 à 4}, cyano- alkyl en C_{1 à 4}-oxy, aminocarbonyl-alkyloxy en C_{1 à 4}, alkyl en C_{1 à 4}-aminocarbonyl-alkyloxy en C_{1 à 4}, di-(alkyl en C_{1 à} 4) - aminocarbonyl-alkyloxy en C_{1 à 4}, pyrrolidin-1-yl-carbonyl-alkyl en C_{1 à 4}-oxy, pipéridin-1-yl-carbonyl-alkyloxy en C_{1 à 4,} morpholin-4-yl-carbonyl-alkyl en C_{1 à 4}-oxy, pipérazin-1-yl-carbonyl-alkyloxy en C_{1 à 4}, 4-(alkyl en C_{1 à 4})-pipérazin-1-yl-carbonyl-alkyloxy en C_{1 à 4},
hydroxy-alkyle en C_{1 à 4}, alkyloxy en C_{1 à 4}-alkyle en C_{1 à 4}, amino-alkyle en C_{1 à 4}, alkylamino en C_{1 à 4}-alkyle en C_{1 à 4}, di-(alkyl en C_{1 à 4}) -amino-alkyle en C_{1 à 4}, pyrrolidin-1-yl-alkyle en C_{1 à 4}, alkyl en C_{1 à 4}-carbonyl-amino-alkyle en C_{1 à 4}, N-(alkyl en C_{1 à 4})-alkylcarbonyl en C_{1 à 4}-amino-alkyle en C_{1 à 4}, 2-oxo-pyrrolidin-1-yl-alkyle en C_{1 à 4}, pipéridin-1-yl-alkyle en C_{1 à 4}, 2-oxo-pipéridin-1-yl-alkyle en C_{1 à 4}, morpholin-4-yl-alkyle en C_{1 à 4}, 3-oxo-morpholin-4-yl-alkyl en C_{1 à 4}, pipérazien C_{1 à 4}, 2-oxo-pipérazin-1-yl-alkyle en C_{1 à 4}, 3-oxo-pipérazin-1-yl-alkyle en C_{1 à 4}, alkyl en C_{1 à 4})-pipérazin-1-yl-alkyle en C_{1 à 4}, 2-oxo-4-(alkyl en C_{1 à 4})-pipérazin-1-yl-alkyle en C_{1 à 4}, 3-oxo-4-(alkyl en C_{1 à 4})-pipérazin-1-yl-alkyl en C_{1 à 4},
hydroxy-alkyloxy en C_{1 à 4}, alkyloxy en C_{1 à 4}-alkyloxy en C_{1 à 4}, alkylsulfanyl en C_{1 à 4}-alkyloxy en C_{1 à 4}, alkylsulfinyl en C_{1 à 4}-alkyloxy, en C_{1 à 4}, alkylsulfonyl en C_{1 à 4}-alkyloxy en C_{1 à 4}, amino-alkyloxy en C_{1 à 4}, alkylamino, en C_{1 à 4}-alkyloxy en C_{1 à 4}, di-(alkyl en C_{1 à 4})-amino-alkyloxy en C_{1 à 4}, pyrrolidin-1-yl-alkyloxy en C_{1 à 4}, 2-oxo-pyrrolidin-1-yl-alkyloxy en C_{1 à 4}, pipéridin-1-yl-alkyloxy en C_{1 à 4}, 2-oxo-pipéridin-1-yl-alkyloxy en C_{1 à 4}, morpholin-4-yl-alkyloxy en C_{1 à 4}, 3-oxo-morpholin-4-yl-alkyloxy en C_{1 à 4}, pipérazin-1-yl-alkyloxy en C_{1 à 4}, 2-oxo-pipérazin-1-yl-alkyloxy en C_{1 à 4}, 3-oxo-pipérazin-1-yl-alkyloxy en C_{1 à 4}, 4-(alkyl en C_{1 à 4})-pipérazin-1-yl-alkyloxy en C_{1 à 4}, 2-oxo-4-(alkyl en C_{1 à 4})-pipérazin-1-yl-alkyloxy en C_{1 à 4}, 3-oxo-4-(alkyl en C_{1 à 4})-pipérazin-1-yl-alkyloxy en C_{1 à 4},
alkylsulfanyle en C_{1 à 4}, alkysulfinyle en C_{1 à 4}, alkylsulfonyle en C_{1 à 4}, alkylsulfonyloxy en C_{1 à 4}, (het)arylsulfonyle, (het)arylsulfonyloxy, trifluorométhylsulfanyle, trifluorométhylsulfinyle, trifluorométhylsulfonyle, cycloalkylsulfanyle en C_{3 à 6}, cycloalkylsulfinyle en C_{3 à 6}, cycloalkylsulfonyle en C_{3 à 6}, cycloalkyl en C₃ à ₆-alkylsulfanyle en C_{1 à 3}, cycloalkyl en C_{3 à 6}-alkylsulfinyle en C_{1 à 3}, cycloalkyl en C_{3 à 6}-alkylsulfonyle en C_{1 à 3},
aminosulfonyle, alkyl en C_{1 à 4}-aminosulfonyle, di-(alkyl en C_{1 à 4})-aminosulfonyle, pyrrolidin-1-yl-sulfonyle, pipéridin-1-yl-sulfonyle, morpholin-4-yl-sulfonyl, pipérazin-1-yl-sulfonyle, ou 4-(alkyl C_{1 a 4})-pipérazin-1-yl-sulfonyle,
où les hétérocycles et cycles cycloalkyle saturés susmentionnés sont facultativement substitués par un ou deux groupes choisis indépendamment l'un de l'autre parmi un atome de fluor, un groupe alkyle en C_{1 à 3}, alcoxy en C_{1 à 3}, alcoxy en C_{1 à 3}-alkyle en C_{1 à 3}, ou hydroxy, ou un sel physiologiquement acceptable de ceux-ci,
y compris les composés exclus de la revendication 1, pour le traitement ou la prévention de maladies ou affections choisies parmi les troubles métaboliques, le diabète sucré de type 1, le diabète sucré de type 2, les complications du diabète (telles que rétinopathie, néphropathie ou neuropathies, pied diabétique, ulcères, macroangiopathies, cicatrisation de plaie lente ou médiocre), acidose métabolique, cétose métabolique, hypoglycémie réactive, hyperinsulinémie, trouble métabolique du glucose, résistance à l'insuline, syndrome métabolique, dyslipidémies de différentes origines, athérosclérose, obésité, tension artérielle élevée, insuffisance cardiaque chronique, oedème, hyperuricémie, dégénérescence des cellules bêta (apoptose ou nécrose des cellules bêta du pancréas), amélioration ou rétablissement de la fonctionnalité des cellules du pancréas, augmentation du nombre et de la taille des cellules bêta du pancréas, insuffisance rénale aiguë, hypertension oculaire, glaucome, ostéoporose, amélioration ou prévention d'un trouble cognitif, anxiété, dépression, tuberculose, lèpre, et psoriasis.

8. Utilisation d'au moins un composé tel que défini dans la revendication 7, pour préparer une composition pharmaceutique qui est appropriée au traitement ou à la prévention de maladies ou affections choisies parmi les troubles métaboliques, le diabète sucré de type 1, le diabète sucré de type 2, les complications du diabète (telles que rétinopathie, néphropathie ou neuropathies, pied diabétique, ulcères, macroangiopathies, cicatrisation de plaie lente ou médiocre), acidose métabolique, cétose métabolique, hypoglycémie réactive, hyperinsulinémie, trouble métabolique du glucose, résistance à l'insuline, syndrome métabolique, dyslipidémies de différentes origines, athérosclérose, obésité, tension artérielle élevée, insuffisance cardiaque chronique, oedème, hyperuricémie, dégénérescence des cellules bêta (apoptose ou nécrose des cellules bêta du pancréas), amélioration ou rétablissement de la fonctionnalité des cellules du pancréas, augmentation du nombre et de la taille des cellules bêta du pancréas, insuffisance rénal aiguë, hypertension oculaire, glaucome, ostéraparase, amélioration ou prévention d'un trouble cognitif, anxiété, dépression, tuberculose, lèpre, et psoriasis.

9. Procédé de préparation d'un composé de formule générale I selon l'une quelconque des revendications 1 à 4 ou d'un sel physiologiquement acceptable selon la revendication 5, **caractérisé en ce que**
l'une des amines de formule générale II 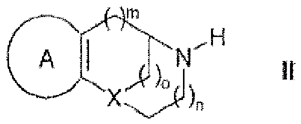 dans laquelle les groupes A, X, m, n and o sont tels que définis dans la revendication 1,
ou de formule générale III 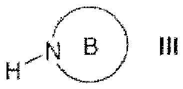 dans laquelle B est tel que défini dans la revendication 1,
réagit avec un dérivé d'acide carbonique Y-CO-Y donnant un composé soit de formule générale IV soit de formule générale V comme intermédiaire 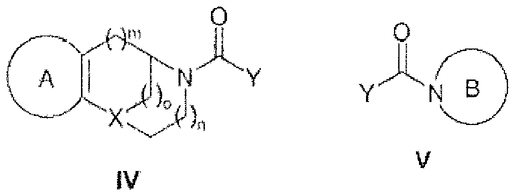 qui est facultativement isolé et facultativement purifié,
et l'intermédiaire de formule IV ou V réagit avec l'autre amine de formule III ou II pour donner le composé de formule I,
où Y est un groupe partant et désigne en particulier un atome de fluor, de chlore, de brome, un groupe cyano, alcoxy en C_{1 à 4}, alcényloxy en C_{2 à 4}, alcynyloxy en C_{2 à 4}, alcoxy en C_{2 à 10} partiellement ou totalement fluoré, oxyarylotriazol, oxyhétéroarylotriazol, hétéroar-N-yle, 3-méthyl-imidazol-1-yle, succinyl-N-oxy, di-(alkyl en C_{1 à 4})aminocarbonyloxy, pyrrolylcarbonyloxy, pipéridinylcarbonyloxy, morpholinylcarbonyloxy, aryloxy, ou hétéroaryloxy,
tandis que les groupes alkyle, alcényle, et alcynyle mentionnés dans la definition des groupes ci-dessus, soit seuls soit comme partie dans l'autre groupe, peuvent être substitués par un ou plusieurs substituants choisis indépendamment des autres parmi un atome de fluor, de chlore, un groupe alkyle en C_{1 à 3}, et alcoxy en C_{1 à 3},
tandis que les groupes aryle mentionnés dans la définition ci-dessus soit seul soit comme partie dans un autre groupe, désignent un groupe phenyle ou naphtyle et les groupes hétéroaryle mentionnés dans la définition des groups ci-dessus soit seules soit comme partie d'un autre groupe désignent un groupe pyridinyle, pyrimidinyle, triazinyle, imidazolyle, pyrazolyle, triazolyle, tétrazolyle, alors que les groupes à la fois aryle et hétéroaryle sont facultativement substitués par un ou plusieurs substituants choisis indépendamment les uns des autres parmi un atome de fluor, de chlore, de brome, et les groupes alkyle en C_{1 à 3}, alkyloxy en C_{1 à 3}, nitro, cyano, et di-(alkyl en C_{1 à 3})amino,
alors que les deux Y dans Y-CO-Y peuvent être identiques ou différents,
alors que le second Y à remplacer peut également être transformé en un Y plus réactif après que le premier Y est remplacé par l'une des deux amines,
facultativement en présence d'une base organique ou d'un autre additif;
et, si nécessaire, tout groupe protecteur utilisé dans les réactions décrites ci-dessus est clivé simultanément ou ultérieurement ;
si on le souhaite, un composé de formule générale 1 ainsi obtenu est résolu en ses stéréoisomères ;
si on le souhaite, un composé de formule générale I ainsi obtenu est converti en ses sels, en particulier pour une utilisation pharmaceutique en ses sels physiologiquement acceptables.
